(19) Europäisches Patentamt
European Patent Office
Office européen des brevets

(11) EP 1 254 897 A1

(12) **EUROPEAN PATENT APPLICATION**
published in accordance with Art. 158(3) EPC

(43) Date of publication:
**06.11.2002 Bulletin 2002/45**

(21) Application number: **00985983.6**

(22) Date of filing: **28.12.2000**

(51) Int Cl.[7]: **C07D 215/12**, C07D 215/14, C07D 215/18, C07D 405/06, C07D 417/06, C07D 413/06, C07D 409/06, A61K 31/55, A61K 31/47, A61P 43/00, A61P 37/08, A61P 29/00

(86) International application number:
**PCT/JP00/09406**

(87) International publication number:
**WO 01/047889 (05.07.2001 Gazette 2001/27)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **28.12.1999 JP 37245599**

(71) Applicant: **Ube Industries, Ltd.**
**Ube-shi, Yamaguchi-ken 755-8633 (JP)**

(72) Inventors:
- **KUROKI, Yoshiaki, Ube Research Laboratory Ube-shi Yamaguchi 755-8633 (JP)**
- **UENO, Hitoshi, Ube Research Laboratory Ube-shi, Yamaguchi 755-8633 (JP)**
- **KATSUBE, Tetsushi, Ube Research Laboratory Ube-shi, Yamaguchi 755-8633 (JP)**
- **KAWAGUCHI, Tetsuo, Ube Research Laboratory Ube-shi, Yamaguchi 755-8633 (JP)**
- **OKANARI, Eiji, Ube Research Laboratory Ube-shi, Yamaguchi 755-8633 (JP)**
- **IKUTA, Takashi, Ube Research Laboratory Ube-shi, Yamaguchi 755-8633 (JP)**

(74) Representative: **Henkel, Feiler, Hänzel**
**Möhlstrasse 37**
**81675 München (DE)**

(54) **TRICYCLIC COMPOUNDS**

(57) The present invention is to provide novel tricyclic compounds having leukotriene antagonistic action and represented by the formula:

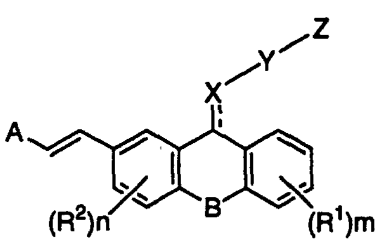

(I)

wherein $R^1$ represents a halogen atom, etc., $R^2$ represents a nitro group, etc., A represents a 5-membered or a 6-membered heteroaromatic ring group containing 1 to 3 hetero atoms selected from the group consisting of a nitrogen atom, an oxygen atom and a sulfur atom, etc., B represents a formula: $-OCH_2-$, etc., X represents a sulfur atom, etc., Y represents $C_1$-$C_{10}$ alkylene group which may have a halogen atom, etc. as a substituent(s), Z represents a carboxyl group whic may be protected, etc., ------ represents a single bond or a double bond,

m is an integer of 1 to 4, n is an integer of 1 to 3, or a pharmaceutically acceptable salt thereof.

EP 1 254 897 A1

## Description

TECHNICAL FIELD

This invention relates to a tricyclic compound or a pharmaceutically acceptable salt thereof having leukotriene $C_4$ antagonistic action and leukotriene $E_4$ antagonistic action in addition to potent leukotriene $D_4$ antagonistic action, and available for an antiallergic agent and an anti-inflammatory agent.

BACKGROUND ART

As a compound having leukotriene $D_4$ antagonistic action as in the present invention and having a similar structure to that of the present invention, there has been known, for example, those disclosed in WO94/19345 publication, and as a compound having a partially similar structure, there has been known compounds such as 5-[3-[3-(2-quinolinylmethoxy)phenoxy]-propyl]-1H-tetrazole(RG7152; J.Med. Chem., 33, 1186(1990)) or 5-[[2-[[4-(2-quinolinylmethoxy)phenoxy]methyl]phenyl]-methyl]-1H-tetrazole (RG12525; J.Med. Chem., 33, 1194(1990)), or compounds disclosed in WO95/18107 publication, etc.

In the present invention, as a result of research for long years about syntheses of compounds having potent leukotriene $D_4$ antagonistic action, as well as having antagonistic action to leukotriene $C_4$ and leukotriene $E_4$ and their pharmaceutical effects, the inventors have found that novel tricyclic compounds have excellent leukotriene $D_4$ antagonistic action, as well as having leukotriene $C_4$ and leukotriene $E_4$ antagonistic action with good balance, and have excellent oral absorbability and durability of the action to accomplish the present invention.

DISCLOSURE OF THE INVENTION

A tricyclic compound represented by the formula (I):

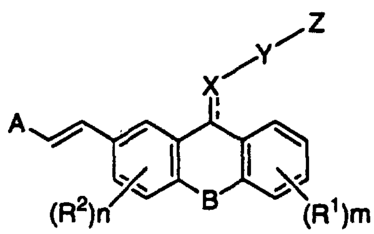

(I)

wherein $R^1$ represents a hydrogen atom, a halogen atom, a hydroxyl group, a nitro group, a cyano group, a carbamoyl group, a formyl group, a carboxyl group, a $C_1$-$C_4$ alkoxy-carbonyl group, a 1H-tetrazol-5-yl group, $C_1$-$C_4$ alkyl group, a fluoro $C_1$-$C_4$ alkyl group, a hydroxy $C_1$-$C_4$ alkyl group, a $C_2$-$C_4$ alkenyl group, a $C_2$-$C_4$ alkynyl group, a $C_1$-$C_4$ alkoxy group, a fluoro $C_1$-$C_4$ alkoxy group, a $C_1$-$C_4$ alkylthio group, a $C_1$-$C_4$ alkylsulfinyl group or a $C_1$-$C_4$ alkylsulfonyl group, $R^2$ represents a hydrogen atom, a halogen atom, a nitro group, a cyano group, $C_1$-$C_4$ alkyl group or a $C_1$-$C_4$ alkoxy group, A represents a 5-membered or a 6-membered heteroaromatic ring group containing 1 to 3 hetero atoms selected from the group consisting of a nitrogen atom, an oxygen atom and a sulfur atom, or a fused heteroaromatic ring group in which the heteroaromatic ring group and a benzene ring are fused, said heteroaromatic ring group or fused heteroaromatic ring group may have a halogen atom, a nitro group, a cyano group, a $C_1$-$C_4$ alkyl group, a fluoro $C_1$-$C_4$ alkyl group, a $C_1$-$C_4$ alkoxy group, a fluoro $C_1$-$C_4$ alkoxy group, a $C_1$-$C_4$ alkylthio group, a fluoro $C_1$-$C_4$ alkylthio group or a $C_3$-$C_4$ alkylene group as a substituent(s), B represents a formula:$-OCH_2-$, a formula: $-CH_2CH_2-$, a formula: $-SCH_2-$, a formula: $-CH_2O-$ or a formula: $-CH_2S-$, X represents an oxygen atom, a sulfur atom, amethylene group or a formula: $=CH-$, Y represents a $C_1$-$C_{10}$ alkylene group, phenylene group or a group of a formula (a):

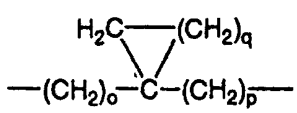

(a)

wherein o and p each is an integer of 0 to 2, and q is an integer of 1 to 4,

each of which may have a halogen atom, a $C_1$-$C_4$ alkyl group or a $C_1$-$C_4$ alkoxy group as a substituent(s), Z represents a carboxyl group which may be protected; a 1H-tetrazol-5-yl group; a formula: $-SO_3H$ group; a formula: $-NH-SO_2-R^3$; or a formula: $-CO-NH-SO_2-R^3$,

wherein $R^3$ represents a $C_1$-$C_4$ alkyl group, a fluoro $C_1$-$C_4$ alkyl group or a phenyl group which may have at least one substituent selected from the group consisting of a halogen atom, a $C_1$-$C_4$ alkyl group, a fluoro $C_1$-$C_4$ alkyl group, a $C_1$-$C_4$ alkoxy group, a fluoro $C_1$-$C_4$ alkoxy group,

a nitro group and a cyano group as a substituent(s),

------ represents a single bond or a double bond,

m is an integer of 1 to 4, and when m is 2 or more, then $R^1$ may be the same or different from each other, and n is an integer of 1 to 3, and when n is 2 or more, then $R^2$ may be the same or different from each other, or a pharmaceutically acceptable salt thereof.

BEST MODE FOR CARRYING OUT THE INVENTION

In the compound represented by the above-mentioned formula (I), as the halogen atom of $R^1$, there may be mentioned, for example, a fluorine, chlorine, bromine or iodine atom, preferably fluorine, chlorine or bromine atom, more preferably fluorine' or chlorine atom, particularly preferably a fluorine atom.

As the $C_1$-$C_4$ alkoxycarbonyl group of $R^1$, there may be mentioned, for example, methoxycarbonyl, ethoxycarbonyl, propoxycarbonyl, isopropoxycarbonyl, butoxycarbonyl, isobutoxycarbonyl, sec-butoxycarbonyl or t-butoxycarbonyl group, preferably methoxycarbonyl, ethoxycarbonyl, propoxycarbonyl or isopropoxycarbonyl group, more preferably a methoxycarbonyl or ethoxycarbonyl group, particularly preferably a methoxycarbonyl group.

As the $C_1$-$C_4$ alkyl group of $R^1$, there may be mentioned methyl, ethyl, propyl, isopropyl, butyl, isobutyl, sec-butyl or t-butyl group, preferably methyl, ethyl, propyl or isopropyl group, more preferably methyl or ethyl group, particularly preferably methyl group.

As the fluoro $C_1$-$C_4$ alkyl group of $R^1$, there may be mentioned, for example, fluoromethyl, difluoromethyl, trifluoromethyl, 2-fluoroethyl, 2,2,2-trifluoroethyl, 2-fluoropropyl, 3-fluor-propyl or 4-fluorobutyl group, preferably fluoromethyl, difluoromethyl, trifluoromethyl or 2-fluoroethyl group, more preferably fluoromethyl, difluoromethyl or trifluoromethyl group, particularly preferably difluoromethyl or trifluoromethyl group.

As the hydroxy $C_1$-$C_4$ alkyl group of $R^1$, there may be mentioned, for example, hydroxymethyl, 1-hydroxyethyl, 2-hydroxyethyl, 1-hydroxy-1-methylethyl, 1-hydroxypropyl, 2-hydroxypropyl, 3-hydroxypropyl, 1-hydroxybutyl or 4-hydroxybutyl group, preferably hydroxymethyl, 1-hydroxyethyl, 2-hydroxyethyl, 1-hydroxy-1-methylethyl, 1-hydroxypropyl or 2-hydroxypropyl group, more preferably hydroxymethyl, 1-hydroxyethyl, 1-hydroxy-1-methylethyl or 1-hydroxypropyl group, particularly preferably hydroxymethyl or 1-hydroxy1-methylethyl group.

As the $C_2$-$C_4$ alkenyl group of $R^1$, there may be mentioned, for example, vinyl, 1-propenyl, allyl, isopropenyl, 1-butenyl, 2-butenyl, 3-butenyl, 1-methyl-1-propenyl, 2-methyl-1-propenyl or 2-methyl-2-propenyl group, preferably vinyl, 1-propenyl, allyl, 1-butenyl, 2-butenyl or 2-methyl-1-propenyl group, more preferably vinyl, 1-propenyl or allyl group, particularly preferably a vinyl group.

As the $C_2$-$C_4$ alkynyl group of $R^1$, there may be mentioned, for example, ethynyl, 1-propynyl, propargyl, 1-butynyl, 2-butynyl or 3-butynyl group, preferably ethynyl, 1-propynyl or 1-butynyl group, more preferably ethynyl or 1-propynyl group, particularly preferably an ethynyl group.

As the $C_1$-$C_4$ alkoxy group of $R^1$, there may be mentioned, for example, methoxy, ethoxy, propoxy, isopropoxy, butoxy, isobutoxy, sec-butoxy or t-butoxy group, preferably methoxy, ethoxy, propoxy or isopropoxy group, more preferably methoxy or ethoxy group, particularly preferably a methoxy group.

As the fluoro $C_1$-$C_4$ alkoxy group of $R^1$, there may be mentioned, for example, a fluoromethoxy, difluoromethoxy, trifluoromethoxy, 2-fluoroethoxy, 2,2,2-trifluoroethoxy, 2-fluoro propoxy, 3-fluoro propoxy or 4-fluoro butoxy group, preferably fluoromethoxy, difluoromethoxy, trifluoromethoxy or 2-fluoroethoxy group, more preferably a fluoromethoxy, difluoromethoxy or trifluoromethoxy group, particularly preferably a difluoromethoxy or trifluoromethoxy group.

As the $C_1$-$C_4$ alkylthio group of $R^1$, there may be mentioned, for example, a methylthio, ethylthio, propylthio, isopropylthio, butylthio, isobutylthio, sec-butylthio or t-butylthio group, preferably methylthio, ethylthio, propylthio or isopropylthio group, more preferably a methylthio or ethylthio group, particularly preferably a methylthio group.

As the $C_1$-$C_4$ alkylsulfinyl group of $R^1$, there may be mentioned, for example, a methylsulfinyl, ethylsulfinyl, propylsulfinyl, isopropylsulfinyl, butylsulfinyl, isobutylsulfinyl, sec-butylsulfinyl or t-butylsulfinyl group, preferably a methylsulfinyl, ethylsulfinyl, propylsulfinyl or isopropylsulfinyl group, more preferably a methylsulfinyl or ethylsulfinyl group, particularly preferably a methylsulfinyl group.

As the $C_1$-$C_4$, alkylsulfonyl group of $R^1$, there may be mentioned, for example, a methylsulfonyl, ethylsulfonyl, propylsulfonyl, isopropylsulfonyl, butylsulfonyl, isobutyl-sulfonyl, sec-butylsulfonyl or t-butylsulfonyl group, preferably a methylsulfonyl, ethylsulfonyl, propylsulfonyl or isopropylsulfonyl group, more preferably a methylsulfonyl or ethylsulfonyl group, particularly preferably a methylsulfonyl group.

In particular, as $R^1$ in the formula (I), there may be preferably mentioned, a hydrogen atom, a fluorine atom, a chlorine atom, a bromine atom, a hydroxyl group, a nitro group, a cyano group, a carbamoyl group, a formyl group, a carboxyl group, a methoxycarbonyl group, an ethoxycarbonyl group, a propoxycarbonyl group, an isopropoxycarbonyl group, a 1H-tetrazol-5-yl group, a methyl group, an ethyl group, a propyl group, an isopropyl group, a fluoromethyl group, a difluoromethyl group, a trifluoromethyl group, a 2-fluoroethyl group, a hydroxymethyl group, a 1-hydroxyethyl group, a 2-hydroxyethyl group, a 1-hydroxy-1-methylethyl group, a 1-hydroxypropyl group, a 2-hydroxypropyl group, a vinyl group, a 1-propenyl group, an allyl group, a 1-butenyl group, a 2-butenyl group, a 2-methyl-1-propenyl group, an ethynyl group, a 1-propynyl group, a 1-butynyl group, a methoxy group, an ethoxy group, a propoxy group, an isopropoxy group, a fluoromethoxy group, a difluoromethoxy group, a trifluoromethoxy group, a 2-fluoroethoxy group, a methylthio group, an ethylthio group, a propylthio group, an isopropylthio group, a methylsulfinyl group, an ethylsulfinyl group, a propylsulfinyl group, an isopropylsulfinyl group, a methylsulfonyl group, an ethylsulfonyl group, a propylsulfonyl group or an isopropylsulfonyl group,

more preferably a hydrogen atom, a fluorine atom, a chlorine atom, a hydroxyl group, a nitro group, a cyano group, a carbamoyl group, a formyl group, a methoxycarbonyl group, an ethoxycarbonyl group, a 1H-tetrazol-5-yl group, a methyl group, an ethyl group, a fluoromethyl group, a difluoromethyl group, a trifluoromethyl group, a hydroxymethyl group, a 1-hydroxyethyl group, a 1-hydroxy-1-methylethyl group, a 1-hydroxypropyl group, a vinyl group, a 1-propenyl group, an allyl group, an ethynyl group, a 1-propynyl group, a 1-butynyl group, a methoxy group, an ethoxy group, a fluoromethoxy group, a difluoromethoxy group, a trifluoromethoxy group, a methylthio group, an ethylthio group, a methylsulfinyl group, an ethylsulfinyl group, a methylsulfonyl group or an ethylsulfonyl group,

still further preferably a hydrogen atom, a fluorine atom, a nitro group, a cyano group, a formyl group, a methoxycarbonyl group, a 1H-tetrazol-5-yl group, a methyl group, a difluoromethyl group, a trifluoromethyl group, a hydroxymethyl group, a 1-hydroxy-1-methylethyl group, a vinyl group, an ethynyl group, a methoxy group, a difluoromethoxy group, a trifluoromethoxy group, a methylthio group, a methylsulfinyl group or a methylsulfonyl group,

particularly preferably a hydrogen atom, a fluorine atom, a cyano group, a trifluoromethyl group or an ethynyl group.

In the formula (I), the halogen atom, a $C_1$-$C_4$ alkyl group and a $C_1$-$C_4$ alkoxy group of $R^2$ each have the same meanings as those mentioned in the above $R^1$, and as $R^2$, it is preferably a hydrogen atom, a fluorine atom, a chlorine atom, a bromine atom, a nitro group, a cyano group, a methyl group, an ethyl group, a propyl group, an isopropyl group, a methoxy group, an ethoxy group, a propoxy group or an isopropoxy group, more preferably a hydrogen atom, a fluorine atom, a chlorine atom, a nitro group, a cyano group, a methyl group, an ethyl group, a methoxy group or an ethoxy group, still further preferably a hydrogen atom, a fluorine atom, a chlorine atom, a methyl group or a methoxy group, particularly preferably a hydrogen atom.

In the formula (I), as "the 5-membered or a 6-membered heteroaromatic ring group containing 1 to 3 hetero atoms selected from the group consisting of a nitrogen atom, an oxygen atom and a sulfur atom, or a fused heteroaromatic ring group in which the heteroaromatic ring group and a benzene ring are fused" of A, there may be mentioned, for example, a 5-membered heteroaromatic ring group such as furan, thiophene, oxazole, thiazole, imidazole, pyrazole or thiadiazole; a 6-membered heteroaromatic ring group such as pyridine, pyrimidine, pyridazine or pyrazine; or a fused heteroaromatic ring group such as benzofuran, benzothiophene, benzoxazole, benzothiazole, benzimidazole, quinoline, quinazoline or quinoxaline group,

preferably an oxazole, thiazole, imidazole, pyrazole, thiadiazole, pyridine, pyrimidine, pyridazine, pyrazine, benzoxazole, benzothiazole, benzimidazole, quinoline, quinazoline or quinoxaline group, more preferably a thiazole, thiadiazole, pyridine, pyrimidine, benzoxazole, benzothiazole, quinolone or quinazoline group, particularly more preferably a pyridine, benzothiazole or quinoline group.

The above-mentioned heteroaromatic ring group or fused heteroaromatic ring group may have a substituent (s), and as a substituent(s), there may be mentioned, for example, a halogen atom having the same meaning as in $R^1$, a nitro group, a cyano group, a $C_1$-$C_4$ alkyl group having the same meaning as in $R^1$, a fluoro$C_1$-$C_4$ alkyl group having the same meaning as in $R^1$, a $C_1$-$C_4$ alkoxy group having the same meaning as in $R^1$, a fluoro $C_1$-$C_4$ alkoxy group having the same meaning as in $R^1$, a $C_1$-$C_4$ alkylthio group having the same meaning as in $R^1$, or a $C_3$-$C_4$ alkylene group such as a trimethylene, tetramethylene group (said alkylene group forms a 5-membered ring or a 6-membered ring by biding to a carbon atom adjacent thereto on a heteroaromatic ring),

preferably a fluorine, chlorine, bromine atom, a nitro, cyano, methyl, ethyl, propyl, isopropyl, butyl, isobutyl, t-butyl, fluoromethyl, difluoromethyl, trifluoromethyl, 2-fluoroethyl, methoxy, ethoxy, propoxy, isopropoxy, fluoromethoxy, difluoromethoxy, trifluoromethoxy, 2-fluoroethoxy, methylthio, ethylthio, propylthio, isopropylthio, trimethylene or tetramethylene group,

more preferably a fluorine, chlorine atom, a nitro, cyano, methyl, ethyl, isopropyl, t-butyl, fluoromethyl, difluoromethyl, trifluoromethyl, methoxy, ethoxy, fluoromethoxy, difluoromethoxy, trifluoromethoxy, methylthio, ethylthio, trimethylene or tetramethylene group, still further preferably a fluorine, chlorine atom, a nitro, cyano, methyl, isopropyl,

t-butyl, difluoromethyl, trifluoromethyl, methoxy, difluoromethoxy, trifluoromethoxy, methylthio or tetramethylene group, particularly preferably a fluorine, chlorine atom, a trifluoromethyl or tetramethylene group.

A number of the substituent(s) on the heteroaromatic ring group or fused heteroaromatic ring group is 1 to 4, preferably 1 to 2.

As A in the formula (I), there may be specifically mentioned, preferably a 2-oxazolyl, 2-thiazolyl, 2- or 4-imidazolyl, 3-pyrazolyl, 1,3,4-thiadiazol-2-yl, 2-pyridyl, 2-or 4-pyrimidinyl, 3-pyridazinyl, 2-pyrazinyl, 2-benzoxazolyl, 2-benzothiazolyl, 2-benzimidazolyl, quinolin-2-yl, quinazolin-2-yl, quinoxaline-2-yl, 4-methyl-2-thiazolyl, 4-ethyl-2-thiazolyl, 4-isopropyl-2-thiazolyl, 4-t-butyl-2-thiazolyl, 4-trifluoromethyl-2-thiazolyl, 5-methyl-1,3,4-thiadiazol-2-yl, 5-ethyl-1,3,4-thiadiazol-2-yl, 5-isopropyl-1,3,4-thiadiazol-2-yl, 5-t-butyl-1,3,4-thiadiazol-2-yl, 5-trifluoromethyl-1,3,4-thiadiazol-2-yl, 5,6-difluoro-2-pyridyl, 5,6-dichloro-2-pyridyl, 5,6-dimethyl-2-pyridyl, 5,6-diethyl-2-pyridyl, 6-trifluoromethyl-2-pyridyl, 6-methylthio-2-pyridyl, 5,6-dihydroclopenta[b]pyridin-2-yl, 5,6,7,8-tetrahydroquinolin-2-yl, 5,6-difluoro-2-pyrimidinyl, 5,6-dichloro-2-pyrimidinyl, 5,6-dimethyl-2-pyrimidinyl, 6-trifluoromethyl-2-pyrimidinyl, 5,6-dihydrocyclopenta-[d]pyrimidin-2-yl, 5,6,7,8-tetrahydroquinazolin-2-yl, 6-fluoro-2-benzoxazolyl, 5-fluoro-2-benzoxazolyl, 5,6-difluoro-2-benzoxazolyl, 6-chloro-2-benzoxazolyl, 5-chloro-2-benzoxazolyl, 5,6-dichloro-2-benzoxazolyl, 5-chloro-6-fluoro-2-benzoxazolyl, 5-methyl-2-benzoxazolyl, 5-cyano-2-benzoxazolyl, 5-trifluoromethyl-2-benzoxazolyl, 5-methylthio-2-benzoxazolyl, 6-fluoro-2-benzothiazolyl, 5-fluoro-2-benzothiazolyl, 5,6-difluoro-2-benzothiazolyl, 6-chloro-2-benzothiazolyl, 5-chloro-2-benzothiazolyl, 5,6-dichloro-2-benzothiazolyl, 5-chloro-6-fluoro-2-benzothiazolyl, 5-methyl-2-benzothiazolyl, 5-cyano-2-benzothiazolyl, 5-trifluoromethyl-2-benzothiazolyl, 5-methylthio-2-benzothiazolyl, 5-fluoroquinolin-2-yl, 6-fluoroquinolin-2-yl, 7-fluoroquinolin-2-yl, 5-chloroquinolin-2-yl, 6-chloroquinolin-2-yl, 7-chloroquinolin-2-yl, 7-methylquinolin-2-yl, 7-trifluoromethylquinolin-2-yl, 7-methoxyquinolin-2-yl, 7-difluoromethoxyquinolin-2-yl, 7-trifluoromethoxyquinolin-2-yl, 5,7-difluoroquinolin-2-yl, 6,7-difluoroquinolin-2-yl, 5,7-dichloroquinolin-2-yl, 6,7-dichloroquinolin-2-yl, 5-chloro-7-fluoroquinolin-2-yl, 6-chloro-7-fluoroquinolin-2-yl, 7-chloro-5-fluoroquinolin-2-yl, 7-chloro-6-fluoroquinolin-2-yl, 7-chloro-6-cyanoquinolin-2-yl, 7-cyano-6-fluoroquinolin-2-yl, 6-fluoro-7-trifluoromethylquinolin-2-yl, 5,6,7-trifluoroquinolin-2-yl, 5-fluoroquinazolin-2-yl, 6-fluoroquinazolin-2-yl, 7-fluoroquinazolin-2-yl, 5-chloroquinazolin-2-yl, 6-chloroquinazolin-2-yl, 7-chloroquinazolin-2-yl, 7-methylquinazolin-2-yl, 7-trifluoromethylquinazolin-2-yl, 7-methoxyquinazolin-2-yl, 7-difluoromethoxyquinazolin-2-yl, 7-trifluoromethoxyquinazolin-2-yl, 5,7-difluoroquinazolin-2-yl, 6,7-difluoroquinazolin-2-yl, 5,7-dichloroquinazolin-2-yl, 6,7-dichloroquinazolin-2-yl, 5-chloro-7-fluoroquinazolin-2-yl, 6-chloro-7-fluoroquinazolin-2-yl, 7-chloro-5-fluoroquinazolin-2-yl, 7-chloro-6-fluoroquinazolin-2-yl, 7-chloro-6-cyanoquinazolin-2-yl, 7-cyano-6-fluoroquinazolin-2-yl, 6-fluoro-7-trifluoromethylquinazolin-2-yl or 5,6,7-trifluoroquinazolin-2-yl group, more preferably a 2-thiazolyl, 1,3,4-thiadiazol-2-yl, 2-pyridyl, 2-pyrimidinyl, 2-benzoxazolyl, 2-benzothiazolyl, quinolin-2-yl, quinazolin-2-yl, 4-methyl-2-thiazolyl, 4-isopropyl-2-thiazolyl, 4-t-butyl-2-thiazolyl, 4-trifluoromethyl-2-thiazolyl, 5-methyl-1,3,4-thiadiazol-2-yl, 5-isopropyl-1,3,4-thiadiazol-2-yl, 5-t-butyl-1,3,4-thiadiazol-2-yl, 5-trifluoromethyl-1,3,4-thiadiazol-2-yl, 5,6-difluoro-2-pyridyl, 5,6-dichloro-2-pyridyl, 5,6-dimethyl-2-pyridyl, 5,6-dihydroclopenta-[b]pyridin-2-yl, 5,6,7,8-tetrahydroquinolin-2-yl, 5,6-difluoro-2-pyrimidinyl, 5,6-dichloro-2-.pyrimidinyl, 5,6-dimethyl-2-pyrimidinyl, 6-trifluoromethyl-2-pyrimidinyl, 5,6-dihydrocyclopenta[d]pyrimidine-2-yl, 5,6,7,8-tetrahydroquinazolin-2-yl, 6-fluoro-2-benzoxazolyl, 5-fluoro-2-benzoxazolyl, 5,6-difluoro-2-benzoxazolyl, 6-chloro-2-benzoxazolyl, 5-chloro-2-benzoxazolyl, 5,6-dichloro-2-benzoxazolyl, 5-chloro-6-fluoro-2-benzoxazolyl, 5-methyl-2-benzoxazolyl, 5-cyano-2-benzoxazolyl, 5'-trifluoromethyl-2-benzoxazolyl, 5-methylthio-2-benzoxazolyl, 6-fluoro-2-benzothiazolyl, 5-fluoro-2-benzothiazolyl, 5,6-difluoro-2-benzothiazolyl, 6-chloro-2-benzothiazolyl, 5-chloro-2-benzothiazolyl, 5,6-dichloro-2-benzothiazolyl, 5-chloro-6-fluoro-2-benzothiazolyl, 5-methyl-2-benzothiazolyl, 5-cyano-2-benzothiazolyl, 5-trifluoromethyl-2-benzothiazolyl, 5-methylthio-2-benzothiazolyl, 5-fluoroquinolin-2-yl, 6-fluoroquinolin-2-yl, 7-fluoroquinolin-2-yl, 5-chloroquinolin-2-yl, 6-chloroquinolin-2-yl, 7-chloroquinolin-2-yl, 7-methylquinolin-2-yl, 7-trifluoromethylquinolin-2-yl, 7-methoxyquinolin-2-yl, 7-difluoromethoxyquinolin-2-yl, 7-trifluoromethoxyquinolin-2-yl, 5,7-difluoroquinolin-2-yl, 6,7-difluoroquinolin-2-yl, 5,7-dichloroquinolin-2-yl, 6,7-dichloroquinolin-2-yl, 5-chloro-7-fluoroquinolin-2-yl, 6-chloro-7-fluoroquinolin-2-yl, 7-chloro-5-fluoroquinolin-2-yl, 7-chloro-6-fluoroquinolin-2-yl, 7-chloro-6-cyano-quinolin-2-yl, 7-cyano-6-fluoroquinolin-2-yl, 6-fluoro-7-trifluoromethylquinolin-2-yl, 5,6,7-trifluoroquinolin-2-yl, 5-fluoroquinazolin-2-yl, 6-fluoroquinazolin-2-yl, 7-fluoroquinazolin-2-yl, 5-chloroquinazolin-2-yl, 6-chloroquinazolin-2-yl, 7-chloroquinazolin-2-yl, 7-methylquinazolin-2-yl, 7-trifluoromethylquinazolin-2-yl, 7-methoxyquinazolin-2-yl, 7-difluoromethoxyquinazolin-2-yl, 7-trifluoromethoxyquinazolin-2-yl, 5,7-difluoroquinazolin-2-yl, 6,7-difluoroquinazolin-2-yl, 5,7-dichloroquinazolin-2-yl, 6,7-dichloroquinazolin-2-yl, 5-chloro-7-fluoroquinazolin-2-yl, 6-chloro-7-fluoroquinazolin-2-yl, 7-chloro-5-fluoroquinazolin-2-yl, 7-chloro-6-fluoroquinazolin-2-yl, 7-chloro-6-cyano-quinazolin-2-yl, 7-cyano-6-fluoroquinazolin-2-yl, 6-fluoro-7-trifluoromethylquinazolin-2-yl or 5,6,7-trifluoroquinazolin-2-yl group,

still further preferably a 2-pyridyl, 2-benzothiazolyl, quinolin-2-yl, 5,6-difluoro-2-pyridyl, 5,6-dichloro-2-pyridyl, 5,6-dimethyl-2-pyridyl, 5,6,7,8-tetrahydroquinolin-2-yl, 6-fluoro-2-benzothiazolyl, 5-fluoro-2-benzothiazolyl, 5,6-difluoro-2-benzothiazolyl, 6-chloro-2-benzothiazolyl, 5-chloro-2-benzothiazolyl, 5,6-dichloro-2-benzothiazolyl, 5-chloro-6-fluoro-2-benzothiazolyl, 5-methyl-2-benzothiazolyl, 5-cyano-2-benzothiazolyl, 5-trifluoromethyl-2-benzothiazolyl, 5-methylthio-2-benzothiazolyl, 5-fluoroquinolin-2-yl, 6-fluoroquinolin-2-yl, 7-fluoroquinolin-2-yl, 5-chloroquinolin-2-yl, 6-chloroquinolin-2-yl, 7-chloroquinolin-2-yl, 7-methylquinolin-2-yl, 7-trifluoromethylquinolin-2-yl, 7-methoxyquinolin-2-yl, 7-difluoromethoxyquinolin-2-yl, 7-trifluoromethoxyquinolin-2-yl, 5,7-difluoroquinolin-2-yl, 6,7-difluoroquinolin-2-yl, 5,7-dichloroquinolin-2-yl, 6,7-dichloroquinolin-2-yl, 5-chloro-7-fluoroquinolin-2-yl, 6-chloro-7-fluoroquinolin-2-yl, 7-chloro-5-fluoroquinolin-2-yl, 7-chloro-6-fluoroquinolin-2-yl, 7-chloro-6-cyanoquinolin-2-yl, 7-cyano-6-fluoroquinolin-2-yl, 6-fluoro-7-trifluoromethylquinolin-2-yl or 5,6,7-trifluoroquinolin-2-yl group,

particularly preferably a 7-fluoroquinolin-2-yl, 7-chloroquinolin-2-yl, 6,7-difluoroquinolin-2-yl, 6,7-dichloroquinolin-2-yl, 7-chloro-6-fluoroquinolin-2-yl, 6-fluoro-7-trifluoromethylquinolin-2-yl or 5,6,7,8-tetrahydroquinolin-2-yl group.

In the above-mentioned formula (I), B represents a formula: $-OCH_2-$, a formula: $-CH_2CH_2-$, a formula: $-SCH_2-$, a formula: $-CH_2O-$ or a formula: $-CH_2S-$, preferably a formula: $-OCH_2-$, a formula: $-CH_2O-$ or a formula: $-CH_2CH_2-$.

In the above-mentioned formula (I), X is an oxygen atom, a sulfur atom, methylene group or a formula: =CH-, preferably a methylene group, an oxygen atom or a sulfur atom.

As the $C_1$-$C_{10}$ alkylene group of Y in the above-mentioned formula (I), there may be mentioned, for example, a methylene, ethylene, trimethylene, tetramethylene, pentamethylene, hexamethylene, heptamethylene, octamethylene, nonamethylene or decamethylene group, preferably a $C_1$-$C_5$ alkylene group, more preferably a methylene, ethylene or trimethylene group, particularly preferably an ethylene or trimethylene group.

The above-mentioned alkylene group may have a substituent(s), and a halogen atom, a $C_1$-$C_4$, alkyl group and a $C_1$-$C_4$ alkoxy group as said substituent (s) are the same as the above-mentioned halogen atom, the $C_1$-$C_4$ alkyl group and the $C_1$-$C_4$ alkoxy group.

As a substituent(s) for an alkylene group of Y, there may be preferably mentioned a fluorine, chlorine atom, a methyl, ethyl, propyl, methoxy, ethoxy or propoxy group, more preferably a fluorine atom, a methyl, ethyl or methoxy group, particularly preferably a fluorine atom or a methyl group.

As the phenylene group of Y, there may be mentioned a 1,2-phenylene, 1,3-phenylene or 1,4-phenylene group, preferably a 1, 2-phenylene or 1, 3-phenylene group, more preferably a 1,2-phenylene group.

As a group shown by the formula (a) of Y, there may be preferably mentioned a group wherein o=0, p=0 and q=1 (hereinafter referred to as group (a-1)), a group wherein o=0, p=1 and q=1 (hereinafter referred to as group (a-2)), a group whereino=0, p=1 and q=2 (hereinafter referred to as group (a-3)), a group wherein o=1, p=0 and q=1 (hereinafter referred to as group (a-4)), a group wherein o=1, p=1 and q=1 (hereinafter referred to as group group (a-5)), a group wherein o=1, p=1 and q=2 (hereinafter referred to as group (a-6)) or a group wherein o=1, p=1 and q=3 (hereinafter referred to as group (a-7)), more preferably a group (a-4), a group (a-5) or a group (a-6), particularly more preferably a group (a-5).

As the preferred group in the formula (I) of Y, there may be specifically mentioned, a methylene, ethylene, trimethylene, tetramethylene, pentamethylene, fluoromethylene, difluoromethylene, 1-fluoroethylene, 2-fluoroethylene, 1,1-difluoroethylene, 2,2-difluoroethylene, 1-methylethylene, 2-methylethylene, 1,1-dimethylethylene, 2,2-dimethylethylene, 1-ethylethylene, 2-ethylethylene, 1-methoxyethylene, 2-methoxyethylene, 1-fluorotrimethylene, 2-fluorotrimethylene, 3-fluorotrimethylene, 1,1-difluorotrimethylene, 2,2- difluorotrimethylene, 3,3-difluorotrimethylene, 1-methyltrimethylene, 2-methyltrimethylene, 1,1-dimethyltrimethylene, 2,2-dimethyltrimethylene, 3,3-dimethyltrimethylene, 2,2-diethyltrimethylene, 2-methoxytrimethylene, 3-methoxytrimethylene, 2,2-dimethoxytrimethylene, 3,3-dimethoxytrimethylene, 1,2-phenylene or 1,4-phenylene group, the above-mentioned group (a-1), group (a-2), group (a-3), group (a-4), group (a-5) or group (a-6),

further preferably a methylene, ethylene, trimethylene, fluoromethylene, difluoromethylene, 1-fluoroethylene, 2-fluoroethylene, 1,1-difluoroethylene, 2,2-difluoroethylene, 1-methylethylene, 2-methylethylene, 1,1-dimethylethylene, 2,2-dimethylethylene, 1-ethylethylene, 2-ethylethylene, 1-fluorotrimethylene, 2-fluorotrimethylene, 3-fluorotrimethylene, 1,1-difluorotrimethylene, 2,2-difluorotrimethylene, 3,3-difluorotrimethylene, 1-methyltrimethylene, 2-methyltrimethylene, 1,1-dimethyltrimethylene, 2,2-dimethyltrimethylene, 3,3-dimethyltrimethylene, 1,2-phenylene group, a group (a-4), a group (a-5) or a group (a-6),

further more preferably a methylene, ethylene, trimethylene, difluoromethylene, 1-fluoroethylene, 2-fluoroethylene, 1,1-difluoroethylene, 2,2-difluoroethylene, 1-methylethylene, 2-methylethylene, 1,1-dimethylethylene, 2,2-dimethylethylene, 1-ethylethylene, 2-ethylethylene, 2,2-difluorotrimethylene, 1-methyltrimethylene, 2-methyltrimethylene, 1,1-dimethyltrimethylene, 2,2-dimethyltrimethylene, 3,3-dimethyltrimethylene, 1,2-phenylene group or a group (a-5), particularly preferably methylene,ethylene, trimethylene, 1-methylethylene, 2-methylethylene, 1,1-dimethylethylene, 2,2-dimethylethylene, 1-ethylethylene, 2-ethylethylene, 1,2-phenylene group or a group (a-5).

In the group represented by the formula: $-NH-SO_2-R^3$ or a formula: $-CO-NH-SO_2-R^3$ of Z,
the halogen, $C_1$-$C_4$ alkyl, fluoro $C_1$-$C_4$ alkyl, $C_1$-$C_4$ alkoxy and fluoro $C_1$-$C_4$ alkoxy group which are a substituent(s) on the $C_1$-$C_4$ alkyl group, the fluoro $C_1$-$C_4$ alkyl group and the phenyl group of $R^3$, have the same meanings as the halogen atom, the $C_1$-$C_4$ alkyl group, the fluoro $C_1$-$C_4$ alkyl group, the $C_1$-$C_4$ alkoxy group and the fluoro $C_1$-$C_4$ alkoxy group of the above-metioned $R^1$, respectively.

As $R^3$, it is preferably a methyl, ethyl, propyl, fluoromethyl, difluoromethyl, trifluoromethyl, 2-fluoroethyl, 2,2,2-trifluoroethyl, phenyl, (o, m or p-)fluorophenyl, (o, m or p-)chlorophenyl, (o, m or p-)methylphenyl, (o, m or p-) ethylphenyl, (o, m or p-)(trifluoromethyl)phenyl, (o, m or p-)methoxyphenyl, (o, m or p-)ethoxyphenyl, (o, m or p-)-(difluoromethoxy)phenyl, (o, m or p-)(trifluoromethoxy)-phenyl, (o, morp-)nitrophenyl or (o, morp-)cyanophenyl group,

further preferably a methyl, ethyl, trifluoromethyl, 2-fluoroethyl, 2,2,2-trifluoroethyl, phenyl, (o or p-)-fluorophenyl, (o or p-)chlorophenyl, (o or p-)methylphenyl, (o or p-) (trifluoromethyl)phenyl, (o or p-)methoxyphenyl, (o or p-)(difluoromethoxy)phenyl, (o or p-)(trifluoromethoxy)-phenyl, (o or p-)nitrophenyl or (o or p-)cyanophenyl group,

further more preferably a methyl, ethyl, trifluoromethyl, phenyl, p-fluorophenyl, p-chlorophenyl, (o or p-) methylphenyl, p-(trifluoromethyl)phenyl, (o or p-)methoxyphenyl, p-(difluoromethoxy)phenyl, p-(trifluoromethoxy)phenyl, p-nitrophenyl or p-cyanophenyl group,

particularly preferably a methyl, trifluoromethyl, phenyl, o-methylphenyl or p-methylphenyl group.

As the preferred group of Z in the formula (I), there may be preferably mentioned, a carboxyl, a formula: $-SO_3H$, 1H-tetrazol-5-yl, methanesulfonylamino, ethanesulfonylamino, trifluoromethanesulfonylamino, phenylsulfonylamino, p-fluorophenylsulfonylamino, p-chlorophenylsulfonylamino, o-methylphenylsulfonylamino, p-methylphenylsulfonylamino, p-trifluoromethylphenylsulfonylamino, o-methoxyphenylsulfonylamino, p-methoxyphenylsulfonylamino, p-difluoromethoxyphenylsulfonylamino, p-trifluoromethoxyphenylsulfonylamino, p-nitrophenylsulfonylamino, p-cyanophenylsulfonylamino, methanesulfonylaminocarbonyl, ethanesulfonylaminocarbonyl, trifluoromethanesulfonylaminocarbonyl, phenylsulfonylaminocarbonyl, p-fluorophenylsulfonylaminocarbonyl, p-chlorophenylsulfonylaminocarbonyl, o-methylphenylsulfonylaminocarbonyl, p-methylphenylsulfonylaminocarbonyl, p-trifluoromethylphenylsulfonylaminocarbonyl, o-methoxyphenylsulfonylaminocarbonyl, p-methoxyphenylsulfonylaminocarbonyl, p-difluoromethoxyphenylsulfonylaminocarbonyl, p-trifluoromethoxyphenylsulfonylaminocarbonyl, p-nitrophenylsulfonylaminocarbonyl or p-cyanophenylsulfonylaminocarbonyl group,

more preferably a carboxyl, a formula: $-SO_3H$, 1H-tetrazol-5-yl, methanesulfonylamino, trifluoromethanesulfonylamino, phenylsulfonylamino, o-methylphenylsulfonylamino, p-methylphenylsulfonylamino, methanesulfonylaminocarbonyl, trifluoromethanesulfonylaminocarbonyl, phenylsulfonylaminocarbonyl, o-methylphenylsulfonylaminocarbonyl or p-methylphenylsulfonylaminocarbonyl group,

particularly preferably a carboxyl, a formula: $-SO_3H$, methanesulfonylamino, trifluoromethanesulfonylamino, methanesulfonylaminocarbonyl or trifluoromethanesulfonylaminocarbonyl group.

Incidentally, when Z is a carboxyl group, the carboxyl group may be protected by a protective group. As the protective group, it is not particularly limited so long as, it can be easily converted into a carboxyl group *in vivo,* there may be mentioned, for example, the $C_1$-$C_4$ alkyl group which has the same meanings as defined in $R^1$; a $C_7$-$C_{10}$ aralkyl group such as a benzyl, phenylethyl or phenylpropyl group; a $C_1$-$C_4$ alkyl group substituted by a $C_2$-$C_5$ alkanoyloxy group such as an acetoxymethyl, 1-acetoxyethyl, 1-acetoxypropyl, 1-acetoxybutyl, propanoyloxymethyl, 1-propanoyloxyethyl, butanoyloxymethyl, 1-butanoyloxymethyl, pivaloyloxymethyl, 1-pivaloyloxyethyl, 1-pivaloyloxypropyl or 1-pivaloyloxybutyl group; a $C_1$-$C_4$ alkyl group substituted by a ($C_1$-$C_4$ alkoxy)carbonyloxy group such as a methoxycarbonyloxymethyl, 1-(methoxycarbonyloxy)ethyl, ethoxycarbonyloxymethyl, 1-(ethoxycarbonyloxy)ethyl, propoxycarbonyloxymethyl, isopropoxycarbonyloxymethyl, 1-(isopropoxycarbonyloxy)ethyl, butoxycarbonyloxymethyl, 1-(butoxycarbonyloxy)-ethyl, t-butoxycarbonyloxymethyl or 1-(t-butoxycarbonyloxy) ethyl group; a $C_1$-$C_4$ alkyl group substituted by a N,N-di ($C_1$-$C_4$ alkyl)aminocarbonyl group such as N,N-dimethylaminocarbonylmethyl,. 2-(N,N-dimethylaminocarbonyl)ethyl or N,N-diethylaminocarbonylmethyl group; a $C_1$-$C_4$ alkyl group substituted by a N,N-di ($C_1$-$C_4$ alkyl) amino group or by a 5 or 6-membered cyclic amino group which may contain an oxygen atom such as a 2-(N,N-dimethylamino) ethyl, 2-(N,N- diethylamino)ethyl, 3-(N,N-dimethylamino)propyl, 2-piperidinoethyl, 2-(4-methyl)piperidinoethyl, 3-piperidinopropyl or 2-morpholinoethyl group; or a (5-methyl-2-oxo-1,3-dioxolen-4-yl)methyl group and the like.

As a protective group for a carboxyl group, there may be preferably mentioned a $C_1$-$C_4$ alkyl group, a benzyl group, a $C_1$-$C_2$ alkyl group substituted by a $C_2$-$C_5$ alkanoyloxy group, a $C_1$-$C_2$ alkyl group substituted by a ($C_1$-$C_4$ alkoxy)carbonyloxy group, or a (5-methyl-2-oxo-1,3-dioxolen-4-yl)methyl group,

more preferably a methyl, ethyl, propyl, isopropyl, butyl, isobutyl, acetoxymethyl, 1-acetoxyethyl, pivaloyloxymethyl or 1-pivaloyloxyethyl group.

In the above-mentioned formula (I), m is an integer of 1 to 4, preferably m is 1, 2 or 3, particularly preferably 1 or 2. When m is 2 or more, $R^1$ may be different from each other.

In the above-mentioned formula (I), n is an integer of 1 to 3, preferably n is 1 or 2, particularly preferably 1. When n is 2 or more, $R^2$ may be different from each other.

In the Compound (I) of the present invention, there exist an optical isomer(s) (including diastereomer) due to an asymmetric carbon atoms) in the molecule, or there exist a case in which a geometric isomer due to a double bond exists, and these respective isomers are included in the present invention.

Also, the Compound (I) of the present invention can be converted into a pharmaceutically acceptable salt, if necessary. Such a salt may be mentioned an acid addition salt of a mineral acid such as hydrochloride, hydrobromide, hydroiodide, sulfate or phosphate; an acid addition salt of an organic acid such as a trifluoroacetic acid salt, a methanesulfonic acid salt, an ethanesulfonic acid salt, a benzenesulfonic acid salt, a p-toluenesulfonic acid salt, an oxalate, a maleate, a fumarate, a tartarate or a citrate; a metal salt of a carboxylic acid such as a sodium salt, a potassium salt, a calcium salt, a magnesium salt, a manganese salt, an iron salt or an aluminum salt; or a salt with an organic base

such as an ammonium salt, a triethylamine salt, a guanidine salt, a hydrazine salt, a quinine salt or a cinchonine salt, and the like.

Incidentally, the Compound (I) of the present invention can also exist as a hydrate.

In the tricyclic compounds having the above-mentioned formula (I) according to the present invention, there may be preferably mentioned

(1) tricyclic compounds wherein $R^1$ in the compound represented by the formula (I) is selected from the group consisting of a hydrogen atom, a fluorine atom, a chlorine atom, a bromine atom, a hydroxyl group, a nitro group, a cyano group, a carbamoyl group, a formyl group, a carboxyl group, a methoxycarbonyl group, an ethoxycarbonyl group, a propoxycarbonyl group, an isopropoxycarbonyl group, a 1H-tetrazol-5-yl group, a methyl group, an ethyl group, a propyl group, an isopropyl group, a fluoromethyl group, a difluoromethyl group, a trifluoromethyl group, a 2-fluoroethyl group, a hydroxymethyl group, a 1-hydroxyethyl group, a 2-hydroxyethyl group, a 1-hydroxy-1-methylethyl group, a 1-hydroxypropyl group, a 2-hydroxypropyl group, a vinyl group, a 1-propenyl group, an allyl group, a 1-butenyl group, a 2-butenyl group, a 2-methyl-1-propenyl group, an ethynyl group, a 1-propynyl group, a 1-butynyl group, a methoxy group, an ethoxy group, a propoxy group, an isopropoxy group, a fluoromethoxy group, a difluoromethoxy group, a trifluoromethoxy group, a 2-fluoroethoxy group, a methylthio group, an ethylthio group, a propylthio group, an isopropylthio group, a methylsulfinyl group, an ethylsulfinyl group, a propylsulfinyl group, an isopropylsulfinyl group, a methylsulfonyl group, an ethylsulfonyl group, a propylsulfonyl group and an isopropylsulfonyl group,

(2). tricyclic compounds wherein $R^1$ in the compound represented by the formula (I) is selected from the group consisting of a hydrogen atom, a fluorine atom, a chlorine atom, a hydroxyl group, a nitro group, a cyano group, a carbamoyl group, a formyl group, a methoxycarbonyl group, an ethoxycarbonyl group; a 1H-tetrazol-5-yl group, a methyl group, an ethyl group, a fluoromethyl group, a difluoromethyl group, a trifluoromethyl group, a hydroxymethyl group, a 1-hydroxyethyl group, a 1-hydroxy-1-methylethyl group, a 1-hydroxypropyl group, a vinyl group, a 1-propenyl group, an allyl group, an ethynyl group, a 1-propynyl group, a 1-butynyl group, a methoxy group, an ethoxy group, a fluoromethoxy group, a difluoromethoxy group, a trifluoromethoxy group, a methylthio group, an ethylthio group, a methylsulfinyl group, an ethylsulfinyl group, a methylsulfonyl group and an ethylsulfonyl group,

(3). tricyclic compounds wherein $R^1$ in the compound represented by the formula (I) is selected from the group consisting of a hydrogen atom, a fluorine atom, a nitro group, a cyano group, a formyl group, a methoxycarbonyl group, a 1H-tetrazol-5-yl group, methyl group, a difluoromethyl group, a trifluoromethyl group, a hydroxymethyl group, a 1-hydroxy-1-methylethyl group, a vinyl group, an ethynyl group, a methoxy group, a difluoromethoxy group, a trifluoromethoxy group, a methylthio group, a methylsulfinyl group and a methylsulfonyl group,

(4). tricyclic compounds wherein $R^1$ in the compound represented by the formula (I) is selected from the group consisting of a hydrogen atom, a fluorine atom, a cyano group, a trifluoromethyl group and an ethynyl group,

(5). tricyclic compounds wherein $R^2$ in the compound represented by the formula (I) is selected from the group consisting of a hydrogen atom, a fluorine atom, a chlorine atom, a nitro group, a cyano group, a methyl group, an ethyl group, a methoxy group and an ethoxy group,

(6). tricyclic compounds wherein $R^2$ in the compound represented by the formula (I) is selected from the group consisting of a hydrogen atom, a fluorine atom, a chlorine atom, a methyl group and a methoxy group,

(7). tricyclic compounds wherein $R^2$ in the compound represented by the formula (I) is a hydrogen atom,

(8). tricyclic compounds wherein the ring shown by A in the compound represented by the formula (I) is selected from the group consisting of a furan, thiophene, oxazole, thiazole, imidazole, pyrazole, thiadiazole, pyridine, pyrimidine, pyridazine, pyrazine, benzofuran, benzothiophene, benzoxazole, benzothiazole, benzimidazole, quinoline, quinazoline and quinoxaline rings,

(9). tricyclic compounds wherein the ring shown by A in the compound represented by the formula (I) is selected from the group consisting of an oxazole, thiazole, imidazole, pyrazole, thiadiazole, pyridine, pyrimidine, pyridazine, pyrazine, benzoxazole, benzothiazole, benzimidazole, quinoline, quinazoline and quinoxaline rings,

(10). tricyclic compounds wherein the ring shown by A in the compound represented by the formula (I) is selected from the group consisting of a thiazole, thiadiazole, pyridine, pyrimidine, benzoxazole, benzothiazole, quinoline

and quinazoline rings,

(11). tricyclic compounds wherein the ring shown by A in the compound represented by the formula (I) is selected from the group consisting of a pyridine, benzothiazole and quinoline rings,

(12). tricyclic compounds wherein the heteroaromatic ring group or fused heteroaromatic ring group is substituted by at least one substituent(s) selected from a fluorine, chlorine, bromine atom, a nitro, cyano, methyl, ethyl, propyl, isopropyl, butyl, isobutyl, t-butyl, fluoromethyl, difluoromethyl, trifluoromethyl, 2-fluoroethyl, methoxy, ethoxy, propoxy, isopropoxy, fluoromethoxy, difluoromethoxy, trifluoromethoxy, 2-fluoroethoxy, methylthio, ethylthio, propylthio, isopropylthio, trimethylene and tetramethylene groups,

(13). tricyclic compounds wherein the heteroaromatic ring group or fused heteroaromatic ring group is substituted by at least one substituent(s) selected from a fluorine, chlorine atom, a nitro, cyano, methyl, ethyl, isopropyl, t-butyl, fluoromethyl, difluoromethyl, trifluoromethyl, methoxy, ethoxy, fluoromethoxy, difluoromethoxy, trifluoromethoxy, methylthio, ethylthio, trimethylene and tetramethylene groups,

(14). tricyclic compounds wherein the heteroaromatic ring group or fused heteroaromatic ring group is substituted by at least one substituent(s) selected from a fluorine, chlorine atom, a nitro, cyano, methyl, isopropyl, t-butyl, difluoromethyl, trifluoromethyl, methoxy, difluoromethoxy, trifluoromethoxy, methylthio and tetramethylene groups,

(15). tricyclic compounds wherein the heteroaromatic ring group or fused heteroaromatic ring group is substituted by at least one substituent(s) selected from a fluorine, chlorine atom, a trifluoromethyl and tetramethylene groups,

(16), tricyclic compounds wherein A in the compound represented by the formula (I) is selected from the group consisting of a 2-oxazolyl, 2-thiazolyl, 2- or 4-imidazolyl, 3-pyrazolyl, 1,3,4-thiadiazol-2-yl, 2-pyridyl, 2- or 4-pyrimidinyl, 3-pyridazinyl, 2-pyrazinyl, 2-benzoxazolyl, 2-benzothiazolyl, 2-benzimidazolyl, quinolin-2-yl, quinazolin-2-yl, quinoxaline-2-yl, 4-methyl-2-thiazolyl, 4-ethyl- 2-thiazolyl, 4-isopropyl-2-thiazolyl, 4-t-butyl-2-thiazolyl, 4-trifluoromethyl-2-thiazolyl, 5-methyl-1,3,4-thiadiazol-2-yl, 5-ethyl-1,3,4-thiadiazol-2-yl, 5-isopropyl-1,3,4-thiadiazol-2-yl, 5-t-butyl-1,3,4-thiadiazol-2-yl, 5-trifluoromethyl-1,3,4-thiadiazol-2-yl, 5,6-difluoro-2-pyridyl, 5,6-dichloro-2-pyridyl, 5,6-dimethyl-2-pyridyl, 5,6-diethyl-2-pyridyl, 6-trifluoromethyl-2-pyridyl, 6-methylthio-2-pyridyl, 5,6-dihydroclopenta[b]pyridin-2-yl, 5,6,7,8-tetrahydroquinolin-2-yl, 5,6-difluoro-2-pyrimidinyl, 5,6-dichloro-2-pyrimidinyl, 5,6-dimethyl-2-pyrimidinyl, 6-trifluoromethyl-2-pyrimidinyl, 5,6-dihydrocyclopenta-[d]pyrimidine-2-yl, 5,6,7,8-tetrahydroquinazolin-2-yl, 6-fluoro-2-benzoxazolyl, 5-fluoro-2-benzoxazolyl, 5,6-difluoro-2-benzoxazolyl, 6-chloro-2-benzoxazolyl, 5-chloro-2-benzoxazolyl, 5,6-dichloro-2-benzoxazolyl, 5-chloro-6-fluoro-2-benzoxazolyl, 5-methyl-2-benzoxazolyl, 5-cyano-2-benzoxazolyl, 5-trifluoromethyl-2-benzoxazolyl, 5-methylthio-2-benzoxazolyl, 6-fluoro-2-benzothiazolyl, 5-fluoro-2-benzothiazolyl, 5,6-difluoro-2-benzothiazolyl, 6-chloro-2-benzothiazolyl, 5-chloro-2-benzothiazolyl, 5,6-dichloro-2-benzothiazolyl, 5-chloro-6-fluoro-2-benzothiazolyl, 5-methyl-2-benzothiazolyl, 5-cyano-2-benzothiazolyl, 5-trifluoromethyl-2-benzothiazolyl, 5-methylthio-2-benzothiazolyl, 5-fluoroquinolin-2-yl, 6-fluoroquinolin-2-yl, 7-fluoroquinolin-2-yl, 5-chloroquinolin-2-yl, 6-chloroquinolin-2-yl, 7-chloroquinolin-2-yl, 7-methylquinolin-2-yl, 7-trifluoromethylquinolin-2-yl, 7-methoxyquinolin-2-yl, 7-difluoromethoxyquinolin-2-yl, 7-trifluoromethoxyquinolin-2-yl, 5,7-difluoroquinolin-2-yl, 6,7-difluoroquinolin-2-yl, 5,7-dichloroquinolin-2-yl, 6,7-dichloroquinolin-2-yl, 5-chloro-7-fluoroquinolin-2-yl, 6-chloro-7-fluoroquinolin-2-yl, 7-chloro-5-fluoroquinolin-2-yl, 7-chloro-6-fluoroquinolin-2-yl, 7-chloro-6-cyano-quinolin-2-yl, 7-cyano-6-fluoroquinolin-2-yl, 6-fluoro-7-trifluoromethylquinolin-2-yl, 5, 6,7-trifluoroquinolin-2-yl, 5-fluoroquinazolin-2-yl, 6-fluoroquinazolin-2-yl, 7-fluoroquinazolin-2-yl, 5-chloroquinazolin-2-yl, 6-chloroquinazolin-2-yl, 7-chloroquinazolin-2-yl, 7-methylquinazolin-2-yl, 7-trifluoromethylquinazolin-2-yl, 7-methoxyquinazolin-2-yl, 7-difluoromethoxyquinazolin-2-yl, 7-trifluoromethoxyquinazolin-2-yl, 5,7-difluoroquinazolin-2-yl, 6,7-difluoroquinazolin-2-yl, 5,7-dichloroquinazolin-2-yl, 6,7-dichloroquinazolin-2-yl, 5-chloro-7-fluoroquinazolin-2-yl, 6-chloro-7-fluoroquinazolin-2-yl, 7-chloro-5-fluoroquinazolin-2-yl, 7-chloro-6-fluoroquinazolin-2-yl, 7-chloro-6-cyano-quinazolin-2-yl, 7-cyano-6-fluoroquinazolin-2-yl, 6-fluoro-7-trifluoromethylquinazolin-2-yl and 5,6,7-trifluoroquinazolin-2-yl groups,

(17). tricyclic compounds wherein A in the compound represented by the formula (I) is selected from the group consisting of a 2-thiazolyl, 1,3,4-thiadiazol-2-yl, 2-pyridyl, 2-pyrimidinyl, 2-benzoxazolyl, 2-benzothiazolyl, quinolin-2-yl, quinazolin-2-yl, 4-methyl-2-thiazolyl, 4-isopropyl-2-thiazolyl, 4-t-butyl-2-thiazolyl, 4-trifluoromethyl-2-thiazolyl, 5-methyl-1,3,4-thiadiazol-2-yl, 5-isopropyl-1,3,4-thiadiazol-2-yl, 5-t-butyl-1,3,4-thiadiazol-2-yl, 5-trifluoromethyl-1,3,4-thiadiazol-2-yl, 5,6-difluoro-2-pyridyl, 5,6-dichloro-2-pyridyl, 5,6-dimethyl-2-pyridyl, 5,6-dihydroclopenta[b]pyridin-2-yl, 5,6,7,8-tetrahydroquinolin-2-yl, 5,6-difluoro-2-pyrimidinyl, 5,6-dichloro-2-pyrimidinyl, 5,6-dimethyl-2-pyrimidinyl, 6-trifluoromethyl-2-pyrimidinyl, 5,6-dihydrocyclopenta[d]pyrimidine-2-yl, 5,6,7,8-tetrahydroquinazolin-2-yl, 6-fluoro-2-benzoxazolyl, 5-fluoro-2-benzoxazolyl, 5,6-difluoro-2-benzoxazolyl, 6-chloro-2-benzoxazolyl, 5-chloro-2-benzoxazolyl, 5,6-dichloro-2-benzoxazolyl, 5-chloro-6-fluoro-2-benzoxazolyl, 5-methyl-2-benzoxazolyl, 5-cyano-2-benzoxazolyl, 5-trifluoromethyl-2-benzoxazolyl, 5-methylthio-2-benzoxazolyl, 6-fluoro-2-benzothiazolyl, 5-fluoro-2-benzothiazolyl, 5,6-difluoro-2-benzothiazolyl, 6-chloro-2-benzothiazolyl, 5-chloro-2-benzo-thiazolyl, 5,6-dichloro-2-benzothiazolyl, 5-chloro-6-fluoro-2-benzothiazolyl, 5-methyl-2-benzothiazolyl, 5-cyano-2-benzothiazolyl, 5-trifluoromethyl-2-benzothiazolyl, 5-methylthio-2-benzothiazolyl, 5-fluoroquinolin-2-yl, 6-fluoroquinolin-2-yl, 7-fluoroquinolin-2-yl, 5-chloroquinolin-2-yl, 6-chloroquinolin-2-yl, 7-chloroquinolin-2-yl, 7-methylquinolin-2-yl, 7-trifluoromethylquinolin-2-yl, 7-methoxyquinolin-2-yl, 7-difluoromethoxyquinolin-2-yl, 7-trifluoromethoxyquinolin-2-yl, 5,7-difluoroquinolin-2-yl, 6,7-difluoroquinolin-2-yl, 5,7-dichloroquinolin-2-yl, 6,7-dichloroquinolin-2-yl, 5-chloro-7-fluoroquinolin-2-yl, 6-chloro-7-fluoroquinolin-2-yl, 7-chloro-5-fluoroquinolin-2-yl, 7-chloro-6-fluoroquinolin-2-yl, 7-chloro-6-cyanoquinolin-2-yl, 7-cyano-6-fluoroquinolin-2-yl, 6-fluoro-7-trifluoromethylquinolin-2-yl, 5,6,7-trifluoroquinolin-2-yl, 5-fluoroquinazolin-2-yl, 6-fluoroquinazolin-2-yl, 7-fluoroquinazolin-2-yl, 5-chloroquinazolin-2-yl, 6-chloroquinazolin-2-yl, 7-chloroquinazolin-2-yl, 7-methylquinazolin-2-yl, 7-trifluoromethylquinazolin-2-yl, 7-methoxyquinazolin-2-yl, 7-difluoromethoxyquinazolin-2-yl, 7-trifluoromethoxyquinazolin-2-yl, 5,7-difluoroquinazolin-2-yl, 6,7-difluoroquinazolin-2-yl, 5,7-dichloroquinazolin-2-yl, 6,7-dichloroquinazolin-2-yl, 5-chloro-7-fluoroquinazolin-2-yl, 6-chloro-7-fluoroquinazolin-2-yl, 7-chloro-5-fluoroquinazolin-2-yl, 7-chloro-6-fluoroquinazolin-2-yl, 7-chloro-6-cyanoquinazolin-2-yl, 7-cyano-6-fluoroquinazolin-2-yl, 6-fluoro-7-trifluoromethylquinazolin-2-yl and 5,6,7-trifluoroquinazolin-2-yl groups,

(18). tricyclic compounds wherein A in the compound represented by the formula (I) is selected from the group consisting of a 2-pyridyl, 2-benzothiazolyl, quinolin-2-yl, 5,6-difluoro-2-pyridyl, 5,6-dichloro-2-pyridyl, 5,6-dimethyl-2-pyridyl, 5,6,7,8-tetrahydroquinolin-2-yl, 6-fluoro-2-benzothiazolyl, 5-fluoro-2-benzothiazolyl, 5,6-difluoro-2-benzothiazolyl, 6-chloro-2-benzothiazolyl, 5-chloro-2-benzothiazolyl, 5,6-dichloro-2-benzothiazolyl, 5-chloro-6-fluoro-2-benzothiazolyl, 5-methyl-2-benzothiazolyl, 5-cyano-2-benzothiazolyl, 5-trifluoromethyl-2-benzothiazolyl, 5-methylthio-2-benzothiazolyl, 5-fluoroquinolin-2-yl, 6-fluoroquinolin-2-yl, 7-fluoroquinolin-2-yl, 5-chloroquinolin-2-yl, 6-chloroquinolin-2-yl, 7-chloroquinolin-2-yl, 7-methylquinolin-2-yl, 7-trifluoromethylquinolin-2-yl, 7-methoxyquinolin-2-yl, 7-difluoromethoxyquinolin-2-yl, 7-trifluoromethoxyquinolin-2-yl, 5,7-difluoroquinolin-2-yl, 6,7-difluoroquinolin-2-yl, 5,7-dichloroquinolin-2-yl, 6,7-dichloroquinolin-2-yl, 5-chloro-7-fluoroquinolin-2-yl, 6-chloro-7-fluoroquinolin-2-yl, 7-chloro-5-fluoroquinolin-2-yl, 7-chloro-6-fluoroquinolin-2-yl, 7-chloro-6-cyanoquinolin-2-yl, 7-cyano-6-fluoroquinolin-2-yl, 6-fluoro-7-trifluoromethylquinolin-2-yl and 5,6,7-trifluoroquinolin-2-yl groups,

(19). tricyclic compounds wherein A in the compound represented by the formula (I) is selected from the group consisting of a 7-fluoroquinolin-2-yl, 7-chloroquinolin-2-yl, 6,7-difluoroquinolin-2-yl, 6,7-dichloroquinolin-2-yl, 7-chloro-6-fluoroquinolin-2-yl, 6-fluoro-7-trifluoromethylquinolin-2-yl and 5,6,7,8-tetrahydroquinolin-2-yl groups,

(20). tricyclic compounds wherein B in the formula (I) is a formula: $-OCH_2-$, a formula: $-CH_2O-$ or a formula: $-CH_2CH_2-$,

(21). tricyclic compounds wherein X in the formula (I) is a methylene group, a sulfur or an oxygen atom,

(22). tricyclic compounds wherein Y in the formula (I) is selected from the group consisting of a methylene, ethylene, trimethylene, tetramethylene, pentamethylene, fluoromethylene, difluoromethylene, 1-fluoroethylene, 2-fluoroethylene, 1,1-difluoroethylene, 2,2-difluoroethylene, 1-methylethylene, 2-methylethylene, 1,1-dimethylethylene, 2,2-dimethylethylene, 1-ethylethylene, 2-ethylethylene, 1-methoxyethylene, 2-methoxyethylene, 1-fluorotrimethylene, 2-fluorotrimethylene, 3-fluorotrimethylene, 1,1-difluorotrimethylene, 2,2-difluorotrimethylene, 3,3-difluorotrimethylene, 1-methyltrimethylene, 2-methyltrimethylene, 1,1-dimethyltrimethylene, 2,2-dimethyltrimethylene, 3,3-dimethyltrimethylene, 2,2-diethyltrimethylene, 2-methoxytrimethylene, 3-methoxytrimethylene, 2,2-dimethoxytrimethylene, 3,3-dimethoxytrimethylene, 1,2-phenylene, 1, 3-phenylene group, a group shown by a group (a) wherein o=0, p=0 and q=1, a group wherein o=0, p=1 and q=1, a group wherein o=0, p=1 and q=2, a group wherein o=1, p=0 and q=1, a group wherein o=1, p=1 and q=1 and a group wherein o=1, p=1 and q=2,

(23). tricyclic compounds wherein Y in the formula (I) is selected from the group consisting of a methylene, ethylene, trimethylene, fluoromethylene, difluoromethylene, 1-fluoroethylene, 2-fluoroethylene, 1,1-difluoroethylene, 2,2-difluoroethylene, 1-methylethylene, 2-methylethylene, 1,1-dimethylethylene, 2,2-dimethylethylene, 1-ethylethylene, 2-ethylethylene, 1-fluorotrimethylene, 2-fluorotrimethylene, 3-fluorotrimethylene, 1,1-difluorotrimethylene, 2,2-difluorotrimethylene, 3,3-difluorotrimethylene, 1-methyltrimethylene, 2-methyltrimethylene, 1,1-dimethyltrimethylene, 2,2-dimethyltrimethylene, 3,3-dimethyltrimethylene, 1,2-phenylene group, in a group shown by the formula (a), a group wherein o=1, p=0 and q=1, a group wherein o=1, p=1 and q=1 and a group wherein o=1, p=1 and q=2,

(24). tricyclic compounds wherein Y in the formula (I) is selected from the group consisting of a methylene, ethylene, trimethylene, difluoromethylene, 1-fluoroethylene, 2-fluoroethylene, 1,1-difluoroethylene, 2,2-difluoroethylene, 1-methylethylene, 2-methylethylene, 1,1-dimethylethylene, 2,2-dimethylethylene, 1-ethylethylene, 2-ethylethylene, 2,2-difluorotrimethylene, 1-methyltrimethylene, 2-methyltrimethylene, 1,1-dimethyltrimethylene, 2,2-dimethyltrimethylene, 3,3-dimethyltrimethylene, 1,2-phenylene and in a group shown by the formula (a), a group wherein o=1, p=1 and q=1,

(25). tricyclic compounds wherein Y in the formula (I) is selected from the group consisting of a methylene, ethylene, trimethylene, 1-methylethylene, 2-methylethylene, 1,1-dimethylethylene, 2,2-dimethylethylene, 1-ethylethylene, 2-ethylethylene, 1,2-phenylene and in a group shown by the formula (a), a group wherein o=1, p=1 and q=1,

(26). tricyclic compounds wherein Z in the formula (I) is selected from the group consisting of a carboxyl, a 1H-tetrazol-5-yl, a formula: $-SO_3H$, methanesulfonylamino, ethanesulfonylamino, trifluoromethanesulfonylamino, phenylsulfonylamino, p-fluorophenylsulfonylamino, p-chlorophenylsulfonylamino, o-methylphenylsulfonylamino, p-methylphenylsulfonylamino, p-trifluoromethylphenylsulfonylamino, o-methoxyphenylsulfonylamino, p-methoxyphenylsulfonylamino, p-difluoromethoxyphenylsulfonylamino, p-trifluoromethoxyphenylsulfonylamino, p-nitrophenylsulfonylamino, p-cyanophenylsulfonylamino, methanesulfonylaminocarbonyl, ethanesulfonylaminocarbonyl, trifluoromethanesulfonylaminocarbonyl, phenylsulfonylaminocarbonyl, p-fluorophenylsulfonylaminocarbonyl, p-chlorophenylsulfonylaminocarbonyl, o-methylphenylsulfonylaminocarbonyl, p-methylphenylsulfonylaminocarbonyl, p-trifluoromethylphenylsulfonylaminocarbonyl, o-methoxyphenylsulfonylaminocarbonyl, p-methoxyphenylsulfonylaminocarbonyl, p-difluoromethoxyphenylsulfonylaminocarbonyl, p-trifluoromethoxyphenylsulfonylaminocarbonyl, p-nitrophenylsulfonylaminocarbonyl and p-cyanophenylsulfonylaminocarbonyl groups,

(27). tricyclic compounds wherein Z in the formula (I) is selected from the group consisting of a carboxyl, a 1H-tetrazol-5-yl, a formula: $-SO_3H$, methanesulfonylamino, trifluoromethanesulfonylamino, phenylsulfonylamino, o-methylphenylsulfonylamino, p-methylphenylsulfonylamino, methanesulfonylaminocarbonyl, trifluoromethanesulfonylaminocarbonyl, phenylsulfonylaminocarbonyl, o-methylphenylsulfonylaminocarbonyl and p-methylphenylsulfonylaminocarbonyl groups,

(28). tricyclic compounds wherein Z in the formula (I) is selected from the group consisting of a carboxyl, a formula: $-SO_3H$, methanesulfonylamino, trifluoromethanesulfonylamino, methanesulfonylaminocarbonyl and trifluoromethanesulfonylaminocarbonyl groups,

(29). tricyclic compounds wherein m in the formula (I) is an integer of 1, 2 or 3,

(30). tricyclic compounds wherein n in the formula (I) is an integer of 1 or 2,
and with regard to $R^1$, a preferred order raises from (1) to (5) in this order, with regard to $R^2$, a preferred order raises from (6) to(8) in this order, with regard to A, a preferred order raises from (9) to (12), from (13) to (16) and from (17) to (20) in this order, with regard to Y, a preferred order raises from (23) to (26) in this order, with regard to Z, a preferred order raises from (27) to (29) in this order.

Also, as the tricyclic compounds having the above-mentioned formula (I), tricyclic compounds comprising the combination of two or more of the above-mentioned (1) to (30) are also preferred.

As the preferred compounds in the Compound (I), the compounds shown in the following Table 1 can be specifically exemplified.

Table 1

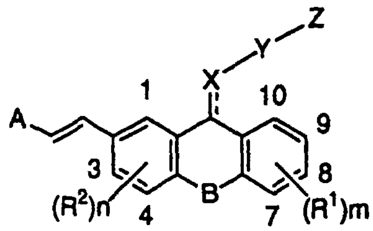

(I)

| No. | A | B | $(R^2)n$ | $(R^1)m$ | X-Y-Z |
|---|---|---|---|---|---|
| 1 | 6, 7-diF-Q | $-OCH_2-$ | H | H | $-OCH_2COOH$ |
| 2 | 6, 7-diF-Q | $-OCH_2-$ | H | H | $-OCH_2CH_2COOH$ |
| 3 | 6, 7-diF-Q | $-OCH_2-$ | H | H | $-OCH_2CH(CH_3)COOH$ |
| 4 | 6, 7-diF-Q | $-OCH_2-$ | H | H | $-OCH_2C(CH_2CH_2)CH_2COOH$ |
| 5 | 6, 7-diF-Q | $-OCH_2-$ | H | H | $-SCH_2COOH$ |
| 6 | 6, 7-diF-Q | $-OCH_2-$ | H | H | $-SCH_2CH_2COOH$ |
| 7 | 6, 7-diF-Q | $-OCH_2-$ | H | H | $-SCH_2CH(CH_3)COOH$ |
| 8 | 6, 7-diF-Q | $-OCH_2-$ | H | H | $-SCH_2C(CH_3)_2COOH$ |
| 9 | 6, 7-diF-Q | $-OCH_2-$ | H | H | $-SCH_2C(CH_2CH_2)COOH$ |
| 10 | 6, 7-diF-Q | $-OCH_2-$ | H | H | $-SCH_2CH(CH_2CH_3)COOH$ |
| 11 | 6, 7-diF-Q | $-OCH_2-$ | H | H | $-SCH(CH_3)CH_2COOH$ |
| 12 | 6, 7-diF-Q | $-OCH_2-$ | H | H | $-SC(CH_3)_2CH_2COOH$ |
| 13 | 6, 7-diF-Q | $-OCH_2-$ | H | H | $-SCH_2CH(CH_3)CH_2COOH$ |
| 14 | 6, 7-diF-Q | $-OCH_2-$ | H | H | $-SCH_2C(CH_2CH_2)CH_2COOH$ |
| 15 | 6, 7-diF-Q | $-OCH_2-$ | H | H | $-SCH_2C(CH_2)CCOOH$ |
| 16 | 6, 7-diF-Q | $-OCH_2-$ | H | H | -S(2-COOH-Ph) |
| 17 | 6, 7-diF-Q | $-OCH_2-$ | H | H | -S(3-COOH-Ph) |
| 18 | 6, 7-diF-Q | $-OCH_2-$ | H | H | -S(4-COOH-Ph) |
| 19 | 6, 7-diF-Q | $-OCH_2-$ | H | H | $-SCH_2$-Tet |
| 20 | 6, 7-diF-Q | $-OCH_2-$ | H | H | $-SCH_2CH_2$-Tet |
| 21 | 6, 7-diF-Q | $-OCH_2-$ | H | H | $-SCH_2NHSO_2CF_3$ |
| 22 | 6, 7-diF-Q | $-OCH_2-$ | H | H | $-SCH_2CONHSO_2CH_3$ |
| 23 | 6, 7-diF-Q | $-OCH_2-$ | H | H | $-SCH_2CONHSO_2CF_3$ |
| 24 | 6, 7-diF-Q | $-OCH_2-$ | H | H | $-SCH_2CONHSO_2Ph$ |
| 25 | 6, 7-diF-Q | $-OCH_2-$ | H | H | $-SCH_2CONHSO_2(2-CH_3-Ph)$ |
| 26 | 6, 7-diF-Q | $-OCH_2-$ | H | H | $-SCH_2CH_2NHSO_2CF_3$ |
| 27 | 6, 7-diF-Q | $-OCH_2-$ | H | H | $-SCH_2CH_2CONHSO_2CH_3$ |
| 28 | 6, 7-diF-Q | $-OCH_2-$ | H | H | $-SCH_2CH_2CONHSO_2CF_3$ |
| 29 | 6, 7-diF-Q | $-OCH_2-$ | H | H | $-SCH_2CH_2CONHSO_2Ph$ |
| 30 | 6, 7-diF-Q | $-OCH_2-$ | H | H | $-SCH_2CH_2CONHSO_2(2-CH_3-Ph)$ |
| 31 | 6, 7-diF-Q | $-OCH_2-$ | H | H | $-SCH_2CH_2SO_3H$ |
| 32 | 6, 7-diF-Q | $-OCH_2-$ | H | H | $-CH_2CH_2CH_2COOH$ |
| 33 | 6, 7-diF-Q | $-OCH_2-$ | H | H | $=CHCH_2CH_2COOH$ |
| 34 | 6, 7-diF-Q | $-OCH_2-$ | H | H | $=CHCH=CHCOOH$ |

| | | | | | |
|---|---|---|---|---|---|
| 35 | 6, 7-diF-Q | $-OCH_2-$ | H | 7-F | $-SCH_2COOH$ |
| 36 | 6, 7-diF-Q | $-OCH_2-$ | H | 7-F | $-SCH_2CH_2COOH$ |
| 37 | 6, 7-diF-Q | $-OCH_2-$ | H | 7-F | $-SCH_2CH(CH_3)COOH$ |
| 38 | 6, 7-diF-Q | $-OCH_2-$ | H | 7-F | $-SCH_2CH(CH_2CH_3)COOH$ |
| 39 | 6, 7-diF-Q | $-OCH_2-$ | H | 7-F | $-SCH_2C(CH_3)_2COOH$ |
| 40 | 6, 7-diF-Q | $-OCH_2-$ | H | 7-F | $-SC(CH_3)_2CH_2COOH$ |
| 41 | 6, 7-diF-Q | $-OCH_2-$ | H | 7-F | $-SCH_2CH(CH_3)CH_2COOH$ |
| 42 | 6, 7-diF-Q | $-OCH_2-$ | H | 7-F | $-SCH_2C(CH_2CH_2)CH_2COOH$ |
| 43 | 6, 7-diF-Q | $-OCH_2-$ | H | 8-F | $-SCH_2COOH$ |
| 44 | 6, 7-diF-Q | $-OCH_2-$ | H | 8-F | $-SCH_2CH_2COOH$ |
| 45 | 6, 7-diF-Q | $-OCH_2-$ | H | 8-F | $-SCH_2CH(CH_3)COOH$ |
| 46 | 6, 7-diF-Q | $-OCH_2-$ | H | 8-F | $-SCH_2CH(CH_2CH_3)COOH$ |
| 47 | 6, 7-diF-Q | $-OCH_2-$ | H | 8-F | $-SCH_2C(CH_3)_2COOH$ |
| 48 | 6, 7-diF-Q | $-OCH_2-$ | H | 8-F | $-SC(CH_3)_2CH_2COOH$ |
| 49 | 6, 7-diF-Q | $-OCH_2-$ | H | 8-F | $-SCH_2CH(CH_3)CH_2COOH$ |
| 50 | 6, 7-diF-Q | $-OCH_2-$ | H | 8-F | $-SCH_2C(CH_2CH_2)CH_2COOH$ |
| 51 | 6, 7-diF-Q | $-OCH_2-$ | H | 9-F | $-SCH_2COOH$ |
| 52 | 6, 7-diF-Q | $-OCH_2-$ | H | 9-F | $-SCH_2CH_2COOH$ |
| 53 | 6, 7-diF-Q | $-OCH_2-$ | H | 9-F | $-SCH_2CH(CH_3)COOH$ |
| 54 | 6, 7-diF-Q | $-OCH_2-$ | H | 9-F | $-SCH_2CH(CH_2CH_3)COOH$ |
| 55 | 6, 7-diF-Q | $-OCH_2-$ | H | 9-F | $-SCH_2C(CH_3)_2COOH$ |
| 56 | 6, 7-diF-Q | $-OCH_2-$ | H | 9-F | $-SC(CH_3)_2CH_2COOH$ |
| 57 | 6, 7-diF-Q | $-OCH_2-$ | H | 9-F | $-SCH_2CH(CH_3)CH_2COOH$ |
| 58 | 6, 7-diF-Q | $-OCH_2-$ | H | 9-F | $-SCH_2C(CH_2CH_2)CH_2COOH$ |
| 59 | 6, 7-diF-Q | $-OCH_2-$ | H | 7-CN | $-SCH_2COOH$ |
| 60 | 6, 7-diF-Q | $-OCH_2-$ | H | 7-CN | $-SCH_2CH_2COOH$ |
| 61 | 6, 7-diF-Q | $-OCH_2-$ | H | 7-CN | $-SCH_2CH(CH_3)COOH$ |
| 62 | 6, 7-diF-Q | $-OCH_2-$ | H | 7-CN | $-SCH_2CH(CH_2CH_3)COOH$ |
| 63 | 6, 7-diF-Q | $-OCH_2-$ | H | 7-CN | $-SCH_2C(CH_3)_2COOH$ |
| 64 | 6, 7-diF-Q | $-OCH_2-$ | H | 7-CN | $-SC(CH_3)_2CH_2COOH$ |
| 65 | 6, 7-diF-Q | $-OCH_2-$ | H | 7-CN | $-SCH_2CH(CH_3)CH_2COOH$ |
| 66 | 6, 7-diF-Q | $-OCH_2-$ | H | 7-CN | $-SCH_2C(CH_2CH_2)CH_2COOH$ |
| 67 | 6, 7-diF-Q | $-OCH_2-$ | H | 8-CN | $-SCH_2COOH$ |
| 68 | 6, 7-diF-Q | $-OCH_2-$ | H | 8-CN | $-SCH_2CH_2COOH$ |
| 69 | 6, 7-diF-Q | $-OCH_2-$ | H | 8-CN | $-SCH_2CH(CH_3)COOH$ |
| 70 | 6, 7-diF-Q | $-OCH_2-$ | H | 8-CN | $-SCH_2CH(CH_2CH_3)COOH$ |
| 71 | 6, 7-diF-Q | $-OCH_2-$ | H | 8-CN | $-SCH_2C(CH_3)_2COOH$ |
| 72 | 6, 7-diF-Q | $-OCH_2-$ | H | 8-CN | $-SC(CH_3)_2CH_2COOH$ |
| 73 | 6, 7-diF-Q | $-OCH_2-$ | H | 8-CN | $-SCH_2CH(CH_3)CH_2COOH$ |
| 74 | 6, 7-diF-Q | $-OCH_2-$ | H | 8-CN | $-SCH_2C(CH_2CH_2)CH_2COOH$ |
| 75 | 6, 7-diF-Q | $-OCH_2-$ | H | 9-CN | $-SCH_2COOH$ |
| 76 | 6, 7-diF-Q | $-OCH_2-$ | H | 9-CN | $-SCH_2CH_2COOH$ |
| 77 | 6, 7-diF-Q | $-OCH_2-$ | H | 9-CN | $-SCH_2CH(CH_3)COOH$ |
| 78 | 6, 7-diF-Q | $-OCH_2-$ | H | 9-CN | $-SCH_2CH(CH_2CH_3)COOH$ |
| 79 | 6, 7-diF-Q | $-OCH_2-$ | H | 9-CN | $-SCH_2C(CH_3)_2COOH$ |

| | | | | | |
|---|---|---|---|---|---|
| 80 | 6, 7-diF-Q | $-OCH_2-$ | H | 9-CN | $-SC(CH_3)_2CH_2COOH$ |
| 81 | 6, 7-diF-Q | $-OCH_2-$ | H | 9-CN | $-SCH_2CH(CH_3)CH_2COOH$ |
| 82 | 6, 7-diF-Q | $-OCH_2-$ | H | 9-CN | $-SCH_2C(CH_2CH_2)CH_2COOH$ |
| 83 | 6, 7-diF-Q | $-OCH_2-$ | H | $7-CF_3$ | $-SCH_2COOH$ |
| 84 | 6, 7-diF-Q | $-OCH_2-$ | H | $7-CF_3$ | $-SCH_2CH_2COOH$ |
| 85 | 6, 7-diF-Q | $-OCH_2-$ | H | $7-CF_3$ | $-SCH_2CH(CH_3)COOH$ |
| 86 | 6, 7-diF-Q | $-OCH_2-$ | H | $7-CF_3$ | $-SCH_2CH(CH_2CH_3)COOH$ |
| 87 | 6, 7-diF-Q | $-OCH_2-$ | H | $7-CF_3$ | $-SCH_2C(CH_3)_2COOH$ |
| 88 | 6, 7-diF-Q | $-OCH_2-$ | H | $7-CF_3$ | $-SC(CH_3)_2CH_2COOH$ |
| 89 | 6, 7-diF-Q | $-OCH_2-$ | H | $7-CF_3$ | $-SCH_2CH(CH_3)CH_2COOH$ |
| 90 | 6, 7-diF-Q | $-OCH_2-$ | H | $7-CF_3$ | $-SCH_2C(CH_2CH_2)CH_2COOH$ |
| 91 | 6, 7-diF-Q | $-OCH_2-$ | H | $8-CF_3$ | $-SCH_2COOH$ |
| 92 | 6, 7-diF-Q | $-OCH_2-$ | H | $8-CF_3$ | $-SCH_2CH_2COOH$ |
| 93 | 6, 7-diF-Q | $-OCH_2-$ | H | $8-CF_3$ | $-SCH_2CH(CH_3)COOH$ |
| 94 | 6, 7-diF-Q | $-OCH_2-$ | H | $8-CF_3$ | $-SCH_2CH(CH_2CH_3)COOH$ |
| 95 | 6, 7-diF-Q | $-OCH_2-$ | H | $8-CF_3$ | $-SCH_2C(CH_3)_2COOH$ |
| 96 | 6, 7-diF-Q | $-OCH_2-$ | H | $8-CF_3$ | $-SC(CH_3)_2CH_2COOH$ |
| 97 | 6, 7-diF-Q | $-OCH_2-$ | H | $8-CF_3$ | $-SCH_2CH(CH_3)CH_2COOH$ |
| 98 | 6, 7-diF-Q | $-OCH_2-$ | H | $8-CF_3$ | $-SCH_2C(CH_2CH_2)CH_2COOH$ |
| 99 | 6, 7-diF-Q | $-OCH_2-$ | H | $9-CF_3$ | $-SCH_2COOH$ |
| 100 | 6, 7-diF-Q | $-OCH_2-$ | H | $9-CF_3$ | $-SCH_2CH_2COOH$ |
| 101 | 6, 7-diF-Q | $-OCH_2-$ | H | $9-CF_3$ | $-SCH_2CH(CH_3)COOH$ |
| 102 | 6, 7-diF-Q | $-OCH_2-$ | H | $9-CF_3$ | $-SCH_2CH(CH_2CH_3)COOH$ |
| 103 | 6, 7-diF-Q | $-OCH_2-$ | H | $9-CF_3$ | $-SCH_2C(CH_3)_2COOH$ |
| 104 | 6, 7-diF-Q | $-OCH_2-$ | H | $9-CF_3$ | $-SC(CH_3)_2CH_2COOH$ |
| 105 | 6, 7-diF-Q | $-OCH_2-$ | H | $9-CF_3$ | $-SCH_2CH(CH_3)CH_2COOH$ |
| 106 | 6, 7-diF-Q | $-OCH_2-$ | H | $9-CF_3$ | $-SCH_2C(CH_2CH_2)CH_2COOH$ |
| 107 | 6, 7-diF-Q | $-OCH_2-$ | H | $7-C\equiv CH$ | $-SCH_2COOH$ |
| 108 | 6, 7-diF-Q | $-OCH_2-$ | H | $7-C\equiv CH$ | $-SCH_2CH_2COOH$ |
| 109 | 6, 7-diF-Q | $-OCH_2-$ | H | $7-C\equiv CH$ | $-SCH_2CH(CH_3)COOH$ |
| 110 | 6, 7-diF-Q | $-OCH_2-$ | H | $7-C\equiv CH$ | $-SCH_2CH(CH_2CH_3)COOH$ |
| 111 | 6, 7-diF-Q | $-OCH_2-$ | H | $7-C\equiv CH$ | $-SCH_2C(CH_3)_2COOH$ |
| 112 | 6, 7-diF-Q | $-OCH_2-$ | H | $7-C\equiv CH$ | $-SC(CH_3)_2CH_2COOH$ |
| 113 | 6, 7-diF-Q | $-OCH_2-$ | H | $7-C\equiv CH$ | $-SCH_2CH(CH_3)CH_2COOH$ |
| 114 | 6, 7-diF-Q | $-OCH_2-$ | H | $7-C\equiv CH$ | $-SCH_2C(CH_2CH_2)CH_2COOH$ |
| 115 | 6, 7-diF-Q | $-OCH_2-$ | H | $8-C\equiv CH$ | $-SCH_2COOH$ |
| 116 | 6, 7-diF-Q | $-OCH_2-$ | H | $8-C\equiv CH$ | $-SCH_2CH_2COOH$ |
| 117 | 6, 7-diF-Q | $-OCH_2-$ | H | $8-C\equiv CH$ | $-SCH_2CH(CH_3)COOH$ |
| 118 | 6, 7-diF-Q | $-OCH_2-$ | H | $8-C\equiv CH$ | $-SCH_2CH(CH_2CH_3)COOH$ |
| 119 | 6, 7-diF-Q | $-OCH_2-$ | H | $8-C\equiv CH$ | $-SCH_2C(CH_3)_2COOH$ |
| 120 | 6, 7-diF-Q | $-OCH_2-$ | H | $8-C\equiv CH$ | $-SC(CH_3)_2CH_2COOH$ |
| 121 | 6, 7-diF-Q | $-OCH_2-$ | H | $8-C\equiv CH$ | $-SCH_2CH(CH_3)CH_2COOH$ |
| 122 | 6, 7-diF-Q | $-OCH_2-$ | H | $8-C\equiv CH$ | $-SCH_2C(CH_2CH_2)CH_2COOH$ |
| 123 | 6, 7-diF-Q | $-OCH_2-$ | H | $9-C\equiv CH$ | $-SCH_2COOH$ |

| | | | | | |
|---|---|---|---|---|---|
| 124 | 6, 7-diF-Q | $-OCH_2-$ | H | $9-C\equiv CH$ | $-SCH_2CH_2COOH$ |
| 125 | 6, 7-diF-Q | $-OCH_2-$ | H | $9-C\equiv CH$ | $-SCH_2CH(CH_3)COOH$ |
| 126 | 6, 7-diF-Q | $-OCH_2-$ | H | $9-C\equiv CH$ | $-SCH_2CH(CH_2CH_3)COOH$ |
| 127 | 6, 7-diF-Q | $-OCH_2-$ | H | $9-C\equiv CH$ | $-SCH_2C(CH_3)_2COOH$ |
| 128 | 6, 7-diF-Q | $-OCH_2-$ | H | $9-C\equiv CH$ | $-SC(CH_3)_2CH_2COOH$ |
| 129 | 6, 7-diF-Q | $-OCH_2-$ | H | $9-C\equiv CH$ | $-SCH_2CH(CH_3)CH_2COOH$ |
| 130 | 6, 7-diF-Q | $-OCH_2-$ | H | $9-C\equiv CH$ | $-SCH_2C(CH_2CH_2)CH_2COOH$ |
| 131 | 6, 7-diF-Q | $-OCH_2-$ | H | $7-C(CH_3)_2OH$ | $-SCH_2COOH$ |
| 132 | 6, 7-diF-Q | $-OCH_2-$ | H | $7-C(CH_3)_2OH$ | $-SCH_2CH_2COOH$ |
| 133 | 6, 7-diF-Q | $-OCH_2-$ | H | $7-C(CH_3)_2OH$ | $-SCH_2CH(CH_3)COOH$ |
| 134 | 6, 7-diF-Q | $-OCH_2-$ | H | $7-C(CH_3)_2OH$ | $-SCH_2CH(CH_2CH_3)COOH$ |
| 135 | 6, 7-diF-Q | $-OCH_2-$ | H | $7-C(CH_3)_2OH$ | $-SCH_2C(CH_3)_2COOH$ |
| 136 | 6, 7-diF-Q | $-OCH_2-$ | H | $7-C(CH_3)_2OH$ | $-SC(CH_3)_2CH_2COOH$ |
| 137 | 6, 7-diF-Q | $-OCH_2-$ | H | $7-C(CH_3)_2OH$ | $-SCH_2CH(CH_3)CH_2COOH$ |
| 138 | 6, 7-diF-Q | $-OCH_2-$ | H | $7-C(CH_3)_2OH$ | $-SCH_2C(CH_2CH_2)CH_2COOH$ |
| 139 | 6, 7-diF-Q | $-OCH_2-$ | H | $8-C(CH_3)_2OH$ | $-SCH_2COOH$ |
| 140 | 6, 7-diF-Q | $-OCH_2-$ | H | $8-C(CH_3)_2OH$ | $-SCH_2CH_2COOH$ |
| 141 | 6, 7-diF-Q | $-OCH_2-$ | H | $8-C(CH_3)_2OH$ | $-SCH_2CH(CH_3)COOH$ |
| 142 | 6, 7-diF-Q | $-OCH_2-$ | H | $8-C(CH_3)_2OH$ | $-SCH_2CH(CH_2CH_3)COOH$ |
| 143 | 6, 7-diF-Q | $-OCH_2-$ | H | $8-C(CH_3)_2OH$ | $-SCH_2C(CH_3)_2COOH$ |
| 144 | 6, 7-diF-Q | $-OCH_2-$ | H | $8-C(CH_3)_2OH$ | $-SC(CH_3)_2CH_2COOH$ |
| 145 | 6, 7-diF-Q | $-OCH_2-$ | H | $8-C(CH_3)_2OH$ | $-SCH_2CH(CH_3)CH_2COOH$ |
| 146 | 6, 7-diF-Q | $-OCH_2-$ | H | $8-C(CH_3)_2OH$ | $-SCH_2C(CH_2CH_2)CH_2COOH$ |
| 147 | 6, 7-diF-Q | $-OCH_2-$ | H | $9-C(CH_3)_2OH$ | $-SCH_2COOH$ |
| 148 | 6, 7-diF-Q | $-OCH_2-$ | H | $9-C(CH_3)_2OH$ | $-SCH_2CH_2COOH$ |
| 149 | 6, 7-diF-Q | $-OCH_2-$ | H | $9-C(CH_3)_2OH$ | $-SCH_2CH(CH_3)COOH$ |
| 150 | 6, 7-diF-Q | $-OCH_2-$ | H | $9-C(CH_3)_2OH$ | $-SCH_2CH(CH_2CH_3)COOH$ |
| 151 | 6, 7-diF-Q | $-OCH_2-$ | H | $9-C(CH_3)_2OH$ | $-SCH_2C(CH_3)_2COOH$ |
| 152 | 6, 7-diF-Q | $-OCH_2-$ | H | $9-C(CH_3)_2OH$ | $-SC(CH_3)_2CH_2COOH$ |
| 153 | 6, 7-diF-Q | $-OCH_2-$ | H | $9-C(CH_3)_2OH$ | $-SCH_2CH(CH_3)CH_2COOH$ |
| 154 | 6, 7-diF-Q | $-OCH_2-$ | H | $9-C(CH_3)_2OH$ | $-SCH_2C(CH_2CH_2)CH_2COOH$ |
| 155 | 6-F, 7-Cl-Q | $-OCH_2-$ | H | H | $-SCH_2CH_2COOH$ |
| 156 | 6-F, 7-Cl-Q | $-OCH_2-$ | H | H | $-SCH_2CH(CH_3)COOH$ |
| 157 | 6-F, 7-Cl-Q | $-OCH_2-$ | H | H | $-SCH_2C(CH_3)_2COOH$ |
| 158 | 6-F, 7-Cl-Q | $-OCH_2-$ | H | H | $-SCH_2C(CH_2CH_2)CH_2COOH$ |
| 159 | 6-F, 7-Cl-Q | $-OCH_2-$ | H | 7-F | $-SCH_2CH_2COOH$ |
| 160 | 6-F, 7-Cl-Q | $-OCH_2-$ | H | 7-F | $-SCH_2CH(CH_3)COOH$ |
| 161 | 6-F, 7-Cl-Q | $-OCH_2-$ | H | 7-F | $-SCH_2C(CH_3)_2COOH$ |
| 162 | 6-F, 7-Cl-Q | $-OCH_2-$ | H | 7-F | $-SCH_2C(CH_2CH_2)CH_2COOH$ |
| 163 | 6-F, 7-Cl-Q | $-OCH_2-$ | H | 8-F | $-SCH_2CH_2COOH$ |
| 164 | 6-F, 7-Cl-Q | $-OCH_2-$ | H | 8-F | $-SCH_2CH(CH_3)COOH$ |
| 165 | 6-F, 7-Cl-Q | $-OCH_2-$ | H | 8-F | $-SCH_2C(CH_3)_2COOH$ |
| 166 | 6-F, 7-Cl-Q | $-OCH_2-$ | H | 8-F | $-SCH_2C(CH_2CH_2)CH_2COOH$ |
| 167 | 6-F, 7-Cl-Q | $-OCH_2-$ | H | 9-F | $-SCH_2CH_2COOH$ |

| | | | | | |
|---|---|---|---|---|---|
| 168 | 6-F, 7-Cl-Q | $-OCH_2-$ | H | 9-F | $-SCH_2CH(CH_3)COOH$ |
| 169 | 6-F, 7-Cl-Q | $-OCH_2-$ | H | 9-F | $-SCH_2C(CH_3)_2COOH$ |
| 170 | 6-F, 7-Cl-Q | $-OCH_2-$ | H | 9-F | $-SCH_2C(CH_2CH_2)CH_2COOH$ |
| 171 | 7-F-Q | $-OCH_2-$ | H | H | $-SCH_2CH_2COOH$ |
| 172 | 7-F-Q | $-OCH_2-$ | H | H | $-SCH_2CH(CH_3)COOH$ |
| 173 | 7-F-Q | $-OCH_2-$ | H | H | $-SCH_2C(CH_3)_2COOH$ |
| 174 | 7-F-Q | $-OCH_2-$ | H | H | $-SCH_2C(CH_2CH_2)CH_2COOH$ |
| 175 | 7-F-Q | $-OCH_2-$ | H | 7-F | $-SCH_2CH_2COOH$ |
| 176 | 7-F-Q | $-OCH_2-$ | H | 7-F | $-SCH_2CH(CH_3)COOH$ |
| 177 | 7-F-Q | $-OCH_2-$ | H | 7-F | $-SCH_2C(CH_3)_2COOH$ |
| 178 | 7-F-Q | $-OCH_2-$ | H | 7-F | $-SCH_2C(CH_2CH_2)CH_2COOH$ |
| 179 | 7-F-Q | $-OCH_2-$ | H | 8-F | $-SCH_2CH_2COOH$ |
| 180 | 7-F-Q | $-OCH_2-$ | H | 8-F | $-SCH_2CH(CH_3)COOH$ |
| 181 | 7-F-Q | $-OCH_2-$ | H | 8-F | $-SCH_2C(CH_3)_2COOH$ |
| 182 | 7-F-Q | $-OCH_2-$ | H | 8-F | $-SCH_2C(CH_2CH_2)CH_2COOH$ |
| 183 | 7-F-Q | $-OCH_2-$ | H | 9-F | $-SCH_2CH_2COOH$ |
| 184 | 7-F-Q | $-OCH_2-$ | H | 9-F | $-SCH_2CH(CH_3)COOH$ |
| 185 | 7-F-Q | $-OCH_2-$ | H | 9-F | $-SCH_2C(CH_3)_2COOH$ |
| 186 | 7-F-Q | $-OCH_2-$ | H | 9-F | $-SCH_2C(CH_2CH_2)CH_2COOH$ |
| 187 | 7-F-Q | $-OCH_2-$ | H | 7-C≡CH | $-SCH_2CH_2COOH$ |
| 188 | 7-F-Q | $-OCH_2-$ | H | 7-C≡CH | $-SCH_2CH(CH_3)COOH$ |
| 189 | 7-F-Q | $-OCH_2-$ | H | 7-C≡CH | $-SCH_2C(CH_3)_2COOH$ |
| 190 | 7-F-Q | $-OCH_2-$ | H | 7-C≡CH | $-SCH_2C(CH_2CH_2)CH_2COOH$ |
| 191 | 7-F-Q | $-OCH_2-$ | H | 8-C≡CH | $-SCH_2CH_2COOH$ |
| 192 | 7-F-Q | $-OCH_2-$ | H | 8-C≡CH | $-SCH_2CH(CH_3)COOH$ |
| 193 | 7-F-Q | $-OCH_2-$ | H | 8-C≡CH | $-SCH_2C(CH_3)_2COOH$ |
| 194 | 7-F-Q | $-OCH_2-$ | H | 8-C≡CH | $-SCH_2C(CH_2CH_2)CH_2COOH$ |
| 195 | 7-F-Q | $-OCH_2-$ | H | 9-C≡CH | $-SCH_2CH_2COOH$ |
| 196 | 7-F-Q | $-OCH_2-$ | H | 9-C≡CH | $-SCH_2CH(CH_3)COOH$ |
| 197 | 7-F-Q | $-OCH_2-$ | H | 9-C≡CH | $-SCH_2C(CH_3)_2COOH$ |
| 198 | 7-F-Q | $-OCH_2-$ | H | 9-C≡CH | $-SCH_2C(CH_2CH_2)CH_2COOH$ |
| 199 | 7-F-Q | $-OCH_2-$ | H | $7-CF_3$ | $-SCH_2CH_2COOH$ |
| 200 | 7-F-Q | $-OCH_2-$ | H | $7-CF_3$ | $-SCH_2CH(CH_3)COOH$ |
| 201 | 7-F-Q | $-OCH_2-$ | H | $7-CF_3$ | $-SCH_2C(CH_3)_2COOH$ |
| 202 | 7-F-Q | $-OCH_2-$ | H | $7-CF_3$ | $-SCH_2C(CH_2CH_2)CH_2COOH$ |
| 203 | 7-F-Q | $-OCH_2-$ | H | $8-CF_3$ | $-SCH_2CH_2COOH$ |
| 204 | 7-F-Q | $-OCH_2-$ | H | $8-CF_3$ | $-SCH_2CH(CH_3)COOH$ |
| 205 | 7-F-Q | $-OCH_2-$ | H | $8-CF_3$ | $-SCH_2C(CH_3)_2COOH$ |
| 206 | 7-F-Q | $-OCH_2-$ | H | $8-CF_3$ | $-SCH_2C(CH_2CH_2)CH_2COOH$ |
| 207 | 7-F-Q | $-OCH_2-$ | H | $9-CF_3$ | $-SCH_2CH_2COOH$ |
| 208 | 7-F-Q | $-OCH_2-$ | H | $9-CF_3$ | $-SCH_2CH(CH_3)COOH$ |
| 209 | 7-F-Q | $-OCH_2-$ | H | $9-CF_3$ | $-SCH_2C(CH_3)_2COOH$ |
| 210 | 7-F-Q | $-OCH_2-$ | H | $9-CF_3$ | $-SCH_2C(CH_2CH_2)CH_2COOH$ |
| 211 | 7-F-Q | $-OCH_2-$ | H | $7-C(CH_3)_2O$ | $-SCH_2CH_2COOH$ |

| | | | | | |
|---|---|---|---|---|---|
| | | | | H | |
| 212 | 7-F-Q | $-OCH_2-$ | H | $7-C(CH_3)_2OH$ | $-SCH_2CH(CH_3)COOH$ |
| 213 | 7-F-Q | $-OCH_2-$ | H | $7-C(CH_3)_2OH$ | $-SCH_2C(CH_3)_2COOH$ |
| 214 | 7-F-Q | $-OCH_2-$ | H | $7-C(CH_3)_2OH$ | $-SCH_2C(CH_2CH_2)CH_2COOH$ |
| 215 | 7-F-Q | $-OCH_2-$ | H | $8-C(CH_3)_2OH$ | $-SCH_2CH_2COOH$ |
| 216 | 7-F-Q | $-OCH_2-$ | H | $8-C(CH_3)_2OH$ | $-SCH_2CH(CH_3)COOH$ |
| 217 | 7-F-Q | $-OCH_2-$ | H | $8-C(CH_3)_2OH$ | $-SCH_2C(CH_3)_2COOH$ |
| 218 | 7-F-Q | $-OCH_2-$ | H | $8-C(CH_3)_2OH$ | $-SCH_2C(CH_2CH_2)CH_2COOH$ |
| 219 | 7-F-Q | $-OCH_2-$ | H | $9-C(CH_3)_2OH$ | $-SCH_2CH_2COOH$ |
| 220 | 7-F-Q | $-OCH_2-$ | H | $9-C(CH_3)_2OH$ | $-SCH_2CH(CH_3)COOH$ |
| 221 | 7-F-Q | $-OCH_2-$ | H | $9-C(CH_3)_2OH$ | $-SCH_2C(CH_3)_2COOH$ |
| 222 | 7-F-Q | $-OCH_2-$ | H | $9-C(CH_3)_2OH$ | $-SCH_2C(CH_2CH_2)CH_2COOH$ |
| 223 | 7-Cl-Q | $-OCH_2-$ | H | H | $-SCH_2COOH$ |
| 224 | 7-Cl-Q | $-OCH_2-$ | H | H | $-SCH_2CH_2COOH$ |
| 225 | 7-Cl-Q | $-OCH_2-$ | H | H | $-SCH_2CH(CH_3)COOH$ |
| 226 | 7-Cl-Q | $-OCH_2-$ | H | H | $-SCH_2C(CH_3)_2COOH$ |
| 227 | 7-Cl-Q | $-OCH_2-$ | H | H | $-SCH_2C(CH_2CH_2)CH_2COOH$ |
| 228 | TQ | $-OCH_2-$ | H | H | $-SCH_2COOH$ |
| 229 | TQ | $-OCH_2-$ | H | H | $-SCH_2CH_2COOH$ |
| 230 | TQ | $-OCH_2-$ | H | H | $-SCH_2CH(CH_3)COOH$ |
| 231 | TQ | $-OCH_2-$ | H | H | $-SCH_2CH(CH_2CH_3)COOH$ |
| 232 | TQ | $-OCH_2-$ | H | H | $-SCH_2C(CH_3)_2COOH$ |
| 233 | TQ | $-OCH_2-$ | H | H | $-SC(CH_3)_2CH_2COOH$ |
| 234 | TQ | $-OCH_2-$ | H | H | $-SCH_2CH(CH_3)CH_2COOH$ |
| 235 | TQ | $-OCH_2-$ | H | H | $-SCH_2C(CH_2CH_2)CH_2COOH$ |
| 236 | TQ | $-OCH_2-$ | H | 7-F | $-SCH_2COOH$ |
| 237 | TQ | $-OCH_2-$ | H | 7-F | $-SCH_2CH_2COOH$ |
| 238 | TQ | $-OCH_2-$ | H | 7-F | $-SCH_2CH(CH_3)COOH$ |
| 239 | TQ | $-OCH_2-$ | H | 7-F | $-SCH_2CH(CH_2CH_3)COOH$ |
| 240 | TQ | $-OCH_2-$ | H | 7-F | $-SCH_2C(CH_3)_2COOH$ |
| 241 | TQ | $-OCH_2-$ | H | 7-F | $-SC(CH_3)_2CH_2COOH$ |
| 242 | TQ | $-OCH_2-$ | H | 7-F | $-SCH_2CH(CH_3)CH_2COOH$ |
| 243 | TQ | $-OCH_2-$ | H | 7-F | $-SCH_2C(CH_2CH_2)CH_2COOH$ |
| 244 | TQ | $-OCH_2-$ | H | 8-F | $-SCH_2COOH$ |
| 245 | TQ | $-OCH_2-$ | H | 8-F | $-SCH_2CH_2COOH$ |
| 246 | TQ | $-OCH_2-$ | H | 8-F | $-SCH_2CH(CH_3)COOH$ |
| 247 | TQ | $-OCH_2-$ | H | 8-F | $-SCH_2CH(CH_2CH_3)COOH$ |
| 248 | TQ | $-OCH_2-$ | H | 8-F | $-SCH_2C(CH_3)_2COOH$ |
| 249 | TQ | $-OCH_2-$ | H | 8-F | $-SC(CH_3)_2CH_2COOH$ |
| 250 | TQ | $-OCH_2-$ | H | 8-F | $-SCH_2CH(CH_3)CH_2COOH$ |
| 251 | TQ | $-OCH_2-$ | H | 8-F | $-SCH_2C(CH_2CH_2)CH_2COOH$ |
| 252 | TQ | $-OCH_2-$ | H | 9-F | $-SCH_2COOH$ |
| 253 | TQ | $-OCH_2-$ | H | 9-F | $-SCH_2CH_2COOH$ |
| 254 | TQ | $-OCH_2-$ | H | 9-F | $-SCH_2CH(CH_3)COOH$ |
| 255 | TQ | $-OCH_2-$ | H | 9-F | $-SCH_2CH(CH_2CH_3)COOH$ |

| | | | | | |
|---|---|---|---|---|---|
| 256 | TQ | $-OCH_2-$ | H | 9-F | $-SCH_2C(CH_3)_2COOH$ |
| 257 | TQ | $-OCH_2-$ | H | 9-F | $-SC(CH_3)_2CH_2COOH$ |
| 258 | TQ | $-OCH_2-$ | H | 9-F | $-SCH_2CH(CH_3)CH_2COOH$ |
| 259 | TQ | $-OCH_2-$ | H | 9-F | $-SCH_2C(CH_2CH_2)CH_2COOH$ |
| 260 | TQ | $-OCH_2-$ | H | 7-C≡CH | $-SCH_2COOH$ |
| 261 | TQ | $-OCH_2-$ | H | 7-C≡CH | $-SCH_2CH_2COOH$ |
| 262 | TQ | $-OCH_2-$ | H | 7-C≡CH | $-SCH_2CH(CH_3)COOH$ |
| 263 | TQ | $-OCH_2-$ | H | 7-C≡CH | $-SCH_2CH(CH_2CH_3)COOH$ |
| 264 | TQ | $-OCH_2-$ | H | 7-C≡CH | $-SCH_2C(CH_3)_2COOH$ |
| 265 | TQ | $-OCH_2-$ | H | 7-C≡CH | $-SC(CH_3)_2CH_2COOH$ |
| 266 | TQ | $-OCH_2-$ | H | 7-C≡CH | $-SCH_2CH(CH_3)CH_2COOH$ |
| 267 | TQ | $-OCH_2-$ | H | 7-C≡CH | $-SCH_2C(CH_2CH_2)CH_2COOH$ |
| 268 | TQ | $-OCH_2-$ | H | 8-C≡CH | $-SCH_2COOH$ |
| 269 | TQ | $-OCH_2-$ | H | 8-C≡CH | $-SCH_2CH_2COOH$ |
| 270 | TQ | $-OCH_2-$ | H | 8-C≡CH | $-SCH_2CH(CH_3)COOH$ |
| 271 | TQ | $-OCH_2-$ | H | 8-C≡CH | $-SCH_2CH(CH_2CH_3)COOH$ |
| 272 | TQ | $-OCH_2-$ | H | 8-C≡CH | $-SCH_2C(CH_3)_2COOH$ |
| 273 | TQ | $-OCH_2-$ | H | 8-C≡CH | $-SC(CH_3)_2CH_2COOH$ |
| 274 | TQ | $-OCH_2-$ | H | 8-C≡CH | $-SCH_2CH(CH_3)CH_2COOH$ |
| 275 | TQ | $-OCH_2-$ | H | 8-C≡CH | $-SCH_2C(CH_2CH_2)CH_2COOH$ |
| 276 | TQ | $-OCH_2-$ | H | 9-C≡CH | $-SCH_2COOH$ |
| 277 | TQ | $-OCH_2-$ | H | 9-C≡CH | $-SCH_2CH_2COOH$ |
| 278 | TQ | $-OCH_2-$ | H | 9-C≡CH | $-SCH_2CH(CH_3)COOH$ |
| 279 | TQ | $-OCH_2-$ | H | 9-C≡CH | $-SCH_2CH(CH_2CH_3)COOH$ |
| 280 | TQ | $-OCH_2-$ | H | 9-C≡CH | $-SCH_2C(CH_3)_2COOH$ |
| 281 | TQ | $-OCH_2-$ | H | 9-C≡CH | $-SC(CH_3)_2CH_2COOH$ |
| 282 | TQ | $-OCH_2-$ | H | 9-C≡CH | $-SCH_2CH(CH_3)CH_2COOH$ |
| 283 | TQ | $-OCH_2-$ | H | 9-C≡CH | $-SCH_2C(CH_2CH_2)CH_2COOH$ |
| 284 | TQ | $-OCH_2-$ | H | $7-CF_3$ | $-SCH_2COOH$ |
| 285 | TQ | $-OCH_2-$ | H | $7-CF_3$ | $-SCH_2CH_2COOH$ |
| 286 | TQ | $-OCH_2-$ | H | $7-CF_3$ | $-SCH_2CH(CH_3)COOH$ |
| 287 | TQ | $-OCH_2-$ | H | $7-CF_3$ | $-SCH_2CH(CH_2CH_3)COOH$ |
| 288 | TQ | $-OCH_2-$ | H | $7-CF_3$ | $-SCH_2C(CH_3)_2COOH$ |
| 289 | TQ | $-OCH_2-$ | H | $7-CF_3$ | $-SC(CH_3)_2CH_2COOH$ |
| 290 | TQ | $-OCH_2-$ | H | $7-CF_3$ | $-SCH_2CH(CH_3)CH_2COOH$ |
| 291 | TQ | $-OCH_2-$ | H | $7-CF_3$ | $-SCH_2C(CH_2CH_2)CH_2COOH$ |
| 292 | TQ | $-OCH_2-$ | H | $8-CF_3$ | $-SCH_2COOH$ |
| 293 | TQ | $-OCH_2-$ | H | $8-CF_3$ | $-SCH_2CH_2COOH$ |
| 294 | TQ | $-OCH_2-$ | H | $8-CF_3$ | $-SCH_2CH(CH_3)COOH$ |
| 295 | TQ | $-OCH_2-$ | H | $8-CF_3$ | $-SCH_2CH(CH_2CH_3)COOH$ |
| 296 | TQ | $-OCH_2-$ | H | $8-CF_3$ | $-SCH_2C(CH_3)_2COOH$ |
| 297 | TQ | $-OCH_2-$ | H | $8-CF_3$ | $-SC(CH_3)_2CH_2COOH$ |
| 298 | TQ | $-OCH_2-$ | H | $8-CF_3$ | $-SCH_2CH(CH_3)CH_2COOH$ |

| | | | | | |
|---|---|---|---|---|---|
| 299 | TQ | $-OCH_2-$ | H | $8-CF_3$ | $-SCH_2C(CH_2CH_2)CH_2COOH$ |
| 300 | TQ | $-OCH_2-$ | H | $9-CF_3$ | $-SCH_2COOH$ |
| 301 | TQ | $-OCH_2-$ | H | $9-CF_3$ | $-SCH_2CH_2COOH$ |
| 302 | TQ | $-OCH_2-$ | H | $9-CF_3$ | $-SCH_2CH(CH_3)COOH$ |
| 303 | TQ | $-OCH_2-$ | H | $9-CF_3$ | $-SCH_2CH(CH_2CH_3)COOH$ |
| 304 | TQ | $-OCH_2-$ | H | $9-CF_3$ | $-SCH_2C(CH_3)_2COOH$ |
| 305 | TQ | $-OCH_2-$ | H | $9-CF_3$ | $-SC(CH_3)_2CH_2COOH$ |
| 306 | TQ | $-OCH_2-$ | H | $9-CF_3$ | $-SCH_2CH(CH_3)CH_2COOH$ |
| 307 | TQ | $-OCH_2-$ | H | $9-CF_3$ | $-SCH_2C(CH_2CH_2)CH_2COOH$ |
| 308 | TQ | $-OCH_2-$ | H | $7-C(CH_3)_2OH$ | $-SCH_2COOH$ |
| 309 | TQ | $-OCH_2-$ | H | $7-C(CH_3)_2OH$ | $-SCH_2CH_2COOH$ |
| 310 | TQ | $-OCH_2-$ | H | $7-C(CH_3)_2OH$ | $-SCH_2CH(CH_3)COOH$ |
| 311 | TQ | $-OCH_2-$ | H | $7-C(CH_3)_2OH$ | $-SCH_2CH(CH_2CH_3)COOH$ |
| 312 | TQ | $-OCH_2-$ | H | $7-C(CH_3)_2OH$ | $-SCH_2C(CH_3)_2COOH$ |
| 313 | TQ | $-OCH_2-$ | H | $7-C(CH_3)_2OH$ | $-SC(CH_3)_2CH_2COOH$ |
| 314 | TQ | $-OCH_2-$ | H | $7-C(CH_3)_2OH$ | $-SCH_2CH(CH_3)CH_2COOH$ |
| 315 | TQ | $-OCH_2-$ | H | $7-C(CH_3)_2OH$ | $-SCH_2C(CH_2CH_2)CH_2COOH$ |
| 316 | TQ | $-OCH_2-$ | H | $8-C(CH_3)_2OH$ | $-SCH_2COOH$ |
| 317 | TQ | $-OCH_2-$ | H | $8-C(CH_3)_2OH$ | $-SCH_2CH_2COOH$ |
| 318 | TQ | $-OCH_2-$ | H | $8-C(CH_3)_2OH$ | $-SCH_2CH(CH_3)COOH$ |
| 319 | TQ | $-OCH_2-$ | H | $8-C(CH_3)_2OH$ | $-SCH_2CH(CH_2CH_3)COOH$ |
| 320 | TQ | $-OCH_2-$ | H | $8-C(CH_3)_2OH$ | $-SCH_2C(CH_3)_2COOH$ |
| 321 | TQ | $-OCH_2-$ | H | $8-C(CH_3)_2OH$ | $-SC(CH_3)_2CH_2COOH$ |
| 322 | TQ | $-OCH_2-$ | H | $8-C(CH_3)_2OH$ | $-SCH_2CH(CH_3)CH_2COOH$ |
| 323 | TQ | $-OCH_2-$ | H | $8-C(CH_3)_2OH$ | $-SCH_2C(CH_2CH_2)CH_2COOH$ |
| 324 | TQ | $-OCH_2-$ | H | $9-C(CH_3)_2OH$ | $-SCH_2COOH$ |
| 325 | TQ | $-OCH_2-$ | H | $9-C(CH_3)_2OH$ | $-SCH_2CH_2COOH$ |
| 326 | TQ | $-OCH_2-$ | H | $9-C(CH_3)_2OH$ | $-SCH_2CH(CH_3)COOH$ |
| 327 | TQ | $-OCH_2-$ | H | $9-C(CH_3)_2OH$ | $-SCH_2CH(CH_2CH_3)COOH$ |
| 328 | TQ | $-OCH_2-$ | H | $9-C(CH_3)_2OH$ | $-SCH_2C(CH_3)_2COOH$ |
| 329 | TQ | $-OCH_2-$ | H | $9-C(CH_3)_2OH$ | $-SC(CH_3)_2CH_2COOH$ |
| 330 | TQ | $-OCH_2-$ | H | $9-C(CH_3)_2OH$ | $-SCH_2CH(CH_3)CH_2COOH$ |
| 331 | TQ | $-OCH_2-$ | H | $9-C(CH_3)_2OH$ | $-SCH_2C(CH_2CH_2)CH_2COOH$ |
| 332 | Q | $-OCH_2-$ | H | H | $-SCH_2CH_2COOH$ |
| 333 | Q | $-OCH_2-$ | H | H | $-SCH_2CH(CH_3)COOH$ |
| 334 | Q | $-OCH_2-$ | H | H | $-SCH_2C(CH_3)_2COOH$ |
| 335 | Q | $-OCH_2-$ | H | H | $-SCH_2C(CH_2CH_2)CH_2COOH$ |
| 336 | 4-Cl-Q | $-OCH_2-$ | H | H | $-SCH_2CH_2COOH$ |
| 337 | 4-Cl-Q | $-OCH_2-$ | H | H | $-SCH_2CH(CH_3)COOH$ |
| 338 | 4-Cl-Q | $-OCH_2-$ | H | H | $-SCH_2C(CH_3)_2COOH$ |
| 339 | 4-Cl-Q | $-OCH_2-$ | H | H | $-SCH_2C(CH_2CH_2)CH_2COOH$ |
| 340 | 5-F-Q | $-OCH_2-$ | H | H | $-SCH_2CH_2COOH$ |
| 341 | 5-F-Q | $-OCH_2-$ | H | H | $-SCH_2CH(CH_3)COOH$ |
| 342 | 5-F-Q | $-OCH_2-$ | H | H | $-SCH_2C(CH_3)_2COOH$ |
| 343 | 5-F-Q | $-OCH_2-$ | H | H | $-SCH_2C(CH_2CH_2)CH_2COOH$ |

| | | | | | |
|---|---|---|---|---|---|
| 344 | 7-$CF_3$-Q | -$OCH_2$- | H | H | -$SCH_2CH_2COOH$ |
| 345 | 7-$CF_3$-Q | -$OCH_2$- | H | H | -$SCH_2CH(CH_3)COOH$ |
| 346 | 7-$CF_3$-Q | -$OCH_2$- | H | H | -$SCH_2C(CH_3)_2COOH$ |
| 347 | 7-$CF_3$-Q | -$OCH_2$- | H | H | -$SCH_2C(CH_2CH_2)CH_2COOH$ |
| 348 | 5, 7-diF-Q | -$OCH_2$- | H | H | -$SCH_2CH_2COOH$ |
| 349 | 5, 7-diF-Q | -$OCH_2$- | H | H | -$SCH_2CH(CH_3)COOH$ |
| 350 | 5, 7-diF-Q | -$OCH_2$- | H | H | -$SCH_2C(CH_3)_2COOH$ |
| 351 | 5, 7-diF-Q | -$OCH_2$- | H | H | -$SCH_2C(CH_2CH_2)CH_2COOH$ |
| 352 | 6-F, 7-$CF_3$-Q | -$OCH_2$- | H | H | -$SCH_2CH_2COOH$ |
| 353 | 6-F, 7-$CF_3$-Q | -$OCH_2$- | H | H | -$SCH_2CH(CH_3)COOH$ |
| 354 | 6-F, 7-$CF_3$-Q | -$OCH_2$- | H | H | -$SCH_2C(CH_3)_2COOH$ |
| 355 | 6-F, 7-$CF_3$-Q | -$OCH_2$- | H | H | -$SCH_2C(CH_2CH_2)CH_2COOH$ |
| 356 | 5, 6, 7-triF-Q | -$OCH_2$- | H | H | -$SCH_2CH_2COOH$ |
| 357 | 5, 6, 7-triF-Q | -$OCH_2$- | H | H | -$SCH_2CH(CH_3)COOH$ |
| 358 | 5, 6, 7-triF-Q | -$OCH_2$- | H | H | -$SCH_2C(CH_3)_2COOH$ |
| 359 | 5, 6, 7-triF-Q | -$OCH_2$- | H | H | -$SCH_2C(CH_2CH_2)CH_2COOH$ |
| 360 | 4-t-Bu-T | -$OCH_2$- | H | H | -$SCH_2CH_2COOH$ |
| 361 | 4-t-Bu-T | -$OCH_2$- | H | H | -$SCH_2CH(CH_3)COOH$ |
| 362 | 4-t-Bu-T | -$OCH_2$- | H | H | -$SCH_2C(CH_3)_2COOH$ |
| 363 | 4-t-Bu-T | -$OCH_2$- | H | H | -$SCH_2C(CH_2CH_2)CH_2COOH$ |
| 364 | 5-F-BT | -$OCH_2$- | H | H | -$SCH_2CH_2COOH$ |
| 365 | 5-F-BT | -$OCH_2$- | H | H | -$SCH_2CH(CH_3)COOH$ |
| 366 | 5-F-BT | -$OCH_2$- | H | H | -$SCH_2C(CH_3)_2COOH$ |
| 367 | 5-F-BT | -$OCH_2$- | H | H | -$SCH_2C(CH_2CH_2)CH_2COOH$ |
| 368 | 5, 6-diF-BT | -$OCH_2$- | H | H | -$SCH_2CH_2COOH$ |
| 369 | 5, 6-diF-BT | -$OCH_2$- | H | H | -$SCH_2CH(CH_3)COOH$ |
| 370 | 5, 6-diF-BT | -$OCH_2$- | H | H | -$SCH_2C(CH_3)_2COOH$ |
| 371 | 5, 6-diF-BT | -$OCH_2$- | H | H | -$SCH_2C(CH_2CH_2)CH_2COOH$ |
| 372 | 5-F-BO | -$OCH_2$- | H | H | -$SCH_2CH_2COOH$ |
| 373 | 5-F-BO | -$OCH_2$- | H | H | -$SCH_2CH(CH_3)COOH$ |
| 374 | 5-F-BO | -$OCH_2$- | H | H | -$SCH_2C(CH_3)_2COOH$ |
| 375 | 5-F-BO | -$OCH_2$- | H | H | -$SCH_2C(CH_2CH_2)CH_2COOH$ |
| 376 | 6-t-Bu-Py | -$OCH_2$- | H | H | -$SCH_2CH_2COOH$ |
| 377 | 6-t-Bu-Py | -$OCH_2$- | H | H | -$SCH_2CH(CH_3)COOH$ |
| 378 | 6-t-Bu-Py | -$OCH_2$- | H | H | -$SCH_2C(CH_3)_2COOH$ |
| 379 | 6-t-Bu-Py | -$OCH_2$- | H | H | -$SCH_2C(CH_2CH_2)CH_2COOH$ |
| 380 | 5-Me, 6-i-Pr-Py | -$OCH_2$- | H | H | -$SCH_2CH_2COOH$ |
| 381 | 5-Me, 6-i-Pr-Py | -$OCH_2$- | H | H | -$SCH_2CH(CH_3)COOH$ |
| 382 | 5-Me, 6-i-Pr-Py | -$OCH_2$- | H | H | -$SCH_2C(CH_3)_2COOH$ |
| 383 | 5-Me, 6-i-Pr-Py | -$OCH_2$- | H | H | -$SCH_2C(CH_2CH_2)CH_2COOH$ |
| 384 | 6, 7-diF-Q | -$CH_2CH_2$- | H | H | -$SCH_2CH_2COOH$ |
| 385 | 6, 7-diF-Q | -$CH_2CH_2$- | H | H | -$SCH_2CH(CH_3)COOH$ |
| 386 | 6, 7-diF-Q | -$CH_2CH_2$- | H | H | -$SCH_2C(CH_3)_2COOH$ |
| 387 | 6, 7-diF-Q | -$CH_2CH_2$- | H | H | -$SCH_2C(CH_2CH_2)CH_2COOH$ |
| 388 | 6, 7-diF-Q | -$CH_2CH_2$- | H | H | -$SCH_2CH_2COOH$ |

| | | | | | |
|---|---|---|---|---|---|
| 389 | 6-F, 7-Cl-Q | $-CH_2CH_2-$ | H | H | $-SCH_2CH(CH_3)COOH$ |
| 390 | 6-F, 7-Cl-Q | $-CH_2CH_2-$ | H | H | $-SCH_2C(CH_3)_2COOH$ |
| 391 | 6-F, 7-Cl-Q | $-CH_2CH_2-$ | H | H | $-SCH_2C(CH_2CH_2)CH_2COOH$ |
| 392 | 7-F-Q | $-CH_2CH_2-$ | H | H | $-SCH_2CH_2COOH$ |
| 393 | 7-F-Q | $-CH_2CH_2-$ | H | H | $-SCH_2CH(CH_3)COOH$ |
| 394 | 7-F-Q | $-CH_2CH_2-$ | H | H | $-SCH_2C(CH_3)_2COOH$ |
| 395 | 7-F-Q | $-CH_2CH_2-$ | H | H | $-SCH_2C(CH_2CH_2)CH_2COOH$ |
| 396 | TQ | $-CH_2CH_2-$ | H | H | $-SCH_2CH_2COOH$ |
| 397 | TQ | $-CH_2CH_2-$ | H | H | $-SCH_2CH(CH_3)COOH$ |
| 398 | TQ | $-CH_2CH_2-$ | H | H | $-SCH_2C(CH_3)_2COOH$ |
| 399 | TQ | $-CH_2CH_2-$ | H | H | $-SCH_2C(CH_2CH_2)CH_2COOH$ |
| 400 | 6, 7-diF-Q | $-SCH_2-$ | H | H | $-SCH_2CH_2COOH$ |
| 401 | 6, 7-diF-Q | $-SCH_2-$ | H | H | $-SCH_2CH(CH_3)COOH$ |
| 402 | 6, 7-diF-Q | $-SCH_2-$ | H | H | $-SCH_2C(CH_3)_2COOH$ |
| 403 | 6, 7-diF-Q | $-SCH_2-$ | H | H | $-SCH_2C(CH_2CH_2)CH_2COOH$ |
| 404 | 6, 7-diF-Q | $-CH_2O-$ | H | H | $-SCH_2CH_2COOH$ |
| 405 | 6, 7-diF-Q | $-CH_2O-$ | H | H | $-SCH_2CH(CH_3)COOH$ |
| 406 | 6, 7-diF-Q | $-CH_2O-$ | H | H | $-SCH_2C(CH_3)_2COOH$ |
| 407 | 6, 7-diF-Q | $-CH_2O-$ | H | H | $-SCH_2C(CH_2CH_2)CH_2COOH$ |
| 408 | 6, 7-diF-Q | $-CH_2S-$ | H | H | $-SCH_2CH_2COOH$ |
| 409 | 6, 7-diF-Q | $-CH_2S-$ | H | H | $-SCH_2CH(CH_3)COOH$ |
| 410 | 6, 7-diF-Q | $-CH_2S-$ | H | H | $-SCH_2C(CH_3)_2COOH$ |
| 411 | 6, 7-diF-Q | $-CH_2S-$ | H | H | $-SCH_2C(CH_2CH_2)CH_2COOH$ |
| 412 | 6, 7-diF-Q | $-OCH_2-$ | H | 7-$CH_2OH$ | $-SCH_2COOH$ |
| 413 | 6, 7-diF-Q | $-OCH_2-$ | H | 7-$CH_2OH$ | $-SCH_2CH_2COOH$ |
| 414 | 6, 7-diF-Q | $-OCH_2-$ | H | 7-$CH_2OH$ | $-SCH_2CH(CH_3)COOH$ |
| 415 | 6, 7-diF-Q | $-OCH_2-$ | H | 7-$CH_2OH$ | $-SCH_2CH(CH_2CH_3)COOH$ |
| 416 | 6, 7-diF-Q | $-OCH_2-$ | H | 7-$CH_2OH$ | $-SCH_2C(CH_3)_2COOH$ |
| 417 | 6, 7-diF-Q | $-OCH_2-$ | H | 7-$CH_2OH$ | $-SC(CH_3)_2CH_2COOH$ |
| 418 | 6, 7-diF-Q | $-OCH_2-$ | H | 7-$CH_2OH$ | $-SCH_2CH(CH_3)CH_2COOH$ |
| 419 | 6, 7-diF-Q | $-OCH_2-$ | H | 7-$CH_2OH$ | $-SCH_2C(CH_2CH_2)CH_2COOH$ |
| 420 | 6, 7-diF-Q | $-OCH_2-$ | H | 8-$CH_2OH$ | $-SCH_2COOH$ |
| 421 | 6, 7-diF-Q | $-OCH_2-$ | H | 8-$CH_2OH$ | $-SCH_2CH_2COOH$ |
| 422 | 6, 7-diF-Q | $-OCH_2-$ | H | 8-$CH_2OH$ | $-SCH_2CH(CH_3)COOH$ |
| 423 | 6, 7-diF-Q | $-OCH_2-$ | H | 8-$CH_2OH$ | $-SCH_2CH(CH_2CH_3)COOH$ |
| 424 | 6, 7-diF-Q | $-OCH_2-$ | H | 8-$CH_2OH$ | $-SCH_2C(CH_3)_2COOH$ |
| 425 | 6, 7-diF-Q | $-OCH_2-$ | H | 8-$CH_2OH$ | $-SC(CH_3)_2CH_2COOH$ |
| 426 | 6, 7-diF-Q | $-OCH_2-$ | H | 8-$CH_2OH$ | $-SCH_2CH(CH_3)CH_2COOH$ |
| 427 | 6, 7-diF-Q | $-OCH_2-$ | H | 8-$CH_2OH$ | $-SCH_2C(CH_2CH_2)CH_2COOH$ |
| 428 | 6, 7-diF-Q | $-OCH_2-$ | H | 9-$CH_2OH$ | $-SCH_2COOH$ |
| 429 | 6, 7-diF-Q | $-OCH_2-$ | H | 9-$CH_2OH$ | $-SCH_2CH_2COOH$ |
| 430 | 6, 7-diF-Q | $-OCH_2-$ | H | 9-$CH_2OH$ | $-SCH_2CH(CH_3)COOH$ |
| 431 | 6, 7-diF-Q | $-OCH_2-$ | H | 9-$CH_2OH$ | $-SCH_2CH(CH_2CH_3)COOH$ |
| 432 | 6, 7-diF-Q | $-OCH_2-$ | H | 9-$CH_2OH$ | $-SCH_2C(CH_3)_2COOH$ |
| 433 | 6, 7-diF-Q | $-OCH_2-$ | H | 9-$CH_2OH$ | $-SC(CH_3)_2CH_2COOH$ |

| | | | | | |
|---|---|---|---|---|---|
| 434 | 6, 7-diF-Q | $-OCH_2-$ | H | 9-$CH_2OH$ | $-SCH_2CH(CH_3)CH_2COOH$ |
| 435 | 6, 7-diF-Q | $-OCH_2-$ | H | 9-$CH_2OH$ | $-SCH_2C(CH_2CH_2)CH_2COOH$ |
| 436 | 6, 7-diF-Q | $-OCH_2-$ | H | 7-$CH=CH_2$ | $-SCH_2COOH$ |
| 437 | 6, 7-diF-Q | $-OCH_2-$ | H | 7-$CH=CH_2$ | $-SCH_2CH_2COOH$ |
| 438 | 6, 7-diF-Q | $-OCH_2-$ | H | 7-$CH=CH_2$ | $-SCH_2CH(CH_3)COOH$ |
| 439 | 6, 7-diF-Q | $-OCH_2-$ | H | 7-$CH=CH_2$ | $-SCH_2CH(CH_2CH_3)COOH$ |
| 440 | 6, 7-diF-Q | $-OCH_2-$ | H | 7-$CH=CH_2$ | $-SCH_2C(CH_3)_2COOH$ |
| 441 | 6, 7-diF-Q | $-OCH_2-$ | H | 7-$CH=CH_2$ | $-SC(CH_3)_2CH_2COOH$ |
| 442 | 6, 7-diF-Q | $-OCH_2-$ | H | 7-$CH=CH_2$ | $-SCH_2CH(CH_3)CH_2COOH$ |
| 443 | 6, 7-diF-Q | $-OCH_2-$ | H | 7-$CH=CH_2$ | $-SCH_2C(CH_2CH_2)CH_2COOH$ |
| 444 | 6, 7-diF-Q | $-OCH_2-$ | H | 8-$CH=CH_2$ | $-SCH_2COOH$ |
| 445 | 6, 7-diF-Q | $-OCH_2-$ | H | 8-$CH=CH_2$ | $-SCH_2CH_2COOH$ |
| 446 | 6, 7-diF-Q | $-OCH_2-$ | H | 8-$CH=CH_2$ | $-SCH_2CH(CH_3)COOH$ |
| 447 | 6, 7-diF-Q | $-OCH_2-$ | H | 8-$CH=CH_2$ | $-SCH_2CH(CH_2CH_3)COOH$ |
| 448 | 6, 7-diF-Q | $-OCH_2-$ | H | 8-$CH=CH_2$ | $-SCH_2C(CH_3)_2COOH$ |
| 449 | 6, 7-diF-Q | $-OCH_2-$ | H | 8-$CH=CH_2$ | $-SC(CH_3)_2CH_2COOH$ |
| 450 | 6, 7-diF-Q | $-OCH_2-$ | H | 8-$CH=CH_2$ | $-SCH_2CH(CH_3)CH_2COOH$ |
| 451 | 6, 7-diF-Q | $-OCH_2-$ | H | 8-$CH=CH_2$ | $-SCH_2C(CH_2CH_2)CH_2COOH$ |
| 452 | 6, 7-diF-Q | $-OCH_2-$ | H | 9-$CH=CH_2$ | $-SCH_2COOH$ |
| 453 | 6, 7-diF-Q | $-OCH_2-$ | H | 9-$CH=CH_2$ | $-SCH_2CH_2COOH$ |
| 454 | 6, 7-diF-Q | $-OCH_2-$ | H | 9-$CH=CH_2$ | $-SCH_2CH(CH_3)COOH$ |
| 455 | 6, 7-diF-Q | $-OCH_2-$ | H | 9-$CH=CH_2$ | $-SCH_2CH(CH_2CH_3)COOH$ |
| 456 | 6, 7-diF-Q | $-OCH_2-$ | H | 9-$CH=CH_2$ | $-SCH_2C(CH_3)_2COOH$ |
| 457 | 6, 7-diF-Q | $-OCH_2-$ | H | 9-$CH=CH_2$ | $-SC(CH_3)_2CH_2COOH$ |
| 458 | 6, 7-diF-Q | $-OCH_2-$ | H | 9-$CH=CH_2$ | $-SCH_2CH(CH_3)CH_2COOH$ |
| 459 | 6, 7-diF-Q | $-OCH_2-$ | H | 9-$CH=CH_2$ | $-SCH_2C(CH_2CH_2)CH_2COOH$ |
| 460 | 6, 7-diF-Q | $-OCH_2-$ | H | 7-$OCH_3$ | $-SCH_2COOH$ |
| 461 | 6, 7-diF-Q | $-OCH_2-$ | H | 7-$OCH_3$ | $-SCH_2CH_2COOH$ |
| 462 | 6, 7-diF-Q | $-OCH_2-$ | H | 7-$OCH_3$ | $-SCH_2CH(CH_3)COOH$ |
| 463 | 6, 7-diF-Q | $-OCH_2-$ | H | 7-$OCH_3$ | $-SCH_2CH(CH_2CH_3)COOH$ |
| 464 | 6, 7-diF-Q | $-OCH_2-$ | H | 7-$OCH_3$ | $-SCH_2C(CH_3)_2COOH$ |
| 465 | 6, 7-diF-Q | $-OCH_2-$ | H | 7-$OCH_3$ | $-SC(CH_3)_2CH_2COOH$ |
| 466 | 6, 7-diF-Q | $-OCH_2-$ | H | 7-$OCH_3$ | $-SCH_2CH(CH_3)CH_2COOH$ |
| 467 | 6, 7-diF-Q | $-OCH_2-$ | H | 7-$OCH_3$ | $-SCH_2C(CH_2CH_2)CH_2COOH$ |
| 468 | 6, 7-diF-Q | $-OCH_2-$ | H | 8-$OCH_3$ | $-SCH_2COOH$ |
| 469 | 6, 7-diF-Q | $-OCH_2-$ | H | 8-$OCH_3$ | $-SCH_2CH_2COOH$ |
| 470 | 6, 7-diF-Q | $-OCH_2-$ | H | 8-$OCH_3$ | $-SCH_2CH(CH_3)COOH$ |
| 471 | 6, 7-diF-Q | $-OCH_2-$ | H | 8-$OCH_3$ | $-SCH_2CH(CH_2CH_3)COOH$ |
| 472 | 6, 7-diF-Q | $-OCH_2-$ | H | 8-$OCH_3$ | $-SCH_2C(CH_3)_2COOH$ |
| 473 | 6, 7-diF-Q | $-OCH_2-$ | H | 8-$OCH_3$ | $-SC(CH_3)_2CH_2COOH$ |
| 474 | 6, 7-diF-Q | $-OCH_2-$ | H | 8-$OCH_3$ | $-SCH_2CH(CH_3)CH_2COOH$ |
| 475 | 6, 7-diF-Q | $-OCH_2-$ | H | 8-$OCH_3$ | $-SCH_2C(CH_2CH_2)CH_2COOH$ |
| 476 | 6, 7-diF-Q | $-OCH_2-$ | H | 9-$OCH_3$ | $-SCH_2COOH$ |
| 477 | 6, 7-diF-Q | $-OCH_2-$ | H | 9-$OCH_3$ | $-SCH_2CH_2COOH$ |
| 478 | 6, 7-diF-Q | $-OCH_2-$ | H | 9-$OCH_3$ | $-SCH_2CH(CH_3)COOH$ |

| | | | | | |
|---|---|---|---|---|---|
| 479 | 6, 7-diF-Q | $-OCH_2-$ | H | $9-OCH_3$ | $-SCH_2CH(CH_2CH_3)COOH$ |
| 480 | 6, 7-diF-Q | $-OCH_2-$ | H | $9-OCH_3$ | $-SCH_2C(CH_3)_2COOH$ |
| 481 | 6, 7-diF-Q | $-OCH_2-$ | H | $9-OCH_3$ | $-SC(CH_3)_2CH_2COOH$ |
| 482 | 6, 7-diF-Q | $-OCH_2-$ | H | $9-OCH_3$ | $-SCH_2CH(CH_3)CH_2COOH$ |
| 483 | 6, 7-diF-Q | $-OCH_2-$ | H | $9-OCH_3$ | $-SCH_2C(CH_2CH_2)CH_2COOH$ |
| 484 | 6, 7-diF-Q | $-OCH_2-$ | H | $7-OCHF_2$ | $-SCH_2COOH$ |
| 485 | 6, 7-diF-Q | $-OCH_2-$ | H | $7-OCHF_2$ | $-SCH_2CH_2COOH$ |
| 486 | 6, 7-diF-Q | $-OCH_2-$ | H | $7-OCHF_2$ | $-SCH_2CH(CH_3)COOH$ |
| 487 | 6, 7-diF-Q | $-OCH_2-$ | H | $7-OCHF_2$ | $-SCH_2CH(CH_2CH_3)COOH$ |
| 488 | 6, 7-diF-Q | $-OCH_2-$ | H | $7-OCHF_2$ | $-SCH_2C(CH_3)_2COOH$ |
| 489 | 6, 7-diF-Q | $-OCH_2-$ | H | $7-OCHF_2$ | $-SC(CH_3)_2CH_2COOH$ |
| 490 | 6, 7-diF-Q | $-OCH_2-$ | H | $7-OCHF_2$ | $-SCH_2CH(CH_3)CH_2COOH$ |
| 491 | 6, 7-diF-Q | $-OCH_2-$ | H | $7-OCHF_2$ | $-SCH_2C(CH_2CH_2)CH_2COOH$ |
| 492 | 6, 7-diF-Q | $-OCH_2-$ | H | $8-OCHF_2$ | $-SCH_2COOH$ |
| 493 | 6, 7-diF-Q | $-OCH_2-$ | H | $8-OCHF_2$ | $-SCH_2CH_2COOH$ |
| 494 | 6, 7-diF-Q | $-OCH_2-$ | H | $8-OCHF_2$ | $-SCH_2CH(CH_3)COOH$ |
| 495 | 6, 7-diF-Q | $-OCH_2-$ | H | $8-OCHF_2$ | $-SCH_2CH(CH_2CH_3)COOH$ |
| 496 | 6, 7-diF-Q | $-OCH_2-$ | H | $8-OCHF_2$ | $-SCH_2C(CH_3)_2COOH$ |
| 497 | 6, 7-diF-Q | $-OCH_2-$ | H | $8-OCHF_2$ | $-SC(CH_3)_2CH_2COOH$ |
| 498 | 6, 7-diF-Q | $-OCH_2-$ | H | $8-OCHF_2$ | $-SCH_2CH(CH_3)CH_2COOH$ |
| 499 | 6, 7-diF-Q | $-OCH_2-$ | H | $8-OCHF_2$ | $-SCH_2C(CH_2CH_2)CH_2COOH$ |
| 500 | 6, 7-diF-Q | $-OCH_2-$ | H | $9-OCHF_2$ | $-SCH_2COOH$ |
| 501 | 6, 7-diF-Q | $-OCH_2-$ | H | $9-OCHF_2$ | $-SCH_2CH_2COOH$ |
| 502 | 6, 7-diF-Q | $-OCH_2-$ | H | $9-OCHF_2$ | $-SCH_2CH(CH_3)COOH$ |
| 503 | 6, 7-diF-Q | $-OCH_2-$ | H | $9-OCHF_2$ | $-SCH_2CH(CH_2CH_3)COOH$ |
| 504 | 6, 7-diF-Q | $-OCH_2-$ | H | $9-OCHF_2$ | $-SCH_2C(CH_3)_2COOH$ |
| 505 | 6, 7-diF-Q | $-OCH_2-$ | H | $9-OCHF_2$ | $-SC(CH_3)_2CH_2COOH$ |
| 506 | 6, 7-diF-Q | $-OCH_2-$ | H | $9-OCHF_2$ | $-SCH_2CH(CH_3)CH_2COOH$ |
| 507 | 6, 7-diF-Q | $-OCH_2-$ | H | $9-OCHF_2$ | $-SCH_2C(CH_2CH_2)CH_2COOH$ |
| 508 | 6, 7-diF-Q | $-OCH_2-$ | H | $7-OCF_3$ | $-SCH_2COOH$ |
| 509 | 6, 7-diF-Q | $-OCH_2-$ | H | $7-OCF_3$ | $-SCH_2CH_2COOH$ |
| 510 | 6, 7-diF-Q | $-OCH_2-$ | H | $7-OCF_3$ | $-SCH_2CH(CH_3)COOH$ |
| 511 | 6, 7-diF-Q | $-OCH_2-$ | H | $7-OCF_3$ | $-SCH_2CH(CH_2CH_3)COOH$ |
| 512 | 6, 7-diF-Q | $-OCH_2-$ | H | $7-OCF_3$ | $-SCH_2C(CH_3)_2COOH$ |
| 513 | 6, 7-diF-Q | $-OCH_2-$ | H | $7-OCF_3$ | $-SC(CH_3)_2CH_2COOH$ |
| 514 | 6, 7-diF-Q | $-OCH_2-$ | H | $7-OCF_3$ | $-SCH_2CH(CH_3)CH_2COOH$ |
| 515 | 6, 7-diF-Q | $-OCH_2-$ | H | $7-OCF_3$ | $-SCH_2C(CH_2CH_2)CH_2COOH$ |
| 516 | 6, 7-diF-Q | $-OCH_2-$ | H | $8-OCF_3$ | $-SCH_2COOH$ |
| 517 | 6, 7-diF-Q | $-OCH_2-$ | H | $8-OCF_3$ | $-SCH_2CH_2COOH$ |
| 518 | 6, 7-diF-Q | $-OCH_2-$ | H | $8-OCF_3$ | $-SCH_2CH(CH_3)COOH$ |
| 519 | 6, 7-diF-Q | $-OCH_2-$ | H | $8-OCF_3$ | $-SCH_2CH(CH_2CH_3)COOH$ |
| 520 | 6, 7-diF-Q | $-OCH_2-$ | H | $8-OCF_3$ | $-SCH_2C(CH_3)_2COOH$ |
| 521 | 6, 7-diF-Q | $-OCH_2-$ | H | $8-OCF_3$ | $-SC(CH_3)_2CH_2COOH$ |
| 522 | 6, 7-diF-Q | $-OCH_2-$ | H | $8-OCF_3$ | $-SCH_2CH(CH_3)CH_2COOH$ |
| 523 | 6, 7-diF-Q | $-OCH_2-$ | H | $8-OCF_3$ | $-SCH_2C(CH_2CH_2)CH_2COOH$ |

| | | | | | |
|---|---|---|---|---|---|
| 524 | 6, 7-diF-Q | $-OCH_2-$ | H | $9-OCF_3$ | $-SCH_2COOH$ |
| 525 | 6, 7-diF-Q | $-OCH_2-$ | H | $9-OCF_3$ | $-SCH_2CH_2COOH$ |
| 526 | 6, 7-diF-Q | $-OCH_2-$ | H | $9-OCF_3$ | $-SCH_2CH(CH_3)COOH$ |
| 527 | 6, 7-diF-Q | $-OCH_2-$ | H | $9-OCF_3$ | $-SCH_2CH(CH_2CH_3)COOH$ |
| 528 | 6, 7-diF-Q | $-OCH_2-$ | H | $9-OCF_3$ | $-SCH_2C(CH_3)_2COOH$ |
| 529 | 6, 7-diF-Q | $-OCH_2-$ | H | $9-OCF_3$ | $-SC(CH_3)_2CH_2COOH$ |
| 530 | 6, 7-diF-Q | $-OCH_2-$ | H | $9-OCF_3$ | $-SCH_2CH(CH_3)CH_2COOH$ |
| 531 | 6, 7-diF-Q | $-OCH_2-$ | H | $9-OCF_3$ | $-SCH_2C(CH_2CH_2)CH_2COOH$ |
| 532 | 6, 7-diF-Q | $-OCH_2-$ | H | $7-SOCH_3$ | $-SCH_2COOH$ |
| 533 | 6, 7-diF-Q | $-OCH_2-$ | H | $7-SOCH_3$ | $-SCH_2CH_2COOH$ |
| 534 | 6, 7-diF-Q | $-OCH_2-$ | H | $7-SOCH_3$ | $-SCH_2CH(CH_3)COOH$ |
| 535 | 6, 7-diF-Q | $-OCH_2-$ | H | $7-SOCH_3$ | $-SCH_2CH(CH_2CH_3)COOH$ |
| 536 | 6, 7-diF-Q | $-OCH_2-$ | H | $7-SOCH_3$ | $-SCH_2C(CH_3)_2COOH$ |
| 537 | 6, 7-diF-Q | $-OCH_2-$ | H | $7-SOCH_3$ | $-SC(CH_3)_2CH_2COOH$ |
| 538 | 6, 7-diF-Q | $-OCH_2-$ | H | $7-SOCH_3$ | $-SCH_2CH(CH_3)CH_2COOH$ |
| 539 | 6, 7-diF-Q | $-OCH_2-$ | H | $7-SOCH_3$ | $-SCH_2C(CH_2CH_2)CH_2COOH$ |
| 540 | 6, 7-diF-Q | $-OCH_2-$ | H | $8-SOCH_3$ | $-SCH_2COOH$ |
| 541 | 6, 7-diF-Q | $-OCH_2-$ | H | $8-SOCH_3$ | $-SCH_2CH_2COOH$ |
| 542 | 6, 7-diF-Q | $-OCH_2-$ | H | $8-SOCH_3$ | $-SCH_2CH(CH_3)COOH$ |
| 543 | 6, 7-diF-Q | $-OCH_2-$ | H | $8-SOCH_3$ | $-SCH_2CH(CH_2CH_3)COOH$ |
| 544 | 6, 7-diF-Q | $-OCH_2-$ | H | $8-SOCH_3$ | $-SCH_2C(CH_3)_2COOH$ |
| 545 | 6, 7-diF-Q | $-OCH_2-$ | H | $8-SOCH_3$ | $-SC(CH_3)_2CH_2COOH$ |
| 546 | 6, 7-diF-Q | $-OCH_2-$ | H | $8-SOCH_3$ | $-SCH_2CH(CH_3)CH_2COOH$ |
| 547 | 6, 7-diF-Q | $-OCH_2-$ | H | $8-SOCH_3$ | $-SCH_2C(CH_2CH_2)CH_2COOH$ |
| 548 | 6, 7-diF-Q | $-OCH_2-$ | H | $9-SOCH_3$ | $-SCH_2COOH$ |
| 549 | 6, 7-diF-Q | $-OCH_2-$ | H | $9-SOCH_3$ | $-SCH_2CH_2COOH$ |
| 550 | 6, 7-diF-Q | $-OCH_2-$ | H | $9-SOCH_3$ | $-SCH_2CH(CH_3)COOH$ |
| 551 | 6, 7-diF-Q | $-OCH_2-$ | H | $9-SOCH_3$ | $-SCH_2CH(CH_2CH_3)COOH$ |
| 552 | 6, 7-diF-Q | $-OCH_2-$ | H | $9-SOCH_3$ | $-SCH_2C(CH_3)_2COOH$ |
| 553 | 6, 7-diF-Q | $-OCH_2-$ | H | $9-SOCH_3$ | $-SC(CH_3)_2CH_2COOH$ |
| 554 | 6, 7-diF-Q | $-OCH_2-$ | H | $9-SOCH_3$ | $-SCH_2CH(CH_3)CH_2COOH$ |
| 555 | 6, 7-diF-Q | $-OCH_2-$ | H | $9-SOCH_3$ | $-SCH_2C(CH_2CH_2)CH_2COOH$ |
| 556 | 6, 7-diF-Q | $-OCH_2-$ | H | $7-SO_2CH_3$ | $-SCH_2COOH$ |
| 557 | 6, 7-diF-Q | $-OCH_2-$ | H | $7-SO_2CH_3$ | $-SCH_2CH_2COOH$ |
| 558 | 6, 7-diF-Q | $-OCH_2-$ | H | $7-SO_2CH_3$ | $-SCH_2CH(CH_3)COOH$ |
| 559 | 6, 7-diF-Q | $-OCH_2-$ | H | $7-SO_2CH_3$ | $-SCH_2CH(CH_2CH_3)COOH$ |
| 560 | 6, 7-diF-Q | $-OCH_2-$ | H | $7-SO_2CH_3$ | $-SCH_2C(CH_3)_2COOH$ |
| 561 | 6, 7-diF-Q | $-OCH_2-$ | H | $7-SO_2CH_3$ | $-SC(CH_3)_2CH_2COOH$ |
| 562 | 6, 7-diF-Q | $-OCH_2-$ | H | $7-SO_2CH_3$ | $-SCH_2CH(CH_3)CH_2COOH$ |
| 563 | 6, 7-diF-Q | $-OCH_2-$ | H | $7-SO_2CH_3$ | $-SCH_2C(CH_2CH_2)CH_2COOH$ |
| 564 | 6, 7-diF-Q | $-OCH_2-$ | H | $8-SO_2CH_3$ | $-SCH_2COOH$ |
| 565 | 6, 7-diF-Q | $-OCH_2-$ | H | $8-SO_2CH_3$ | $-SCH_2CH_2COOH$ |
| 566 | 6, 7-diF-Q | $-OCH_2-$ | H | $8-SO_2CH_3$ | $-SCH_2CH(CH_3)COOH$ |
| 567 | 6, 7-diF-Q | $-OCH_2-$ | H | $8-SO_2CH_3$ | $-SCH_2CH(CH_2CH_3)COOH$ |
| 568 | 6, 7-diF-Q | $-OCH_2-$ | H | $8-SO_2CH_3$ | $-SCH_2C(CH_3)_2COOH$ |

| | | | | | |
|---|---|---|---|---|---|
| 569 | 6, 7-diF-Q | $-OCH_2-$ | H | 8-$SO_2CH_3$ | $-SC(CH_3)_2CH_2COOH$ |
| 570 | 6, 7-diF-Q | $-OCH_2-$ | H | 8-$SO_2CH_3$ | $-SCH_2CH(CH_3)CH_2COOH$ |
| 571 | 6, 7-diF-Q | $-OCH_2-$ | H | 8-$SO_2CH_3$ | $-SCH_2C(CH_2CH_2)CH_2COOH$ |
| 572 | 6, 7-diF-Q | $-OCH_2-$ | H | 9-$SO_2CH_3$ | $-SCH_2COOH$ |
| 573 | 6, 7-diF-Q | $-OCH_2-$ | H | 9-$SO_2CH_3$ | $-SCH_2CH_2COOH$ |
| 574 | 6, 7-diF-Q | $-OCH_2-$ | H | 9-$SO_2CH_3$ | $-SCH_2CH(CH_3)COOH$ |
| 575 | 6, 7-diF-Q | $-OCH_2-$ | H | 9-$SO_2CH_3$ | $-SCH_2CH(CH_2CH_3)COOH$ |
| 576 | 6, 7-diF-Q | $-OCH_2-$ | H | 9-$SO_2CH_3$ | $-SCH_2C(CH_3)_2COOH$ |
| 577 | 6, 7-diF-Q | $-OCH_2-$ | H | 9-$SO_2CH_3$ | $-SC(CH_3)_2CH_2COOH$ |
| 578 | 6, 7-diF-Q | $-OCH_2-$ | H | 9-$SO_2CH_3$ | $-SCH_2CH(CH_3)CH_2COOH$ |
| 579 | 6, 7-diF-Q | $-OCH_2-$ | H | 9-$SO_2CH_3$ | $-SCH_2C(CH_2CH_2)CH_2COOH$ |
| 580 | 6, 7-diF-Q | $-OCH_2-$ | 1-F | H | $-SCH_2COOH$ |
| 581 | 6, 7-diF-Q | $-OCH_2-$ | 1-F | H | $-SCH_2CH_2COOH$ |
| 582 | 6, 7-diF-Q | $-OCH_2-$ | 1-F | H | $-SCH_2CH(CH_3)COOH$ |
| 583 | 6, 7-diF-Q | $-OCH_2-$ | 1-F | H | $-SCH_2CH(CH_2CH_3)COOH$ |
| 584 | 6, 7-diF-Q | $-OCH_2-$ | 1-F | H | $-SCH_2C(CH_3)_2COOH$ |
| 585 | 6, 7-diF-Q | $-OCH_2-$ | 1-F | H | $-SC(CH_3)_2CH_2COOH$ |
| 586 | 6, 7-diF-Q | $-OCH_2-$ | 1-F | H | $-SCH_2CH(CH_3)CH_2COOH$ |
| 587 | 6, 7-diF-Q | $-OCH_2-$ | 1-F | H | $-SCH_2C(CH_2CH_2)CH_2COOH$ |
| 588 | 6, 7-diF-Q | $-OCH_2-$ | 3-F | H | $-SCH_2COOH$ |
| 589 | 6, 7-diF-Q | $-OCH_2-$ | 3-F | H | $-SCH_2CH_2COOH$ |
| 590 | 6, 7-diF-Q | $-OCH_2-$ | 3-F | H | $-SCH_2CH(CH_3)COOH$ |
| 591 | 6, 7-diF-Q | $-OCH_2-$ | 3-F | H | $-SCH_2CH(CH_2CH_3)COOH$ |
| 592 | 6, 7-diF-Q | $-OCH_2-$ | 3-F | H | $-SCH_2C(CH_3)_2COOH$ |
| 593 | 6, 7-diF-Q | $-OCH_2-$ | 3-F | H | $-SC(CH_3)_2CH_2COOH$ |
| 594 | 6, 7-diF-Q | $-OCH_2-$ | 3-F | H | $-SCH_2CH(CH_3)CH_2COOH$ |
| 595 | 6, 7-diF-Q | $-OCH_2-$ | 3-F | H | $-SCH_2C(CH_2CH_2)CH_2COOH$ |
| 596 | 6, 7-diF-Q | $-OCH_2-$ | 4-F | H | $-SCH_2COOH$ |
| 597 | 6, 7-diF-Q | $-OCH_2-$ | 4-F | H | $-SCH_2CH_2COOH$ |
| 598 | 6, 7-diF-Q | $-OCH_2-$ | 4-F | H | $-SCH_2CH(CH_3)COOH$ |
| 599 | 6, 7-diF-Q | $-OCH_2-$ | 4-F | H | $-SCH_2CH(CH_2CH_3)COOH$ |
| 600 | 6, 7-diF-Q | $-OCH_2-$ | 4-F | H | $-SCH_2C(CH_3)_2COOH$ |
| 601 | 6, 7-diF-Q | $-OCH_2-$ | 4-F | H | $-SC(CH_3)_2CH_2COOH$ |
| 602 | 6, 7-diF-Q | $-OCH_2-$ | 4-F | H | $-SCH_2CH(CH_3)CH_2COOH$ |
| 603 | 6, 7-diF-Q | $-OCH_2-$ | 4-F | H | $-SCH_2C(CH_2CH_2)CH_2COOH$ |
| 604 | 6, 7-diF-Q | $-OCH_2-$ | 1-Cl | H | $-SCH_2COOH$ |
| 605 | 6, 7-diF-Q | $-OCH_2-$ | 1-Cl | H | $-SCH_2CH_2COOH$ |
| 606 | 6, 7-diF-Q | $-OCH_2-$ | 1-Cl | H | $-SCH_2CH(CH_3)COOH$ |
| 607 | 6, 7-diF-Q | $-OCH_2-$ | 1-Cl | H | $-SCH_2CH(CH_2CH_3)COOH$ |
| 608 | 6, 7-diF-Q | $-OCH_2-$ | 1-Cl | H | $-SCH_2C(CH_3)_2COOH$ |
| 609 | 6, 7-diF-Q | $-OCH_2-$ | 1-Cl | H | $-SC(CH_3)_2CH_2COOH$ |
| 610 | 6, 7-diF-Q | $-OCH_2-$ | 1-Cl | H | $-SCH_2CH(CH_3)CH_2COOH$ |
| 611 | 6, 7-diF-Q | $-OCH_2-$ | 1-Cl | H | $-SCH_2C(CH_2CH_2)CH_2COOH$ |
| 612 | 6, 7-diF-Q | $-OCH_2-$ | 3-Cl | H | $-SCH_2COOH$ |
| 613 | 6, 7-diF-Q | $-OCH_2-$ | 3-Cl | H | $-SCH_2CH_2COOH$ |

| | | | | | |
|---|---|---|---|---|---|
| 614 | 6, 7-diF-Q | $-OCH_2-$ | 3-Cl | H | $-SCH_2CH(CH_3)COOH$ |
| 615 | 6, 7-diF-Q | $-OCH_2-$ | 3-Cl | H | $-SCH_2CH(CH_2CH_3)COOH$ |
| 616 | 6, 7-diF-Q | $-OCH_2-$ | 3-Cl | H | $-SCH_2C(CH_3)_2COOH$ |
| 617 | 6, 7-diF-Q | $-OCH_2-$ | 3-Cl | H | $-SC(CH_3)_2CH_2COOH$ |
| 618 | 6, 7-diF-Q | $-OCH_2-$ | 3-Cl | H | $-SCH_2CH(CH_3)CH_2COOH$ |
| 619 | 6, 7-diF-Q | $-OCH_2-$ | 3-Cl | H | $-SCH_2C(CH_2CH_2)CH_2COOH$ |
| 620 | 6, 7-diF-Q | $-OCH_2-$ | 4-Cl | H | $-SCH_2COOH$ |
| 621 | 6, 7-diF-Q | $-OCH_2-$ | 4-Cl | H | $-SCH_2CH_2COOH$ |
| 622 | 6, 7-diF-Q | $-OCH_2-$ | 4-Cl | H | $-SCH_2CH(CH_3)COOH$ |
| 623 | 6, 7-diF-Q | $-OCH_2-$ | 4-Cl | H | $-SCH_2CH(CH_2CH_3)COOH$ |
| 624 | 6, 7-diF-Q | $-OCH_2-$ | 4-Cl | H | $-SCH_2C(CH_3)_2COOH$ |
| 625 | 6, 7-diF-Q | $-OCH_2-$ | 4-Cl | H | $-SC(CH_3)_2CH_2COOH$ |
| 626 | 6, 7-diF-Q | $-OCH_2-$ | 4-Cl | H | $-SCH_2CH(CH_3)CH_2COOH$ |
| 627 | 6, 7-diF-Q | $-OCH_2-$ | 4-Cl | H | $-SCH_2C(CH_2CH_2)CH_2COOH$ |
| 628 | 6, 7-diF-Q | $-OCH_2-$ | $1-NO_2$ | H | $-SCH_2COOH$ |
| 629 | 6, 7-diF-Q | $-OCH_2-$ | $1-NO_2$ | H | $-SCH_2CH_2COOH$ |
| 630 | 6, 7-diF-Q | $-OCH_2-$ | $1-NO_2$ | H | $-SCH_2CH(CH_3)COOH$ |
| 631 | 6, 7-diF-Q | $-OCH_2-$ | $1-NO_2$ | H | $-SCH_2CH(CH_2CH_3)COOH$ |
| 632 | 6, 7-diF-Q | $-OCH_2-$ | $1-NO_2$ | H | $-SCH_2C(CH_3)_2COOH$ |
| 633 | 6, 7-diF-Q | $-OCH_2-$ | $1-NO_2$ | H | $-SC(CH_3)_2CH_2COOH$ |
| 634 | 6, 7-diF-Q | $-OCH_2-$ | $1-NO_2$ | H | $-SCH_2CH(CH_3)CH_2COOH$ |
| 635 | 6, 7-diF-Q | $-OCH_2-$ | $1-NO_2$ | H | $-SCH_2C(CH_2CH_2)CH_2COOH$ |
| 636 | 6, 7-diF-Q | $-OCH_2-$ | $3-NO_2$ | H | $-SCH_2COOH$ |
| 637 | 6, 7-diF-Q | $-OCH_2-$ | $3-NO_2$ | H | $-SCH_2CH_2COOH$ |
| 638 | 6, 7-diF-Q | $-OCH_2-$ | $3-NO_2$ | H | $-SCH_2CH(CH_3)COOH$ |
| 639 | 6, 7-diF-Q | $-OCH_2-$ | $3-NO_2$ | H | $-SCH_2CH(CH_2CH_3)COOH$ |
| 640 | 6, 7-diF-Q | $-OCH_2-$ | $3-NO_2$ | H | $-SCH_2C(CH_3)_2COOH$ |
| 641 | 6, 7-diF-Q | $-OCH_2-$ | $3-NO_2$ | H | $-SC(CH_3)_2CH_2COOH$ |
| 642 | 6, 7-diF-Q | $-OCH_2-$ | $3-NO_2$ | H | $-SCH_2CH(CH_3)CH_2COOH$ |
| 643 | 6, 7-diF-Q | $-OCH_2-$ | $3-NO_2$ | H | $-SCH_2C(CH_2CH_2)CH_2COOH$ |
| 644 | 6, 7-diF-Q | $-OCH_2-$ | $4-NO_2$ | H | $-SCH_2COOH$ |
| 645 | 6, 7-diF-Q | $-OCH_2-$ | $4-NO_2$ | H | $-SCH_2CH_2COOH$ |
| 646 | 6, 7-diF-Q | $-OCH_2-$ | $4-NO_2$ | H | $-SCH_2CH(CH_3)COOH$ |
| 647 | 6, 7-diF-Q | $-OCH_2-$ | $4-NO_2$ | H | $-SCH_2CH(CH_2CH_3)COOH$ |
| 648 | 6, 7-diF-Q | $-OCH_2-$ | $4-NO_2$ | H | $-SCH_2C(CH_3)_2COOH$ |
| 649 | 6, 7-diF-Q | $-OCH_2-$ | $4-NO_2$ | H | $-SC(CH_3)_2CH_2COOH$ |
| 650 | 6, 7-diF-Q | $-OCH_2-$ | $4-NO_2$ | H | $-SCH_2CH(CH_3)CH_2COOH$ |
| 651 | 6, 7-diF-Q | $-OCH_2-$ | $4-NO_2$ | H | $-SCH_2C(CH_2CH_2)CH_2COOH$ |
| 652 | 6, 7-diF-Q | $-OCH_2-$ | 1-CN | H | $-SCH_2COOH$ |
| 653 | 6, 7-diF-Q | $-OCH_2-$ | 1-CN | H | $-SCH_2CH_2COOH$ |
| 654 | 6, 7-diF-Q | $-OCH_2-$ | 1-CN | H | $-SCH_2CH(CH_3)COOH$ |
| 655 | 6, 7-diF-Q | $-OCH_2-$ | 1-CN | H | $-SCH_2CH(CH_2CH_3)COOH$ |
| 656 | 6, 7-diF-Q | $-OCH_2-$ | 1-CN | H | $-SCH_2C(CH_3)_2COOH$ |
| 657 | 6, 7-diF-Q | $-OCH_2-$ | 1-CN | H | $-SC(CH_3)_2CH_2COOH$ |
| 658 | 6, 7-diF-Q | $-OCH_2-$ | 1-CN | H | $-SCH_2CH(CH_3)CH_2COOH$ |

| | | | | | |
|---|---|---|---|---|---|
| 659 | 6, 7-diF-Q | $-OCH_2-$ | 1-CN | H | $-SCH_2C(CH_2CH_2)CH_2COOH$ |
| 660 | 6, 7-diF-Q | $-OCH_2-$ | 3-CN | H | $-SCH_2COOH$ |
| 661 | 6, 7-diF-Q | $-OCH_2-$ | 3-CN | H | $-SCH_2CH_2COOH$ |
| 662 | 6, 7-diF-Q | $-OCH_2-$ | 3-CN | H | $-SCH_2CH(CH_3)COOH$ |
| 663 | 6, 7-diF-Q | $-OCH_2-$ | 3-CN | H | $-SCH_2CH(CH_2CH_3)COOH$ |
| 664 | 6, 7-diF-Q | $-OCH_2-$ | 3-CN | H | $-SCH_2C(CH_3)_2COOH$ |
| 665 | 6, 7-diF-Q | $-OCH_2-$ | 3-CN | H | $-SC(CH_3)_2CH_2COOH$ |
| 666 | 6, 7-diF-Q | $-OCH_2-$ | 3-CN | H | $-SCH_2CH(CH_3)CH_2COOH$ |
| 667 | 6, 7-diF-Q | $-OCH_2-$ | 3-CN | H | $-SCH_2C(CH_2CH_2)CH_2COOH$ |
| 668 | 6, 7-diF-Q | $-OCH_2-$ | 4-CN | H | $-SCH_2COOH$ |
| 669 | 6, 7-diF-Q | $-OCH_2-$ | 4-CN | H | $-SCH_2CH_2COOH$ |
| 670 | 6, 7-diF-Q | $-OCH_2-$ | 4-CN | H | $-SCH_2CH(CH_3)COOH$ |
| 671 | 6, 7-diF-Q | $-OCH_2-$ | 4-CN | H | $-SCH_2CH(CH_2CH_3)COOH$ |
| 672 | 6, 7-diF-Q | $-OCH_2-$ | 4-CN | H | $-SCH_2C(CH_3)_2COOH$ |
| 673 | 6, 7-diF-Q | $-OCH_2-$ | 4-CN | H | $-SC(CH_3)_2CH_2COOH$ |
| 674 | 6, 7-diF-Q | $-OCH_2-$ | 4-CN | H | $-SCH_2CH(CH_3)CH_2COOH$ |
| 675 | 6, 7-diF-Q | $-OCH_2-$ | 4-CN | H | $-SCH_2C(CH_2CH_2)CH_2COOH$ |
| 676 | 6, 7-diF-Q | $-OCH_2-$ | $1-CH_3$ | H | $-SCH_2COOH$ |
| 677 | 6, 7-diF-Q | $-OCH_2-$ | $1-CH_3$ | H | $-SCH_2CH_2COOH$ |
| 678 | 6, 7-diF-Q | $-OCH_2-$ | $1-CH_3$ | H | $-SCH_2CH(CH_3)COOH$ |
| 679 | 6, 7-diF-Q | $-OCH_2-$ | $1-CH_3$ | H | $-SCH_2CH(CH_2CH_3)COOH$ |
| 680 | 6, 7-diF-Q | $-OCH_2-$ | $1-CH_3$ | H | $-SCH_2C(CH_3)_2COOH$ |
| 681 | 6, 7-diF-Q | $-OCH_2-$ | $1-CH_3$ | H | $-SC(CH_3)_2CH_2COOH$ |
| 682 | 6, 7-diF-Q | $-OCH_2-$ | $1-CH_3$ | H | $-SCH_2CH(CH_3)CH_2COOH$ |
| 683 | 6, 7-diF-Q | $-OCH_2-$ | $1-CH_3$ | H | $-SCH_2C(CH_2CH_2)CH_2COOH$ |
| 684 | 6, 7-diF-Q | $-OCH_2-$ | $3-CH_3$ | H | $-SCH_2COOH$ |
| 685 | 6, 7-diF-Q | $-OCH_2-$ | $3-CH_3$ | H | $-SCH_2CH_2COOH$ |
| 686 | 6, 7-diF-Q | $-OCH_2-$ | $3-CH_3$ | H | $-SCH_2CH(CH_3)COOH$ |
| 687 | 6, 7-diF-Q | $-OCH_2-$ | $3-CH_3$ | H | $-SCH_2CH(CH_2CH_3)COOH$ |
| 688 | 6, 7-diF-Q | $-OCH_2-$ | $3-CH_3$ | H | $-SCH_2C(CH_3)_2COOH$ |
| 689 | 6, 7-diF-Q | $-OCH_2-$ | $3-CH_3$ | H | $-SC(CH_3)_2CH_2COOH$ |
| 690 | 6, 7-diF-Q | $-OCH_2-$ | $3-CH_3$ | H | $-SCH_2CH(CH_3)CH_2COOH$ |
| 691 | 6, 7-diF-Q | $-OCH_2-$ | $3-CH_3$ | H | $-SCH_2C(CH_2CH_2)CH_2COOH$ |
| 692 | 6, 7-diF-Q | $-OCH_2-$ | $4-CH_3$ | H | $-SCH_2COOH$ |
| 693 | 6, 7-diF-Q | $-OCH_2-$ | $4-CH_3$ | H | $-SCH_2CH_2COOH$ |
| 694 | 6, 7-diF-Q | $-OCH_2-$ | $4-CH_3$ | H | $-SCH_2CH(CH_3)COOH$ |
| 695 | 6, 7-diF-Q | $-OCH_2-$ | $4-CH_3$ | H | $-SCH_2CH(CH_2CH_3)COOH$ |
| 696 | 6, 7-diF-Q | $-OCH_2-$ | $4-CH_3$ | H | $-SCH_2C(CH_3)_2COOH$ |
| 697 | 6, 7-diF-Q | $-OCH_2-$ | $4-CH_3$ | H | $-SC(CH_3)_2CH_2COOH$ |
| 698 | 6, 7-diF-Q | $-OCH_2-$ | $4-CH_3$ | H | $-SCH_2CH(CH_3)CH_2COOH$ |
| 699 | 6, 7-diF-Q | $-OCH_2-$ | $4-CH_3$ | H | $-SCH_2C(CH_2CH_2)CH_2COOH$ |
| 700 | 6, 7-diF-Q | $-OCH_2-$ | $1-OCH_3$ | H | $-SCH_2COOH$ |
| 701 | 6, 7-diF-Q | $-OCH_2-$ | $1-OCH_3$ | H | $-SCH_2CH_2COOH$ |
| 702 | 6, 7-diF-Q | $-OCH_2-$ | $1-OCH_3$ | H | $-SCH_2CH(CH_3)COOH$ |
| 703 | 6, 7-diF-Q | $-OCH_2-$ | $1-OCH_3$ | H | $-SCH_2CH(CH_2CH_3)COOH$ |

| | | | | | |
|---|---|---|---|---|---|
| 704 | 6, 7-diF-Q | $-OCH_2-$ | $1-OCH_3$ | H | $-SCH_2C(CH_3)_2COOH$ |
| 705 | 6, 7-diF-Q | $-OCH_2-$ | $1-OCH_3$ | H | $-SC(CH_3)_2CH_2COOH$ |
| 706 | 6, 7-diF-Q | $-OCH_2-$ | $1-OCH_3$ | H | $-SCH_2CH(CH_3)CH_2COOH$ |
| 707 | 6, 7-diF-Q | $-OCH_2-$ | $1-OCH_3$ | H | $-SCH_2C(CH_2CH_2)CH_2COOH$ |
| 708 | 6, 7-diF-Q | $-OCH_2-$ | $3-OCH_3$ | H | $-SCH_2COOH$ |
| 709 | 6, 7-diF-Q | $-OCH_2-$ | $3-OCH_3$ | H | $-SCH_2CH_2COOH$ |
| 710 | 6, 7-diF-Q | $-OCH_2-$ | $3-OCH_3$ | H | $-SCH_2CH(CH_3)COOH$ |
| 711 | 6, 7-diF-Q | $-OCH_2-$ | $3-OCH_3$ | H | $-SCH_2CH(CH_2CH_3)COOH$ |
| 712 | 6, 7-diF-Q | $-OCH_2-$ | $3-OCH_3$ | H | $-SCH_2C(CH_3)_2COOH$ |
| 713 | 6, 7-diF-Q | $-OCH_2-$ | $3-OCH_3$ | H | $-SC(CH_3)_2CH_2COOH$ |
| 714 | 6, 7-diF-Q | $-OCH_2-$ | $3-OCH_3$ | H | $-SCH_2CH(CH_3)CH_2COOH$ |
| 715 | 6, 7-diF-Q | $-OCH_2-$ | $3-OCH_3$ | H | $-SCH_2C(CH_2CH_2)CH_2COOH$ |
| 716 | 6, 7-diF-Q | $-OCH_2-$ | $4-OCH_3$ | H | $-SCH_2COOH$ |
| 717 | 6, 7-diF-Q | $-OCH_2-$ | $4-OCH_3$ | H | $-SCH_2CH_2COOH$ |
| 718 | 6, 7-diF-Q | $-OCH_2-$ | $4-OCH_3$ | H | $-SCH_2CH(CH_3)COOH$ |
| 719 | 6, 7-diF-Q | $-OCH_2-$ | $4-OCH_3$ | H | $-SCH_2CH(CH_2CH_3)COOH$ |
| 720 | 6, 7-diF-Q | $-OCH_2-$ | $4-OCH_3$ | H | $-SCH_2C(CH_3)_2COOH$ |
| 721 | 6, 7-diF-Q | $-OCH_2-$ | $4-OCH_3$ | H | $-SC(CH_3)_2CH_2COOH$ |
| 722 | 6, 7-diF-Q | $-OCH_2-$ | $4-OCH_3$ | H | $-SCH_2CH(CH_3)CH_2COOH$ |
| 723 | 6, 7-diF-Q | $-OCH_2-$ | $4-OCH_3$ | H | $-SCH_2C(CH_2CH_2)CH_2COOH$ |
| 724 | 6, 7-diF-Q | $-CH_2CH_2-$ | H | H | $-SCH_2COOH$ |
| 725 | 6, 7-diF-Q | $-CH_2CH_2-$ | H | H | $-SCH_2CH(CH_2CH_3)COOH$ |
| 726 | 6, 7-diF-Q | $-CH_2CH_2-$ | H | H | $-SC(CH_3)_2CH_2COOH$ |
| 727 | 6, 7-diF-Q | $-CH_2CH_2-$ | H | H | $-SCH_2CH(CH_3)CH_2COOH$ |
| 728 | 6, 7-diF-Q | $-CH_2CH_2-$ | H | 7-F | $-SCH_2COOH$ |
| 729 | 6, 7-diF-Q | $-CH_2CH_2-$ | H | 7-F | $-SCH_2CH_2COOH$ |
| 730 | 6, 7-diF-Q | $-CH_2CH_2-$ | H | 7-F | $-SCH_2CH(CH_3)COOH$ |
| 731 | 6, 7-diF-Q | $-CH_2CH_2-$ | H | 7-F | $-SCH_2CH(CH_2CH_3)COOH$ |
| 732 | 6, 7-diF-Q | $-CH_2CH_2-$ | H | 7-F | $-SCH_2C(CH_3)_2COOH$ |
| 733 | 6, 7-diF-Q | $-CH_2CH_2-$ | H | 7-F | $-SC(CH_3)_2CH_2COOH$ |
| 734 | 6, 7-diF-Q | $-CH_2CH_2-$ | H | 7-F | $-SCH_2CH(CH_3)CH_2COOH$ |
| 735 | 6, 7-diF-Q | $-CH_2CH_2-$ | H | 7-F | $-SCH_2C(CH_2CH_2)CH_2COOH$ |
| 736 | 6, 7-diF-Q | $-CH_2CH_2-$ | H | 8-F | $-SCH_2COOH$ |
| 737 | 6, 7-diF-Q | $-CH_2CH_2-$ | H | 8-F | $-SCH_2CH_2COOH$ |
| 738 | 6, 7-diF-Q | $-CH_2CH_2-$ | H | 8-F | $-SCH_2CH(CH_3)COOH$ |
| 739 | 6, 7-diF-Q | $-CH_2CH_2-$ | H | 8-F | $-SCH_2CH(CH_2CH_3)COOH$ |
| 740 | 6, 7-diF-Q | $-CH_2CH_2-$ | H | 8-F | $-SCH_2C(CH_3)_2COOH$ |
| 741 | 6, 7-diF-Q | $-CH_2CH_2-$ | H | 8-F | $-SC(CH_3)_2CH_2COOH$ |
| 742 | 6, 7-diF-Q | $-CH_2CH_2-$ | H | 8-F | $-SCH_2CH(CH_3)CH_2COOH$ |
| 743 | 6, 7-diF-Q | $-CH_2CH_2-$ | H | 8-F | $-SCH_2C(CH_2CH_2)CH_2COOH$ |
| 744 | 6, 7-diF-Q | $-CH_2CH_2-$ | H | 9-F | $-SCH_2COOH$ |
| 745 | 6, 7-diF-Q | $-CH_2CH_2-$ | H | 9-F | $-SCH_2CH_2COOH$ |
| 746 | 6, 7-diF-Q | $-CH_2CH_2-$ | H | 9-F | $-SCH_2CH(CH_3)COOH$ |
| 747 | 6, 7-diF-Q | $-CH_2CH_2-$ | H | 9-F | $-SCH_2CH(CH_2CH_3)COOH$ |
| 748 | 6, 7-diF-Q | $-CH_2CH_2-$ | H | 9-F | $-SCH_2C(CH_3)_2COOH$ |

| | | | | | |
|---|---|---|---|---|---|
| 749 | 6, 7-diF-Q | $-CH_2CH_2-$ | H | 9-F | $-SC(CH_3)_2CH_2COOH$ |
| 750 | 6, 7-diF-Q | $-CH_2CH_2-$ | H | 9-F | $-SCH_2CH(CH_3)CH_2COOH$ |
| 751 | 6, 7-diF-Q | $-CH_2CH_2-$ | H | 9-F | $-SCH_2C(CH_2CH_2)CH_2COOH$ |
| 752 | 6, 7-diF-Q | $-CH_2CH_2-$ | H | 7-CN | $-SCH_2COOH$ |
| 753 | 6, 7-diF-Q | $-CH_2CH_2-$ | H | 7-CN | $-SCH_2CH_2COOH$ |
| 754 | 6, 7-diF-Q | $-CH_2CH_2-$ | H | 7-CN | $-SCH_2CH(CH_3)COOH$ |
| 755 | 6, 7-diF-Q | $-CH_2CH_2-$ | H | 7-CN | $-SCH_2CH(CH_2CH_3)COOH$ |
| 756 | 6, 7-diF-Q | $-CH_2CH_2-$ | H | 7-CN | $-SCH_2C(CH_3)_2COOH$ |
| 757 | 6, 7-diF-Q | $-CH_2CH_2-$ | H | 7-CN | $-SC(CH_3)_2CH_2COOH$ |
| 758 | 6, 7-diF-Q | $-CH_2CH_2-$ | H | 7-CN | $-SCH_2CH(CH_3)CH_2COOH$ |
| 759 | 6, 7-diF-Q | $-CH_2CH_2-$ | H | 7-CN | $-SCH_2C(CH_2CH_2)CH_2COOH$ |
| 760 | 6, 7-diF-Q | $-CH_2CH_2-$ | H | 8-CN | $-SCH_2COOH$ |
| 761 | 6, 7-diF-Q | $-CH_2CH_2-$ | H | 8-CN | $-SCH_2CH_2COOH$ |
| 762 | 6, 7-diF-Q | $-CH_2CH_2-$ | H | 8-CN | $-SCH_2CH(CH_3)COOH$ |
| 763 | 6, 7-diF-Q | $-CH_2CH_2-$ | H | 8-CN | $-SCH_2CH(CH_2CH_3)COOH$ |
| 764 | 6, 7-diF-Q | $-CH_2CH_2-$ | H | 8-CN | $-SCH_2C(CH_3)_2COOH$ |
| 765 | 6, 7-diF-Q | $-CH_2CH_2-$ | H | 8-CN | $-SC(CH_3)_2CH_2COOH$ |
| 766 | 6, 7-diF-Q | $-CH_2CH_2-$ | H | 8-CN | $-SCH_2CH(CH_3)CH_2COOH$ |
| 767 | 6, 7-diF-Q | $-CH_2CH_2-$ | H | 8-CN | $-SCH_2C(CH_2CH_2)CH_2COOH$ |
| 768 | 6, 7-diF-Q | $-CH_2CH_2-$ | H | 9-CN | $-SCH_2COOH$ |
| 769 | 6, 7-diF-Q | $-CH_2CH_2-$ | H | 9-CN | $-SCH_2CH_2COOH$ |
| 770 | 6, 7-diF-Q | $-CH_2CH_2-$ | H | 9-CN | $-SCH_2CH(CH_3)COOH$ |
| 771 | 6, 7-diF-Q | $-CH_2CH_2-$ | H | 9-CN | $-SCH_2CH(CH_2CH_3)COOH$ |
| 772 | 6, 7-diF-Q | $-CH_2CH_2-$ | H | 9-CN | $-SCH_2C(CH_3)_2COOH$ |
| 773 | 6, 7-diF-Q | $-CH_2CH_2-$ | H | 9-CN | $-SC(CH_3)_2CH_2COOH$ |
| 774 | 6, 7-diF-Q | $-CH_2CH_2-$ | H | 9-CN | $-SCH_2CH(CH_3)CH_2COOH$ |
| 775 | 6, 7-diF-Q | $-CH_2CH_2-$ | H | 9-CN | $-SCH_2C(CH_2CH_2)CH_2COOH$ |
| 776 | 6, 7-diF-Q | $-CH_2CH_2-$ | H | $7-CF_3$ | $-SCH_2COOH$ |
| 777 | 6, 7-diF-Q | $-CH_2CH_2-$ | H | $7-CF_3$ | $-SCH_2CH_2COOH$ |
| 778 | 6, 7-diF-Q | $-CH_2CH_2-$ | H | $7-CF_3$ | $-SCH_2CH(CH_3)COOH$ |
| 779 | 6, 7-diF-Q | $-CH_2CH_2-$ | H | $7-CF_3$ | $-SCH_2CH(CH_2CH_3)COOH$ |
| 780 | 6, 7-diF-Q | $-CH_2CH_2-$ | H | $7-CF_3$ | $-SCH_2C(CH_3)_2COOH$ |
| 781 | 6, 7-diF-Q | $-CH_2CH_2-$ | H | $7-CF_3$ | $-SC(CH_3)_2CH_2COOH$ |
| 782 | 6, 7-diF-Q | $-CH_2CH_2-$ | H | $7-CF_3$ | $-SCH_2CH(CH_3)CH_2COOH$ |
| 783 | 6, 7-diF-Q | $-CH_2CH_2-$ | H | $7-CF_3$ | $-SCH_2C(CH_2CH_2)CH_2COOH$ |
| 784 | 6, 7-diF-Q | $-CH_2CH_2-$ | H | $8-CF_3$ | $-SCH_2COOH$ |
| 785 | 6, 7-diF-Q | $-CH_2CH_2-$ | H | $8-CF_3$ | $-SCH_2CH_2COOH$ |
| 786 | 6, 7-diF-Q | $-CH_2CH_2-$ | H | $8-CF_3$ | $-SCH_2CH(CH_3)COOH$ |
| 787 | 6, 7-diF-Q | $-CH_2CH_2-$ | H | $8-CF_3$ | $-SCH_2CH(CH_2CH_3)COOH$ |
| 788 | 6, 7-diF-Q | $-CH_2CH_2-$ | H | $8-CF_3$ | $-SCH_2C(CH_3)_2COOH$ |
| 789 | 6, 7-diF-Q | $-CH_2CH_2-$ | H | $8-CF_3$ | $-SC(CH_3)_2CH_2COOH$ |
| 790 | 6, 7-diF-Q | $-CH_2CH_2-$ | H | $8-CF_3$ | $-SCH_2CH(CH_3)CH_2COOH$ |
| 791 | 6, 7-diF-Q | $-CH_2CH_2-$ | H | $8-CF_3$ | $-SCH_2C(CH_2CH_2)CH_2COOH$ |
| 792 | 6, 7-diF-Q | $-CH_2CH_2-$ | H | $9-CF_3$ | $-SCH_2COOH$ |
| 793 | 6, 7-diF-Q | $-CH_2CH_2-$ | H | $9-CF_3$ | $-SCH_2CH_2COOH$ |

| | | | | | |
|---|---|---|---|---|---|
| 794 | 6, 7-diF-Q | $-CH_2CH_2-$ | H | $9-CF_3$ | $-SCH_2CH(CH_3)COOH$ |
| 795 | 6, 7-diF-Q | $-CH_2CH_2-$ | H | $9-CF_3$ | $-SCH_2CH(CH_2CH_3)COOH$ |
| 796 | 6, 7-diF-Q | $-CH_2CH_2-$ | H | $9-CF_3$ | $-SCH_2C(CH_3)_2COOH$ |
| 797 | 6, 7-diF-Q | $-CH_2CH_2-$ | H | $9-CF_3$ | $-SC(CH_3)_2CH_2COOH$ |
| 798 | 6, 7-diF-Q | $-CH_2CH_2-$ | H | $9-CF_3$ | $-SCH_2CH(CH_3)CH_2COOH$ |
| 799 | 6, 7-diF-Q | $-CH_2CH_2-$ | H | $9-CF_3$ | $-SCH_2C(CH_2CH_2)CH_2COOH$ |
| 800 | 6, 7-diF-Q | $-CH_2CH_2-$ | H | 7-C≡CH | $-SCH_2COOH$ |
| 801 | 6, 7-diF-Q | $-CH_2CH_2-$ | H | 7-C≡CH | $-SCH_2CH_2COOH$ |
| 802 | 6, 7-diF-Q | $-CH_2CH_2-$ | H | 7-C≡CH | $-SCH_2CH(CH_3)COOH$ |
| 803 | 6, 7-diF-Q | $-CH_2CH_2-$ | H | 7-C≡CH | $-SCH_2CH(CH_2CH_3)COOH$ |
| 804 | 6, 7-diF-Q | $-CH_2CH_2-$ | H | 7-C≡CH | $-SCH_2C(CH_3)_2COOH$ |
| 805 | 6, 7-diF-Q | $-CH_2CH_2-$ | H | 7-C≡CH | $-SC(CH_3)_2CH_2COOH$ |
| 806 | 6, 7-diF-Q | $-CH_2CH_2-$ | H | 7-C≡CH | $-SCH_2CH(CH_3)CH_2COOH$ |
| 807 | 6, 7-diF-Q | $-CH_2CH_2-$ | H | 7-C≡CH | $-SCH_2C(CH_2CH_2)CH_2COOH$ |
| 808 | 6, 7-diF-Q | $-CH_2CH_2-$ | H | 8-C≡CH | $-SCH_2COOH$ |
| 809 | 6, 7-diF-Q | $-CH_2CH_2-$ | H | 8-C≡CH | $-SCH_2CH_2COOH$ |
| 810 | 6, 7-diF-Q | $-CH_2CH_2-$ | H | 8-C≡CH | $-SCH_2CH(CH_3)COOH$ |
| 811 | 6, 7-diF-Q | $-CH_2CH_2-$ | H | 8-C≡CH | $-SCH_2CH(CH_2CH_3)COOH$ |
| 812 | 6, 7-diF-Q | $-CH_2CH_2-$ | H | 8-C≡CH | $-SCH_2C(CH_3)_2COOH$ |
| 813 | 6, 7-diF-Q | $-CH_2CH_2-$ | H | 8-C≡CH | $-SC(CH_3)_2CH_2COOH$ |
| 814 | 6, 7-diF-Q | $-CH_2CH_2-$ | H | 8-C≡CH | $-SCH_2CH(CH_3)CH_2COOH$ |
| 815 | 6, 7-diF-Q | $-CH_2CH_2-$ | H | 8-C≡CH | $-SCH_2C(CH_2CH_2)CH_2COOH$ |
| 816 | 6, 7-diF-Q | $-CH_2CH_2-$ | H | 9-C≡CH | $-SCH_2COOH$ |
| 817 | 6, 7-diF-Q | $-CH_2CH_2-$ | H | 9-C≡CH | $-SCH_2CH_2COOH$ |
| 818 | 6, 7-diF-Q | $-CH_2CH_2-$ | H | 9-C≡CH | $-SCH_2CH(CH_3)COOH$ |
| 819 | 6, 7-diF-Q | $-CH_2CH_2-$ | H | 9-C≡CH | $-SCH_2CH(CH_2CH_3)COOH$ |
| 820 | 6, 7-diF-Q | $-CH_2CH_2-$ | H | 9-C≡CH | $-SCH_2C(CH_3)_2COOH$ |
| 821 | 6, 7-diF-Q | $-CH_2CH_2-$ | H | 9-C≡CH | $-SC(CH_3)_2CH_2COOH$ |
| 822 | 6, 7-diF-Q | $-CH_2CH_2-$ | H | 9-C≡CH | $-SCH_2CH(CH_3)CH_2COOH$ |
| 823 | 6, 7-diF-Q | $-CH_2CH_2-$ | H | 9-C≡CH | $-SCH_2C(CH_2CH_2)CH_2COOH$ |
| 824 | 6, 7-diF-Q | $-CH_2CH_2-$ | H | $7-C(CH_3)_2OH$ | $-SCH_2COOH$ |
| 825 | 6, 7-diF-Q | $-CH_2CH_2-$ | H | $7-C(CH_3)_2OH$ | $-SCH_2CH_2COOH$ |
| 826 | 6, 7-diF-Q | $-CH_2CH_2-$ | H | $7-C(CH_3)_2OH$ | $-SCH_2CH(CH_3)COOH$ |
| 827 | 6, 7-diF-Q | $-CH_2CH_2-$ | H | $7-C(CH_3)_2OH$ | $-SCH_2CH(CH_2CH_3)COOH$ |
| 828 | 6, 7-diF-Q | $-CH_2CH_2-$ | H | $7-C(CH_3)_2OH$ | $-SCH_2C(CH_3)_2COOH$ |
| 829 | 6, 7-diF-Q | $-CH_2CH_2-$ | H | $7-C(CH_3)_2OH$ | $-SC(CH_3)_2CH_2COOH$ |
| 830 | 6, 7-diF-Q | $-CH_2CH_2-$ | H | $7-C(CH_3)_2OH$ | $-SCH_2CH(CH_3)CH_2COOH$ |
| 831 | 6, 7-diF-Q | $-CH_2CH_2-$ | H | $7-C(CH_3)_2OH$ | $-SCH_2C(CH_2CH_2)CH_2COOH$ |
| 832 | 6, 7-diF-Q | $-CH_2CH_2-$ | H | $8-C(CH_3)_2OH$ | $-SCH_2COOH$ |
| 833 | 6, 7-diF-Q | $-CH_2CH_2-$ | H | $8-C(CH_3)_2OH$ | $-SCH_2CH_2COOH$ |
| 834 | 6, 7-diF-Q | $-CH_2CH_2-$ | H | $8-C(CH_3)_2OH$ | $-SCH_2CH(CH_3)COOH$ |
| 835 | 6, 7-diF-Q | $-CH_2CH_2-$ | H | $8-C(CH_3)_2OH$ | $-SCH_2CH(CH_2CH_3)COOH$ |
| 836 | 6, 7-diF-Q | $-CH_2CH_2-$ | H | $8-C(CH_3)_2OH$ | $-SCH_2C(CH_3)_2COOH$ |

| | | | | | |
|---|---|---|---|---|---|
| 837 | 6, 7-diF-Q | $-CH_2CH_2-$ | H | 8-$C(CH_3)_2OH$ | $-SC(CH_3)_2CH_2COOH$ |
| 838 | 6, 7-diF-Q | $-CH_2CH_2-$ | H | 8-$C(CH_3)_2OH$ | $-SCH_2CH(CH_3)CH_2COOH$ |
| 839 | 6, 7-diF-Q | $-CH_2CH_2-$ | H | 8-$C(CH_3)_2OH$ | $-SCH_2C(CH_2CH_2)CH_2COOH$ |
| 840 | 6, 7-diF-Q | $-CH_2CH_2-$ | H | 9-$C(CH_3)_2OH$ | $-SCH_2COOH$ |
| 841 | 6, 7-diF-Q | $-CH_2CH_2-$ | H | 9-$C(CH_3)_2OH$ | $-SCH_2CH_2COOH$ |
| 842 | 6, 7-diF-Q | $-CH_2CH_2-$ | H | 9-$C(CH_3)_2OH$ | $-SCH_2CH(CH_3)COOH$ |
| 843 | 6, 7-diF-Q | $-CH_2CH_2-$ | H | 9-$C(CH_3)_2OH$ | $-SCH_2CH(CH_2CH_3)COOH$ |
| 844 | 6, 7-diF-Q | $-CH_2CH_2-$ | H | 9-$C(CH_3)_2OH$ | $-SCH_2C(CH_3)_2COOH$ |
| 845 | 6, 7-diF-Q | $-CH_2CH_2-$ | H | 9-$C(CH_3)_2OH$ | $-SC(CH_3)_2CH_2COOH$ |
| 846 | 6, 7-diF-Q | $-CH_2CH_2-$ | H | 9-$C(CH_3)_2OH$ | $-SCH_2CH(CH_3)CH_2COOH$ |
| 847 | 6, 7-diF-Q | $-CH_2CH_2-$ | H | 9-$C(CH_3)_2OH$ | $-SCH_2C(CH_2CH_2)CH_2COOH$ |
| 848 | 6, 7-diF-Q | $-CH_2O-$ | H | H | $-SCH_2COOH$ |
| 849 | 6, 7-diF-Q | $-CH_2O-$ | H | H | $-SCH_2CH(CH_2CH_3)COOH$ |
| 850 | 6, 7-diF-Q | $-CH_2O-$ | H | H | $-SC(CH_3)_2CH_2COOH$ |
| 851 | 6, 7-diF-Q | $-CH_2O-$ | H | H | $-SCH_2CH(CH_3)CH_2COOH$ |
| 852 | 6, 7-diF-Q | $-CH_2O-$ | H | 7-F | $-SCH_2COOH$ |
| 853 | 6, 7-diF-Q | $-CH_2O-$ | H | 7-F | $-SCH_2CH_2COOH$ |
| 854 | 6, 7-diF-Q | $-CH_2O-$ | H | 7-F | $-SCH_2CH(CH_3)COOH$ |
| 855 | 6, 7-diF-Q | $-CH_2O-$ | H | 7-F | $-SCH_2CH(CH_2CH_3)COOH$ |
| 856 | 6, 7-diF-Q | $-CH_2O-$ | H | 7-F | $-SCH_2C(CH_3)_2COOH$ |
| 857 | 6, 7-diF-Q | $-CH_2O-$ | H | 7-F | $-SC(CH_3)_2CH_2COOH$ |
| 858 | 6, 7-diF-Q | $-CH_2O-$ | H | 7-F | $-SCH_2CH(CH_3)CH_2COOH$ |
| 859 | 6, 7-diF-Q | $-CH_2O-$ | H | 7-F | $-SCH_2C(CH_2CH_2)CH_2COOH$ |
| 860 | 6, 7-diF-Q | $-CH_2O-$ | H | 8-F | $-SCH_2COOH$ |
| 861 | 6, 7-diF-Q | $-CH_2O-$ | H | 8-F | $-SCH_2CH_2COOH$ |
| 862 | 6, 7-diF-Q | $-CH_2O-$ | H | 8-F | $-SCH_2CH(CH_3)COOH$ |
| 863 | 6, 7-diF-Q | $-CH_2O-$ | H | 8-F | $-SCH_2CH(CH_2CH_3)COOH$ |
| 864 | 6, 7-diF-Q | $-CH_2O-$ | H | 8-F | $-SCH_2C(CH_3)_2COOH$ |
| 865 | 6, 7-diF-Q | $-CH_2O-$ | H | 8-F | $-SC(CH_3)_2CH_2COOH$ |
| 866 | 6, 7-diF-Q | $-CH_2O-$ | H | 8-F | $-SCH_2CH(CH_3)CH_2COOH$ |
| 867 | 6, 7-diF-Q | $-CH_2O-$ | H | 8-F | $-SCH_2C(CH_2CH_2)CH_2COOH$ |
| 868 | 6, 7-diF-Q | $-CH_2O-$ | H | 9-F | $-SCH_2COOH$ |
| 869 | 6, 7-diF-Q | $-CH_2O-$ | H | 9-F | $-SCH_2CH_2COOH$ |
| 870 | 6, 7-diF-Q | $-CH_2O-$ | H | 9-F | $-SCH_2CH(CH_3)COOH$ |
| 871 | 6, 7-diF-Q | $-CH_2O-$ | H | 9-F | $-SCH_2CH(CH_2CH_3)COOH$ |
| 872 | 6, 7-diF-Q | $-CH_2O-$ | H | 9-F | $-SCH_2C(CH_3)_2COOH$ |
| 873 | 6, 7-diF-Q | $-CH_2O-$ | H | 9-F | $-SC(CH_3)_2CH_2COOH$ |
| 874 | 6, 7-diF-Q | $-CH_2O-$ | H | 9-F | $-SCH_2CH(CH_3)CH_2COOH$ |
| 875 | 6, 7-diF-Q | $-CH_2O-$ | H | 9-F | $-SCH_2C(CH_2CH_2)CH_2COOH$ |
| 876 | 6, 7-diF-Q | $-CH_2O-$ | H | 7-CN | $-SCH_2COOH$ |
| 877 | 6, 7-diF-Q | $-CH_2O-$ | H | 7-CN | $-SCH_2CH_2COOH$ |
| 878 | 6, 7-diF-Q | $-CH_2O-$ | H | 7-CN | $-SCH_2CH(CH_3)COOH$ |
| 879 | 6, 7-diF-Q | $-CH_2O-$ | H | 7-CN | $-SCH_2CH(CH_2CH_3)COOH$ |
| 880 | 6, 7-diF-Q | $-CH_2O-$ | H | 7-CN | $-SCH_2C(CH_3)_2COOH$ |
| 881 | 6, 7-diF-Q | $-CH_2O-$ | H | 7-CN | $-SC(CH_3)_2CH_2COOH$ |

| | | | | | |
|---|---|---|---|---|---|
| 882 | 6, 7-diF-Q | $-CH_2O-$ | H | 7-CN | $-SCH_2CH(CH_3)CH_2COOH$ |
| 883 | 6, 7-diF-Q | $-CH_2O-$ | H | 7-CN | $-SCH_2C(CH_2CH_2)CH_2COOH$ |
| 884 | 6, 7-diF-Q | $-CH_2O-$ | H | 8-CN | $-SCH_2COOH$ |
| 885 | 6, 7-diF-Q | $-CH_2O-$ | H | 8-CN | $-SCH_2CH_2COOH$ |
| 886 | 6, 7-diF-Q | $-CH_2O-$ | H | 8-CN | $-SCH_2CH(CH_3)COOH$ |
| 887 | 6, 7-diF-Q | $-CH_2O-$ | H | 8-CN | $-SCH_2CH(CH_2CH_3)COOH$ |
| 888 | 6, 7-diF-Q | $-CH_2O-$ | H | 8-CN | $-SCH_2C(CH_3)_2COOH$ |
| 889 | 6, 7-diF-Q | $-CH_2O-$ | H | 8-CN | $-SC(CH_3)_2CH_2COOH$ |
| 890 | 6, 7-diF-Q | $-CH_2O-$ | H | 8-CN | $-SCH_2CH(CH_3)CH_2COOH$ |
| 891 | 6, 7-diF-Q | $-CH_2O-$ | H | 8-CN | $-SCH_2C(CH_2CH_2)CH_2COOH$ |
| 892 | 6, 7-diF-Q | $-CH_2O-$ | H | 9-CN | $-SCH_2COOH$ |
| 893 | 6, 7-diF-Q | $-CH_2O-$ | H | 9-CN | $-SCH_2CH_2COOH$ |
| 894 | 6, 7-diF-Q | $-CH_2O-$ | H | 9-CN | $-SCH_2CH(CH_3)COOH$ |
| 895 | 6, 7-diF-Q | $-CH_2O-$ | H | 9-CN | $-SCH_2CH(CH_2CH_3)COOH$ |
| 896 | 6, 7-diF-Q | $-CH_2O-$ | H | 9-CN | $-SCH_2C(CH_3)_2COOH$ |
| 897 | 6, 7-diF-Q | $-CH_2O-$ | H | 9-CN | $-SC(CH_3)_2CH_2COOH$ |
| 898 | 6, 7-diF-Q | $-CH_2O-$ | H | 9-CN | $-SCH_2CH(CH_3)CH_2COOH$ |
| 899 | 6, 7-diF-Q | $-CH_2O-$ | H | 9-CN | $-SCH_2C(CH_2CH_2)CH_2COOH$ |
| 900 | 6, 7-diF-Q | $-CH_2O-$ | H | $7-CF_3$ | $-SCH_2COOH$ |
| 901 | 6, 7-diF-Q | $-CH_2O-$ | H | $7-CF_3$ | $-SCH_2CH_2COOH$ |
| 902 | 6, 7-diF-Q | $-CH_2O-$ | H | $7-CF_3$ | $-SCH_2CH(CH_3)COOH$ |
| 903 | 6, 7-diF-Q | $-CH_2O-$ | H | $7-CF_3$ | $-SCH_2CH(CH_2CH_3)COOH$ |
| 904 | 6, 7-diF-Q | $-CH_2O-$ | H | $7-CF_3$ | $-SCH_2C(CH_3)_2COOH$ |
| 905 | 6, 7-diF-Q | $-CH_2O-$ | H | $7-CF_3$ | $-SC(CH_3)_2CH_2COOH$ |
| 906 | 6, 7-diF-Q | $-CH_2O-$ | H | $7-CF_3$ | $-SCH_2CH(CH_3)CH_2COOH$ |
| 907 | 6, 7-diF-Q | $-CH_2O-$ | H | $7-CF_3$ | $-SCH_2C(CH_2CH_2)CH_2COOH$ |
| 908 | 6, 7-diF-Q | $-CH_2O-$ | H | $8-CF_3$ | $-SCH_2COOH$ |
| 909 | 6, 7-diF-Q | $-CH_2O-$ | H | $8-CF_3$ | $-SCH_2CH_2COOH$ |
| 910 | 6, 7-diF-Q | $-CH_2O-$ | H | $8-CF_3$ | $-SCH_2CH(CH_3)COOH$ |
| 911 | 6, 7-diF-Q | $-CH_2O-$ | H | $8-CF_3$ | $-SCH_2CH(CH_2CH_3)COOH$ |
| 912 | 6, 7-diF-Q | $-CH_2O-$ | H | $8-CF_3$ | $-SCH_2C(CH_3)_2COOH$ |
| 913 | 6, 7-diF-Q | $-CH_2O-$ | H | $8-CF_3$ | $-SC(CH_3)_2CH_2COOH$ |
| 914 | 6, 7-diF-Q | $-CH_2O-$ | H | $8-CF_3$ | $-SCH_2CH(CH_3)CH_2COOH$ |
| 915 | 6, 7-diF-Q | $-CH_2O-$ | H | $8-CF_3$ | $-SCH_2C(CH_2CH_2)CH_2COOH$ |
| 916 | 6, 7-diF-Q | $-CH_2O-$ | H | $9-CF_3$ | $-SCH_2COOH$ |
| 917 | 6, 7-diF-Q | $-CH_2O-$ | H | $9-CF_3$ | $-SCH_2CH_2COOH$ |
| 918 | 6, 7-diF-Q | $-CH_2O-$ | H | $9-CF_3$ | $-SCH_2CH(CH_3)COOH$ |
| 919 | 6, 7-diF-Q | $-CH_2O-$ | H | $9-CF_3$ | $-SCH_2CH(CH_2CH_3)COOH$ |
| 920 | 6, 7-diF-Q | $-CH_2O-$ | H | $9-CF_3$ | $-SCH_2C(CH_3)_2COOH$ |
| 921 | 6, 7-diF-Q | $-CH_2O-$ | H | $9-CF_3$ | $-SC(CH_3)_2CH_2COOH$ |
| 922 | 6, 7-diF-Q | $-CH_2O-$ | H | $9-CF_3$ | $-SCH_2CH(CH_3)CH_2COOH$ |
| 923 | 6, 7-diF-Q | $-CH_2O-$ | H | $9-CF_3$ | $-SCH_2C(CH_2CH_2)CH_2COOH$ |
| 924 | 6, 7-diF-Q | $-CH_2O-$ | H | $7-C\equiv CH$ | $-SCH_2COOH$ |
| 925 | 6, 7-diF-Q | $-CH_2O-$ | H | $7-C\equiv CH$ | $-SCH_2CH_2COOH$ |

| | | | | | |
|---|---|---|---|---|---|
| 926 | 6, 7-diF-Q | $-CH_2O-$ | H | 7-C≡CH | $-SCH_2CH(CH_3)COOH$ |
| 927 | 6, 7-diF-Q | $-CH_2O-$ | H | 7-C≡CH | $-SCH_2CH(CH_2CH_3)COOH$ |
| 928 | 6, 7-diF-Q | $-CH_2O-$ | H | 7-C≡CH | $-SCH_2C(CH_3)_2COOH$ |
| 929 | 6, 7-diF-Q | $-CH_2O-$ | H | 7-C≡CH | $-SC(CH_3)_2CH_2COOH$ |
| 930 | 6, 7-diF-Q | $-CH_2O-$ | H | 7-C≡CH | $-SCH_2CH(CH_3)CH_2COOH$ |
| 931 | 6, 7-diF-Q | $-CH_2O-$ | H | 7-C≡CH | $-SCH_2C(CH_2CH_2)CH_2COOH$ |
| 932 | 6, 7-diF-Q | $-CH_2O-$ | H | 8-C≡CH | $-SCH_2COOH$ |
| 933 | 6, 7-diF-Q | $-CH_2O-$ | H | 8-C≡CH | $-SCH_2CH_2COOH$ |
| 934 | 6, 7-diF-Q | $-CH_2O-$ | H | 8-C≡CH | $-SCH_2CH(CH_3)COOH$ |
| 935 | 6, 7-diF-Q | $-CH_2O-$ | H | 8-C≡CH | $-SCH_2CH(CH_2CH_3)COOH$ |
| 936 | 6, 7-diF-Q | $-CH_2O-$ | H | 8-C≡CH | $-SCH_2C(CH_3)_2COOH$ |
| 937 | 6, 7-diF-Q | $-CH_2O-$ | H | 8-C≡CH | $-SC(CH_3)_2CH_2COOH$ |
| 938 | 6, 7-diF-Q | $-CH_2O-$ | H | 8-C≡CH | $-SCH_2CH(CH_3)CH_2COOH$ |
| 939 | 6, 7-diF-Q | $-CH_2O-$ | H | 8-C≡CH | $-SCH_2C(CH_2CH_2)CH_2COOH$ |
| 940 | 6, 7-diF-Q | $-CH_2O-$ | H | 9-C≡CH | $-SCH_2COOH$ |
| 941 | 6, 7-diF-Q | $-CH_2O-$ | H | 9-C≡CH | $-SCH_2CH_2COOH$ |
| 942 | 6, 7-diF-Q | $-CH_2O-$ | H | 9-C≡CH | $-SCH_2CH(CH_3)COOH$ |
| 943 | 6, 7-diF-Q | $-CH_2O-$ | H | 9-C≡CH | $-SCH_2CH(CH_2CH_3)COOH$ |
| 944 | 6, 7-diF-Q | $-CH_2O-$ | H | 9-C≡CH | $-SCH_2C(CH_3)_2COOH$ |
| 945 | 6, 7-diF-Q | $-CH_2O-$ | H | 9-C≡CH | $-SC(CH_3)_2CH_2COOH$ |
| 946 | 6, 7-diF-Q | $-CH_2O-$ | H | 9-C≡CH | $-SCH_2CH(CH_3)CH_2COOH$ |
| 947 | 6, 7-diF-Q | $-CH_2O-$ | H | 9-C≡CH | $-SCH_2C(CH_2CH_2)CH_2COOH$ |
| 948 | 6, 7-diF-Q | $-CH_2O-$ | H | $7-C(CH_3)_2OH$ | $-SCH_2COOH$ |
| 949 | 6, 7-diF-Q | $-CH_2O-$ | H | $7-C(CH_3)_2OH$ | $-SCH_2CH_2COOH$ |
| 950 | 6, 7-diF-Q | $-CH_2O-$ | H | $7-C(CH_3)_2OH$ | $-SCH_2CH(CH_3)COOH$ |
| 951 | 6, 7-diF-Q | $-CH_2O-$ | H | $7-C(CH_3)_2OH$ | $-SCH_2CH(CH_2CH_3)COOH$ |
| 952 | 6, 7-diF-Q | $-CH_2O-$ | H | $7-C(CH_3)_2OH$ | $-SCH_2C(CH_3)_2COOH$ |
| 953 | 6, 7-diF-Q | $-CH_2O-$ | H | $7-C(CH_3)_2OH$ | $-SC(CH_3)_2CH_2COOH$ |
| 954 | 6, 7-diF-Q | $-CH_2O-$ | H | $7-C(CH_3)_2OH$ | $-SCH_2CH(CH_3)CH_2COOH$ |
| 955 | 6, 7-diF-Q | $-CH_2O-$ | H | $7-C(CH_3)_2OH$ | $-SCH_2C(CH_2CH_2)CH_2COOH$ |
| 956 | 6, 7-diF-Q | $-CH_2O-$ | H | $8-C(CH_3)_2OH$ | $-SCH_2COOH$ |
| 957 | 6, 7-diF-Q | $-CH_2O-$ | H | $8-C(CH_3)_2OH$ | $-SCH_2CH_2COOH$ |
| 958 | 6, 7-diF-Q | $-CH_2O-$ | H | $8-C(CH_3)_2OH$ | $-SCH_2CH(CH_3)COOH$ |
| 959 | 6, 7-diF-Q | $-CH_2O-$ | H | $8-C(CH_3)_2OH$ | $-SCH_2CH(CH_2CH_3)COOH$ |
| 960 | 6, 7-diF-Q | $-CH_2O-$ | H | $8-C(CH_3)_2OH$ | $-SCH_2C(CH_3)_2COOH$ |
| 961 | 6, 7-diF-Q | $-CH_2O-$ | H | $8-C(CH_3)_2OH$ | $-SC(CH_3)_2CH_2COOH$ |
| 962 | 6, 7-diF-Q | $-CH_2O-$ | H | $8-C(CH_3)_2OH$ | $-SCH_2CH(CH_3)CH_2COOH$ |
| 963 | 6, 7-diF-Q | $-CH_2O-$ | H | $8-C(CH_3)_2OH$ | $-SCH_2C(CH_2CH_2)CH_2COOH$ |
| 964 | 6, 7-diF-Q | $-CH_2O-$ | H | $9-C(CH_3)_2OH$ | $-SCH_2COOH$ |
| 965 | 6, 7-diF-Q | $-CH_2O-$ | H | $9-C(CH_3)_2OH$ | $-SCH_2CH_2COOH$ |
| 966 | 6, 7-diF-Q | $-CH_2O-$ | H | $9-C(CH_3)_2OH$ | $-SCH_2CH(CH_3)COOH$ |
| 967 | 6, 7-diF-Q | $-CH_2O-$ | H | $9-C(CH_3)_2OH$ | $-SCH_2CH(CH_2CH_3)COOH$ |
| 968 | 6, 7-diF-Q | $-CH_2O-$ | H | $9-C(CH_3)_2OH$ | $-SCH_2C(CH_3)_2COOH$ |
| 969 | 6, 7-diF-Q | $-CH_2O-$ | H | $9-C(CH_3)_2OH$ | $-SC(CH_3)_2CH_2COOH$ |

| | | | | | |
|---|---|---|---|---|---|
| 970 | 6, 7-diF-Q | $-CH_2O-$ | H | $9-C(CH_3)_2OH$ | $-SCH_2CH(CH_3)CH_2COOH$ |
| 971 | 6, 7-diF-Q | $-CH_2O-$ | H | $9-C(CH_3)_2OH$ | $-SCH_2C(CH_2CH_2)CH_2COOH$ |
| 972 | 6, 7-diCl-Q | $-OCH_2-$ | H | H | $-SCH_2COOH$ |
| 973 | 6, 7-diCl-Q | $-OCH_2-$ | H | H | $-SCH_2CH_2COOH$ |
| 974 | 6, 7-diCl-Q | $-OCH_2-$ | H | H | $-SCH_2CH(CH_3)COOH$ |
| 975 | 6, 7-diCl-Q | $-OCH_2-$ | H | H | $-SCH_2CH(CH_2CH_3)COOH$ |
| 976 | 6, 7-diCl-Q | $-OCH_2-$ | H | H | $-SCH_2C(CH_3)_2COOH$ |
| 977 | 6, 7-diCl-Q | $-OCH_2-$ | H | H | $-SC(CH_3)_2CH_2COOH$ |
| 978 | 6, 7-diCl-Q | $-OCH_2-$ | H | H | $-SCH_2CH(CH_3)CH_2COOH$ |
| 979 | 6, 7-diCl-Q | $-OCH_2-$ | H | H | $-SCH_2C(CH_2CH_2)CH_2COOH$ |
| 980 | 6-Cl, 7-F-Q | $-OCH_2-$ | H | H | $-SCH_2COOH$ |
| 981 | 6-Cl, 7-F-Q | $-OCH_2-$ | H | H | $-SCH_2CH_2COOH$ |
| 982 | 6-Cl, 7-F-Q | $-OCH_2-$ | H | H | $-SCH_2CH(CH_3)COOH$ |
| 983 | 6-Cl, 7-F-Q | $-OCH_2-$ | H | H | $-SCH_2CH(CH_2CH_3)COOH$ |
| 984 | 6-Cl, 7-F-Q | $-OCH_2-$ | H | H | $-SCH_2C(CH_3)_2COOH$ |
| 985 | 6-Cl, 7-F-Q | $-OCH_2-$ | H | H | $-SC(CH_3)_2CH_2COOH$ |
| 986 | 6-Cl, 7-F-Q | $-OCH_2-$ | H | H | $-SCH_2CH(CH_3)CH_2COOH$ |
| 987 | 6-Cl, 7-F-Q | $-OCH_2-$ | H | H | $-SCH_2C(CH_2CH_2)CH_2COOH$ |
| 988 | TQ | $-CH_2CH_2-$ | H | H | $-SCH_2COOH$ |
| 989 | TQ | $-CH_2CH_2-$ | H | H | $-SCH_2CH(CH_2CH_3)COOH$ |
| 990 | TQ | $-CH_2CH_2-$ | H | H | $-SC(CH_3)_2CH_2COOH$ |
| 991 | TQ | $-CH_2CH_2-$ | H | H | $-SCH_2CH(CH_3)CH_2COOH$ |
| 996 | TQ | $-CH_2CH_2-$ | H | 7-F | $-SCH_2COOH$ |
| 997 | TQ | $-CH_2CH_2-$ | H | 7-F | $-SCH_2CH_2COOH$ |
| 998 | TQ | $-CH_2CH_2-$ | H | 7-F | $-SCH_2CH(CH_3)COOH$ |
| 999 | TQ | $-CH_2CH_2-$ | H | 7-F | $-SCH_2CH(CH_2CH_3)COOH$ |
| 1000 | TQ | $-CH_2CH_2-$ | H | 7-F | $-SCH_2C(CH_3)_2COOH$ |
| 1001 | TQ | $-CH_2CH_2-$ | H | 7-F | $-SC(CH_3)_2CH_2COOH$ |
| 1002 | TQ | $-CH_2CH_2-$ | H | 7-F | $-SCH_2CH(CH_3)CH_2COOH$ |
| 1003 | TQ | $-CH_2CH_2-$ | H | 7-F | $-SCH_2C(CH_2CH_2)CH_2COOH$ |
| 1004 | TQ | $-CH_2CH_2-$ | H | 8-F | $-SCH_2COOH$ |
| 1005 | TQ | $-CH_2CH_2-$ | H | 8-F | $-SCH_2CH_2COOH$ |
| 1006 | TQ | $-CH_2CH_2-$ | H | 8-F | $-SCH_2CH(CH_3)COOH$ |
| 1007 | TQ | $-CH_2CH_2-$ | H | 8-F | $-SCH_2CH(CH_2CH_3)COOH$ |
| 1008 | TQ | $-CH_2CH_2-$ | H | 8-F | $-SCH_2C(CH_3)_2COOH$ |
| 1009 | TQ | $-CH_2CH_2-$ | H | 8-F | $-SC(CH_3)_2CH_2COOH$ |
| 1010 | TQ | $-CH_2CH_2-$ | H | 8-F | $-SCH_2CH(CH_3)CH_2COOH$ |
| 1011 | TQ | $-CH_2CH_2-$ | H | 8-F | $-SCH_2C(CH_2CH_2)CH_2COOH$ |
| 1012 | TQ | $-CH_2CH_2-$ | H | 9-F | $-SCH_2COOH$ |
| 1013 | TQ | $-CH_2CH_2-$ | H | 9-F | $-SCH_2CH_2COOH$ |
| 1014 | TQ | $-CH_2CH_2-$ | H | 9-F | $-SCH_2CH(CH_3)COOH$ |
| 1015 | TQ | $-CH_2CH_2-$ | H | 9-F | $-SCH_2CH(CH_2CH_3)COOH$ |
| 1016 | TQ | $-CH_2CH_2-$ | H | 9-F | $-SCH_2C(CH_3)_2COOH$ |
| 1017 | TQ | $-CH_2CH_2-$ | H | 9-F | $-SC(CH_3)_2CH_2COOH$ |
| 1018 | TQ | $-CH_2CH_2-$ | H | 9-F | $-SCH_2CH(CH_3)CH_2COOH$ |

| | | | | |
|---|---|---|---|---|
| 1019 TQ | $-CH_2CH_2-$ | H | 9-F | $-SCH_2C(CH_2CH_2)CH_2COOH$ |
| 1020 TQ | $-CH_2CH_2-$ | H | 7-CN | $-SCH_2COOH$ |
| 1021 TQ | $-CH_2CH_2-$ | H | 7-CN | $-SCH_2CH_2COOH$ |
| 1022 TQ | $-CH_2CH_2-$ | H | 7-CN | $-SCH_2CH(CH_3)COOH$ |
| 1023 TQ | $-CH_2CH_2-$ | H | 7-CN | $-SCH_2CH(CH_2CH_3)COOH$ |
| 1024 TQ | $-CH_2CH_2-$ | H | 7-CN | $-SCH_2C(CH_3)_2COOH$ |
| 1025 TQ | $-CH_2CH_2-$ | H | 7-CN | $-SC(CH_3)_2CH_2COOH$ |
| 1026 TQ | $-CH_2CH_2-$ | H | 7-CN | $-SCH_2CH(CH_3)CH_2COOH$ |
| 1027 TQ | $-CH_2CH_2-$ | H | 7-CN | $-SCH_2C(CH_2CH_2)CH_2COOH$ |
| 1028 TQ | $-CH_2CH_2-$ | H | 8-CN | $-SCH_2COOH$ |
| 1029 TQ | $-CH_2CH_2-$ | H | 8-CN | $-SCH_2CH_2COOH$ |
| 1030 TQ | $-CH_2CH_2-$ | H | 8-CN | $-SCH_2CH(CH_3)COOH$ |
| 1031 TQ | $-CH_2CH_2-$ | H | 8-CN | $-SCH_2CH(CH_2CH_3)COOH$ |
| 1032 TQ | $-CH_2CH_2-$ | H | 8-CN | $-SCH_2C(CH_3)_2COOH$ |
| 1033 TQ | $-CH_2CH_2-$ | H | 8-CN | $-SC(CH_3)_2CH_2COOH$ |
| 1034 TQ | $-CH_2CH_2-$ | H | 8-CN | $-SCH_2CH(CH_3)CH_2COOH$ |
| 1035 TQ | $-CH_2CH_2-$ | H | 8-CN | $-SCH_2C(CH_2CH_2)CH_2COOH$ |
| 1036 TQ | $-CH_2CH_2-$ | H | 9-CN | $-SCH_2COOH$ |
| 1037 TQ | $-CH_2CH_2-$ | H | 9-CN | $-SCH_2CH_2COOH$ |
| 1038 TQ | $-CH_2CH_2-$ | H | 9-CN | $-SCH_2CH(CH_3)COOH$ |
| 1039 TQ | $-CH_2CH_2-$ | H | 9-CN | $-SCH_2CH(CH_2CH_3)COOH$ |
| 1040 TQ | $-CH_2CH_2-$ | H | 9-CN | $-SCH_2C(CH_3)_2COOH$ |
| 1041 TQ | $-CH_2CH_2-$ | H | 9-CN | $-SC(CH_3)_2CH_2COOH$ |
| 1042 TQ | $-CH_2CH_2-$ | H | 9-CN | $-SCH_2CH(CH_3)CH_2COOH$ |
| 1043 TQ | $-CH_2CH_2-$ | H | 9-CN | $-SCH_2C(CH_2CH_2)CH_2COOH$ |
| 1044 TQ | $-CH_2CH_2-$ | H | $7-CF_3$ | $-SCH_2COOH$ |
| 1045 TQ | $-CH_2CH_2-$ | H | $7-CF_3$ | $-SCH_2CH_2COOH$ |
| 1046 TQ | $-CH_2CH_2-$ | H | $7-CF_3$ | $-SCH_2CH(CH_3)COOH$ |
| 1047 TQ | $-CH_2CH_2-$ | H | $7-CF_3$ | $-SCH_2CH(CH_2CH_3)COOH$ |
| 1048 TQ | $-CH_2CH_2-$ | H | $7-CF_3$ | $-SCH_2C(CH_3)_2COOH$ |
| 1049 TQ | $-CH_2CH_2-$ | H | $7-CF_3$ | $-SC(CH_3)_2CH_2COOH$ |
| 1050 TQ | $-CH_2CH_2-$ | H | $7-CF_3$ | $-SCH_2CH(CH_3)CH_2COOH$ |
| 1051 TQ | $-CH_2CH_2-$ | H | $7-CF_3$ | $-SCH_2C(CH_2CH_2)CH_2COOH$ |
| 1052 TQ | $-CH_2CH_2-$ | H | $8-CF_3$ | $-SCH_2COOH$ |
| 1053 TQ | $-CH_2CH_2-$ | H | $8-CF_3$ | $-SCH_2CH_2COOH$ |
| 1054 TQ | $-CH_2CH_2-$ | H | $8-CF_3$ | $-SCH_2CH(CH_3)COOH$ |
| 1055 TQ | $-CH_2CH_2-$ | H | $8-CF_3$ | $-SCH_2CH(CH_2CH_3)COOH$ |
| 1056 TQ | $-CH_2CH_2-$ | H | $8-CF_3$ | $-SCH_2C(CH_3)_2COOH$ |
| 1057 TQ | $-CH_2CH_2-$ | H | $8-CF_3$ | $-SC(CH_3)_2CH_2COOH$ |
| 1058 TQ | $-CH_2CH_2-$ | H | $8-CF_3$ | $-SCH_2CH(CH_3)CH_2COOH$ |
| 1059 TQ | $-CH_2CH_2-$ | H | $8-CF_3$ | $-SCH_2C(CH_2CH_2)CH_2COOH$ |
| 1060 TQ | $-CH_2CH_2-$ | H | $9-CF_3$ | $-SCH_2COOH$ |
| 1061 TQ | $-CH_2CH_2-$ | H | $9-CF_3$ | $-SCH_2CH_2COOH$ |
| 1062 TQ | $-CH_2CH_2-$ | H | $9-CF_3$ | $-SCH_2CH(CH_3)COOH$ |
| 1063 TQ | $-CH_2CH_2-$ | H | $9-CF_3$ | $-SCH_2CH(CH_2CH_3)COOH$ |

| | | | | |
|---|---|---|---|---|
| 1064 TQ | $-CH_2CH_2-$ | H | $9-CF_3$ | $-SCH_2C(CH_3)_2COOH$ |
| 1065 TQ | $-CH_2CH_2-$ | H | $9-CF_3$ | $-SC(CH_3)_2CH_2COOH$ |
| 1066 TQ | $-CH_2CH_2-$ | H | $9-CF_3$ | $-SCH_2CH(CH_3)CH_2COOH$ |
| 1067 TQ | $-CH_2CH_2-$ | H | $9-CF_3$ | $-SCH_2C(CH_2CH_2)CH_2COOH$ |
| 1068 TQ | $-CH_2CH_2-$ | H | $7-C\equiv CH$ | $-SCH_2COOH$ |
| 1069 TQ | $-CH_2CH_2-$ | H | $7-C\equiv CH$ | $-SCH_2CH_2COOH$ |
| 1070 TQ | $-CH_2CH_2-$ | H | $7-C\equiv CH$ | $-SCH_2CH(CH_3)COOH$ |
| 1071 TQ | $-CH_2CH_2-$ | H | $7-C\equiv CH$ | $-SCH_2CH(CH_2CH_3)COOH$ |
| 1072 TQ | $-CH_2CH_2-$ | H | $7-C\equiv CH$ | $-SCH_2C(CH_3)_2COOH$ |
| 1073 TQ | $-CH_2CH_2-$ | H | $7-C\equiv CH$ | $-SC(CH_3)_2CH_2COOH$ |
| 1074 TQ | $-CH_2CH_2-$ | H | $7-C\equiv CH$ | $-SCH_2CH(CH_3)CH_2COOH$ |
| 1075 TQ | $-CH_2CH_2-$ | H | $7-C\equiv CH$ | $-SCH_2C(CH_2CH_2)CH_2COOH$ |
| 1076 TQ | $-CH_2CH_2-$ | H | $8-C\equiv CH$ | $-SCH_2COOH$ |
| 1077 TQ | $-CH_2CH_2-$ | H | $8-C\equiv CH$ | $-SCH_2CH_2COOH$ |
| 1078 TQ | $-CH_2CH_2-$ | H | $8-C\equiv CH$ | $-SCH_2CH(CH_3)COOH$ |
| 1079 TQ | $-CH_2CH_2-$ | H | $8-C\equiv CH$ | $-SCH_2CH(CH_2CH_3)COOH$ |
| 1080 TQ | $-CH_2CH_2-$ | H | $8-C\equiv CH$ | $-SCH_2C(CH_3)_2COOH$ |
| 1081 TQ | $-CH_2CH_2-$ | H | $8-C\equiv CH$ | $-SC(CH_3)_2CH_2COOH$ |
| 1082 TQ | $-CH_2CH_2-$ | H | $8-C\equiv CH$ | $-SCH_2CH(CH_3)CH_2COOH$ |
| 1083 TQ | $-CH_2CH_2-$ | H | $8-C\equiv CH$ | $-SCH_2C(CH_2CH_2)CH_2COOH$ |
| 1084 TQ | $-CH_2CH_2-$ | H | $9-C\equiv CH$ | $-SCH_2COOH$ |
| 1085 TQ | $-CH_2CH_2-$ | H | $9-C\equiv CH$ | $-SCH_2CH_2COOH$ |
| 1086 TQ | $-CH_2CH_2-$ | H | $9-C\equiv CH$ | $-SCH_2CH(CH_3)COOH$ |
| 1087 TQ | $-CH_2CH_2-$ | H | $9-C\equiv CH$ | $-SCH_2CH(CH_2CH_3)COOH$ |
| 1088 TQ | $-CH_2CH_2-$ | H | $9-C\equiv CH$ | $-SCH_2C(CH_3)_2COOH$ |
| 1089 TQ | $-CH_2CH_2-$ | H | $9-C\equiv CH$ | $-SC(CH_3)_2CH_2COOH$ |
| 1090 TQ | $-CH_2CH_2-$ | H | $9-C\equiv CH$ | $-SCH_2CH(CH_3)CH_2COOH$ |
| 1091 TQ | $-CH_2CH_2-$ | H | $9-C\equiv CH$ | $-SCH_2C(CH_2CH_2)CH_2COOH$ |
| 1092 TQ | $-CH_2CH_2-$ | H | $7-C(CH_3)_2OH$ | $-SCH_2COOH$ |
| 1093 TQ | $-CH_2CH_2-$ | H | $7-C(CH_3)_2OH$ | $-SCH_2CH_2COOH$ |
| 1094 TQ | $-CH_2CH_2-$ | H | $7-C(CH_3)_2OH$ | $-SCH_2CH(CH_3)COOH$ |
| 1095 TQ | $-CH_2CH_2-$ | H | $7-C(CH_3)_2OH$ | $-SCH_2CH(CH_2CH_3)COOH$ |
| 1096 TQ | $-CH_2CH_2-$ | H | $7-C(CH_3)_2OH$ | $-SCH_2C(CH_3)_2COOH$ |
| 1097 TQ | $-CH_2CH_2-$ | H | $7-C(CH_3)_2OH$ | $-SC(CH_3)_2CH_2COOH$ |
| 1098 TQ | $-CH_2CH_2-$ | H | $7-C(CH_3)_2OH$ | $-SCH_2CH(CH_3)CH_2COOH$ |
| 1099 TQ | $-CH_2CH_2-$ | H | $7-C(CH_3)_2OH$ | $-SCH_2C(CH_2CH_2)CH_2COOH$ |
| 1100 TQ | $-CH_2CH_2-$ | H | $8-C(CH_3)_2OH$ | $-SCH_2COOH$ |
| 1101 TQ | $-CH_2CH_2-$ | H | $8-C(CH_3)_2OH$ | $-SCH_2CH_2COOH$ |
| 1102 TQ | $-CH_2CH_2-$ | H | $8-C(CH_3)_2OH$ | $-SCH_2CH(CH_3)COOH$ |
| 1103 TQ | $-CH_2CH_2-$ | H | $8-C(CH_3)_2OH$ | $-SCH_2CH(CH_2CH_3)COOH$ |
| 1104 TQ | $-CH_2CH_2-$ | H | $8-C(CH_3)_2OH$ | $-SCH_2C(CH_3)_2COOH$ |
| 1105 TQ | $-CH_2CH_2-$ | H | $8-C(CH_3)_2OH$ | $-SC(CH_3)_2CH_2COOH$ |
| 1106 TQ | $-CH_2CH_2-$ | H | $8-C(CH_3)_2OH$ | $-SCH_2CH(CH_3)CH_2COOH$ |

| | | | | |
|---|---|---|---|---|
| 1107 TQ | $-CH_2CH_2-$ | H | $8-C(CH_3)_2OH$ | $-SCH_2C(CH_2CH_2)CH_2COOH$ |
| 1108 TQ | $-CH_2CH_2-$ | H | $9-C(CH_3)_2OH$ | $-SCH_2COOH$ |
| 1109 TQ | $-CH_2CH_2-$ | H | $9-C(CH_3)_2OH$ | $-SCH_2CH_2COOH$ |
| 1110 TQ | $-CH_2CH_2-$ | H | $9-C(CH_3)_2OH$ | $-SCH_2CH(CH_3)COOH$ |
| 1111 TQ | $-CH_2CH_2-$ | H | $9-C(CH_3)_2OH$ | $-SCH_2CH(CH_2CH_3)COOH$ |
| 1112 TQ | $-CH_2CH_2-$ | H | $9-C(CH_3)_2OH$ | $-SCH_2C(CH_3)_2COOH$ |
| 1113 TQ | $-CH_2CH_2-$ | H | $9-C(CH_3)_2OH$ | $-SC(CH_3)_2CH_2COOH$ |
| 1114 TQ | $-CH_2CH_2-$ | H | $9-C(CH_3)_2OH$ | $-SCH_2CH(CH_3)CH_2COOH$ |
| 1115 TQ | $-CH_2CH_2-$ | H | $9-C(CH_3)_2OH$ | $-SCH_2C(CH_2CH_2)CH_2COOH$ |
| 1116 TQ | $-OCH_2-$ | H | $7-COOCH_3$ | $-SCH_2COOH$ |
| 1117 TQ | $-OCH_2-$ | H | $7-COOCH_3$ | $-SCH_2CH_2COOH$ |
| 1118 TQ | $-OCH_2-$ | H | $7-COOCH_3$ | $-SCH_2CH(CH_3)COOH$ |
| 1119 TQ | $-OCH_2-$ | H | $7-COOCH_3$ | $-SCH_2CH(CH_2CH_3)COOH$ |
| 1120 TQ | $-OCH_2-$ | H | $7-COOCH_3$ | $-SCH_2C(CH_3)_2COOH$ |
| 1121 TQ | $-OCH_2-$ | H | $7-COOCH_3$ | $-SC(CH_3)_2CH_2COOH$ |
| 1122 TQ | $-OCH_2-$ | H | $7-COOCH_3$ | $-SCH_2CH(CH_3)CH_2COOH$ |
| 1123 TQ | $-OCH_2-$ | H | $7-COOCH_3$ | $-SCH_2C(CH_2CH_2)CH_2COOH$ |
| 1124 TQ | $-OCH_2-$ | H | $8-COOCH_3$ | $-SCH_2COOH$ |
| 1125 TQ | $-OCH_2-$ | H | $8-COOCH_3$ | $-SCH_2CH_2COOH$ |
| 1126 TQ | $-OCH_2-$ | H | $8-COOCH_3$ | $-SCH_2CH(CH_3)COOH$ |
| 1127 TQ | $-OCH_2-$ | H | $8-COOCH_3$ | $-SCH_2CH(CH_2CH_3)COOH$ |
| 1128 TQ | $-OCH_2-$ | H | $8-COOCH_3$ | $-SCH_2C(CH_3)_2COOH$ |
| 1129 TQ | $-OCH_2-$ | H | $8-COOCH_3$ | $-SC(CH_3)_2CH_2COOH$ |
| 1130 TQ | $-OCH_2-$ | H | $8-COOCH_3$ | $-SCH_2CH(CH_3)CH_2COOH$ |
| 1131 TQ | $-OCH_2-$ | H | $8-COOCH_3$ | $-SCH_2C(CH_2CH_2)CH_2COOH$ |
| 1132 TQ | $-OCH_2-$ | H | $9-COOCH_3$ | $-SCH_2COOH$ |
| 1133 TQ | $-OCH_2-$ | H | $9-COOCH_3$ | $-SCH_2CH_2COOH$ |
| 1134 TQ | $-OCH_2-$ | H | $9-COOCH_3$ | $-SCH_2CH(CH_3)COOH$ |
| 1135 TQ | $-OCH_2-$ | H | $9-COOCH_3$ | $-SCH_2CH(CH_2CH_3)COOH$ |
| 1136 TQ | $-OCH_2-$ | H | $9-COOCH_3$ | $-SCH_2C(CH_3)_2COOH$ |
| 1137 TQ | $-OCH_2-$ | H | $9-COOCH_3$ | $-SC(CH_3)_2CH_2COOH$ |
| 1138 TQ | $-OCH_2-$ | H | $9-COOCH_3$ | $-SCH_2CH(CH_3)CH_2COOH$ |
| 1139 TQ | $-OCH_2-$ | H | $9-COOCH_3$ | $-SCH_2C(CH_2CH_2)CH_2COOH$ |

Incidentally, in the above Table 1, the abbreviations mean the following groups
t-Bu, t-butyl group; BO, 2-benzoxazolyl group; BT, 2-benzothiazolyl group; Tet, a 1H-tetrazol-5-yl group; Me, methyl group; i-Pr, isopropyl group; Ph, phenyl group; Py, 2-pyridyl group; Q, quinolin-2-yl group; T, 2-thiazolyl group; TQ, 5,6,7,8-tetrahydroquinolin-2-yl group.

In the above Table 1, the numerical value of the formula (I) shows a number of the position.

More preferred compounds may include Compounds Nos. 1, 2, 3, 4, 5, 6, 7, 8, 9, 10,11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 59, 60, 61, 62, 63, 64, 65, 66, 67, 68, 69, 70, 71, 72, 73, 74, 83, 84, 85, 86, 87, 88, 89, 90, 91, 92, 93, 94, 95, 96, 97, 98, 107, 108, 109, 110, 111, 112, 113, 114, 115, 116, 117, 1119, 120, 121, 122, 131, 132, 133, 134, 135, 136, 137, 138, 139, 140, 141, 142, 143, 144, 145, 146, 155, 156, 157, 158, 159, 160, 161, 162, 163, 164, 165, 166, 171, 172, 173, 174, 175, 176, 177, 178, 179, 180, 181, 182, 187, 188, 189, 190, 191, 192, 193, 194, 199, 200, 201, 202, 203, 204, 205, 206, 211, 212, 213, 214, 215, 216, 217, 218, 223, 224, 225, 226, 227, 228, 229, 230, 231, 232, 233, 234, 235, 236, 237, 238, 239, 240, 241, 242, 243, 244, 245, 246, 247, 248, 249, 250, 251, 260, 261, 262, 263,

264, 265, 266, 267, 268, 269, 270, 271, 272, 273, 272, 275, 284, 285, 286, 287, 288, 290, 291, 292, 293, 284, 295, 296, 297, 298, 299, 308, 309, 310, 311, 312, 313, 314, 315, 316, 317, 318, 319, 320, 321, 322, 323, 332, 333, 334, 335, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 384, 385, 386, 387, 388, 389, 390, 391, 392, 393, 394, 395, 396, 397, 398, 399, 404, 405, 406, 407, 412, 413, 414, 415, 416, 417, 418, 419, 420, 421, 422, 423, 424, 425, 426, 427, 436, 437, 438, 439, 440, 441, 442, 443, 444, 445, 446, 447, 448, 449, 450, 451, 460, 461, 462, 463, 464, 465, 466, 467, 468, 469, 470, 471, 472, 473, 474, 475, 484, 485, 486, 487, 488, 489, 490, 491, 492, 493, 494, 495, 496, 497, 498, 499, 508, 509, 510, 511, 512, 513, 514, 515, 516, 517, 518, 519, 520, 521, 522, 523, 532, 533, 534, 535, 536, 537, 538, 539, 540, 541, 542, 543, 544, 545, 546, 547, 548, 556,557, 558, 559, 560, 561, 562, 563, 564, 565, 566, 567, 568, 569, 570, 571, 588, 589, 590, 591, 592, 593, 594, 595, 596, 597, 598, 599, 600, 601, 602, 603, 612, 613, 614, 615, 616, 617, 618, 619, 620, 621, 622, 623, 624, 625, 626, 627, 636, 637, 638, 639, 640, 641, 642, 643, 644, 645, 646, 647, 648, 649, 650, 651, 660, 661, 662, 663, 664, 665, 666, 667, 668, 669, 670, 671, 672, 673, 674, 675, 684, 685, 686, 687, 688, 689, 690, 691, 692, 693, 694, 695, 696, 697, 698, 699, 708, 709, 710, 711, 712, 713, 714, 715, 716, 717, 718, 719, 720, 721, 722, 723, 724, 725, 726, 727, 728, 729, 730, 731, 732, 733, 734, 735, 736, 737, 738, 739, 740, 741, 742, 743, 752, 753, 754, 755, 756, 757, 758, 759, 760, 761, 762, 763, 764, 765, 766, 767, 776, 777, 778, 779, 780, 781, 782, 783, 784, 785, 786, 787, 788, 789, 790, 791, 800, 801, 802, 803, 804, 805, 806, 807, 808, 809, 810, 811, 812, 813, 814, 815, 824, 825, 826, 827, 828, 829, 830, 831, 832, 833, 834, 835, 836, 837, 838, 839, 848, 849, 850, 851, 852, 853, 854, 855, 856, 857, 858, 859, 860, 861, 862, 863, 864, 865, 866, 867, 876, 877, 878, 879, 880, 881, 882, 883, 884, 885, 886, 887, 888, 889, 890, 891, 900, 901, 902, 903, 904, 905, 906, 907, 908, 909, 910, 911, 912, 913, 914, 915, 924, 925, 926, 927, 928, 929, 930, 931, 932, 933, 934, 935, 936, 937, 938, 939, 948, 949, 950, 951, 952, 953, 954, 955, 956, 957, 958, 959, 960, 961, 962, 963, 972, 973, 974, 975, 976, 977, 978, 979, 980, 981, 982, 983, 984, 985, 986, 987, 988, 989, 990, 991, 992, 993, 994, 995, 996, 997, 998, 999, 1000, 1001, 1003, 1004, 1005, 1006, 1007, 1007, 1009, 1010, 1011, 1020, 1021, 1022, 1023, 1024, 1025, 1026, 1027, 1028, 1029, 1030, 1031, 1032, 1033, 1034, 1035, 1044, 1045, 1046, 1046, 1048; 1049, 1050, 1051, 1052, 1053, 1054, 1055, 1056, 1057, 1058, 1059, 1068, 1069, 1070, 1071, 1072, 1073, 1074, 1075, 1076, 1077, 1078, 1079, 1080, 1081, 1082, 1083, 1092, 1093, 1094, 1095, 1096, 1097, 1098, 1099, 1100, 1101, 1102, 1103, 1104, 1105, 1106 or 1107,

more preferably Compounds Nos. 1, 2, 3, 4, 5, 6, 7, 8, 9, 10, 11, 12, 13, 14, 15, 16, 19, 20, 21, 22, 23, 24, 25, 26, 27, 28, 29, 30, 31, 32, 35, 36, 37, 38, 39, 40, 41, 42, 43, 44, 45, 46, 47, 48, 49, 50, 59, 60, 61, 62, 63, 64, 65, 66, 83, 84, 85, 86, 87, 88, 89, 90, 91, 92, 93, 94, 95, 96, 97, 98, 107, 108, 109, 110,111, 112, 113, 114, 115, 116, 117, 1119, 120, 121, 122, 131, 132, 133, 134, 135, 136, 137, 138, 155, 156, 157, 158, 159, 160, 161, 162, 163, 164, 165, 166, 171, 172, 173, 174, 175, 176, 177, 178, 179, 180, 181, 182, 187, 188, 189, 190, 191, 192, 193, 194, 199, 200, 201, 202, 203, 204, 205, 206, 211, 212, 213, 214, 223, 224, 225, 226, 227, 228, 229, 230, 231, 232, 233, 234, 235, 236, 237, 238, 239, 240, 241, 242, 243, 244, 245, 246, 247, 248, 249, 250, 251, 260, 261, 262, 263, 264, 265, 266, 267, 268, 269, 270, 271, 272, 273, 272, 275, 284, 285, 286, 287, 288, 290, 291, 292, 293, 284, 295, 296, 297, 298, 299, 308, 309, 310, 311, 312, 313, 314, 315, 323, 332, 333, 334, 335, 352, 353, 354, 355, 384, 385, 386, 387, 388, 389, 390, 391, 392, 393, 394, 395, 396, 397, 398, 399, 404, 405, 406, 407, 412, 413, 414, 415, 416, 417, 418, 419, 436, 437, 438, 439, 440, 441, 442, 443, 460, 461, 462, 463, 464, 465, 466, 467, 484, 485, 486, 487, 488, 489, 490, 491, 508, 509, 510, 511, 512, 513, 514, 515, 532, 533, 534, 535, 536, 537, 538, 539, 556, 557, 558, 559, 560, 561, 562, 563, 588, 589, 590, 591, 592, 593, 594, 595, 596, 597, 598, 599, 600, 601, 602, 603, 612, 613, 614, 615, 616, 617, 618, 619, 620, 621, 622, 623, 624, 625, 626, 627, 684, 685, 686, 687, 688, 689, 690, 691, 692, 693, 694, 695, 696, 697, 698, 699, 708, 709, 710, 711, 712, 713, 714, 715, 716, 717, 718, 719, 720, 721, 722, 723, 724, 725, 726, 727, 728, 729, 730, 731, 732, 733, 734, 735, 736, 737, 738, 739, 740, 741, 742, 743, 752, 753, 754, 755, 756, 757, 758, 759, 776, 777, 778, 779, 780, 781, 782, 783, 784, 785, 786, 787, 788, 789, 790, 791, 800, 801, 802, 803, 804, 805, 806, 807, 808, 809, 810, 811, 812, 813, 814, 815, 824, 825, 826, 827, 828, 829, 830, 831, 848, 849, 850, 851, 852, 853, 854, 855, 856, 857, 858, 859, 860, 861, 862, 863, 864, 865, 866, 867, 876, 877, 878, 879, 880, 881, 882, 883, 900, 901, 902, 903, 904, 905, 906, 907, 908, 909, 910, 911, 912, 913, 914, 915, 924, 925, 926, 927, 928, 929, 930, 931, 932, 933, 934, 935, 936, 937, 938, 939, 948, 949, 950, 951, 952, 953, 954, 955, 972, 973, 974, 975, 976, 977, 978, 979, 980, 981, 982, 983, 984, 985, 986, 987, 988, 989, 990, 991, 992, 993, 994, 995, 996, 997, 998, 999, 1000, 1001, 1003, 1004, 1005, 1006, 1007, 1007, 1009, 1010, 1011, 1020, 1021, 1022, 1023, 1024, 1025, 1026, 1027, 1044, 1045, 1046, 1046, 1048, 1049, 1050, 1051, 1052, 1053, 1054, 1055, 1056, 1057, 1058, 1059, 1068, 1069, 1070, 1071, 1072, 1073, 1074, 1075, 1076, 1077, 1078, 1079, 1080, 1081, 1082, 1083, 1092, 1093, 1094, 1095, 1096, 1097, 1098 or 1099,

further more preferably Compounds Nos. 1, 2, 3, 4, 5, 6, 7, 8, 10,11, 12, 13, 14, 15, 16, 21, 22, 23, 26, 27, 28, 31, 32, 35, 36, 37, 38, 39, 40, 41, 42, 43, 44, 45, 46, 47, 48, 49, 50, 59, 60, 61, 62, 63, 64, 65, 66, 83, 84, 85, 86, 87, 88, 89, 90, 91, 92, 93, 94, 95, 96, 97, 98, 107, 108, 109, 110,111, 112, 113, 114, 115, 116, 117, 1119, 120, 121, 122, 155, 156, 157, 158, 159, 160, 161, 162, 163, 164, 165, 166, 171, 172, 173, 174, 175, 176, 177, 178, 179, 180, 181, 182, 187, 188, 189, 190, 191, 192, 193, 194, 199, 200, 201, 202, 203, 204, 205, 206, 223, 224, 225, 226, 227, 228,

229, 230, 231, 232, 233, 234, 235, 236, 237, 238, 239, 240, 241, 242, 243, 244, 245, 246, 247, 248, 249, 250, 251, 260, 261, 262, 263, 264, 265, 266, 267, 268, 269, 270, 271, 272, 273, 272, 275, 284, 285, 286, 287, 288, 290, 291, 292, 293, 284, 295, 296, 297, 298, 299, 323, 332, 333, 334, 335, 352, 353, 354, 355, 384, 385, 386, 387, 388, 389, 390, 391, 392, 393, 394, 395, 396, 397, 398, 399, 404, 405, 406, 407, 508, 509, 510, 511, 512, 513, 514, 515, 532, 533, 534, 535, 536, 537, 538, 539, 556, 557, 558, 559, 560, 561, 562, 563, 724, 725, 726, 727, 728, 729, 730, 731, 732, 733, 734, 735, 736, 737, 738, 739, 740, 741, 742, 743, 752, 753, 754, 755, 756, 757, 758, 759, 776, 777, 778, 779, 780, 781, 782, 783, 784, 785, 786, 787, 788, 789, 790, 791, 800, 801, 802, 803, 804, 805, 806, 807, 808, 809, 810, 811, 812, 813, 814, 815, 848, 856, 857, 858, 859, 860, 861, 862, 863, 864, 865, 866, 867, 876, 877, 878, 879, 880, 881, 882, 883, 900, 901, 902, 903, 904, 905, 906, 907, 908, 909, 910, 911, 912, 913, 914, 915, 924, 925, 926, 927, 928, 929, 930, 931, 932, 933, 934, 935, 936, 937, 938, 939, 948, 949, 950, 951, 952, 953, 954, 955, 972, 973, 974, 975, 976, 977, 978, 979, 980, 981, 982, 983, 984, 985, 986, 987, 988, 989, 990, 991, 992, 993, 994, 995, 996, 997, 998, 999, 1000, 1001, 1003, 1004, 1005, 1006, 1007, 1007, 1009, 1010,1011, 1020, 1021, 1022, 1023, 1024, 1025, 1026, 1027, 1044, 1045, 1046, 1046, 1048, 1049, 1050, 1051, 1052, 1053, 1054, 1055, 1056, 1057, 1058, 1059, 1068, 1069, 1070, 1071, 1072, 1073, 1074, 1075, 1076, 1077, 1078, 1079, 1080, 1081, 1082 or 1083,

particularly preferably

Compound No. 1;
[2-[(E)-2-(6,7-difluoro-2-quinolinyl)ethenyl]-6,11-dihydrodibenz[b,e]oxepin-11-yl]oxyacetic acid,
Compound No. 5;
[2-[(E)-2-(6,7-difluoro-2-quinolinyl)ethenyl]-6,11-dihydrodibenz[b,e]oxepin-11-yl] thioacetic acid,
Compound No. 6;
3-{[2-[(E)-2-(6,7-difluoro-2-quinolinyl)ethenyl]-6,11-dihydrodibenz[b,e]oxepin-11-yl]thio}propionic acid,
Compound No. 7;
3-{[2-[(E)-2-(6,7-difluoro-2-quinolinyl)ethenyl]-6,11-dihydrodibenz[b,e]oxepin-11-yl]thio}-2-methylpropionic acid,
Compound No. 8;
3-{[2-[(E)-2-(6,7-difluoro-2-quinolinyl)ethenyl]-6,11-dihydrodibenz[b,e]oxepin-11-yl]thio}-2,2-dimethylpropionic acid,
Compound No. 10;
3-{[2-[(E)-2-(6,7-difluoro-2-quinolinyl)ethenyl]-6,11-dihydrodibenz[b,e]oxepin-11-yl]thio}-2-ethylpropionic acid,
Compound No. 12;
3-{[2-[(E)-2-(6,7-difluoro-2-quinolinyl)ethenyl]-6,11-dihydrodibenz[b,e]oxepin-11-yl]thio}-3,3-dimethylpropionic acid,
Compound No. 14;
{1-[[2-[(E)-2-(6,7-difluoro-2-quinolinyl)ethenyl]-6,11-dihydrodibenz[b,e]oxepin-11-yl]thiomethyl]cyclopropyl}acetic acid,
Compound No. 16;
2-{[2-[(E)-2-(6,7-difluoro-2-quinolinyl)ethenyl]-6,11-dihydrodibenz[b,e]oxepin-11-yl]thio}benzoic acid
Compound No. 22;
[2-[(E)-2-(6,7-difluoro-2-quinolinyl)ethenyl]-6,11-dihydrodibenz[b,e]oxepin-11-yl]thio-N-methanesulfonylacetic amide
Compound No. 27;
3-[2-[(E)-2-(6,7-difluoro-2-quinolinyl)ethenyl]-6,11-dihydrodibenz[b,e]oxepin-11-yl]thio-N-methanesulfonylpropiona mide
Compound No. 31;
2-{[2-[(E)-2-(6,7-difluoro-2-quinolinyl)ethenyl]-6,11-dihydrodibenz[b,e]oxepin-11-yl]thio}ethanesulfonic acid
Compound No. 32;
4-[2-[(E)-2-(6,7-difluoro-2-quinolinyl)ethenyl]-6,11-dihydrodibenz[b,e]oxepin-11-yl]butanoic acid
Compound No. 36;
3-{[2-[(E)-2-(6,7-difluoro-2-quinolinyl)ethenyl]-7-fluoro-6,11-dihydrodibenz[b,e]oxepin-11-yl]thio}propionic acid,
Compound No. 42;
{1-[[2-[(E)-2-(6,7-difluoro-2-quinolinyl)ethenyl]-7-fluoro-6,11-dihydrodibenz[b,e]oxepin-11-yl]thiomethyl]cyclopropyl} acetic acid,
Compound No. 44;
3-{[2-[(E)-2-(6,7-difluoro-2-quinolinyl)ethenyl]-8-fluoro-6,11-dihydrodibenz[b,e]oxepin-11-yl]thio}propionic acid,
Compound No. 60;
3-{[7-cyano-2-[(E)-2-(6,7-difluoro-2-quinolinyl)ethenyl]-6,11-dihydrodibenz[b,e]oxepin-11-yl]thio}propionic acid,
Compound No. 84;
3-{(2-[(E)-2-(6,7-difluoro-2-quinolinyl)ethenyl]-7-trifluoromethyl-6,11-dihydrodibenz[b,e]oxepin-11-yl]thio}propionic acid,
Compound No. 108;
3-{[7-ethynyl-2-[(E)-2-(6,7-difluoro-2-quinolinyl)ethenyl]-6,11-dihydrodibenz[b,e]oxepin-11-yl]thio}propionic acid,
Compound No. 155;
3-{[2-[(E)-2-(7-chloro-6-fluoro-2-quinolinyl)ethenyl]-6,11-dihydrodibenz[b,e]oxepin-11-yl]thio}propionic acid,
Compound No. 159;
3-{[2-[(E)-2-(7-chloro-6-fluoro-2-quinolinyl)ethenyl]-7-fluoro-6,11-dihydrodibenz[b,e]oxepin-11-yl]thio}propionic acid,
Compound No. 162;
{1-[[2-[(E)-2-(7-chloro-6-fluoro-2-quinolinyl)ethenyl]-7-fluoro-6,11-dihydrodibenz[b,e]oxepin-11-yl]thiomethyl]cyclopropyl}acetic acid,
Compound No. 171;
3-{[2-[(E)-2-(7-fluoro-2-quinolinyl)ethenyl]-6,11-dihydrodibenz[b,e]oxepin-11-yl]thio}propionic acid,
Compound No. 172;
3-{[2-[(E)-2-(7-fluoro-2-quinolinyl)ethenyl]-6,11-dihydrodibenz[b,e]oxepin-11-yl]thio}-2-methylpropionic acid,
Compound No. 174;
{1-[[2-[(E)-2-(7-chloro-6-fluoro-2-quinolinyl)ethenyl]-6,11-dihydrodibenz[b,e]oxepin-11-yl]thiomethyl]cyclopropyl}acetic acid,
Compound No. 223;
{2-[(E)-2-(7-chloro-2-quinolinyl)ethenyl]-6,11-dihydrodibenz[b,e]oxepin-11-yl}thioacetic acid,
Compound No. 224;
3-{[2-[(E)-2-(7-chloro-2-quinolinyl)ethenyl]-6,11-dihydrodibenz[b,e]oxepin-11-yl]thio}propionic acid,
Compound No. 225;
3-{[2-[(E)-2-(7-chloro-2-quinolinyl)ethenyl]-6,11-dihydrodibenz[b,e]oxepin-11-yl]thio}-2-methylpropionic acid,
Compound No. 227;
{1-[[2-[(E)-2-(7-chloro-2-quinolinyl)ethenyl]-6,11-dihydrodibenz[b,e]oxepin-11-yl]thiomethyl]cyclopropyl}acetic acid,
Compound No. 229;
3-{[2-[(E)-2-(5,6,7,8-tetrahydro-2-quinolinyl)ethenyl]-6,11-dihydrodibenz[b,e]oxepin-11-yl]thio}propionic acid,
Compound No. 230;
3-{[2-[(E)-2-(5,6,7,8-tetrahydro-2-quinolinyl)ethenyl]-6,11-dihydrodibenz[b,e]oxepin-11-yl]thio}-2-methylpropionic acid,
Compound No. 235;
{1-([2-[(E)-2-(5,6,7,8-tetrahydro-2-quinolinyl)ethenyl]-6,11-dihydrodibenz[b,e]oxepin-11-yl]thiomethyl]cyclopropyl}acetic acid,
Compound No. 352;
3-{[2-[(E)-2-(6-fluoro-7-trifluoromethyl-2-quinolinyl)ethenyl]-6,11-dihydrodibenz[b,e]oxepin-11-yl]thio}propionic acid,
Compound No. 384;
3-{[3-[(E)-2-(6,7-difluoro-2-quinolinyl)ethenyl]-10,11-dihydro-5H-dibenz[a,d]cyclohepten-5-yl]thio}propionic acid,
Compound No. 385;
3-{[3-[(E)-2-(6,7-difluoro-2-quinolinyl)ethenyl]-10,11-dihydro-5H-dibenz[a,d]cyclohepten-5-yl]thio}-2-methylpropionic acid, or
Compound No. 404;
3-{[9-[(E)-2-(6,7-difluoro-2-quinolinyl)ethenyl]-6,11-dihydrodibenz[b,e]oxepin-11-yl]thio}propionic acid.

The compound represented by the formula (I) of the present invention can be produced, for example, by Preparation process A, B, C, D, E, F, G or M shown below.

Preparation process A

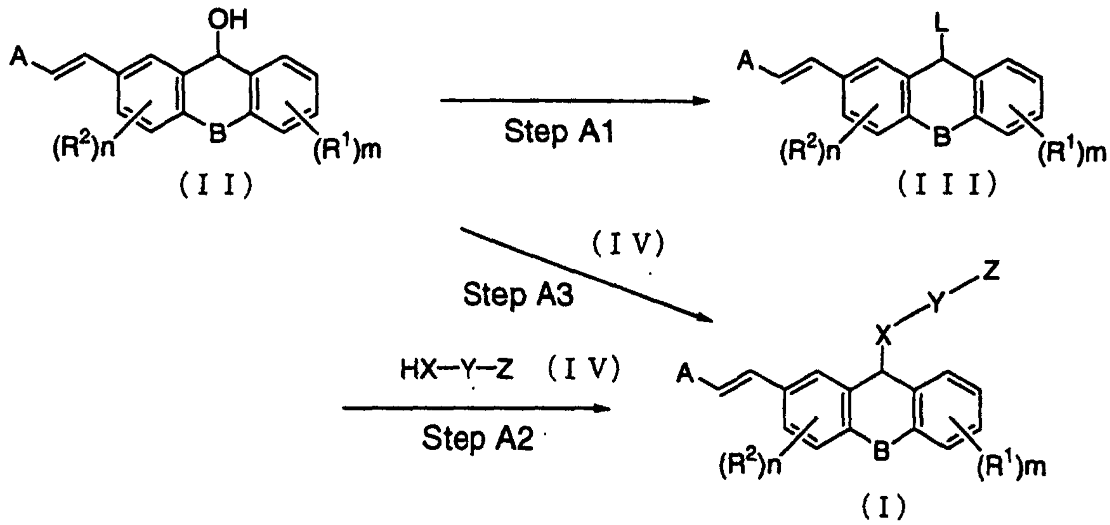


Preparation process B

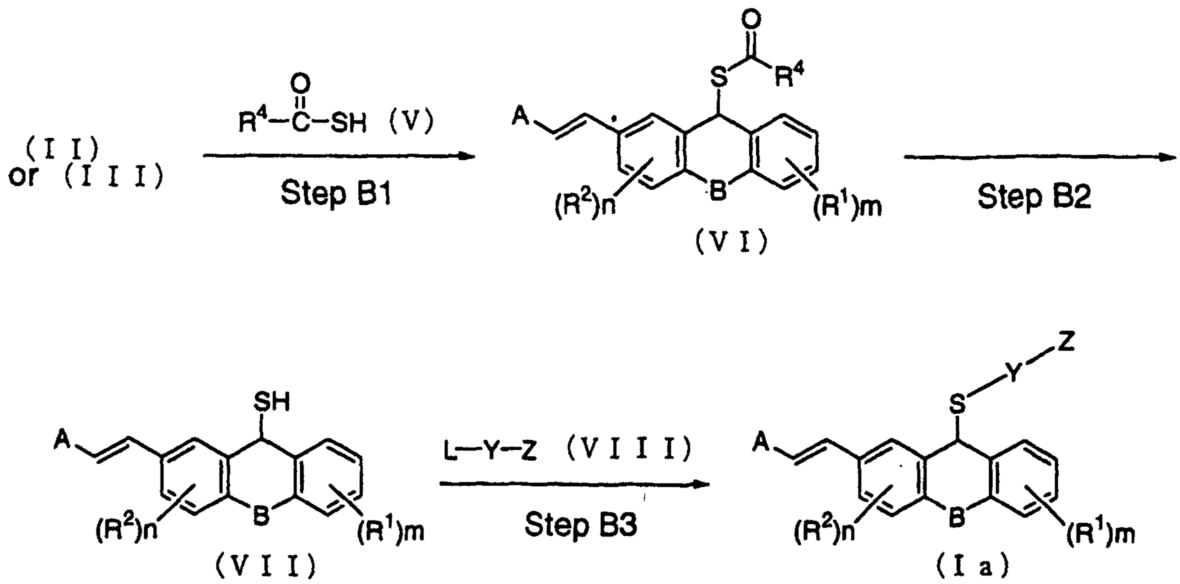

Preparation process C

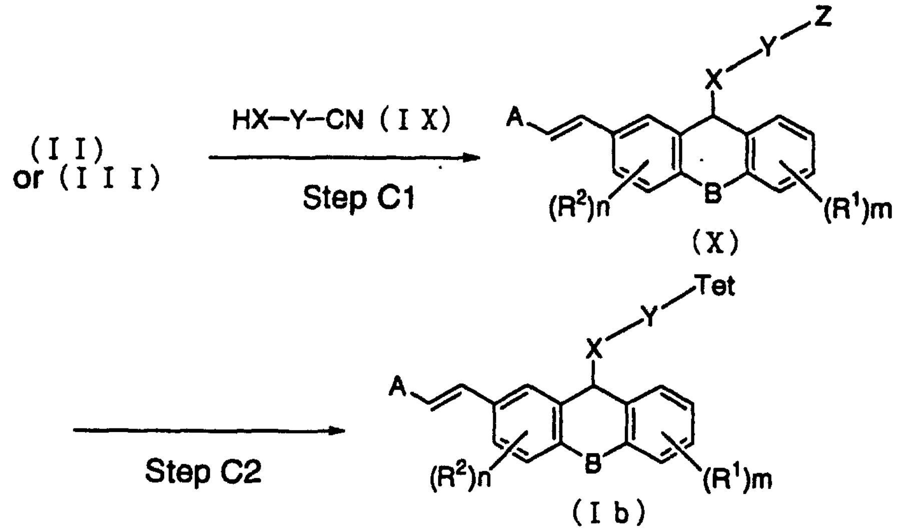


Preparation process D

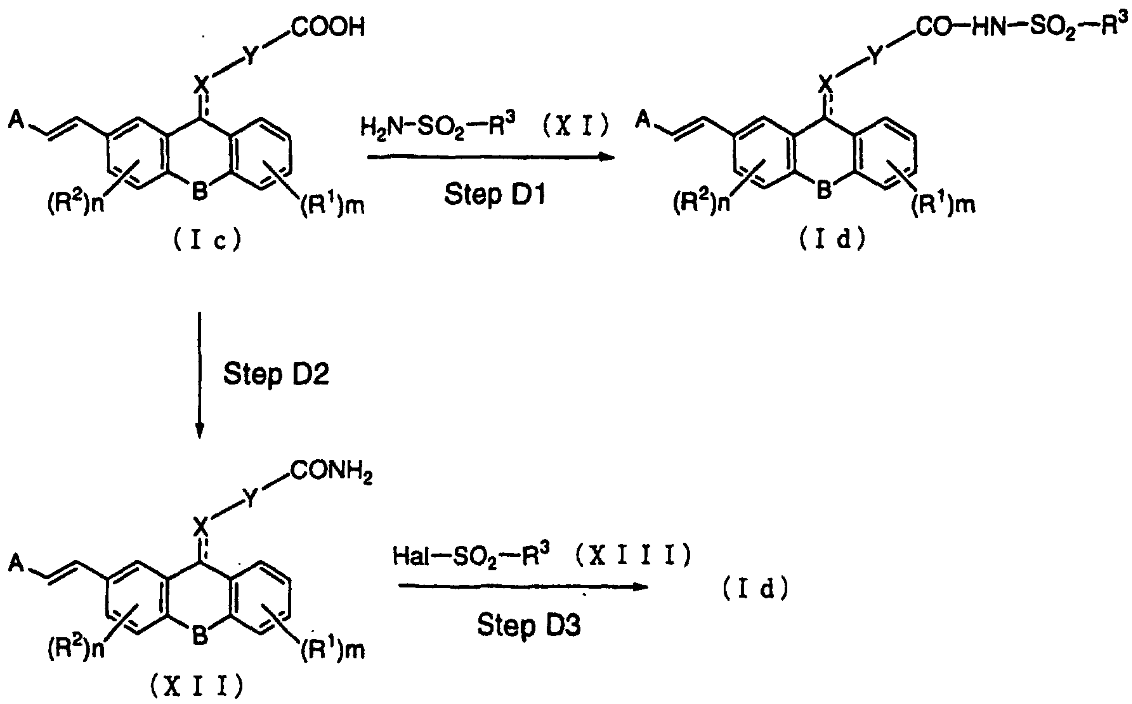

Preparation process E

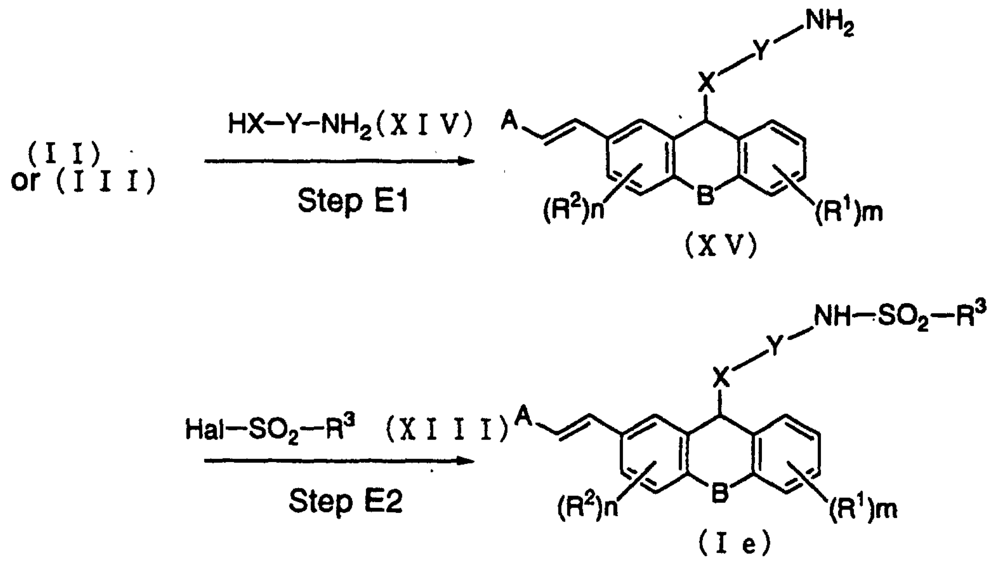


(II) or (III)

(XV)

(Ie)

Preparation process F

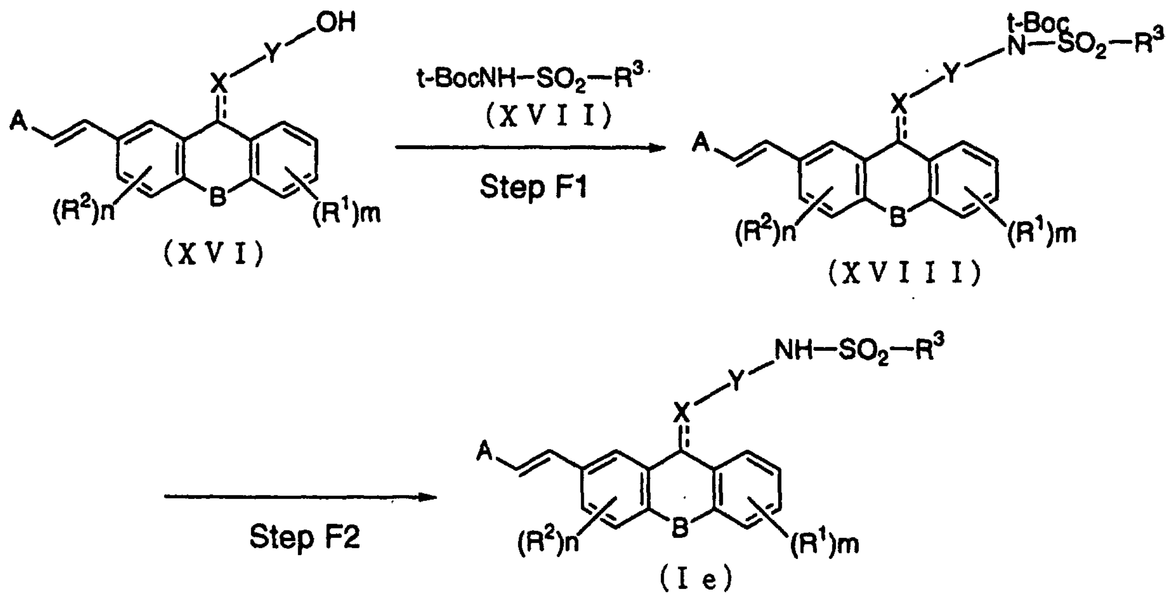


(XVI)

(XVIII)

(Ie)

Preparation process G

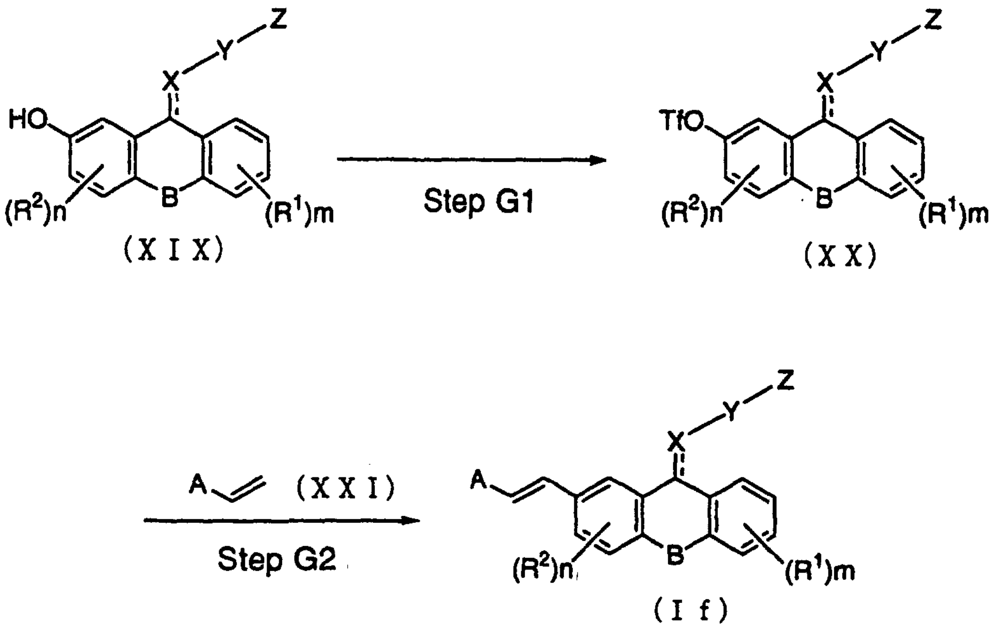


(XIX) (XX) (If)

Preparation process M

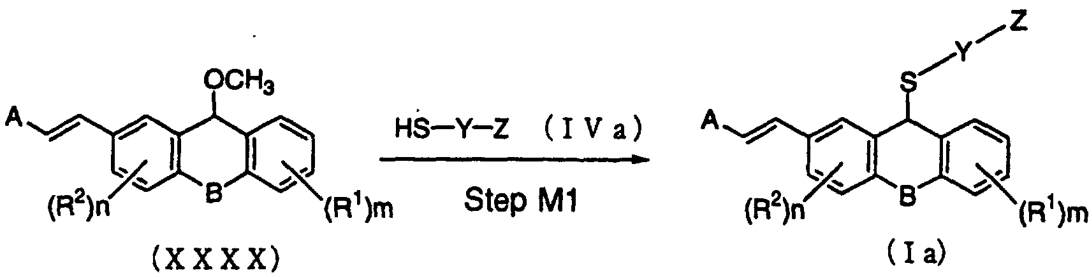


(XXXX) (Ia)

In the chemical formulae described in the above-mentioned preparation processes, $R^1$, $R^2$, $R^3$, A, B, X, Y, Z, m and n have the same meanings as defined above, L represents a halogen atom, a $C_1$-$C_4$ alkylsulfonyloxy group, a fluoro $C_1$-$C_4$ alkylsulfonyloxy group or a phenylsulfonyloxy group which may be substituted (said a substituent(s) is a $C_1$-$C_4$ alkyl group or a halogen atom), $R^4$ represents a $C_1$-$C_4$ alkyl group or a phenyl group which may be substituted (said a substituent(s) is a $C_1$-$C_4$ alkyl group or a halogen atom), Tet means a 1H-tetrazol-5-yl group, Hal means a halogen atom, and t-Boc means a t-butoxycarbonyl group.

Preparation process A is a preparation process of Compound (I).

Step A1 of Preparation process A is a step of synthesizing Compound (III) by halogenating or sulfonylating Compound (II).

Halogenation of Compound (II) can be carried out by reacting Compound (II) and a halogenating agent in a solvent or without solvent (preferably in a solvent).

The solvent to be used is not particularly limited so long as it has no adverse effect and dissolves starting materials with some extends, and there may be mentioned, for example, halogenated hydrocarbons such as methylene chloride, chloroform, carbon tetrachloride and dichloroethane; aromatic hydrocarbons such as benzene and toluene; or aliphatic hydrocarbons such as heptane, hexane and cyclohexane, preferably halogenated hydrocarbons.

As the halogenating agent, there may be mentioned, for example, thionyl chloride, thionyl bromide, phosphorus oxychloride, phosphorus oxybromide or phosphorus pentachloride, etc., preferably thionyl chloride or phosphorus oxychloride. An amount of the halogenating agent to be used is usually in an amount of 1 to 10-fold mole, preferably 1 to 2-fold mole based on Compound (II).

The reaction is usually carried out in the range of -20 to 100°C, preferably -10 to 30°C. The reaction time may vary depending on the reaction temperature and others, and it is usually for 5 minutes to 10 hours, preferably 10

minutes to 5 hours.

Sulfonylation of Compound (II) can be carried out byreacting Compound (II) and a sulfonylating agent in the presence of a base in a solvent.

The solvent to be used is not particularly limited so long as it has no adverse effect and dissolves starting materials with some extends, and there may be mentioned, for example, the same solvent to those used in the above-mentioned halogenating reaction (for example, halogenated hydrocarbons, aromatic hydrocarbons or aliphatic hydrocarbons ), preferably halogenated hydrocarbons.

As the sulfonylating agent, there may be mentioned, for example, methanesulfonyl chloride, trifluoromethanesulfonyl chloride, methanesulfonic anhydride, trifluoromethanesulfonic anhydride, benzenesulfonyl chloride, toluenesulfonylchloride, benzenesulfonyl bromide or toluenesulfonyl bromide, etc., preferably methanesulfonyl chloride, benzenesulfonyl chloride or toluenesulfonyl chloride. An amount of the sulfonylating agent to be used is usually in an amount of 1 to 10-fold mole, preferably 1 to 3-fold mole based on Compound (II).

As the base, there may be mentioned, for example, amines such as triethylamine, tributylamine, diisopropylethylamine, pyridine, picoline, lutidine and 4-dimethylaminopyridine, preferably triethylamine, diisopropylethylamine or pyridine.

An amount of the base to be used is usually in an amount of 1 to 10-fold mole, preferably 1 to 2-fold mole based on the sulfonylating agent.

The reaction is usually carried out in the range of -10 to 100°C, preferably 0 to 30°C. The reaction time may vary depending on the reaction temperature and others, and it is usually for 5 minutes to 10 hours, preferably 30 minutes to 5 hours.

Incidentally, Compound (III) can be separated and purified from the reaction mixture by the usual method, and a crude product obtained by concentrating the reaction mixture can be used as such to the next step.

Step A2 can be carried out by reacting Compound (III) and Compound (IV) in the presence of a base in a solvent.

An amount of Compound (IV) to be used is usually in an amount of 1 to 10-fold mole, preferably 1 to 5-fold mole based on Compound (III).

The solvent to be used is not particularly limited so long as it has no adverse effect and dissolves starting materials with some extends, and there may be mentioned, for example, aprotic polar solvents such as N,N-dimethylformamide, dimethylsulfoxide, N,N-dimethylacetamide and hexamethylphosphoric triamide; halogenated hydrocarbons such as methylene chloride, chloroform and dichloroethane; ketones such as acetone, methyl ethyl ketone and methyl isobutyl ketone; nitriles such as acetonitrile; esters such as ethyl acetate; ethers such as diethyl ether , diisopropylether, tetrahydrofuranordioxane; or a mixed solvent of the above solvents, preferably halogenated hydrocarbons, aprotic polar solvents, ethers or a mixed solvent of the above solvents.

As the base to be used, there may be mentioned, for example, alkali metal hydrides such as sodium hydroxide or lithium hydroxide; alkali metal amides such as sodium amide, etc.; amines such as triethylamine, tributylamine, diisopropylethylamine, pyridine, picoline, lutidine or 4-dimethylaminopyridine, etc.; or alkali metal carbonates such as sodium carbonate, potassium carbonate and sodium hydrogen carbonate, preferably amines or alkali metal hydrides. An amount of the base to be used may vary depending on the kind of the starting compounds, and it is usually in an amount of 1 to 10-fold mole, preferably 1 to 5-fold mole based on Compound (IV).

The reaction is usually carried out in the range of -50 to 150°C, preferably -10 to 100°C. The reaction time may vary depending on the reaction temperature and others, and it is usually for 5 minutes to 10 hours, preferably 30 minutes to 5 hours.

Step A3 is a step to obtain Compound (I) in another method, in particular, it is suitably employed when X is a sulfur atom. This step is carried out by reacting Compound (II) and Compound (IV) in the presence of an acid catalyst in a solvent.

An amount of Compound (IV) to be used is usually in an amount of 1 to 5-fold mole, preferably 1 to 2-fold mole based on Compound (II).

The solvent to be used is not particularly limited so long as it has no adverse effect on the reaction and dissolves starting materials with some extends, and there may be mentioned, for example, halogenated hydrocarbons such as methylene chloride, chloroform and dichloroethane; alcohols such as methanol, ethanol, propanol, isopropanol and butanol; aprotic polar solvents such as N,N-dimethylformamide, dimethylsulfoxide, N,N-dimethylacetamide and hexamethylphosphoric triamide; or ethers such as diethyl ether, diisopropyl ether, tetrahydrofuran and dioxane, preferably halogenated hydrocarbons.

As the acid catalyst to be used, there may be mentioned, for example, mineral acids such as hydrochloric acid, sulfuric acid and phosphoric acid; organic acids such as methanesulfonic acid and trifluoroacetic acid; Lewis acids such as boron trifluoride-diethyl ether complex, zinc chloride, tin tetrachloride and aluminum chloride, preferably organic acids or boron trifluoride-diethyl ether complex.

An amount of the catalyst to be used is usually in an amount of 0.1 to 50-fold mole, preferably 1 to 10-fold

mole based on Compound (II), and when organic acids are used, it may be used in a largely excess amount served as a solvent.

The reaction is usually carried out in the range of -10 to 100°C, preferably 0 to 30°C. The reaction time may vary depending on the reaction temperature and others, and it is usually for 5 minutes to 10 hours, preferably 10 minutes to 5 hours.

Incidentally, in Compound (I), the compound wherein Z is a carboxyl group (Compound Ic mentioned below) is directly produced by using Compound (IV) wherein Z is a carboxyl group, or can be synthesized by firstly leading to Compound (I) where Z is a protected carboxyl group using Compound (IV) where Z is a protected carboxyl group (said protective group is preferably $C_1$-$C_4$ alkyl group) and then by hydrolyzing said protective group under acidic or alkaline conditions according to the conventional method.

Also, in Compound (I), a desired protective group can be easily introduced into Compound (Ic) wherein Z is a carboxyl group according to the conventional method. (for example, written by W.Greene and P.G.H.Wult "Protective Group in Organic Synthesis", 2nd Ed., John Wiley & Sons, see page 224)

Preparation process B is a preparation processs of Compound (Ia).

A method of obtaining Compound (VI) from Compound (II) or Compound (III) and thiocarboxylic acid (V) in Step B1 can be carried out in the same manner as described in the Step A2 or Step A3 of the above-mentioned Preparation process A except for using thiocarboxylic acid (V) in place of Compound (IV).

In Step B2, Compound (VII) can be synthesized by hydrolyzing Compound (VI) under alkaline conditions according to the conventional method.

Step B3 is carried out by reacting Compound (VI) and Compound (VIII) in the presence of a base in a solvent. The present reaction is carried out in the same manner as in the above-mentioned Step A2 except for using Compound (VII) in place of Compound (II), and using Compound (VIII) in place of Compound (IV).

An amount of Compound (VIII) to be used is usually in an amount of 1 to 10-fold mole, preferably 1 to 5-fold mole based on Compound (VII)

Preparation processs C is a preparation processs of Compound (Ib), and in Step C1, the reaction of obtaining Compound (X) from Compound (II) or Compound (III) and Compound (IX) is carried out under the same reaction conditions as those of Preparation process A except for using Compound (IX) in place of Compound (IV).

In Step C2, Compound (Ib) can be synthesized by reacting Compound (X) and an azide compound in a solvent.

As the azide compound to be used, there may be mentioned, for example, alkali metal azides such as sodium azide, potassium azide and lithium azide; alkaline earth metal azides such as calcium azide and magnesium azide; or organic tin azides such as trimethyl tin azide, tributyl tin azide and triphenyl tin azide. An amount of the azide compound to be used is usually in an amount of 1 to 10-fold mole, preferably 1 to 5-fold mole based on Compound (X). In said reaction, the azide compound can be used singly, or may be used in combination with, for example, Lewis acids such as aluminum chloride, stannic chloride, zinc chloride, titanium chloride and boron trifluoride-diethyl ether complex; ammonium salts such as ammonium chloride and tetramethyl ammonium chloride; sulfonic acids such as methanesulfonic acid and ethanesulfonic acid; alkali metal chlorides such as lithium chloride, and others; or amine salts such as triethylamine hydrochloride, and others.

The solvent to be used is not particularly limited so long as it has no adverse effect on the reaction and dissolves starting materials with some extends, and there may be mentioned, for example, aprotic polar solvents such as N,N-dimethylformamide, dimethylsulfoxide, N-methylpyrrolidone and N,N-dimethylacetamide; ethers such as diethyl ether, tetrahydrofuran, dimethoxyethane, diethoxyethane and dioxane; aromatic hydrocarbons such as benzene, toluene and xylene; or aliphatic hydrocarbons such as hexane and petroleum ether.

The reaction is usually carried out in the range of 0 to 200°C, preferably 50 to 150°C. The reaction time may vary depending on the reaction temperature and others, and it is usually for 1 hour to 72 hours, preferably 3 hours to 48 hours.

Preparation process D is a preparation processs of Compound (Id), and it is carried out by a method (Step D1a) in which Compound (Ic) and Compound (XI) are reacted in the presence of a condensing agent, or a method (Step D1c) in which Compound (Ic) is once led to its reactive derivative (Step D1b), then, the reactive derivative and Compound (XI) are reacted in the presence of a base.

As the condensing agent to be used in Step D1a, there may be mentioned, for example, N,N'-dicyclohexylcarbodiimide (DCC), 1-ethyl-3-(3-dimethylaminopropyl)carbodiimide hydrochloride (EDC), N,N'-carbonyldiimidazole (CDI), diphenylphosphoryl azide, benztriazol-1-yloxy-tris-(dimethyamino)phosphonium hexafluorophosphate (BOP), benzotriazol-1-yloxy-tris-pyrrolidinophosphonium hexafluorophosphate (PyBOP), 2-(lH-benzotriazol-1-yl)-1,1,3,3-tetramethyluronium hexafluorophosphate (HBTU), or 2-(1H-benzotriazol-1-yl)-1,1,3,3-tetramethyluronium tetrafluoroborate (TBTU), preferably DCC or EDC.

An amount of the condensing agent to be used is usually 1 to 5-fold mole, preferably 1 to 3-fold mole based on Compound (Ic).

The solvent to be used is not particularly limited so long as it has no adverse effect on the reaction and

dissolves starting materials with some extends, and there may be mentioned, for example, ethers such as diethyl ether, tetrahydrofuran, dimethoxyethane, diethoxyethane and dioxane; nitriles such as acetonitrile; aprotic polar solvents such as N,N-dimethylformamide, dimethylsulfoxide, N-methylpyrrolidone and N,N-dimethylacetamide; or halogenated hydrocarbons such as methylene chloride, chloroform and dichlorodichloroethane, these may be used singly or a mixed solvent.

The reaction is usually carried out in the range of -20 to 100°C, preferably 0 to 50°C. The reaction time may vary depending on the reaction temperature and others, and it is usually for 30 minutes to 24 hours, preferably 1 hour to 10 hours.

As the reactive derivative of Compound (Ic) in Step D1b, there may be mentioned, for example, acid halide derivatives of Compound (Ic) such as an acid bromide or an acid chloride of Compound (Ic); or reactive amide derivatives of Compound (Ic) obtained from Compound (Ic) and imidazole, dimethylpyrazole or triazole, preferably an acid halide derivative.

The acid halide of Compound (Ic) can be prepared according to the conventional method, and it can be synthesized by reacting, for example, Compound (Ic) with a halogenating agent (for example, thionyl chloride, thionyl bromide or phosphorus pentachloride) in a solvent (for example, halogenated hydrocarbons such as methylene chloride, chloroform and dichloroethane).

Also, an activated amide derivative of Compound (Ic) can be prepared according to the conventional method, and it can be synthesized by reacting, for example, in the case of an imidazole amide product of Compound (Ic), Compound (Ic) is reacted with 1,1'-carbonyldiimidazole in a solvent.

The reactive derivative of Compound (Ic) can be used in the next Step D1b as such without separation after formation.

An amount of Compound (XI) to be used in the reaction of the reactive derivative of Compound (Ic) and Compound (XI) in Step D1c is usually 1 to 10-fold mole, preferably 1 to 5-fold mole based on Compound (Ic).

As the base to be used, there may be mentioned, for example, amines such as triethylamine, tributylamine, diisopropylethylamine, pyridine, picoline; lutidine, 4-dimethylaminopyridine, 1,8-diazabicyclo[5.4.0]undecene and 1,5-diazabicyclo[4.3.0]-7-nonene, preferably triethylamine, tributylamine or diisopropylethylamine. An amount of the base to be used is usually in an amount of 1 to 10-fold mole, preferably 1 to 5-fold mole based on Compound (Ic).

The reaction is usually carried out in the range of 0 to 150°C, preferably 10 to 100°C. The reaction time may vary depending on the reaction temperature and others, and it is usually for 5 minutes to 48 hours, preferably 30 minutes to 24 hours.

Also, Compound (Id) can be synthesized by a method via Compound (XII).

Step D2 is a step to obtain Compound (XII) by amidating a carboxyl group of Compound (Ic), and it is carried out by optionally selecting a method from methods conventionally known in the art. For example, Compound (XII) can be easily synthesized by reacting the reactive derivative of the above-mentioned Compound (Ic) and ammonia.

Step D3 can be carried out by reacting Compound (XII) and Compound (XIII) in a solvent in the presence of a base.

An amount of Compound (XIII) is usually in an amount of 1 to 10-fold mole, preferably 1 to 5-fold mole based on Compound (XII).

The solvent and the base to be used are mentioned those described in the above-mentioned Step D1, and the reaction can be carried out under the same conditions as Step D1.

Preparation process E is a preparation processs of Compound (Ie).

In Step E1, the reaction of obtaining Compound (XV) from Compound (II) or Compound (III) and Compound (XIV) canbe carried out in the same manner as mentioned above except forusing Compound (XIV) in place of Compound (IV).

Step E2 is carried out by reacting Compound (XV) and Compound (XIII) in a solvent in the presence of a base.

An amount of Compound (XIII) to be used is usually in an amount of 1 to 10-fold mole, preferably 1 to 5-fold mole based on Compound (XV).

As the solvent to be used, the same solvents as those mentioned in the above Step D1 may be mentioned, and preferably halogenated hydrocarbons or aprotic polar solvents.

As the base to be used, the same base as those mentioned in the above Step D1 may be mentioned, and an amount of the base to be used is usually in an amount of 1 to 10-fold mole, preferably 1 to 5-fold mole based on Compound (XIV). Also, in the present reaction, the base may be used in a largely excess amount served as a solvent.

The reaction is usually carried out in the range of -20 to 100°C, preferably 0 to 50°C. The reaction time may vary depending on the reaction temperature and others, and it is usually for 5 minutes to 10 hours, preferably 30 minutes to 5 hours.

Preparation process F is another preparation processs of Compound (Ie).

The reaction of obtaining Compound (XVIII) from Compound (XVI) in Step F1 is carried out by using a conventionally known method which has been known as "Mitsunobu Reaction". For example, it is carried out by reacting

Compound (XVI) and Compound (XVII), with triphenylphosphine and diethyl azodicarboxylate in tetrahydrofuran. An amount of triphenylphosphine to be used is usually in an amount of 1 to 5-fold mole, preferably 1 to 5-fold mole based on Compound (XVI). An amount of diethylazodicarboxylate to be used is usually in an amount of 1 to 10-fold mole, preferably 1 to 5-fold mole based on Compound (XVI).

The reaction is usually carried out in the range of -50 to 80°C, preferably -10 to 50°C. The reaction time may vary depending on the above-mentioned other conditions, and it is usually for 5 minutes to 24 hours, preferably 30 minutes to 10 hours.

The reaction of obtaining Compound (Ie) from Compound (XVIII) in Step F2 can be carried out by the conventionally known method, for example, by a method of deprotection by reacting with organic acids such as trifluoroacetic acid in tetrahydrofuran.

Preparation process G is a preparation processs of Compound (If).

Step G1 is a step of preparing Compound (XX) by trifrating Compound (XIX).

Trifration of Compound (XIX) can be carried out by reacting Compound (XIX) and a trifrating agent in the presence of a base in a solvent.

The solvent to be used is not particularly limited so long as it has no adverse effect on the reaction and dissolves starting materials with some extends, and there may be mentioned, for example, halogenated hydrocarbons such as methylene chloride, chloroform, carbon tetrachloride and dichloroethane; ethers such as diethyl ether, tetrahydrofuran, dimethoxyethane, diethoxyethane and dioxane; aromatic hydrocarbons such as benzene and toluene; or aliphatic hydrocarbons such as heptane, hexane and cyclohexane, preferably halogenated hydrocarbons or ethers.

As the trifrating agent, there may be mentioned, for example, trifluoromethanesulfonyl chloride, trifluoromethanesulfonic anhydride and the like, preferably trifluoromethanesulfonic anhydride. An amount of the trifrating agent to be used is usually in an amount of 1 to 10-fold mole, preferably 1 to 2-fold mole based on Compound (XIX).

As the base, there may be mentioned, for example, amines such as triethylamine, tributylamine, diisopropylethylamine, pyridine, picoline, lutidine, 4-dimethylaminopyridine and the like, preferably triethylamine, diisopropylethylamine, pyridine. An amount of the base to be used is usually in an amount of 1 to 10-fold mole, preferably 1 to 2-fold mole based on the trifrating agent.

The reaction is usually carried out in the range of -20 to 100°C, preferably -10 to 50°C. The reaction time may vary depending on the reaction temperature and others, and it is usually for 5 minutes to 10 hours, preferably 5 minutes to 5 hours.

Step G2 is carried out by reacting Compound (XX) and Compound (XXI) in an inert gas atmosphere such as nitrogen, helium and argon in the presence of a catalyst (palladium catalyst) and a base in a solvent.

The solvent to be used is not particularly limited so long as it has no adverse effect on the reaction and dissolves starting materials with some extends, and there may be mentioned, for example, polar solvents such as N, N-dimethylformamide, dimethylsulfoxide, N-methylpyrrolidone and N,N-dimethylacetamide, or acetonitrile, etc., preferably N,N-dimethylformamide.

As the base, there may be mentioned, for example, amines such as triethylamine, tributylamine, diisopropylethylamine, pyridine, picoline, lutidine and 4-dimethylaminopyridine, preferably triethylamine, diisopropylethylamine or pyridine. An amount of the base to be used is usually in an amount of 1 to 10-fold mole, preferably 1 to 2-fold mole based on Compound (XX).

Also, in place of the amines, a combination of a phase-transfer catalyst such as tetrabutyl ammonium chloride and tetrabutyl ammonium bromide, and alkali metal carbonates such as potassium carbonate, sodium carbonate and sodium hydrogen carbonate may be used.

As the palladium complex, there may be mentioned, for example, palladium acetate, palladium acetate-triphenylphosphine, dichlorobistriphenylphosphine or tetrakistriphenylphosphine, preferably palladium acetate-triphenylphosphine or tetrakistriphenylphosphine. An amount of the palladium complex to be used is usually in an amount of 0.01 to 1-foldmole, preferably 0.01 to 0.1-foldmole based on Compound (XX) .

Also, lithium chloride or lithium bromide may be copresent in the reaction.

The reaction is usually carried out in the range of 0 to 150°C, preferably 25 to 80°C. The reaction time may vary depending on the reaction temperature and others, and it is usually for 30 minutes to 24 hours, preferably 1 hour to 10 hours.

Preparation processs M is a preparation processs of Compound (Ia).

Step M1 is carried out by reacting Compound (XXXX) and Compound (IVa) in the presence of a catalyst in a solvent.

An amount of Compound (IVa) to be used is usually in an amount of 1 to 5-fold mole, preferably 1 to 2-fold mole based on Compound (XXXX).

The solvent to be used not particularly limited so long as it has no adverse effect on the reaction and dissolves starting materials with some extends, and there may be mentioned, for example, halogenated hydrocarbons such as methylene chloride, chloroform and dichloroethane; aromatic hydrocarbons such as benzene and toluene, preferably

halogenated hydrocarbons.

As the catalyst to be used, there may be used, for example, Lewis acids such as boron trifluoride-diethyl ether complex and others.

An amount of the catalyst to be used is usually in an amount of 0.1 to 10-fold mole, preferably 1 to 5-fold mole based on Compound (XXXX).

The reaction is usually carried out in the range of 0 to 100°C, preferably 0 to 30°C. The reaction time may vary depending on the reaction temperature and others, and it is usually for 5 minutes to 10 hours, preferably 30 minutes to 5 hours.

In the above-mentioned respective reactions, the desired compound can be isolated from the reaction mixture according to the conventional manner. For example, when insoluble material exists, after optionally removing it by filtration, the solvent is distilled, or the solvent was removed under reduced pressure, water is added to the residue, the mixture is extracted with a water-immiscible organic solvent such as ethyl acetate, etc., and if necessary, after drying over anhydrous sodium sulfate, etc., the solvent is removed to obtain the desired compound, and further necessary, it can be further purified by the conventional method, for example, recrystallization, column chromatography, and others.

Also, the compound of the general formula (I) according to the present invention can be converted into a pharmaceutically acceptable salt by treating with an acid or a base according to the conventional method. For example, a desired salt can be obtained by reacting with a desired acid or a base in an inert solvent (preferably ethers such as diethyl ether, tetrahydrofuran, dimethoxyethane, diethoxyethane and dioxane; alcohols such as methanol, ethanol, propanol, isopropanol and butanol; halogenated hydrocarbons such as methylene chloride, chloroform; or water), and removing the solvent, or collecting the precipitated crystal by filtration. Also, it can be separated as a salt directly from a reaction mixture at the final reaction step.

Moreover, in the compound of the formula (I), there exist optical isomer(s) (including diastereomer) due to an asymmetric carbon(s) and/or geometric (E, Z) isomers due to an unsaturated carbon. These respective isomers can be separated by treating the corresponding racemic isomers or geometric isomer mixture by usual optical resolution methods (fractional recrystallization method, optical resolution column chromatography method or diastereimer method, etc.) or separation methods (recrystallization method, column chromatography method, etc.). For example, optical isomers are to be separated, Compound (I) which is racemic mixture is reacted with optically active sulfonic acid compound ((S)or (R)-camphor-10-sulfonic acid, etc.), to obtain one of the diastereomer salts, if necessary, further subjecting to purification, the resulting diastereomer salt is decomposed according to the conventional manner to obtain an optical isomer. Also, when the above reaction is carried out by using the starting compound which has been subjected to optical resolution or separation, desired optical isomer or geometric isomer can be obtained.

Compound (IV), (V), (VIII), (IX), (XI), (XIII), (XIV), (XVI), (XVII) and other sub-starting materials which are used as starting materials in the above-mentioned Preparation processes A, B, C, D, E, F, G and M are each known compounds or can be easily produced according to the conventionally known method. Also, Compound (II) can be produced according to Preparation processs H or Preparation processs J as shown below, Compound (XXIVa) can be produced according to Preparation processs I as shown below, Compound (XIXa) can be produced according to Preparation processs K as shown below, Compound (XXI) can be produced, for example, according to Preparation processs L as shown below, Compound (XXXX) can be produced according to Preparation processs N as shown below.

Preparation processs H

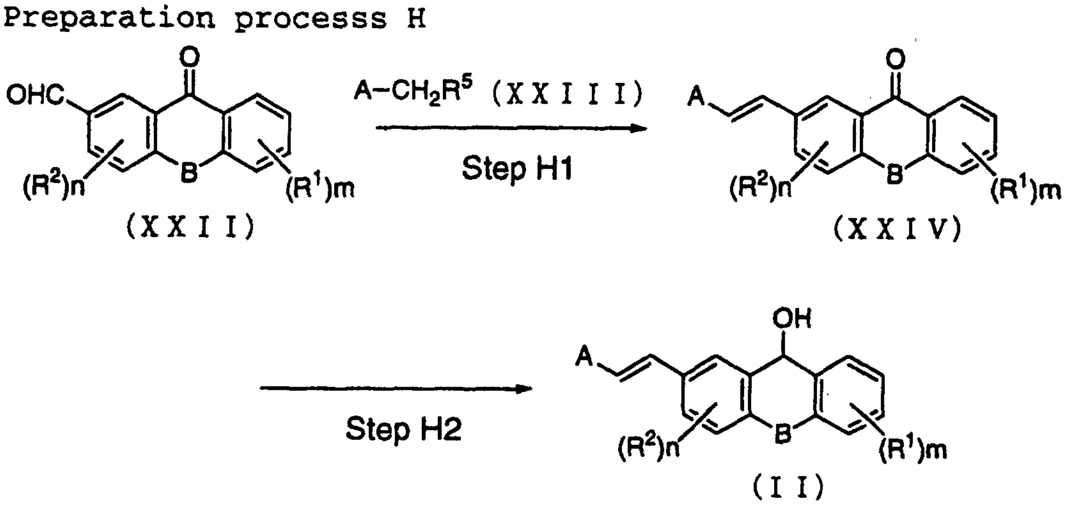


(XXII)

(XXIV)

(II)

Preparation processs I

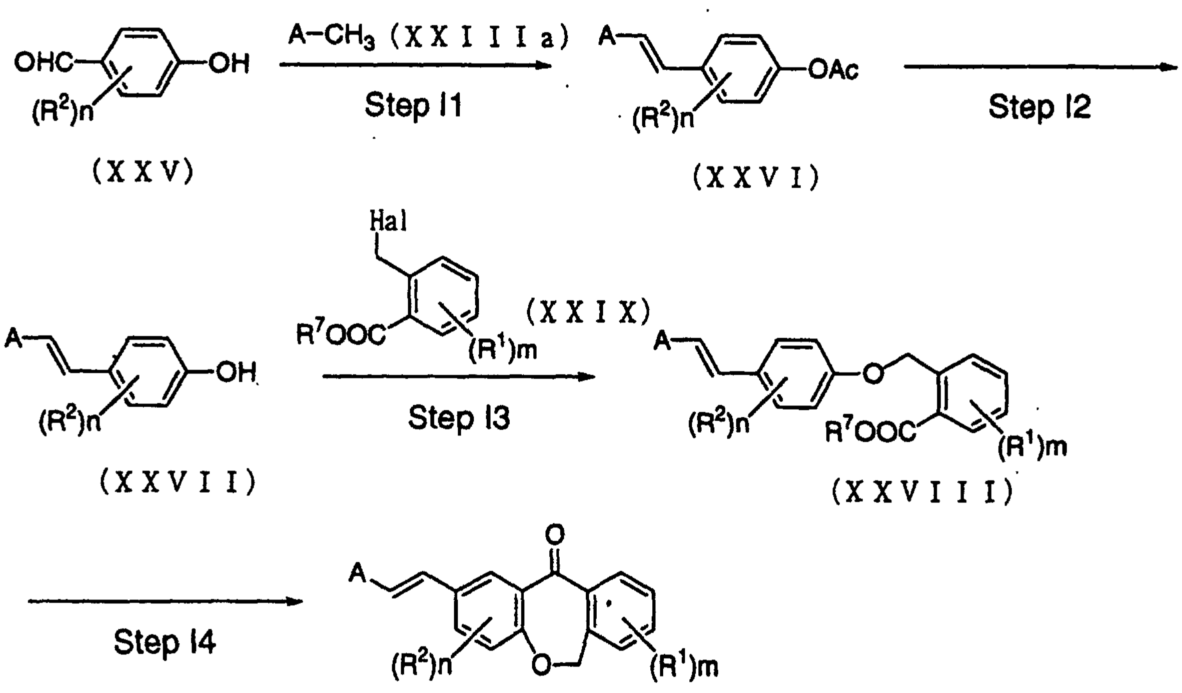


(XXV) (XXVI) (XXVII) (XXVIII) (XXIVa)

Preparation processs J

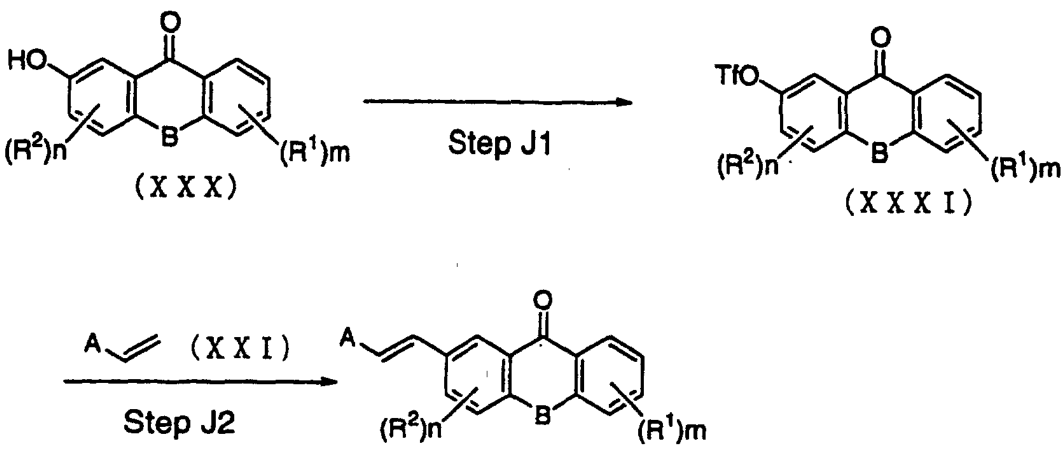


(XXX) (XXXI) (XXIV)

Preparation processs K

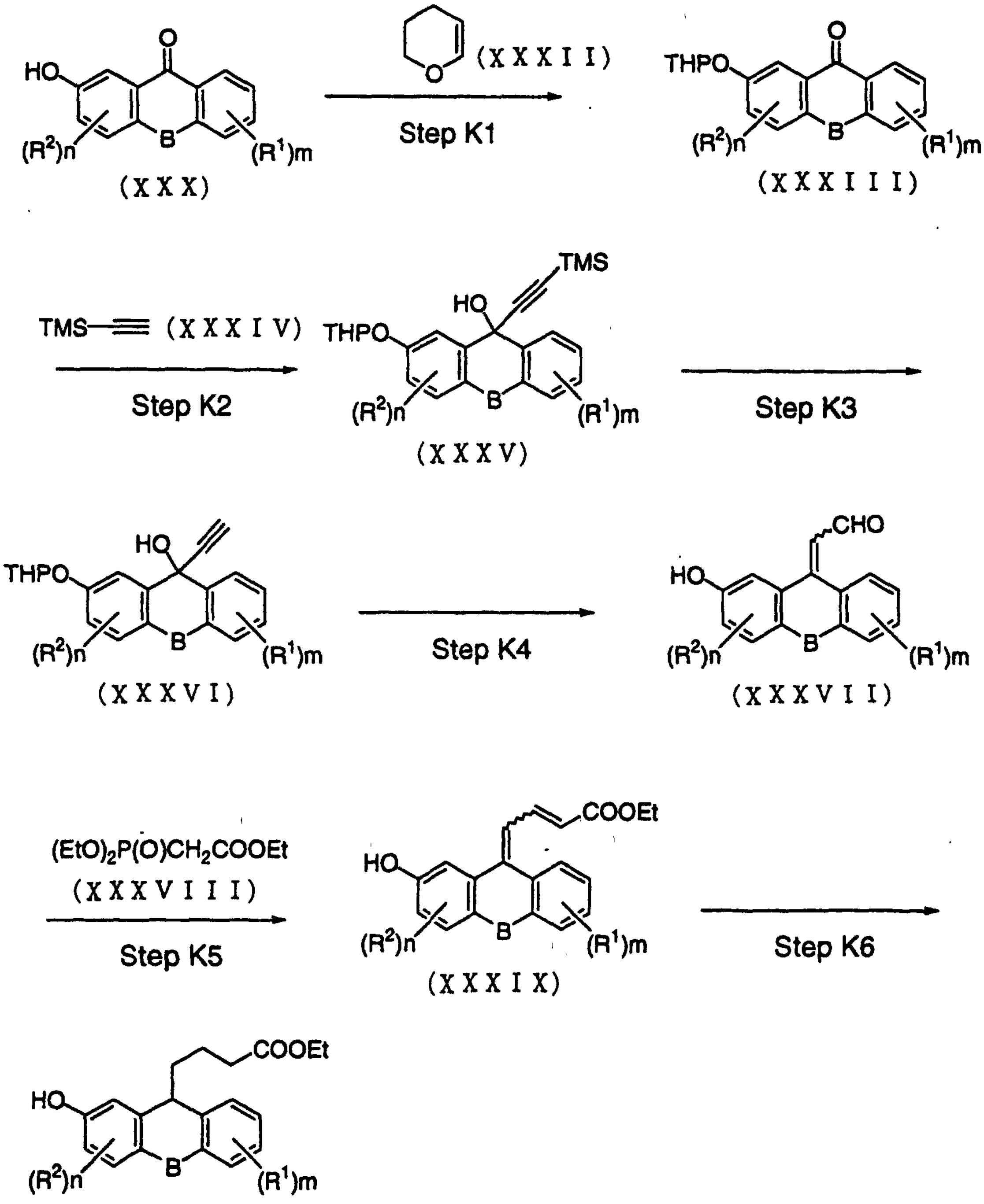


(X X X) (X X X I I I) (X X X V) (X X X V I) (X X X V I I) (X X X I X) (X I X a)

Preparation processs L

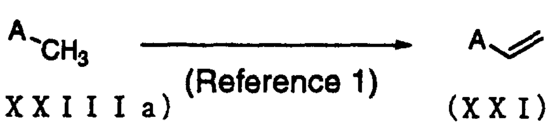


(X X I I I a) (X X I)

(Reference 1) J. Org. Chem., 61, 3398(1996)

Preparation processs N

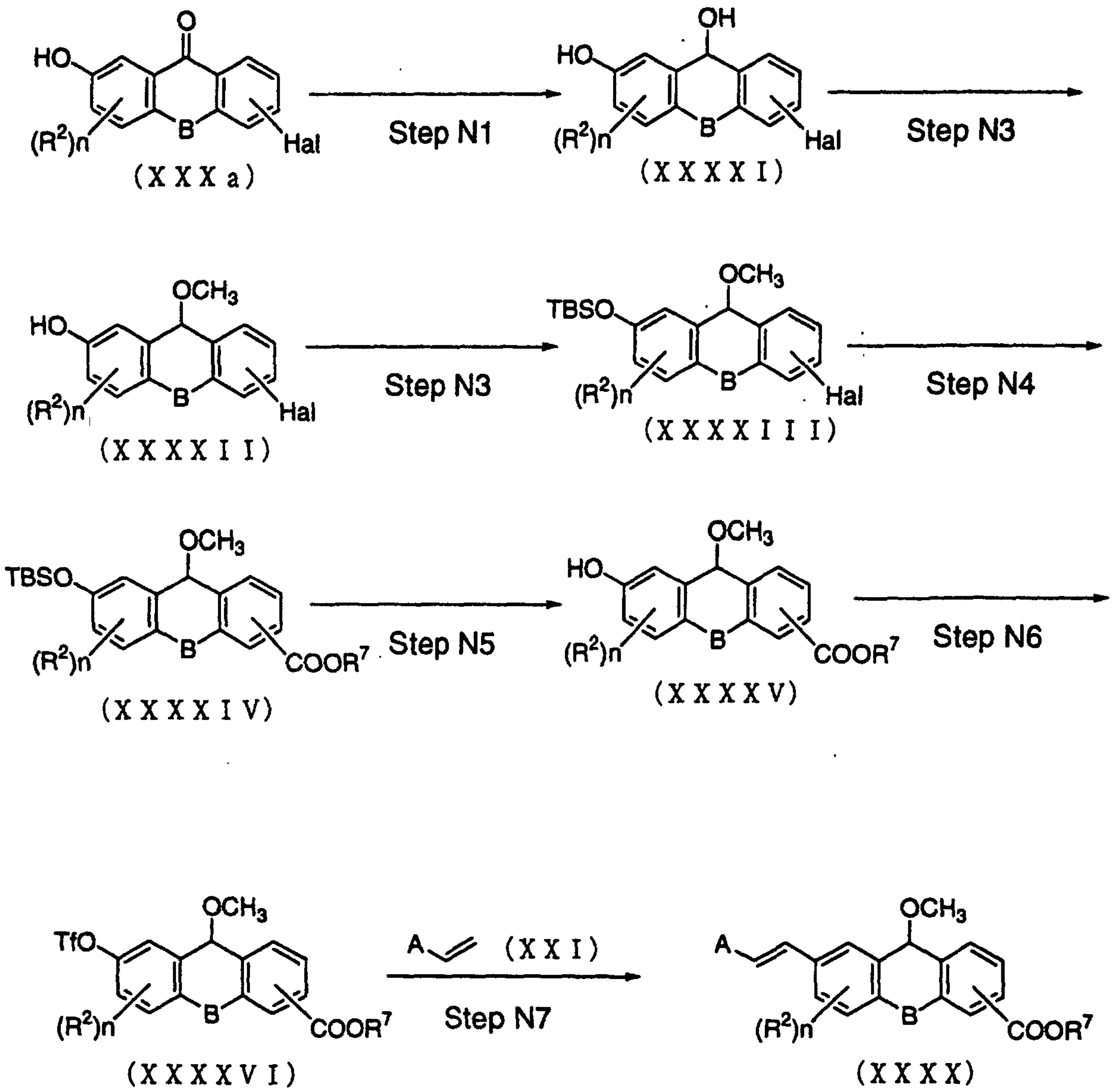


(XXXa) (XXXXI) (XXXXII) (XXXXIII) (XXXXIV) (XXXXV) (XXXXVI) (XXXX)

Preparation processs O

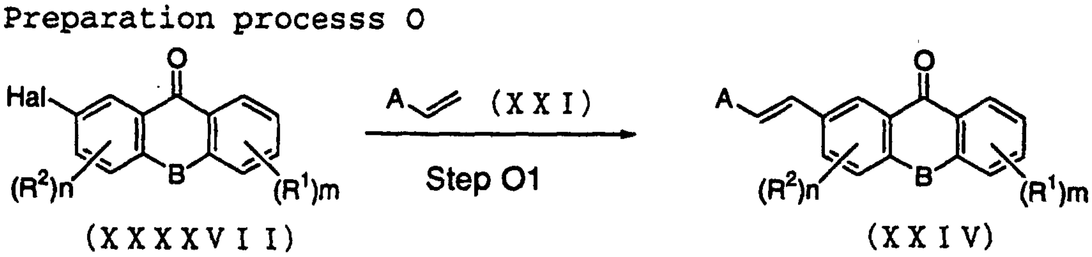


(XXXXVII) (XXIV)

Preparation processs P

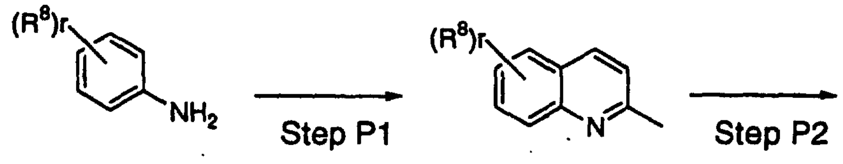


(XXXXVIII) (XXXXIX)

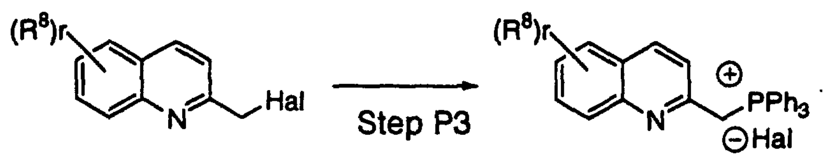


(XXXXX) (XXXXXI)

Preparation processs Q

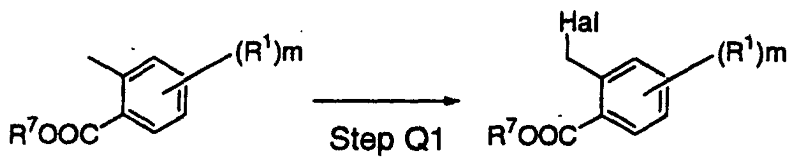


(XXXXXII) (XXIX)

In the above formulae, $R^1$, $R^2$, A, B, Hal, m and n have the same meanings as mentioned above, $R^5$ represents a hydrogen atom or a formula: $-P(R^6)_3 \cdot Hal$ group,

wherein $R^6$ represents a $C_1$-$C_4$ alkyl group or a phenyl group,

Ac represents an acetyl group, $R^7$ represents a $C_1$-$C_4$ alkyl group, $R^8$ represents a halogen atom with the same meaning as in $R^1$, a nitro group, a cyano group, a $C_1$-$C_4$ alkyl group with the same meaning as in $R^1$, a fluoro $C_1$-$C_4$ alkyl group with the same meaning as in $R^1$, a $C_1$-$C_4$ alkoxy group with the same meaning as in $R^1$, a fluoro $C_1$-$C_4$ alkoxy group with the same meaning as in $R^1$ or a $C_1$-$C_4$ alkylthio group with the same meaning as in $R^1$, r is an integer of 1 to 4, when r is 2 or more, $R^8$s may be different from each other, Et means an ethyl group, THP means a tetrahydropyranyl group, TBS means a t-butyldimethylsilyl group.

Preparation processs H is a preparation processs of Compound (II).

In Step H1 of Preparation processs H, as a starting material, there are a method (Step H1a) of using Compound (XXIII, $R^5$ = a hydrogen atom) or a method (Step H1b) of using Compound (XXIII), $R^5$ = a formula: $-P(R^6)_3 \cdot Hal$ group).

In the method of using Compound (XXIII, $R^5$ = a hydrogen atom) of Step H1a, it is carried out by reacting Compound (XXII) and Compound (XXIII, $R^5$ = a hydrogen atom) in acetic anhydride. An amount of Compound (XXIII, $R^5$ = a hydrogen atom) to be used is usually in an amount of 1 to 10-foldmole, preferably 1 to 5-fold mole based on Compound (XXII).

The reaction is usually carried out in the range of 20 to 200°C, preferably 50 to 150°C. The reaction time may vary depending on the reaction temperature and others, it is usually for 1 hour to 200 hours, preferably 3 hours to 100 hours.

In the method of using Compound (XXIII, $R^5$ = a formula: $-P(R^6)_3 \cdot Hal$ group) of Step H1b, it is carried out by reacting Compound (XXII) and Compound (XXIII, $R^5$ = a formula: $-P(R^6)_3 \cdot Hal$ group) in a solvent in the presence of a base. An amount of Compound (XXIII, $R^5$ = a formula: $-P(R^6)_3 \cdot Hal$ group) to be used is usually in an amount of 1 to 5-fold mole, preferably 1 to 2-fold mole based on Compound (XXII).

The solvent to be used is not particularly limited so long as it has no adverse effect on the reaction and dissolves starting materials with some extends, and there may be mentioned, for example, aromatic hydrocarbons

such as benzene, toluene and xylene; ethers such as diethyl ether, dioxane and tetrahydrofuran; halogenated hydrocarbons such as methylene chloride, chloroform and dichloroethane; or aprotic polar solvents such as N,N-dimethylformamide and dimethylsulfoxide, preferably aromatic hydrocarbons or ethers.

As the base tobe used, there may be mentioned, for example, alkali metal hydrides such as sodium hydride, lithium hydride and potassium hydride; alkali metal amides such as sodium amide and lithium diisopropylamide; alkali metal alkoxides such as sodium methoxide, sodium ethoxide and potassium t-butoxide; or alkyl lithiums such as methyl lithium, butyl lithium and t-butyl lithium, preferably sodium hydride, lithium diisopropylamide, potassium t-butoxide, butyl lithium or t-butyl lithium. An amount of the base to be used is usually in an amount of 1 to 3-fold mole, preferably 1 to 1.5-fold mole based on Compound (XXIII, $R^5$ = a formula: $-P(R^6)_3 \cdot Hal$ group).

The reaction is usually carried out in the range of -80 to 100°C, preferably -60 to 50°C. The reaction time may vary depending on the reaction temperature and others, it is usually for 10 minutes to 10 hours, preferably 15 minutes to 6 hours.

The reduction of Compound (XXIV) to Compound (II) in Step H2 is carried out by using a reducing agent in a solvent.

As the reducing agent, there may be mentioned, for example, sodium borohydride , lithium borohydride, sodium cyanoborohydride or lithium aluminum hydride, preferably sodium borohydride.

As the solvent to be used, there may be mentioned, for example, alcohols such as methanol, ethanol, propanol, isopropanol and butanol; ethers such as tetrahydrofuran, dioxane and 1,2-dimethoxyethane; nitriles such as acetonitrile; amides such as N,N-dimethylformamide, N,N-dimethylacetamide and N-methylpyrrolidone; water; or a mixed solvent of the above solvents, preferably methanol, ethanol, tetrahydrofuran, N,N-dimethylformamide, N,N-dimethylacetamide or a mixed solvent of the above solvents.

The reaction is usually carried out in the range of -10 to 150°C, preferably 0 to 100°C. The reaction time may vary depending on the reaction temperature and other conditions, and it is usually for 10 minutes to 10 hours, preferably 30 minutes to 6 hours.

Preparation processs I is a preparation processs of Compound (XXIVa).

Step I1 is carried out by reacting Compound (XXV) and Compound (XXIIIa) in acetic anhydride, and it is carried out by the same method as mentioned in the above Step H1 except for using Compound (XXIIIa) in place of Compound (XXIII, $R^5$ = a hydrogen atom).

In Step I2, Compound (XXVI) is hydrolyzed to Compound (XXVII) according to the conventional manner under alkaline conditions.

Step I3 is carried out by reacting Compound (XXVII) and Compound (XXIX) in a solvent in the presence of a base. An amount of Compound (XXIX) to be used is usually in an amount of 1 to 5-fold mole, preferably 1 to 2-fold mole based on Compound (XXVII).

As the solvent and the base to be used In Step I3, those mentioned in the above Step A2 may be mentioned, and as the reaction conditions, those mentioned in Step A2 can be employed and carried out.

Step I4 contains a step (Step I4a) of obtaining a carboxylic acid material by hydrolyzing an ester group of Compound (XXVIII) and a step (Step I4b) of cyclizing said carboxylic acid material to produce Compound (XXIV).

Hydrolysis of the ester group of Compound (XXVIII) in Step I4a can be easily carried out according to the conventional manner under alkaline or acidic conditions.

Step I4b is carried out by reacting the carboxylic acid material of Compound (XXVIII) obtained as mentioned above in a solvent in the presence of a catalyst (a dehydrating agent) .

The solvent to be used is not particularly limited so long as it has no adverse effect on the reaction and dissolves starting materials with some extends, and there may be mentioned, for example, halogenated hydrocarbons such as methylene chloride, chloroform, carbon tetrachloride and dichloroethane; or nitrobenzene or carbon disulfide, preferably halogenated hydrocarbons.

As the catalyst to be used, there may be mentioned, for example, mineral acids such as sulfuric acid, phosphoric acid and polyphosphoric acid; acid anhydrides such as methanesulfonic anhydride and trifluoroacetic acid anhydride; or Lewis acids such as boron trifluoride-diethyl ether complex, aluminum chloride and zinc chloride, preferably polyphosphoric acid, methanesulfonic anhydride, trifluoroacetic acid anhydride or boron trifluoride-diethyl ether complex. Also, a mixture of trifluoroacetic acid anhydride and boron trifluoride-diethyl ether complex is suitably used.

An amount of the catalyst to be used is usually in an amount of 1 to 10-fold mole, preferably 1 to 5-fold mole based on Compound (XXVIII) or its carboxylic acid material.

The reaction is usually carried out in the range of 0 to 100°C, preferably 0 to 50°C. The reaction timemayvarydepending on the reaction temperature and others, and it is usually for 5 minutes to 24 hours, preferably 30 minutes to 18 hours.

Preparation processs J is another preparation processs of Compound (XXIV).

Step J1 is a step to obtain Compound (XX) by trifrating Compound (XXX), and it is carried out in the same manner as in the above-mentioned Step G1 except for using Compound (XXX) in place of Compound (XIX).

Step J2 is a step to obtain Compound (XXIV) by subjecting Compound (XXXI) and Compound (XXI) to coupling reaction, and the present reaction is carried out in the same manner as in the above-mentioned Step G2 except for using Compound (XXXI) in place of Compound (XX).

Preparation processs K is a preparation processs of Compound (XIXa).

Step K1 is carried out by the method of reacting Compound (XXXIII) and Compound (XXXII) according to the conventionally known method, for example, by reacting them in ethyl acetate, in the presence of an acid catalyst such as hydrochloric acid, p-toluenesulfonic acid and pyridinium p-toluenesulfonate.

Step K2 is carried out by reacting Compound (XXXIII) and Compound (XXXIV) in a solvent by using a base. The present reaction is carried out in the same method of obtaining Compound (XXIV) by using Compound (XXIII, $R^5$ = a formula: $-P(R^6)_3 \cdot Hal$ group) in the above-mentioned Step H1 except for using Compound (XXXIII) in place of Compound (XXII), and using Compound (XXXIV) in place of Compound (XXIII, $R^5$ = a formula: $-P(R^6)_3 \cdot Hal$ group).

A reaction of producing Compound (XXXVI) from Compound (XXXV) in Step K3 can be carried out by the conventionally known method, for example, by a method of subjecting to deprotecting reaction in tetrahydrofuran using a tetra-n-butyl ammonium fluoride 1.0M tetrahydrofuran solution.

A reaction of producing Compound (XXXVII) from Compound (XXXVI) in Step K4 can be carried out by subjecting to rearrangement reaction in a solvent in the presence of an acid catalyst.

The solvent to be used is not particularly limited so long as it has no adverse effect on the reaction and dissolves starting materials with some extends, and there may be mentioned, for example, a mixed solvent comprising one or several kinds of organic solvents selected from alcohols such as methanol, ethanol, propanol and butanol; ethers such as tetrahydrofuran, dioxane and 1,2-dimethoxyethane; nitriles such as acetonitrile; and amides such as N,N-dimethylformamide, N,N-dimethylacetamide and N-methylpyrrolidone; a mixed solvent with water, preferably a mixed solvent comprising one or two organic solvents selected from tetrahydrofuran and N,N-dimethylformamide with water.

As the acid catalyst to be used, there may be mentioned, for example, mineral acids such as hydrochloric acid, sulfuric acid and phosphoric acid; organic acids such as methanesulfonic acid and trifluoroacetic acid. An amount of the catalyst to be used is usually in an amount of 1 to 100-fold mole, preferably 1 to 50-fold mole based on Compound (II).

The reaction is usually carried out in the range of 0 to 100°C, preferably 0 to 30°C. The reaction time may vary depending on the reaction temperature and others, and it is usually for 5 minutes to 48 hours, preferably 30 minutes to 24 hours.

Step K5 is carried out by reacting Compound (XXXVII) and Compound (XXXVIII) in a solvent in the presence of a base. An amount of Compound (XXXVIII) to be used is usually in an amount of 1 to 10-foldmole, preferably 1 to 5-foldmole based on Compound (XXXVII).

As the base to be used, there may be mentioned, for example, alkali metal hydrides such as sodium hydride and lithium hydride; alkali metal alkoxides such as sodiummethoxide, sodium ethoxide and potassium t-butoxide; alkyl lithiums such as methyl lithium and butyl lithium; or metal amides such as sodium amide and lithium diisopropyl amide, preferably metal hydrides.

An amount of the base to be used is usually in an amount of 1 to 5-fold mole, preferably 1 to 2-fold mole based on Compound (XXXVIII).

In Step K5, the solvent to be used is not particularly limited so long as it has no adverse effect on the reaction and dissolves starting materials with some extends, and there may be mentioned, for example, aromatic hydrocarbons such as benzene and toluene; ethers such as tetrahydrofuran, dioxane, dimethoxyethane and diethoxyethane; or aprotic polar solvents such as N,N-dimethylformamide, N,N-dimethylacetamide and dimethylsulfoxide, preferably ethers.

The reaction is usually carried out in the range of -50°C to 100°C, preferably -10°C to 50°C.

The reaction time is usually for 15 minutes to 12 hours, preferably 30 minutes to 5 hours.

Step K6 is carried out by subjecting Compound (XXXIX) to catalytic reduction by hydrogen in the presence of a catalyst in a solvent.

In Step K6, the solvent to be used is not particularly limited so long as it has no adverse effect on the reaction and dissolves starting materials with some extends, and there may be mentioned, for example, alcohols such as methanol and ethanol; or ethers such as tetrahydrofuran and dioxane, preferably alcohols.

The catalyst to be used in Step K6 may be mentioned, for example, palladium-carbon, platinum-carbon, platinum oxide or rhodium-carbon. In the reaction of Step K6, a partial pressure of hydrogen is usually 1 atm to 10 atm, preferably 1 atm to 3 atm.

The reaction is usually carried out in the range of 0°C to 100°C, preferably 20°C to 80°C. The reaction time may vary depending on the reaction temperature and others, and it is usually for 15 minutes to 72 hours, preferably 30 minutes to 48 hours.

Preparation processs N is a preparation processs of Compound (XXXX).

Step N1 is a reduction of Compound (XXXa) to Compound (XXXXI), and is carried out by using a reducing

agent in a solvent. The present reaction is carried out in the same manner as in the method of obtaining Compound (II) in the above-mentioned Step H2.

Step N2 is carried out by reacting Compound (XXXXI) according to the conventionally known method, for example, by reacting it in methanol in the presence of an acid catalyst such as hydrochloric acid, p-toluenesulfonic acid and pyridinium p-toluenesulfonate.

Step N3 is carried out by reacting Compound (XXXXII) and a silylating agent according to the conventionally known method, for example, by reacting it in tetrahydrofuran in the presence of a base catalyst such as imidazole.

Step N4 is a step of converning Compound (XXXXIII) having a halogen atom into Compound (XXXXIV) having an ester group, and can be carried out by subjecting to lithiation using lithium-halogen exchange reaction between alkyl lithium and an organic halogenated material as described in "Organometallic Chemistry (New Experimental Chemistry Lecture 12)", Maruzen (1975), and then, ① carbon dioxide is reacted for carboxylation, subsequently to treate it with an alkylating agent such as dimethyl sulfate, or ② it is treated with a carbonate ester such as dimethyl carbonate.

A reaction of producing Compound (XXXXV) from Compound (XXXXIV) in Step N5 is carried out by the conventionally known method, for example, by subjecting to deprotection reaction using a tetra-n-butyl ammonium fluoride 1.0M tetrahydrofuran solution in tetrahydrofuran.

Step N6 is carried out by reacting Compound (XXXXV) and trifluoromethanesulfonic anhydride in a solvent in the presence of a base, and it is carried out in the same manner as the process of obtaining Compound (XX) in the above-mentioned Step G1 except for using Compound (XXXXII) in place of Compound (XIX).

Step N7 is a coupling reaction of Compound (XXXXVI) and Compound (XXI). The present reaction is carried out by using a palladium catalyst in an inert gas atmosphere in a solvent. The present reaction is carried out in the same manner as the process of obtaining Compound (If) in the above-mentioned Step G2 except for using Compound (XXXXVI) in place of Compound (XX).

Preparation processs O is a preparation processs of Compound (XXIV).

Step O1 is a coupling reaction of Compound (XXXXVII) and Compound (XXI). The present reaction is carried out by using a palladium catalyst in an inert gas atmosphere in a solvent. The present reaction is carried out in the same method of obtaining Compound (If) in the above-mentioned Step G2 except for using Compound (XXXXVII) in place of Compound (XX), and copresenting neither lithium chloride nor lithium bromide.

Preparation processs P is a method of providing Compound (XXIII) to be used in Step H1 of Preparation processs H.

Step P1 is carried out by using Compound (XXXXVIII) and $\alpha,\beta$-unsaturated aldehyde in a solvent in the presence of an acid catalyst as disclosed in J.Org. Chem., 42, 911 (1977).

Step P2 is carried out, for example, as disclosed in Japanese Provisional Patent Publication No. Hei.9-31059, by using Compound (XXXXIX) in a solvent with a brominating agent such as N-bromosuccin imide, and a radical initiator such as benzoyl peroxide and 2,2'-azobis(isobutyronitrile).

Step P3 is easily carried out by reacting Compound (XXXXX) with triphenylphosphine according to the conventionally known method in a solvent.

Preparation processs Q is a method of producing Compound (XXIX) to be used in Step I1 of Preparation processs I, and can be carried out by using Compound (XXXXXII) according to the conventionally known method, for example, with a brominating agent such as N-bromosuccin imide in the presence of a radical initiator such as benzoyl peroxide and 2,2'-azobis(isobutyronitrile).

In Compound (II), Compound (XIX) and Compound (XXI), there exist optical isomers (including diastereomer) due to an asymmetric carbon(s) and/or geometric (E, Z) isomers due to an unsaturated carbon(s). These respective isomers can be separated by treating the corresponding racemic isomers or geometric isomer mixture by usual optical resolution methods (fractional crystallization method, optical resolution column chromatography method or diastereimer method, etc.) or separation methods (recrystallization method, column chromatography method, etc.). For example, optical isomers are to be separated, Compound (I) which is racemic mixture is reacted with optically active sulfonic acid Compound ((S)or (R)-camphor-10-sulfonic acid, etc.), to obtain one of the diastereomer salts, if necessary, further subjecting to purification, the resulting diastereomer salt is decomposed according to the conventional manner to obtain an optical isomer.

Incidentally, Compounds (XXII), (XXIII), (XXIIIa), (XXV), (XXIX), (XXX), (XXXII), (XXXIV), (XXXVIII), (XXXa), (XXXXVII), (XXXXVIII), (XXXXXII) and other sub-starting materials which are used as starting materials in Preparation processs H, Preparation processs I, Preparation processs J, Preparation processs K, Preparation processs L, Preparation processs N, Preparation processs O, Preparation processs P and Preparation processs Q are each known compound or can be easily produced according to the conventionally known method.

Utilizability in industry

The compound represented by the formula (I) according to the present invention has potent leukotriene antagonistic action, and is extremely useful as an antiallergic agent and an anti-inflammatory agent.

As an administration form for the purposes, there may be mentioned, for example, an oral administration such as a tablet, a capsule, a granule, powder or a syrup, or a non-oral administration such as an intravenous injection, an intramuscular injection, a suppository, an inhalant, an aerosol or an ophthalmic solution. A dose for administration may vary depending on an age, a body weight, symptom and a form of administration as wel as a number of administration, and it is usually administered about 0.1 to 1,000 mg per day once or divided to several times to an adult person.

EXAMPLES

In the following, the present invention is further explained in more detail by referring to Test examples and Examples, but the scope of the present invention is not limited by these.

Test example 1

Leukotriene $D_4$ receptor binding test

<Preparation of receptor sample>

As a receptor sample, a lung cell membrane fraction from guinea pigs was used. Preparation of the membrane fraction was carried out according to the method of Ahn et al. (Eur. J. Pharmacol., 127, 153-155 (1986)). Lungs of Hartleymale guinea pigs (400 to 500 g body weight, Nippon SLC Co.) were extracted, and perfused with a physiological saline, and then, adding 10 mM of PIPES, 10 mM of $MgCl_2$ and 10 mM of $CaCl_2$ buffer (pH 7.5) to the lung tissue and the mixture was homogenized. This homogenate was centrifuged at 70,000xg for 10 minutes to obtain a membrane fraction.

<Leukotriene $D_4$ recepter binding test>

Leukotriene $D_4$ ($LTD_4$) recepter binding test was carried out according to the method of Aharony, et al. (J. Pharmacol. Expl.Ther., 243, 921-926 (1987)). To 0.42 mg of the receptor sample were added 10 mM of PIPES; 10 mM of $MgCl_2$ and 10 mM of $CaCl_2$ buffer (pH 7.5) to make the total amount of 480 µl, and 10 µl of [$^3$H] $LTD_4$ (NEN Life Science Products Co.) and 10 µl of a Test compound in dimethylsulfoxide were added to the mixture, and the resulting mixture was incubated at 25°C for 30 minutes. The mixtures thus incubated were filtered through a glass fiber filter (Whatman Co., GF/C) using cell harvester (Brandel Co., M-30R). The filter were washed with 10mM of Tris. and 100 mM of NaCl buffer (pH 7.5), and 5 ml of a liquid scintillator (NACALAI TESQUE INC., clearsol I), and radioactivity was measured by a liquid scintillation analyzer (Packard Co., 2000CA). When a dissociation constant (Kd) of $LTD_4$ was to be obtained, [$^3$H] $LTD_4$ with 0.03 to 0.5 nM was used, and 1 µM of non-radioactive $LTD_4$ was added. When a binding inhibition constant (Ki) of the Test compound is to be meaured, [$^3$H] $LTD_4$ with 0.2 nM was used. Kd and Ki are calculated according to the method of Bennett et al. (Neurotransmitter Receptor Binding, 2nd ed., edited by H.I. Yamamura et al., pp. 61-89, Raven Press (1985)). The results are shown in Table 2.

Table 2

| Results of leukotriene $D_4$ receptor binding test | | | |
|---|---|---|---|
| Test compound | pKi value | Test compound | pKi value |
| Compound of Example 2(b) | 9.5 | Compound of Example 26 | 9.5 |
| Compound of Example 3 | 9.8 | Compound of Example 32(b) | 9.1 |
| Compound of Example 4 | 9.8 | Compound of Example 34(b) | 9.2 |
| Compound of Example 5(b) | 9.6 | Compound of Example 35(b) | 9.4 |
| Compound of Example 7(b) | 9.7 | Compound of Example 40 | 9.1 |
| Compound of Example 12 | 9.8 | Compound of Example 43 | 9.9 |
| Compound of Example 13(b) | 9.2 | Compound of Example 44 | 9.7 |
| Compound of Example 14 | 9.2 | Compound of Example 45 | 9.8 |
| Compound of Example 15 | 9.8 | Compound of Example 51(c) | 9.8 |
| Compound of Example 19 | 9.7 | Compound of Example 54(b) | 9.9 |
| Compound of Example 23 | 9.3 | Compound of Example 58 | 9.3 |

Table 2 (continued)

| Results of leukotriene $D_4$ receptor binding test | | | |
|---|---|---|---|
| Test compound | pKi value | Test compound | pKi value |
| Compound A | 9.5 | | |
| Compound A: 11-(2-carboxyethyl)thio-2-(7-chloro-6-fluoro-quinolin-2-yl) methoxy-6,11-dihydrodibenz[b,e]oxepine (see WO94/193445 publication) | | | |

Test example 2

Leukotriene $D_4$ induced respiratory constriction test

Respiratory constriction was measured by modifying the method of Konzett and Rössler (Arch. Exp. Pathol. Pharmakol., 195, pp. 71-74 (1940)). Hartley male guinea pigs (400 to 500 g body weight, Nippon SLC Co.) were anesthetized with pentobarbital (50 mg/kg, s.c.), and a cannula was inserted into the trachea to carry out artificial ventilation with an artificial ventilator (manufactured by Harvard Co., Model 683). An inner pressure of the respiratory tract was measured by a differential pressure transducer (Nihon Koden, TP-603T) connected to the respiratory cannula and it is used as an index of respiratory constriction.

$LTD_4$ (0.03, 0.06, 0.13, 0.25, 0.5, 1 and 2 μg/kg, Sigma Co.) was intravenously administered from a cannula inserted into the right jugular vein from a low dose with an interval of 5 minutes to cause a respiratory constriction reaction and an increased amount of a respiratory inner pressure was measured. Test compound was suspended in 0.5% sodium carboxymethyl cellulose aqueous solution, and orally administered 1 hour before administration of $LTD_4$. Animals were fasted for 24 hours before administration of the Test compound. From a dose-reaction curve of $LTD_4$, 50% reaction dose ($ED_{50}$) was measured, and a dose ($A_2$) of the Test compound required for shifting two-times of a dose-reaction curve of a control group to a higher dose side was calculated from the formula shown below.

$$A_2 = (\text{Dose of Compound administered})/\{(ED_{50}\text{ of group to which Compound was added})/(ED_{50}\text{ of control group})-1\}$$

The results are shown in Table 3.

Table 3

| Results of leukotriene $D_4$ induced respiratory constriction test | |
|---|---|
| Test compound | $A_2$ (mg/kg p.o. 1hr) |
| Compound of Example 2(b) | 0.0017 |
| Compound of Example 3 | 0.0036 |
| Compound of Example 4 | 0.0037 |
| Compound of Example 5(b) | 0.0026 |
| Compound of Example 7(b) | 0.0035 |
| Compound of Example 12 | 0.0049 |
| Compound of Example 13(b) | 0.0078 |
| Compound of Example 14 | 0.0044 |
| Compound of Example 15 | 0.0008 |
| Compound of Example 19 | 0.0024 |
| Compound of Example 23 | 0.0057 |
| Compound of Example 32(b) | 0.0094 |
| Compound of Example 34(b) | 0.0077 |
| Compound of Example 35(b) | 0.0066 |
| Compound of Example 40 | 0.0088 |
| Compound of Example 43 | 0.0013 |
| Compound of Example 44 | 0.005 |
| Compound of Example 51(c) | 0.0036 |
| Compound of Example 54(b) | 0.0023 |

Table 3 (continued)

| Results of leukotriene $D_4$ induced respiratory constriction test | |
|---|---|
| Test compound | $A_2$ (mg/kg p.o. 1hr) |
| Compound of Example 58 | 0.0064 |
| Compound A | 0.016 |
| Compound A: 11-(2-carboxyethyl)thio-2-(7-chloro-6-fluoro-quinolin-2-yl)methoxy-6,11-dihydrodibenz[b,e]-oxepine (see WO 94/193445 publication) | |

Example 1

(a) Methyl [2-[(E)-2-(6,7-difluoro-2-quinolinyl)ethenyl]-6,11-dihydrodibenz[b,e]oxepin-11-yl]oxyacetate: (methyl ester of Exemplary compound 1)

In 10ml of tetrahydrofuran was dissolved [2-[(E)-2-(6,7-difluoro-2-quinolinyl)ethenyl]-11-hydroxy-6,11-dihydrodibenz[b,e]oxepine (1.20 g, 2.99 mmol), and after cooling to 0°C, triethylamine (0.85 ml, 5.98 mmol) and methanesulfonyl chloride (0.30 ml, 3.89 mmol) were added to the solution, and the mixture was stirred at 0°C for 1 hour, and further at room temperature for 3 hours.

After completion of the reaction, the solvent was removed under reduced pressure. The resulting residue was dissolved in a mixed solution of 15 ml of N,N-dimethylformamide and 5 ml of tetrahydrofuran, methyl glycolate (0.54 g, 5.98 mmol) was added to the mixture and the mixture was stirred at room temperature overnight.

Then, water was added to the reaction mixture, the resulting mixture was extracted with chloroform, the organic layer was washed with water, and dried over anhydrous sodium sulfate. The solvent was removed under reduced pressure, and the resulting residue was applied to silica gel chromatography (eluent: hexane/ethyl acetate = 2/1 (volume ratio)) to obtain 0.38 g of the desired compound as white solid.

(b) [2-[(E)-2-(6,7-difluoro-2-quinolinyl)ethenyl]-6,11-dihydrodibenz[b,e]oxepin-11-yl]oxyacetic acid: (Exemplary compound 1)

In a mixed solution comprising 15 ml of methyl alcohol and 5ml of tetrahydrofuran was dissolved methyl [2-[(E)-2-(6,7-difluoro-2-quinolinyl)ethenyl]-6,11-dihydrodibenz[b,e]oxepin-11-yl]oxyacetate (0.38 g, 0.81 mmol), an aqueous 1N-sodium hydroxide solution (2.40 ml, 2.40 mmol) was added to the solution, and the mixture was stirred at room temperature for 5 hours.

After completion of the reaction, the reaction solution was adjusted to pH about 6.5 by using a dil. acetic acid aqueous solution and the mixture was concentrated under reduced pressure. Water was added to the residue and the precipitated solid was collected by filtration. The resulting solid was washed with diethyl ether, and then, dried under reduced pressure to obtain 0.26 g of the desired compound as yellowish solid.
m.p.; 206 to 208°C
FAB-MS (m/z); 460($M^+$+1)
$^1$H-NMR(δ, DMSO-$d_6$); 4.03(s, 1H), 5.02(d, J=12.2Hz, 1H), 5.49(s, 1H), 6.06(d, J=12.2Hz, 1H), 88(d, J=8.5Hz, 1H), 7.31-7.50(m, 4H), 7.65(dd, J=8.5, 2.2Hz, 1H), 7.74(d, J=16.4Hz, 1H), 7.80(d, J=1.9Hz, 1H), 7.80(d, J=16.4Hz, 1H), 7.89(d, J=8.80Hz, 1H), 7.96(dd, J=12.0, 8.0Hz, 1H), 8.02(dd, J=11.2, 9.0Hz, 1H), 8.34(d, J=8.8Hz, 1H).

(c) Sodium [2-[(E)-2-(6,7-difluoro-2-quinolinyl)ethenyl]-6,11-dihydrodibenz[b,e]oxepin-11-yl]oxyacetate

In a mixed solution comprising 15 ml of tetrahydrofuran and 5 ml of methanol was dissolved [2-[(E)-2-(6,7-difluoro-2-quinolinyl)ethenyl]-6,11-dihydrodibenz[b,e]oxepin-11-yl]-oxyacetic acid (0.27 g, 0.58 mmol), an aqueous 0.1N-sodium hydroxide solution (5.80 ml, 0.58 mmol) was added to the mixture, and the resulting mixture was stirred at room temperature for 1 hour.

After completion of the reaction, the reaction solution was concentrated, the residue was washed with a mixed solution of ethyl acetate and diethyl ether, and dried under reduced pressure to obtain 0.10 g of the desired compound as pale yellowish powder.
m.p.; 202 to 205°C
FAB-MS(m/z); 482($M^+$+1)

Example 2

(a) 3-{[2-[(E)-2-(7-chloro-6-fluoro-2-quinolinyl)ethenyl]-6,11-dihydrodibenz[b,e]oxepin-11-yl]thio}propionic acid: (Exemplary compound 155)

In a mixed solution comprising 6 ml of trifluoroacetic acid and 40 ml of methylene chloride was dissolved [2-[(E)-2-(7-chloro-6-fluoro-2-quinolinyl)ethenyl]-11-hydroxy-6,11-dihydrodibenz[b,e]oxepine (0.77 g, 1.90 mmol), and under ice-cooling, 3-mercaptopropionic acid (0.18 ml, 2.09 mmol) was added thereto and then, the mixture was stirred at room temperature for 30 minutes.

After completion of the reaction, the reaction solution was concentrated, water was added to the residue and the precipitated solid was collected by filtration. The obtained solid was dissolved in a mixed solution of chloroform : methanol = 4: 1, and the solution was dried over anhydrous sodium sulfate and the solvent was removed. The resulting residue was applied to silica gel chromatography (eluent: toluene/ethyl acetate = 4/1 (volume ratio)), washed with diethyl ether, and then, dried under reduced pressure to obtain 0.22 g of the desired compound as yellowish solid .
m.p.; 204 to 207°C
FAB-MS(m/z); 506($M^+$+1) $^1$H-NMR (δ, DMSO-$d_6$); 2.45-2.80(m, 4H), 5.01(d, J=12.9Hz, 1H), 5.37(s, 1H), 6.18(d, J=12.9Hz, 1H), 6.87(d, J=8.5Hz, 1H), 7.35(d, J=16.1Hz, 1H), 7.30-7.50(m, 4H), 7.56(dd, J=8.5, 2.2Hz, 1H), 7.71(d, J=2.2Hz, 1H), 7.77(d, J=16.4Hz, 1H), 7.93(d, J=8.8Hz, 1H), 7.99(d, J=9.8Hz, 1H), 8.19(d, J=7.1Hz, 1H), 8.35(d, J=8.8Hz, 1H), 11.50-13.00(br.s, 1H)

(b) Sodium 3-{[2-[(E)-2-(7-chloro-6-fluoro-2-quinolinyl)ethenyl]-6,11-dihydrodibenz[b,e]oxepin-11-yl]thio}propionate

In a mixed solution comprising 10 ml of methanol, 20 ml of chloroform and 5 ml of tetrahydrofuran was dissolved 3-{[2-[(E)-2-(7-chloro-6-fluoro-2-quinolinyl)ethenyl]-6,11-dihydrodibenz[b,e]oxepin-11-yl]thio}propionicacid (0.10 g, 0.20 mmol), then, at room temperature, an aqueous 1N-sodium hydroxide solution (0.20 ml, 0.20 mmol) was added to the mixture, and the resulting mixture was stirred at room temperature for 30 minutes.

After completion of the reaction, the reaction solution was concentrated, the residue was washed with diethyl ether, and dried under reduced pressure to obtain 0.09 g of the desired compound as pale yellowish solid.
m.p.; 234 to 244°C (decomposed)
FAB-MS(m/z); 528($M^+$+1)

Example 3

{1-[[2-[(E)-2-(6,7-difluoro-2-quinolinyl)ethenyll-7-fluoro-6,11-dihydrodibenz[b,e]oxepin-11-yl]thiomethyl]cyclopropyl}acetic acid trifluoroacetic acid salt: (trifluoroacetic acid salt of Exemplary compound 42)

In a mixed solution comprising 1.0 ml of trifluoroacetic acid and 20 ml of methylene chloride was dissolved [2-[(E)-2-(6,7-difluoro-2-quinolinyl)ethenyl]-7-fluoro-11-hydroxy-6,11-dihydrodibenz[b,e]oxepine (0.20 g, 0.48 mmol), [1-(mercaptomethyl)cyclopropyl]acetic acid (0.10 g, 0.68 mmol) was added to the solution, and the mixture was stirred at room temperature for 1 hour.

After completion of the reaction, the reaction solution was concentrated, water was added to the residue and the precipitated solid was collected by filtration. The resulting solid was washed with ethyl acetate, and dried under reduced pressure to obtain 0.17 g of the desired compound as yellowish solid.
m.p.; 174 to 179°C
FAB-MS(m/z); 548($M^+$+1)
$^1$H-NMR(δ, DMSO-$d_6$); 0.30-0.50(m, 4H), 2.20-2.40(m, 2H), 2.58(d, J=12.9Hz, 1H), 2.81(d, J=12.7Hz, 1H), 5.31(d, J=13.4Hz, 1H), 5.31(s, 1H), 6.04(d, J=15.9Hz, 1H), 6.92 (d, J=8.5Hz, 1H), 7.35(d, J=16.6Hz, 1H), 7.21-7.38(m, 3H), 7.61(d, J=8.8Hz, 1H), 7.68(s, 1H), 7.80(d, J=16.4Hz, 1H), 7.91(d, J=8.5Hz, 1H), 7.97(dd, J=12.0, 7.8Hz, 1H), 8,04 (dd, J=11.0, 9.0Hz, 1H), 8.37(d, J=8.9Hz, 1H), 11.00-13.00(br.s, 1H)

In the same manner as in Example 2, compounds of the following Examples 4 to 41 were obtained.

Example 4

Sodium [2-[(E)-2-(6,7-difluoro-2-quinolinyl)ethenyl]-6, 11-dihydrodibenz[b,e]oxepin-11-yl]thioacetate: (Exemplary compound 5)
Appearance; yellowish solid.
m.p.; 210 to 225°C
FAB-MS(m/z); 498($M^+$+1)
$^1$H-NMR(δ, DMSO-$d_6$); 3.14(d, J=17.8Hz, 1H), 3.19(d, J=17.5Hz, 1H), 5.01(d, J=12.9Hz, 1H), 5.44(s, 1H), 6.16(d,

J=12.7Hz, 1H), 6.87(d, J=8.3Hz, 1H), 7.31(d, J=16.4Hz, 1H), 7.35-7.45(m, 4H), 7.58(dd, J=8.5, 2.0Hz, 1H), 7.68(d, J=2.0Hz, 1H), 7.77(d, J=16.1Hz, 1H), 7.88(d, J=8.6Hz, 1H), 7.94(dd, J=11.9, 7.8Hz, 1H), 8.00(dd, J=11.0, 9.0Hz, 1H), 8.32(d, J=8.8Hz, 1H)

Example 5

(a) 3-{[2-[(E)-2-(6,7-difluoro-2-quinolinyl)ethenyl]-6,11-dihydrodibenz[b,e]oxepin-11-yl]thio}propionic acid: (Exemplary compound 6)
Appearance; yellowish solid
m.p.; 213 to 216°C
FAB-MS (m/z); 490 ($M^{+}$+1)
$^{1}$H-NMR(δ, DMSO-$d_6$); 2.49-2.72(m, 4H), 5.02(d, J=12.9Hz, 1H), 5.36(s, 1H), 6.18(d, J=12.7Hz, 1H), 6.87(d, J=3.9Hz, 1H), 7.34(d, J=16.4Hz, 1H), 7.36-7.47 (m, 4H), 7.57(dd, J=8.5, 2.2Hz, 1H), 7.71(d, J=2.2Hz, 1H), 7.79 (d, J=16.4Hz, 1H), 7.90(d, J=9.0Hz, 1H), 7.95(dd, J=12.0, 7.8Hz, 1H), 8.04(dd, J=11.2, 9.0Hz, 1H), 8.37(d, J=8.5Hz, 1H)

(b) Sodium 3-{[2-[(E)-2-(6,7-difluoro-2-quinolinyl)ethenyl]-6,11-dihydrodibenz[b,e]oxepin-11-yl]thio}propionate
Appearance: red brownish solid
m.p.; 213 to 216°C
FAB-MS(m/z); 512($M^{+}$+1)

Example 6

(a) 3-{[2-[(E)-2-(6,7-difluoro-2-quinolinyl)ethenyl]-6,11-dihydrodibenz[b,e]oxepin-11-yl]thio}-2,2-dimethylpropionic acid: (Exemplary compound 8)
Appearance; yellowish solid
FAB-MS(m/z); 518(M++1)
$^{1}$H-NMR(δ, DMSO-$d_6$); 1.10(d, J=16.6Hz, 6H), 2.56(d, J=12.9Hz, 1H), 2.85(d, J=12.9Hz, 1H), 5.02(d, J=12.9Hz, 1H), 5.23(s, 1H), 6.21(d, J=12.7Hz, 1H), 6.87(d, J=8.5Hz, 1H), 7.33(d, J=16.4Hz, 1H), 7.38-7.45(m, 4H), 7.58(dd, J=8.5, 2.2Hz, 1H), 7.66(d, J=2.2Hz, 1H), 7.78(d, J=16.4Hz, 1H), 7.88(d, J=8.8Hz, 1H), 7.95(dd, J=12.0, 7.8Hz, 1H), 8.02 (dd, J=9.0, 2.2Hz, 1H), 8.35(d, J=8.8Hz, 1H), 12.43(br.s, 1H)

(b) Sodium 3-{[2-[(E)-2-(6,7-difluoro-2-quinolinyl)ethenyl]-6,11-dihydrodibenz[b,e]oxepin-11-yl]thio}-2,2-dimethylpropionate
Appearance; yellowish solid
m.p.; 181 to 184°C
FAB-MS(m/z); 540($M^{+}$+1)

Example 7

(a) {1-[[2-[(E)-2-(6,7-difluoro-2-quinolinyl)ethenyl]-6,11dihydrodibenz[b,e]oxepin-11-yl]thiomethyl]cyclopropyl}-acetic acid: (Exemplary compound 14)
Appearance; pale yellowish solid
m.p.; 170 to 173°C
FAB-MS(m/z); 530($M^{+}$+1)
$^{1}$H-NMR(δ, DMSO-$d_6$); 0.30-0.60(m, 4H), 2.23(d, J=16.1Hz, 1H), 2.34(d, J=16.1Hz, 1H), 2.53(d, J=12.7Hz, 1H), 2.80(d, J=12.9Hz, 1H), 5.02(d, J=12.7Hz, 1H), 5.23(s, 1H), 6.24(d, J=12.7Hz, 1H), 6.87(d, J=8.5Hz, 1H), 7.30-7.50 (m, 5H), 7.58(dd, J=8.5, 2.0Hz, 1H), 7.66(d, J=2.0Hz, 1H), 7.78(d, J=16.1Hz, 1H), 7.89(d, J=8.8Hz, 1H), 7.95(dd, J=12.0, 8.1Hz, 1H), 8.02(dd, J=11.0, 8.8Hz, 1H), 8.35(d, J=8.8Hz, 1H), 11.5-12.5(br.s, 1H)

(b) Sodium {1-[[2-[(E)-2-(6,7-difluoro-2-quinolinyl)ethenyl]-6,11-dihydrodibenz[b,e]oxepin-11-yl]thiomethyl]cyclopropyl}acetate

Appearance; yellowish solid
m.p.; 137 to 139°C
FAB-MS(m/z); 552($M^+$+1)

Example 8

(a) 2-{[2-[(E)-2-(6,7-difluoro-2-quinolinyl)ethenyl]-6,11-dihydrodibenz[b,e]oxepin-11-yl]thio}benzoic acid: (Exemplary compound 16)
Appearance; yellowish solid
FAB-MS(m/z) ; 538($M^+$+1)
$^1$H-NMR(δ, DMSO-$d_6$); 5.07(d, J=12.7Hz, 1H), 6.37(s, 1H), 6.33(d, J=12.7Hz, 1H), 6.91(d, J=8.5Hz, 1H), 7.16-7.21 (m, 2H), 7.25-7.42(m, 5H), 752-7.60(m, 2H), 7.66-7.76(m, 3H), 7.86(d, J=8.5Hz, 1H), 7.95(dd, J=11.7, 7.8Hz, 1H), 8.03(dd, J=11.0, 9.0Hz, 1H), 8.35(d, J=8.8Hz, 1H)

(b) Sodium 2-{[2-[(E)-2-(6,7-difluoro-2-quinolinyl)ethenyl]-6,11-dihydrodibenz[b,e]oxepin-11-yl]thio}benzoate
Appearance; pale yellowish solid
m.p.; 183 to 186°C
FAB-MS(m/z); 560($M^+$+1)

Example 9

(a) 3-{[2-[(E)-2-(6,7-difluoro-2-quinolinyl)ethenyl]-6,11-dihydrodibenz[b,e]oxepin-11-yl]thio}benzoic acid: (Exemplary compound 17)
Appearance; yellowish solid
FAB-MS(m/z); 538($M^+$+1)
$^1$H-NMR (δ, DMSO-$d_6$); 5.11(d, J=12.9Hz, 1H), 5.79(s, 1H), 6.30(d, J=12.9Hz, 1H), 6.91(d, J=8.5Hz, 1H), 6.99(d, J=6.8Hz, 1H), 7.15(t, J=6.1Hz, 1H), 724-7.32 (m, 2H), 7.40-7.45(m, 2H), 7.56(dd, J=8.5, 2.2Hz, 1H), 7.63(J=7.8Hz, 1H), 7.71-7.76 (m, 2H), 7.84-7.90(m, 3H), 7.95(dd, J=12.0, 7.8Hz, 1H), 8.03(dd, J=11.0, 9.1HZ, 1H), 8.35(d, J=8.8Hz, 1H)

(b) Sodium 3-{[2-[(E)-2-(6,7-difluoro-2-quinolinyl)ethenyl]-6,11-dihydrodibenz[b,e]oxepin-11-yl]thio}benzoate
Appearance; pale yellowish solid
m.p.; 184 to 188°C
FAB-MS(m/z); 560($M^+$+1)

Example 10

(a) 4-{[2-[(E)-2-(6,7-difluoro-2-quinolinyl)ethenyl]-6,11-dihydrodibenz[b,e]oxepin-11-yl]thio}benzoic acid: (Exemplary compound 18)
Appearance; yellowish solid
FAB-MS(m/z); 538($M^+$+1)
$^1$H-NMR(δ, DMSO-$d_6$); 5.11(d, J=13.2Hz, 1H), 5.93(s, 1H), 6.24(d, J=12.9Hz, 1H), 6.92(d, J=8.5Hz, 1H), 7.15-7.33 (m, 4H), 7.43(d, J=7.1Hz 1H), 7.55-7.59(m, 3H), 7.72-7.77(m, 2H), 7.83-7.88(m, 3H), 7.94(dd, J=12.0, 7.8Hz, 1H), 8.03(dd, J=11.2, 9.0Hz, 1H), 8.35(d, J=8.5Hz, 1H), 13.00(br.s, 1H)

(b) Sodium 4-{[2-[(E)-2-(6,7-difluoro-2-quinolinyl)ethenyl]-6,11-dihydrodibenz[b,e]oxepin-11-yl]thio}benzoate
Appearance; pale yellowish solid
m.p.; 285 to 287°C (decomposed)
FAB-MS(m/z); 560($M^+$+1)

Example 11

Sodium 2-{[2-[(E)-2-(6,7-difluoro-2-quinolinyl)-ethenyl]-6,11-dihydrodibenz[b,e]oxepin-11-yl]thio}ethanesulfonate: (Exemplary compound 31)
Appearance; pale brownish solid.
m.p.; 246 to 254°C
FAB-MS(m/z); 548($M^+$+1)
$^1$H-NMR(δ, DMSO-$d_6$); 2.60-2.85(m; 4H), 5.00(d, J=12.7Hz, 1H), 5.38(s, 1H), 6.18(d, J=12.7Hz, 1H), 6.85(d, J=8.5Hz, 1H), 7.34-7.46(m, 5H), 7.55(dd, J=8.5, 2.0Hz, 1H), 7.75(s, 1H), 7.78(d, J=16.1Hz, 1H), 7.89(d, J=8.8Hz, 1H), 7.96(dd, J=11.9, 8.0Hz, 1H), 8.02(dd, J=11.2, 8.9Hz, 1H), 8.35(d, J=8.8Hz, 1H)

Example 12

3-{[2-[(E)-2-(6,7-difluoro-2-quinolinyl)ethenyl]-7-fluoro-6,11-dihydrodibenz[b,e]oxepin-11-yl]thio}propionic acid: (Exemplary compound 36)
m.p.; 224 to 227°C
FAB-MS(m/z); 508($M^+$+1)
$^1$H-NMR(δ, DMSO-$d_6$); 2.54-2.75(m, 4H), 5.31(d, J=13.7Hz, 1H), 5.44(s,1H), 5.98(d, J=13.7Hz, 1H), 6.91(d, J=8.5Hz, 1H), 7.35(d, J=16.4Hz, 1H), 7.24-7.47(m, 3H), 7.59(dd, J=8.5, 2.2Hz, 1H), 7.72(d, J=2.2Hz, 1H), 7.78(d, J=16.4Hz, 1H), 7.89(d, J=8.8Hz, 1H), 7.95(dd, J=11.8, 7.9Hz, 1H), 8.19(dd, J=11.0, 9.0Hz, 1H), 8.35(d, J=8.8Hz, 1H), 11.00-13.00 (br.s, 1H)

Example 13

(a) 3-{[7-cyano-2-[(E)-2-(6,7-difluoro-2-quinolinyl)ethenyl]-6,11-dihydrodibenz[b,e]oxepin-11-yl]thio}propionic acid: (Exemplary compound 60)
Appearance; yellowish solid
m.p.; 261 to 264°C (decomposed)
FAB-MS(m/z); 513($M^+$-1)
$^1$H-NMR(δ, DMSO-$d_6$); 2.55-2.66(m, 2H), 2.68-2.73(m, 2H), 5.27(d, J=13.7Hz, 1H), 5.52(s, 1H), 6.24(d, J=13.7Hz, 1H), 6.95(d, J=8.6Hz, 1H), 7.36(d, J=16.1Hz, 1H), 7.61(t, J=7.8Hz, 1H), 7.62(dd, J=8.6, 2.2Hz, 1H), 7.72 (d, J=2.2Hz, 1H), 7.78 (dd, J=7.8, 1.5Hz, 1H), 7.79(d, J=16.1Hz, 1H), 7.88(dd, J=7.8, 1.5Hz, 1H), 7.90(d, J=8.8Hz, 1H), 7.95(dd, J=11.7, 7.8Hz, 1H), 8.03(dd, J=11.0, 8.8Hz, 1H), 8.36(d, J=8.8Hz, 1H), 11.70(br.s, 1H)

(b) Sodium 3-{[7-cyano-2-[(E)-2-(6,7-difluoro-2-quinolinyl)ethenyl]-6,11-dihydrodibenz[b,e]oxepin-11-yl]thio}propionate
Appearance; yellowish white solid
m.p.; 179 to 182°C
FAB-MS(m/z); 537($M^+$+1)

Example 14

Sodium 3-{[2-[(E)-2-(6,7-difluoro-2-quinolinyl)ethenyl]-7-trifluoromethyl-6,11-dihydrodibenz[b,e]oxepin-11-yl]thio}propionate: (sodium salt of Exemplary compound 84)
Appearance; yellowish white solid
FAB-MS(m/z); 580($M^+$+1) $^1$H-NMR(δ, DMSO-$d_6$); 2.09-2.20(m, 2H), 2.58-2.61(m, 1H), 2.72-2.75(m, 1H), 5.25(d, J=14.2Hz, 1H), 5.65(s, 1H), 6.28(d, J=13.7Hz, 1H), 6.88(d, J=8.6Hz, 1H), 7.37(d, J=16.4Hz, 1H), 7.55-7.62(m, 2H), 7.76-7.81(m, 4H), 7.90(d, J=8.8Hz, 1H), 7.97(dd, J=12.0, 7.8Hz, 1H), 8.02 (dd, J=11.0, 9.0Hz, 1H), 8.35 (d, J=8.8Hz, 1H)

Example 15

Sodium 3-{[2-[(E)-2-(6,7-difluoro-2-quinolinyl)ethenyl]-7-ethynyl-6,11-dihydrodibenz[b,e]oxepin-11-yl]thio}propionate: (sodium salt of Exemplary compound 108)
Appearance; pale orange solid
m.p.; 186 to 189°C

FAB-MS(m/z); 535($M^{+}$+1) $^{1}$H-NMR(δ, DMSO-$d_6$); 2.09-2.25(m, 2H), 2.55-2.61(m, 1H), 2.68-2.78(m, 1H), 5.44(s, 1H), 5.45(d, J=12.9Hz, 1H), 6.15(d, J=12.9Hz, 1H), 6.87(d, J=8.6Hz, 1H), 7.36(d, J=16.4Hz, 1H), 7.37(t, J=7.6Hz, 1H), 7.47-7.52(m, 2H), 7.56(dd, J=8.5, 2.2Hz, 1H), 7.73(d, J=2.2Hz, 1H), 7.78(d, J=16.4Hz, 1H), 7.89(d, J=8.8Hz, 1H), 7.96 (dd, J=12.0, 8.1Hz, 1H), 8.04(dd, J=11.2Hz, 9.0Hz, 1H), 8.34(d, J=8.6Hz, 1H)

Example 16

3-{[2-[(E)-2-(7-chloro-6-fluoro-2-quinolinyl)ethenyl]-7-fluoro-6,11-dihydrodibenz[b,e]oxepin-11-yl]thio}propionic acid: (Exemplary compound 159)
Appearance; yellowish solid
m.p.; 238 to 241°C
FAB-MS(m/z); 524($M^{+}$+1) $^{1}$H-NMR(δ, DMSO-$d_6$); 2.54-2.76(m, 4H), 5.31(d, J=13.4Hz, 1H), 5.45(s, 1H), 5.99(d, J=13.4Hz, 1H), 6.91(d, J=8.5Hz, 1H), 7.30(d, J=16.4Hz, 1H), 7.24-7.47(m, 3H), 7.59(dd, J=8.5, 2.2Hz, 1H), 7.72(d, J=1.9Hz, 1H), 7.78(d, J=16.4Hz, 1H), 7.93(d, J=8.5Hz, 1H), 8.00(d, J=9.8Hz, 1H), 8.19 (d, J=7.3Hz, 1H), 8.36 (d, J=8.8Hz, 1H), 12.32(br.s, 1H)

Example 17

{1-[[2-[(E)-2-(7-chloro-6-fluoro-2-quinolinyl)ethenyl]-7-fluoro-6,11-dihydrodibenz[b,e]oxepin-11-yl]thiomethyl]cyclopropyl}acetic acid: (Exemplary compound 162)
Appearance; yellowish solid
m.p.; 237 to 239°C
FAB-MS(m/z); 564($M^{+}$+1)
$^{1}$H-NMR(δ, DMSO-$d_6$); 0.30-0.50(m, 4H), 2.23(d, J=15.9Hz, 1H), 2.31(d, J=15.9Hz, 1H), 2.58(d, J=12.9Hz, 1H), 2.82 (d, J=12.7Hz, 1H), 5.31(d, J=13.4Hz, 1H), 5.31(s, 1H), 6.04(d, J=13.4Hz, 1H), 6.91(d, J=8.5Hz, 1H), 7.35(d, J=16.4Hz, 1H), 7.21-7.44(m, 3H), 7.61(dd, J=8.5, 1.9Hz, 1H), 7.68(d, J=1.9Hz, 1H), 7.80(d, J=16.4Hz, 1H), 7.94(d, J=8.5Hz, 1H), 8.00(d, J=9.8Hz, 1H), 8.19(d, J=7.3Hz, 1H), 8.36(d, J=8.8Hz, 1H), 12.08(br.s, 1H)

Example 18

(a) 3-{[2-[(E)-2-(7-fluoro-2-quinolinyl)ethenyl]-6,11-dihydrodibenz[b,e]oxepin-11-yl]thio}propionic acid: (Exemplary compound 171)
Appearance; pale yellowish solid
$^{1}$H-NMR(δ, DMSO-$d_6$); 2.45-2.80(m, 4H), 5.02(d, J=12.7Hz, 1H), 5.37(s, 1H), 6.19(d, J=12.7Hz, 1H), 6.87(d, J=8.5Hz, 1H), 7.30-7.55(m, 6H), 7.58(dd, J=8.5, 2.2Hz, 1H), 7.70(dd, J=10.3, 2.4Hz, 1H), 7.72(s, 1H), 7.80(d, J=15.6Hz, 1H), 7.84(d, J=8.5Hz, 1H), 8.04(dd, J=9.0, 6.3Hz, 1H), 8.38(d, J=8.8Hz, 1H), 12.33(br.s, 1H)

(b) Sodium 3-{[2-[(E)-2-(7-fluoro-2-quinolinyl)ethenyl]-6,11-dihydrodibenz[b,e]oxepin-11-yl]thio}propionate
Appearance; yellowish solid
m.p.; 160 to 163°C
FAB-MS(m/z); 494($M^{+}$+1)

Example 19

Sodium 3-{[2-[(E)-2-(7-fluoro-2-quinolinyl)ethenyl]-6,11-dihydrodibenz[b,e]oxepin-11-yl]thio}-2-(R)-methylpropionate: (sodium salt of Exemplary compound 172) Appearance; pale yellowish powder
m.p.; 195 to 205°C
FAB-MS(m/z); 508($M^{+}$+1)
$^{1}$H-NMR(δ, DMSO-$d_6$); 0.98-1.03(m, 3H), 2.15-2.90(m, 3H), 5.00(d, J=12.7Hz, 1H), 5.33(d, J=8.6, 1H), 6.23(dd, J=12.7, 6.1Hz, 1H), 6.85(d, J=8.5Hz, 1H), 7.33-7.51(m, 6H), 7.56(d, J=8.6Hz, 1H), 7.69-7.86(m, 4H), 8.03(dd, J=9.0, 6.6Hz, 1H), 8.38(d, J=8.5Hz, 1H),

Example 20

(a) {1-[[2-[(E)-2-(7-fluoro-2-quinolinyl)ethenyl]-6,11-dihydrodibenz[b,e]oxepin-11-yl]thiomethyl]cyclopropyl}acetic acid: (Exemplary compound 174)
Appearance; yellowish solid
FAB-MS(m/z); 512($M^+$+1)
$^1$H-NMR (δ, DMSO-$d_6$); 0.30-0.60 (m, 4H), 2.23(d, J=15.9Hz, 1H), 2.34(d, J=15.9Hz, 1H), 2.53(d, J=12.7Hz, 1H), 2.81(d, J=12.9Hz, 1H), 5.02(d, J=12.9Hz, 1H), 5.23(s, 1H), 6.24(d, J=12.7Hz, 1H), 6.87(d, J=8.5Hz, 1H), 7.35(d, J=16.1Hz, 1H), 7.30-7.50(m, 4H), 7.48(dd, J=8.5, 2.7Hz, 1H), 7.59(dd, J=8.5, 2.0Hz, 1H), 7.68(d, J=2.2Hz, 1H), 7.70(dd, J=10.5, 2.7Hz, 1H), 7.80(d, J=16.8Hz, 1H), 7.84(d, J=8.8Hz, 1H), 8.03(dd, J=9.0, 6.6Hz, 1H), 8.38(d, J=8.5Hz, 1H), 11.5-12.5(br.s, 1H)

(b) Sodium {1-[[2-[(E)-2-(7-fluoro-2-quinolinyl)ethenyl]-6,11-dihydrodibenz[b,e]oxepin-11-yl]thiomethyl]cyclopropyl}acetate
Appearance; yellowish solid
m.p; 149 to 152°C
FAB-MS(m/z) ;534($M^+$+1)

Example 21

[2-[(E)-2-(7-chloro-2-quinolinyl)ethenyl]-6,11-dihydrodibenz[b,e]oxepin-11-yl]thioacetic acid: (Exemplary compound 223)
Appearance; yellowish red solid
m.p.; 221 to 231°C (decomposed)
FAB-MS(m/z); 474($M^+$+1)
$^1$H-NMR(δ, DMSO-$d_6$); 3.25(s, 2H), 5.04(d, J=12.7Hz, 1H), 5.40(s, 1H), 6.18(d, J=12.7Hz, 1H), 6.91(d, J=8.5Hz, 1H), 7.30-7.50(m, 5H), 7.62(dd, J=8.8, 2.0Hz, 2H), 7.68(d, J=1.7Hz, 1H), 7.88(d, J=16.4Hz, 1H), 7.98(d, J=8.5Hz, 1H), 8.02 (d, J=2.9Hz, 1H)

Example 22

3-{[2-[(E)-2-(7-chloro-2-quinolinyl)ethenyl]-6,11-dihydrodibenz[b,e]oxepin-11-yl]thio}propionic acid: (Exemplary compound 224)
Appearance; yellowish solid
m.p.; 195 to 198°C
FAB-MS(m/z); 488($M^+$+1)
$^1$H-NMR(δ, DMSO-$d_6$); 2.45-2.75(m, 4H), 5.02(d, J=12.7Hz, 1H), 5.37(s, 1H), 6.19(d, J=12.7Hz, 1H), 6.87(d, J=8.5Hz, 1H), 7.35(d, J=16.4Hz, 1H), 7.30-7.50(m, 4H), 7.58(dd, J=8.8, 2.2Hz, 2H), 7.72(d, J=2.2Hz, 1H), 7.79(d, J=16.4Hz, 1H), 7.89(d, J=8.8Hz, 1H), 7.99(d, J=9.0Hz, 1H), 8.01(s, 1H), 8.38(d, J=8.5Hz, 1H), 12.27(br.s, 1H)

Example 23

Sodium 3-{[2-[(E)-2-(7-chloro-2-quinolinyl)ethenyl]-6,11-dihydrodibenz[b,e]oxepin-11-yl]thio}-2-(R)-methylpropionate: (sodium salt of Exemplary compound 225)
Appearance; yellowish powder
m.p.; 207 to 217°C
FAB-MS (m/z); 524 ($M^+$+1)
$^1$H-NMR(δ, DMSO-$d_6$); 0.97-1.04(m, 3H), 2.17-2.90(m, 3H), 4.99(dd, J=12.7, 2.69Hz, 1H), 5.34(d, J=10.0, 1H), 6.23 (dd, J=12.7, 6.8Hz, 1H), 6.84(d, J=8.6Hz, 1H), 7.33-7.42(m, 5H), 7.56(d, J=5.6Hz, 1H), 7.57(dd, J=8.55, 2.2Hz, 1H), 7.74-7.82(m, 2H), 7.89(dd, J=8.7, 1.8Hz, 1H), 7.97-8.02(m, 2H), 8.34(d, J=8.6Hz, 1H)

Example 24

{1-[[2-[(E)-2-(7-chloro-2-quinolinyl)ethenyl]-6,11-dihydrodibenz[b,e]oxepin-11-yl]thiomethyl]cyclopropyl}acetic acid: (Exemplary compound 227)
Appearance; yellowish solid

m.p.; 204 to 206°C
FAB-MS(m/z); 528($M^+$+1)
$^1$H-NMR(δ, DMSO-$d_6$); 0.30-0.60(m, 4H), 2.22(d, J=16.1Hz, 1H), 2.33(d, J=16.1Hz, 1H), 2.53(d, J=12.9Hz, 1H), 2.80 (d, J=12.9Hz, 1H), 5.02(d, J=12.7Hz, 1H), 5.23 (s, 1H), 6.23(d, J=12.7Hz, 1H), 6.87(d, J=8.5Hz, 1H), 7.30-7.50(m, 5H), 7.58(dd, J=8.5, 2.2Hz, 1H), 7.59(dd, J=8.3, 2.2Hz, 1H), 7.67(d, J=2.2Hz, 1H), 7.80(d, J=16.4Hz, 1H), 7.89(d, J=8.8Hz, 1H), 7.99(d, J=8.8Hz, 1H), 8.01(s, 1H), 8.38(d, J=8.5Hz, 1H), 12.08(br.s, 1H)

Example 25

3-{[2-[(E)-2-(5,6,7,8-tetrahydro-2-quinolinyl)ethenyl]-6,11-dihydrodibenz[b,e]oxepin-11-yl]thio}propionic acid: (Exemplary compound 229)
Appearance; pale yellowish solid
m.p.; 150 to 153°C
FAB-MS(m/z); 480($M^+$+H)
$^1$H-NMR(δ, DMSO-$d_6$); 1.65-1.95(m, 4H), 2.00-2.25(m, 2H), 2.45-2.95(m, 6H), 4.96(d, J=12.7Hz, 1H), 5.30(s, 1H), 6.18(d, J=12.7Hz, 1H), 6.79(d, J=8.5Hz, 1H), 7.11(d, J=16.1Hz, 1H), 7.25-7.55(m, 8H), 7.60(d, J=2.2Hz, 1H)

Example 26

Sodium 3-{[2-[(E)-2-(5,6,7,8-tetrahydro-2-quinolinyl)ethenyl]-6,11-dihydrodibenz[b,e]oxepin-11-yl]thio}-2-(R)-methylpropionate: (sodium salt of Exemplary compound 230) Appearance; pale yellowish powder
m.p.; 168 to 173°C
FAB-MS(m/z); 494($M^+$+1)
$^1$H-NMR(δ, DMSO-$d_6$); 1.02(t, 3H), 1.74-1.84 (m, 4H), 2.12-2.26(m, 2H), 2.68-2.88(m, 5H), 4.96(dd, J=12.7, 1.9Hz, 1H), 5.28(d, J=8.3, 1H), 6.20(dd, J=12.7, 5.4Hz, 1H), 6.80(d, J=8.5Hz, 1H), 7.10(dd, J=16.1, 4.6Hz, 1H), 7.27-7.49(m, 8H), 7.61(d, J=2.9Hz, 1H)

Example 27

Sodium 3-{[2-[(E)-2-(5,6,7,8-tetrahydro-2-quinolinyl)ethenyl]-6,11-dihydrodibenz[b,e]oxepin-11-yl]thio}-3-methylbutanoate: (sodium salt of Exemplary compound 234)
Appearance; yellowish white-tinted powder
m.p.; 168 to 175°C
FAB-MS(m/z); 508($M^+$+1)
$^1$H-NMR(δ, DMSO-$d_6$); 1.35(s, 3H), 1.37(s, 3H), 1.74-1.84(m, 4H), 2.26(d, J=13.2Hz, 1H), 2.31(d, J=13.2Hz, 1H), 2.71-2.85(m, 4H), 4.96(d, J=12.7Hz, 1H), 5.56(s, 1H), 6.20(d, J=12.7Hz, 1H), 6.74(d, J=8.5Hz, 1H), 7.09(d, J=16.1Hz, 1H), 7.28-7.50(m, 9H), 7.66(d, J=2.2Hz, 1H)

Example 28

(a) {1-{[2-[(E)-2-(5,6,7,8-tetrahydro-2-quinolinyl)ethenyl]-6,11-dihydrodibenz[b,e]oxepin-11-yl]thiomethyl]cyclopropyl}acetic acid: (Exemplary compound 235)
Appearance; yellow greenish solid
$^1$H-NMR(δ, DMSO-$d_6$); 0.30-0.60(m, 4H), 1.70-1.90(m, 4H), 2.22(d, J=15.9Hz, 1H), 2.32(d, J=15.6Hz, 1H), 2.45-2.90(m, 6H), 4.99(d, J=12.7Hz, 1H), 5.18(s, 1H), 6.20(d, J=12.7Hz, 1H), 6.81(d, J=8.5Hz, 1H), 7.10(d, J=16.1Hz, 1H), 7.15-7.45(m, 4H), 7.47(d, J=16.4Hz, 1H), 7.47(dd, J=8.8, 2.2Hz, 1H), 7.56(d, J=2.2Hz, 1H), 11.0-13.0(br.s, 1H)

(b) Sodium {1-[[2-[(E)-2-(5,6,7,8-tetrahydro-2-quinolinyl)-ethenyl]-6,11-dihydrodibenz[b,e]oxepin-11-yl]thiomethyl] cyclopropyl}acetate
Appearance; pale yellowish solid
m.p.; 142 to 145°C
FAB-MS(m/z); 520($M^+$+1)

Example 29

(a) 3-{[2-[(E)-2-(4-chloro-2-quinolinyl)ethenyl]-6,11-dihydrodibenz[b,e]oxepin-11-yl]thio}propionic acid: (Exemplary compound 336)
Appearance; yellowish solid
FAB-MS(m/z); 488($M^+$+1)
$^1$H-NMR(δ, DMSO-$d_6$); 2.40-2.75(m, 4H), 5.02(d, J=12.9Hz, 1H), 5.36(s, 1H), 6.18(d, J=12.7Hz, 1H), 6.88(d, J=8.5Hz, 1H), 7.34(d, J=16.4Hz, 1H), 7.35-7.50(m, 4H), 7.57(dd, J=8.5, 2.2Hz, 1H), 7.65-7.75(m, 1H), 7.72(d, J=2.4Hz, 1H), 7.85(d, J=16.8Hz, 1H), 7.80-7.90(m, 1H), 8.05(d, J=8.5Hz, 1H), 8.13(s, 1H), 8.16(d, J=8.3Hz, 1H), 12.29(br.s, 1H)

(b) Sodium 3-{[2-[(E)-2-(4-chloro-2-quinolinyl)ethenyl]-6,11-dihydrodibenz[b,e]oxepin-11-yl]thio}propionate
Appearance; yellowish solid
m.p.; 140 to 142°C
FAB-MS(m/z); 510($M^+$+1)

Example 30

(a) 3-{[2-[(E)-2-(5-fluoro-2-quinolinyl)ethenyl]-6,11-dihydrodibenz[b,e]oxepin-11-yl]thio}propionic acid: (Exemplary compound 340)
Appearance; yellowish solid
$^1$H-NMR(δ, DMSO-$d_6$); 2.45-2.80(m, 4H), 5.02(d, J=12.9Hz, 1H), 5.36(s, 1H), 6.19(d, J=12.7Hz, 1H), 7.10-7.50 (m, 6H), 7.60(dd, J=8.5, 2.2Hz, 2H), 7.65-7.80(m, 2H), 7.82(d, J=9.0Hz, 1H), 7.83(d, J=16.6Hz, 1H), 7.94(d, J=8.8Hz, 1H), 8.45(d, J=8.8Hz, 1H), 12.27(br.s, 1H)

(b) Sodium 3-{[2-[(E)-2-(5-fluoro-2-quinolinyl)ethenyl]-6,11-dihydrodibenz[b,e]oxepin-11-yl]thio}propionate
Appearance; yellowish solid
m.p.; 150 to 152°C
FAB-MS(m/z); 494($M^+$+1)

Example 31

(a) {1-[[2-[(E)-2-(5-fluoro-2-quinolinyl)ethenyl]-6,11-dihydrodibenz[b,e]oxepin-11-yl]thiomethyl]cyclopropyl}acetic acid: (Exemplary compound 343)
Appearance; yellowish solid
$^1$H-NMR(δ, DMSO-$d_6$) ; 0.30-0.60 (m, 4H), 2.24(d, J=16.1Hz, 1H), 2.35(d, J=16.1Hz, 1H), 2.55(d, J=12.7Hz, 1H), 2.82(d, J=12.9Hz, 1H), 5.03(d, J=12.7Hz, 1H), 5.24(s, 1H), 6.26(d, J=12.7Hz, 1H), 6.89(d, J=8.5Hz, 1H), 7.10-7.50 (m, 6H), 7.62(dd, J=8.5, 2.0Hz, 1H), 7.71(d, J=2.0Hz, 1H), 7.47(td, J=7.8, 6.1Hz, 1H), 7.83(d, J=8.5Hz, 1H), 7.85 (d, J=16.1Hz, 1H), 7.96(d, J=8.8Hz, 1H), 8.47(d, J=8.5Hz, 1H), 12.13(br.s, 1H)

(b) Sodium {1-[[2-[(E)-2-(5-fluoro-2-quinolinyl)ethenyl]-6,11-dihydrodibenz[b,e]oxepin-11-yl]thiomethyl]cyclopropyl}acetate
Appearance; yellowish solid
m.p.; 142 to 144°C
FAB-MS(m/z) ; 534($M^+$+1)

Example 32

(a) 3-{[2-[(E)-2-(5,7-difluoro-2-quinolinyl)ethenyl]-6,11-dihydrodibenz[b,e]oxepin-11-yl]thio}propionic acid: (Exemplary compound 348)

Appearance; yellowish solid
FAB-MS(m/z); 490 ($M^{+}$+1)
$^{1}$H-NMR(δ, DMSO-$d_6$); 2.40-2.80(m, 4H), 5.02(d, J=12.9Hz, 1H), 5.36(s, 1H), 6.19(d, J=12.7Hz, 1H), 6.88(d, J=8.5Hz, 1H), 7.30-7.50 (m, 5H), 7.52(td, J=10.0, 2.4Hz, 1H), 7.55-7.65(m, 1H), 7.59(dd, J=8.5, 2.2Hz, 1H), 7.73 (d, J=2.2Hz, 1H), 7.85(d, J=16.4Hz, 1H), 7.91(d, J=8.8Hz, 1H), 8.45(d, J=8.8Hz, 1H), 12.0-12.5(br.s, 1H)
(b) Sodium 3-{[2-[(E)-2-(5,7-difluoro-2-quinolinyl)ethenyl]-6,11-dihydrodibenz[b,e]oxepin-11-yl]thio}propionate
Appearance; pale yellowish solid
m.p.; 210 to 213°C
FAB-MS(m/z); 512 ($M^{+}$+1)

Example 33

(a) {1-[[2-[(E)-2-(5,7-difluoro-2-quinolinyl)ethenyl]-6,11-dihydrodibenz[b,e]oxepin-11-yl]thiomethyl]cyclopropyl} acetic acid: (Exemplary compound 351)
Appearance; yellowish solid
$^{1}$H-NMR(δ, DMSO-$d_6$) ; 0.30-0.60(m, 4H), 2.22(d, J=15.9Hz, 1H), 2.33(d, J=15.9Hz, 1H), 2.52(d, J=12.9Hz, 1H), 2.80(d, J=12.9Hz, 1H), 5.02(d, J=12.7Hz, 1H), 5.23(s, 1H), 6.24 (d, J=12.7Hz, 1H), 6.87(d, J=8.5Hz, 1H), 7.36(d, J=16.4Hz, 1H), 7.30-7.50(m, 4H), 7.52(dd, J=10.0, 2.4Hz, 1H), 7.61(dd, J=8.5, 2.0Hz, 2H), 7.69(d, J=2.0Hz, 1H), 7.85(d, J=16.4Hz, 1H), 7.92(d, J=8.8Hz, 1H), 8.45(d, J=8.8Hz, 1H), 11.5-12.5(br.s, 1H)

(b) Sodium {1-[[2-[(E)-2-(5,7-difluoro-2-quinolinyl)ethenyl]-6,11-dihydrodibenz[b,e]oxepin-11-yl]thiomethyl]cyclopropyl}acetate
Appearance; yellowish solid
m.p.; 149 to 152°C
FAB-MS(m/z); 552 ($M^{+}$+1)

Example 34

(a) 3-{[2-[(E)-2-(5,6,7-trifluoro-2-quinolinyl)ethenyl]-6,11-dihydrodibenz[b,e]oxepin-11-yl]thio}propionic acid: (Exemplary compound 356)
Appearance; yellowish solid
FAB-MS(m/z); 508($M^{+}$+1)
$^{1}$H-NMR(δ, DMSO-$d_6$); 2.45-2.80 (m, 4H), 5.02(d, J=12.9Hz, 1H), 5.36(s, 1H), 6.19(d, J=12.7Hz, 1H), 6.87(d, J=8.5Hz, 1H), 7.36(d, J=16.4Hz), 7.35-7.50(m, 4H), 7.58(dd, J=8.5, 2.2Hz, 1H), 7.72(d, J=2.2Hz, 1H), 7.84(d, J=16.4Hz, 1H), 7.87(ddd, J=11.7, 7.1, 2.0Hz, 1H), 7.97(d, J=8.8Hz, 1H), 8.49(d, J=8.8Hz, 1H), 11.5-13.0(br.s, 1H)

(b) Sodium 3-{[2-[(E)-2-(5,6,7-trifluoro-2-quinolinyl)ethenyl]-6,11-dihydrodibenz[b,e]oxepin-11-yl]thio}propionate
Appearance; pale yellowish solid
m.p.; 235 to 237°C
FAB-MS(m/z); 530($M^{+}$+1)

Example 35

(a) {1-[[2-[(E)-2-(5,6,7-trifluoro-2-quinolinyl)ethenyl]-6,11-dihydrodibenz[b,e]oxepin-11-yl]thiomethyl]cyclopropyl} acetic acid: (Exemplary compound 359)
Appearance; yellowish solid
FAB-MS(m/z); 570($M^{+}$+1)
$^{1}$H-NMR(δ, DMSO-$d_6$); 0.30-0.60(m, 4H), 2.22(d, J=15.9Hz, 1H), 2.34(d, J=16.1Hz, 1H), 2.52 (d, J=12.5Hz, 1H), 2.80(d, J=12.7Hz, 1H), 5.02(d, J=12.7Hz, 1H), 5.23(s, 1H), 6.24(d, J=12.7Hz, 1H), 6.87(d, J=8.5Hz, 1H), 7.36(d, J=16.4Hz, 1H), 7.30-7.50(m, 4H), 7.60(dd, J=8.5, 2.0Hz, 2H), 7.68(d, J=1.7Hz, 1H), 7.84(d, J=16.4Hz, 1H), 7.80-7.95(m, 1H), 7.98(d, J=9.0Hz, 1H), 8.49(d, J=8.8Hz, 1H), 12.1(br.s, 1H)

(b) Sodium {1-[[2-[(E)-2-(5,6,7-trifluoro-2-quinolinyl)ethenyl]-6,11-dihydrodibenz[b,e]oxepin-11-yl]thiomethyl]cyclopropyl}acetate
Appearance; yellowish solid
m.p.; 195 to 198°C
FAB-MS(m/z); 570($M^+$+1)

Example 36

(a) 3- {[2-[(E)-2-(5-fluoro-2-benzothiazolyl)ethenyl]-6,11-dihydrodibenz[b,e]oxepin-11-yl]thio}propionic acid: (Exemplary compound 364)
Appearance; yellowish solid
FAB-MS(m/z); 478 ($M^+$+1)
$^1$H-NMR(δ, DMSO-$d_6$); 2.45-2.80(m, 4H), 5.03(d, J=12.9Hz, 1H), 5.32(s, 1H), 6.20(d, J=12.7Hz, 1H), 6.87(d, J=8.5Hz, 1H), 7.33(td, J=9.0, 2.4Hz, 1H), 7.35-7.50(m, 4H), 7.48(d, J=16.1Hz, 1H), 7.62(d, J=16.4Hz, 1H), 7.64 (dd, J=8.5, 2.2Hz, 1H), 7.75(d, J=2.2Hz, 1H), 7.79(dd, J=10.0, 2.4Hz, 1H), 8.13(dd, J=9.0, 5.4Hz, 1H), 12.31(br.s, 1H)

(b) Sodium 3-{[2-[(E)-2-(5-fluoro-2-benzothiazolyl)ethenyl]-6,11-dihydrodibenz[b,e]oxepin-11-yl]thio}propionate
Appearance; pale yellowish solid
m.p.;>300°C
FAB-MS(m/z); 500($M^+$+1)

Example 37

(a) {1-[[2-[(E)-2-(5-fluoro-2-benzothiazolyl)ethenyl]-6,11-dihydrodibenz[b,e]oxepin-11-yl]thiomethyl]cyclopropyl} acetic acid: (Exemplary compound 367)
Appearance; yellowish solid
FAB-MS (m/z); 518($M^+$+1)
$^1$H-NMR (δ, DMSO-$d_6$); 0.30-0.60(m, 4H), 2.22(d, J=16.1Hz, 1H), 2.34(d, J=15.9Hz, 1H), 2.53 (d, J=12.7Hz, 1H), 2.80 (d, J=12.9Hz, 1H), 5.03(d, J=12.7Hz, 1H), 5.19(s, 1H), 6.25(d, J=12.5Hz, 1H), 6.87(d, J=8.5Hz, 1H), 7.10-7.50 (m, 5H), 7.47(d, J=16.1Hz, 1H), 7.63(d, J=15.9Hz, 1H), 7.64(dd, J=8.8, 2.2Hz, 1H), 7.72(d, J=2.2Hz, 1H), 7.79 (dd, J=10.0, .4Hz, 1H), 8.12(dd, J=9.0, 5.4Hz, 1H), 11.5-12.5(br.s, 1H)

(b) Sodium {1-[[2-[(E)-2-(5-fluoro-2-benzothiazolyl)ethenyl]-6,11-dihydrodibenz[b,e]oxepin-11-yl]thiomethyl]cyclopropyl}acetate
Appearance; yellowish solid
m.p.; 155 to 157°C
FAB-MS(m/z); 540($M^+$+1)

Example 38

(a) 3-{[2-[(E)-2-(5-fluoro-2-benzoxazolyl)ethenyl]-6,11-dihydrodibenz[b,e]oxepin-11-yl]thio}propionic acid: (Exemplary compound 372)
Appearance; pale brownish solid
FAB-MS(m/z); 462($M^+$+1)
$^1$H-NMR(δ, DMSO-$d_6$); 2.45-2.75(m, 4H), 5.03(d, J=12.7Hz, 1H), 5.32(s, 1H), 6.20(d, J=13.2Hz, 1H), 6.88(d, J=8.3Hz, 1H), 7.17(d, J=16.1Hz, 1H), 7.25(ddd, J=9.8, 8.8, 2.4Hz, 1H), 7.35-7.50(m, 4H), 7.59(dd, J=8.8, 2.9Hz, 1H), 7.66(dd, J=8.8, 2.0Hz, 1H), 7.74(dd, J=8.8, 4.4Hz, 1H), 7.76(d, J=16.6Hz, 1H), 7.78(d, J=2.4Hz, 1H), 12.0-13.0(br.s, 1H)

(b) Sodium 3-{[2-[(E)-2-(5-fluoro-2-benzoxazolyl)ethenyl]-6,11-dihydrodibenz[b,e]oxepin-11-yl]thio}propionate
Appearance; brownish solid

m.p.; 247 to 250°C
FAB-MS(m/z); 484($M^+$+1)

Example 39

(a) 3-{[2-[(E)-2-(6-isopropyl-5-methyl-2-pyridyl)ethenyl]-6,11-dihydrodibenz[b,e]oxepin-11-yl]thio}propionic acid: (Exemplary compound 380)
Appearance; yellowish solid
FAB-MS(m/z); 460($M^+$+1)
$^1$H-NMR(δ, DMSO-$d_6$); 1.25(d, J=6.6Hz, 6H), 2.27(s, 3H), 2.44-2.68(m, 4H), 3.20-3.35(m, 1H), 4.98(d, J=12.9Hz, 1H), 5.36(s, 1H), 6.17(d, J=12.7Hz, 1H), 7.10(d, J=15.9Hz, 1H), 7.24(d, J=7.8Hz, 1H), 7.37-7.55(m, 7H), 7.59(d, J=2.2Hz, 1H)

(b) Sodium 3-{[2-[(E)-2-(6-isopropyl-5-methyl-2-pyridyl)-ethenyl]-6,11-dihydrodibenz[b,e]oxepin-11-yl]thio}propionate
Appearance; white solid
m.p.; 137 to 139°C
FAB-MS(m/z); 482($M^+$+1)

Example 40

Sodium 3-{[3-[(E)-2-(6,7-difluoro-2-quinolinyl)ethenyl]-10,11-dihydro-5H-dibenz[a,d]cyclohepten-5-yl]thio} propionate: (sodium salt of Exemplary compound 384)
Appearance; yellowish solid
m.p.; 179 to 182°C
FAB-MS(m/z); 509($M^+$)
$^1$H-NMR(δ, DMSO-$d_6$); 2.00-2.20(m, 2H), 2.52-2.70(m, 2H), 2.80-3.00(m, 2H), 3.50-4.00(m, 2H), 5.33(s, 1H), 7.00-7.60 (m, 4H), 7.22(d, J=7.8Hz, 1H), 7.46(d, J=16.1Hz, 1H), 7.54(d, J=7.3Hz, 1H), 7.65-8.20(m, 3H), 7.81(d, J=16.1Hz, 1H), 7.91(d, J=8.8Hz, 1H), 8.36(d, J=8.5Hz, 1H)

Example 41

Sodium 3-{[2-[(E)-2-(6,7-difluoro-2-quinolinyl)ethenyl]-6,11-dihydrodibenz[b,e]thiepin-11-yl]thio}propionate: (sodium salt of Exemplary compound 400)
Appearance; pale brownish solid
m.p.; 144 to 148°C
FAB-MS(m/z); 528($M^+$+1)
$^1$H-NMR(δ, DMSO-$d_6$); 2.10-2.30(m, 2H), 2.55-2.65(m, 2H), 3.60-4.10(br.s, 1H), 5.10-6.00 (br.s, 1H), 5.50(s, 1H), 7.09 (d, J=8.1Hz, 1H), 7.20-7.60(m, 5H), 7.43(d, J=15.6, 1H), 7.77(d, J=16.4, 1H), 7.80-8.20(m, 4H), 8.35(d, J=9.0Hz, 1H)

Example 42

(a) Ethyl 1-{[2-[(E)-2-(6,7-difluoro-2-quinolinyl)ethenyl]-6,11-dihydrodibenz[b,e]oxepin-11-yl]thiomethyl}cyclopropanecarboxylate: (ethyl ester of Exemplary compound 9)

In a mixed solution comprising 10 ml of methylene chloride and 3 ml of trifluoroacetic acid was dissolved [2-[(E)-2-(6,7-difluoro-2-quinolinyl)ethenyl]-11-hydroxy-6,11-dihydrodibenz[b,e]oxepine (1.0 g, 2.49 mmol), and ethyl 1-(mercaptomethyl)cycloprpanecarboxylate (0.80 g, 4.98 mmol) was added to the solution and the mixture was stirred at room temperature for 1 hour.

After completion of the reaction, the reaction solution was concentrated, water was added to the residue, a pH of the mixture was adjusted to about 6.5 with sodium hydrogen carbonate, and then, the mixture was extracted with chloroform. The organic layer was washed with water, dried over anhydrous sodium sulfate and then concentrated, and applied to silica gel chromatography (eluent: hexane/ethyl acetate = 2/1 (volume ratio)) to obtain 1.30 g of the desired compound as pale yellowish viscous oily product.
EI-MS(m/z); 543($M^+$), CI-MS(m/z); 544($M^+$+1)

(b) 1-{[2-[(E)-2-(6,7-difluoro-2-quinolinyl)ethenyl]-6,11-dihydrodibenz[b,e]oxepin-11-yl]thiomethyl}cyclopropanecarboxylic acid: (Exemplary compound 9)

In a mixed solution comprising 20 ml of methanol and 10 ml of tetrahydrofuran was dissolved ethyl 1-{[2-[(E)-2-(6,7-difluoro-2-quinolinyl)ethenyl]-6,11-dihydrodibenz[b,e]oxepin-11-yl]thiomethyl}cyclopropanecarboxylate (1.3 g, 2.4 mmol), and an aqueous 1N-sodium hydroxide solution (7.2 ml, 7.2 mmol) was added to the solution and the mixture was stirred at room temperature for 15 hours.

After completion of the reaction, the reaction solution was adjusted to pH about 6.5 by usig a dil. acetic acid aqueous solution, and the mixture was concentrated under reduced pressure. The residue was applied to silica gel chromatography (eluent: hexane/ethyl acetate = 1/1 (volume ratio)) to obtain 0.39 g of the desired compound as pale yellowish solid.

FAB-MS(m/z); 516($M^+$+1)

$^1$H-NMR(δ, DMSO-$d_6$); 0.53-0.58(m, 1H), 0.82-0.87(m, 1H), 1.02-1.09(m, 1H), 2.60(d, J=13.5Hz, 1H), 2.98(d, J=13.1Hz, 1H), 5.02(d, J=12.7Hz, 1H), 5.33(s, 1H), 6.20(d, J=12.7Hz, 1H), 6.87(d, J=8.5Hz, 1H), 7.30-7.47(m, 5H), 7.58(dd, J=8.5, 2.2Hz, 1H), 7.67(d, J=2.2Hz, 1H), 7.78(d, J=16.3Hz, 1H), 7.90(d, J=8.79Hz, 1H), 7.95(dd, J=12.0, 8.06Hz, 1H), 8.03(dd, J=11.0, 8.8Hz, 1H), 8.34(d, J=8.8Hz, 1H), 12.4(br.s, 1H)

(c) Sodium 1-{[2-[(E)-2-(6,7-difluoro-2-quinolinyl)ethenyl]-6,11-dihydrodibenz[b,e]oxepin-11-yl]thiomethyl}cyclopropanecarboxylate

In a mixed solution comprising 15 ml of tetrahydrofuran and 5 ml of methanol was dissolved 1-{[2-[(E)-2-(6,7-difluoro-2-quinolinyl)ethenyl]-6,11-dihydrodibenz[b,e]oxepin-11-yl]thiomethyl}cyclopropanecarboxylic acid (0.39 g, 0.76 mmol), an aqueous 0.5N-sodium hydroxide solution (1.52 ml, 0.76 mmol) was added to the solution and the mixture was stirred at room temperature for 2 hours.

After completion of the reaction, the reaction solution was concentrated, the residue was washed with diethyl ether, and dried under reduced pressure to obtain 0.35 g of the desired compound as pale yellowish powder.

m.p.; 175 to 185°C

FAB-MS(M/Z); 538 ($M^+$+1)

In the same manner as in Example 42, the following Compounds of Examples 43 to 45 were obtained.

Example 43

Sodium 3-{[2-[(E)-2-(6,7-difluoro-2-quinolinyl)ethenyl]-6,11-dihydrodibenz[b,e]oxepin-11-yl]thio}-2-(S)-methylpropionate: (sodium salt of Exemplary compound 7)

Appearance; ocherous powder

m.p.; 193 to 216°C

FAB-MS(m/z); 526 ($M^+$+1)

$^1$H-NMR(δ, DMSO-$d_6$); 0.95-1.00(m, 3H), 2.14-2.89(m, 3H), 4.98(dd, J=12.5, 2.9Hz, 1H), 5.34(d, J=13.7Hz, 1H), 6.20 (dd, J=12.3, 10.2Hz, 1H), 6.83(dd, J=8.5, 1.22Hz, 1H), 7.33-7.41 (m, 5H), 7.53(dd, J=8.5, 2.2Hz, 1H), 7.75(s, 1H), 7.78(d, J=16.1Hz, 1H), 7.89(dd, J=8.5, 2.6Hz, 1H), 7.97(dd, J=12.0, 8.3Hz, 1H), 8.03(dd, J=11.0, 8.8Hz, 1H), 8.35(d, J=8.8Hz, 1H)

Example 44

Sodium 3-{[2-[(E)-2-(6,7-difluoro-2-quinolinyl)ethenyl]-6,11-dihydrodibenz[b,e]oxepin-11-yl]thio}-2-(R)-methylpropionate: (sodium salt of Exemplary compound 7)

Appearance; ocherous powder

m.p.; 196 to 220°C

FAB-MS(m/z); 526($M^+$+1)

$^1$H-NMR(δ, DMSO-$d_6$); 0.96-1.01 (m, 3H), 2.20-2.89(m, 3H), 4.98(dd, J=12.7, 2.7Hz, 1H), 5.34(d, J=11.7Hz, 1H), 6.21 (dd, J=12.5, 8.3Hz, 1H), 6.83(d, J=8.8Hz, 1H), 7.33-7.44(m, 5H), 7.46(s, 1H), 7.55(d, J=8.5Hz, 1H), 7.77(d, J=16.11 1H), 7.89(d, J=8.5Hz, 1H), 7.97 (dd, J=12.0, 8.1Hz, 1H), 8.03 (dd, J=11.0, 8.8Hz, 1H), 8.34 (d, J=8.8Hz, 1H)

Example 45

Sodium 3-{[2-[(E)-2-(6,7-difluoro-2-quinolinyl)ethenyl]-6,11-dihydrodibenz[b,e]oxepin-11-yl]thio}-2-ethylpropionate: (sodium salt of Exemplary compound 10)

Appearance; pale yellowish powder

m.p.; 209 to 218°C

FAB-MS(m/z); 540($M^+$+1)
$^1$H-NMR(δ, DMSO-$d_6$); 0.78(t, 3H), 1.41(m, 2H), 2.06(m, 1H), 2.54-2.63(m, 2H), 4.97(d, J=12.7Hz, 1H), 5.31(s, 1H), 6.22(d, J=12.5Hz, 1H), 6.83(d, J=8.5Hz, 1H), 7.33-7.41(m, 5H), 7.54(d, J=8.5Hz, 1H), 7.77(d, J=16.4Hz, 1H), 7.78(d, J=2.2Hz, 1H), 7.90(d, J=8.8Hz, 1H), 7.99(dd, J=12.45, 7.57Hz, 1H), 8.03(dd, J=11.2, 9.0Hz, 1H), 8.35(d, J=8.8Hz, 1H)

Example 46

(a) 3-[2-[(E)-2-(6,7-difluoro-2-quinolinyl)ethenyl]-6,11-dihydrodibenz[b,e]oxepin-11-yl]thio-N-trifluoromethanesulfonylpropionamide: (Exemplary compound 28)

In 20 ml of tetrahydrofuran were dissolved 3-[2-[(E)-2-(6,7-difluoro-2-quinolinyl)ethenyl]-6,11-dihydrodibenz [b,e]oxepin-11-yl]thiopropionic acid (0.54 g, 1.1 mmol) and trifluoromethanesulfonamide (0.24 g, 1.6 mmol), and then, dimethylaminopyridine(0.13 g, 1.1 mmol) and ethyl(dimethylaminopropyl)carbodiimide hydrochloride (0.34 g, 1.8 mmol) were successively added to the solution and the mixture was stirred at room temperature for 23 hours.

After completion of the reaction, water was added to the reaction mixture, the resulting mixture was extracted with ethyl acetate, the organic layer was washed with a saturated aqueous sodium chloride solution, and dried over anhydrous sodium sulfate. The solvent was concentrated, and the resulting residue was applied to silica gel chromatography (eluent: hexane/ethyl acetate = 1/1 (volume ratio)) to obtain 0.30 g of the desired compound as yellowish solid.
m.p.; 152 to 156°C
FAB-MS(m/z); 621($M^+$+1)
$^1$H-NMR(δ, DMSO-$d_6$); 2.35-2.70(m, 4H), 5.00(d, J=12.7Hz, 1H), 5.35(s, 1H), 6.18(d, J=12.7Hz, 1H), 6.85 (d, J=8.6Hz, 1H), 7.35(d, J=16.3Hz, 1H), 7.35-7.46(m, 4H), 7.56(dd, J=8.6, 2.2Hz, 1H), 7.72(d, J=2.2Hz, 1H), 7.78 (d, J=16.4Hz, 1H), 7.89(d, J=8.8Hz, 1H), 7.95(dd, J=12.0, 8.2Hz, 1H), 8.02(dd, J=11.0, 9.03Hz, 1H), 8.35(d, J=8.8Hz, 1H)

(b) 3-[2-[(E)-2-(6,7-difluoro-2-quinolinyl)ethenyl]-6,11-dihydrodibenz[b,e]oxepin-11-yl]thio-N-trifluoromethanesulfonylpropionamide hydrochloride

In 2 ml of tetrahydrofuran was dissolved sodium 3-[2-[(E)-2-(6,7-difluoro-2-quinolinyl)ethenyl]-6,11-dihydrodibenz[b,e]oxepin-11-yl]thio-N-trifluoromethanesulfonylpropionamide (0.12 g, 0.19 mmol), 1N-hydrochloric acid (0.20 ml, 0.20 mmol) was added to the solution and the mixture was stirred at room temperature for 10 minutes. The formed precipitate was further diluted with distilled water and the formed precipitate was collected by filtration. The obtained solid was washed with distilled water and dried under reduced pressure to obtain 0.10 g of the desired compound as yellowish solid.
m.p.; 234 to 238°C
FAB-MS(m/z); 621($M^+$+1)
$^1$H-NMR(δ, DMSO-$d_6$); 2.44-2.67(m, 4H), 5.02(d, J=12.7Hz, 1H), 5.36(s, 1H), 6.18(d, J=12.7Hz, 1H), 6.89(d, J=8.5Hz, 1H), 7.35(d, J=16.1Hz, 1H), 7.36(m, 4H), 7.58(dd, J=8.5, 2.2Hz, 1H), 7.74(d, J=2.2Hz, 1H), 7.90(d, J=16, 1Hz, 1H), 7.99(dd, J=11.5, 7.6Hz, 1H), 8.04(d, J=9.3Hz, 1H), 8.13(dd, J=11.0, 8.8Hz, 1H), 8.52(d, J=8.8Hz, 1H)

Example 47

(a) [2-[(E)-2-(6,7-difluoro-2-quinolinyl)ethenyl]-6,11-dihydrodibenz[b,e]oxepin-11-yl]thio-N-methanesulfonylacet amide: (Exemplary compound 22)

In 20 ml of tetrahydrofuran were dissolved [2-[(E)-2-(6,7-difluoro-2-quinolinyl)ethenyl]-6,11-dihydrodibenz[b, e]oxepin-11-yl]thioacetic acid (0.66 g, 1.33 mmol) and trifluoromethanesulfonamide (0.20 g, 2.10 mmol), and then, dimethylaminopyridine (0.39 g, 4.42 mmol) and ethyl(dimethylaminopropyl)carbodiimide hydrochloride (0.37 g, 1.93 mmol) were successively added to the solution, and the mixture was stirred at room temperature for 1.5 hours.

After completion of the reaction, water was added to the reaction solution, and the mixture was adjusted to pH about 5.0 with 1N-hydrochloric acid. The mixture was extracted with ethyl acetate, and the organic layer was washed with a saturated aqueous sodium chloride solution and dried over anhydrous sodium sulfate. The solvent was concentrated and the resulting residue was applied to silica gel chromatography (eluent: hexane/ethyl acetate = 2/3 (volume ratio)) to obtain 0.23 g of the desired compound as yellowish solid.
m.p.; 228 to 234°C (decomposed)
FAB-MS (m/z) ; 553 ($M^+$+1)

$^1H$-NMR(δ, DMSO-$d_6$); 3.25(s, 3H), 3.30-3.39(m, 2H), 5.04(d, J=12.9Hz, 1H), 5.37(s, 1H), 6.16(d, J=12.9Hz, 1H), 6.90(d, J=8.3Hz, 1H), 7.34(d, J=16.1Hz, 1H), 7.37-7.48(m, 4H), 7.61(dd, J=8.6, 2.0Hz, 1H), 7.64(d, J=2.0Hz, 1H), 7.78(d, J=16.4Hz, 1H), 7.87(d, J=8.5Hz, 1H), 7.96(dd, J=12.0, 7.8Hz, 1H), 8.02(dd, J=11.0, 9.0Hz, 1H), 8.35(d, J=8.6Hz, 1H), 11.93(br.s, 1H)

(b) [2-[(E)-2-(6,7-difluoro-2-quinolinyl)ethenyl]-6,11-dihydrodibenz[b,e]oxepin-11-yl]thio-N-methanesulfonylacetamide sodium salt

In 4 ml of tetrahydrofuran was dissolved under heating [2-[(E)-2-(6,7-difluoro-2-quinolinyl)ethenyl]-6,11-dihydrodibenz[b,e]oxepin-11-yl]thio-N-methanesulfonylacetamide (0.11 g, 0.20 mmol), and an aqueous 1N-sodium hydroxide solution (0.2 ml, 0.20 mmol) was added to the solution and the mixture was stirred at room temperature for 1 hour.

After completion of the reaction, the solvent was removed under reduced pressure, diethyl ether was added to the resulting residue to form crystal, and the crystal was collected by filtration, washed with diethyl ether and dried under reduced pressure to obtain 0.07 g of the desired compound as beige color solid.
m.p.; 178 to 183°C
FAB-MS(m/z); 575($M^+$+1)

In the same manner as in Example 47, the compounds of the following Examples 48 to 49 were obtained.

Example 48

[2-[(E)-2-(6,7-difluoro-2-quinolinyl)ethenyl]-6,11-di hydrodibenz[b,e]oxepin-11-yl]thio-N-trifluoromethanesulfonylacetamide sodium salt: (sodium salt of Exemplary compound 23)
Appearance; orange solid.
m.p.; 182 to 186°C
FAB-MS(m/z); 629($M^+$+1)
$^1H$-NNR(δ, DMSO-$d_6$); 2.96(d, J=14.4Hz, 1H), 3.06(d, J=14.7Hz, 1H), 5.01(d, J=12.7Hz, 1H), 5.53(s, 1H), 6.11(d, J=12.9Hz, 1H), 6.87(d, J=8.3Hz, 1H), 7.34(d, J=16.4Hz, 1H), 7.33-7.45(m, 4H), 7.57(dd, J=8.5, 2.0Hz, 1H), 7.71(d, J=2.0Hz, 1H), 7.77(d, J=16.4Hz, 1H), 7.84(d, J=8.6Hz, 1H), 7.96(dd, J=12.0, 8.05Hz, 1H), 8.01(dd, J=11.2, 8.8Hz, 1H), 8.35(d, J=8.6Hz, 1H)

Example 49

(a) 3-[2-[(E)-2-(6,7-difluoro-2-quinolinyl)ethenyl]-6,11-dihydrodibenz[b,e]oxepin-11-yl]thio-N-methanesulfonylpropionamide: (Exemplary compound 27)
Appearance; orange solid.
FAB-MS (m/z) ; 567($M^+$+1)
$^1H$-NMR(δ, DMSO-$d_6$); 2.65-2.72(m, 4H), 3.25(s, 3H), 5.02(d, J=12.9Hz, 1H), 5.35(s, 1H), 6.15(d, J=12.7Hz, 1H), 6.87(d, J=8.3Hz, 1H), 7.35(1H, J=16.4Hz, 1H), 7.39-7.46(m, 4H), 7.57(dd, J=8.6, 2.2Hz, 1H), 7.71(d, J=2.2Hz, 1H), 7.78(d, J=16.4Hz, 1H), 7.87(d, J=8.5Hz, 1H), 7.94(dd, J=12.0, 7.8Hz, 1H), 8.03(dd, J=11.0, 8.8Hz, 1H), 8.36 (d, J=8.5Hz, 1H), 11.83(s, 1H)

(b) 3-[2-[(E)-2-(6,7-difluoro-2-quinolinyl)ethenyl]-6,11-dihydrodibenz[b,e]oxepin-11-yl]thio-N-methanesulfonylpropionamide sodium salt
Appearance; brownish solid
m.p.; 187 to 192°C
FAB-MS (m/z); 589 ($M^+$+1)

Example 50

(a) N-t-butoxycarbonyl-N-{2-[2-[(E)-2-(6,7-difluoro-2quinolinyl)ethenyl]-6,11-dihydrodibenz[b,e]oxepin-11-yl] thioethyl}trifluoromethanesulfonamide

In 10 ml of tetrahydrofuran was dissolved [2-[(E)-2-(6,7-difluoro-2-quinolinyl)ethenyl]-11-(2-hydroxyethyl)thio-6,11-dihydrodibenz[b,e]oxepine (0.29 g, 0.63 mmol), and then, N-t-butoxycarbonyltrifluoromethanesulfonamide (0.26 g, 1.04 mmol) and triphenylphosphine (0.26 g, 0.99 mmol) were added to the solution successively, and

further a diethyl azodicarboxylate 2.2M toluene solution (0.40 ml, 0.88 mmol) was added to the mixture and the mixture was stirred at room temperature for 5 hours.

After completion of the reaction, the reaction solution was concentrated as such and the residue was applied to silica gel chromatography (eluent: hexane/ethyl acetate = 9/1 (volume ratio)) to obtain 0.39 g of the desired compound as orange solid.
FAB-MS(m/z); 693 ($M^+$+1)
$^1$H-NMR (δ, DMSO-$d_6$); 1.46(s, 9H), 2.71-2.75(m, 2H), 3.94-4.07(m, 2H), 5.04(d, J=12.9Hz, 1H), 5.39(s, 1H), 6.18 (d, J=12.9Hz, 1H), 6.90(d, J=8.3Hz, 1H), 7.36(d, J=15.9Hz, 1H), 7.39-7.49 (m, 4H), 7.59(dd, J=8.6, 2.2Hz, 1H), 7.78(d, J=2.2Hz, 1H), 7.85(d, J=16.1Hz, 1H), 7.95(dd, J=11.5, 7.8Hz, 1H), 7.96(d, J=8.5Hz, 1H), 8.09(dd, J=11.0, 8.8Hz, 1H), 8.46(d, J=8.8Hz, 1H)

(b) N-{2-[2-[(E)-2-(6,7-difluoro-2-quinolinyl)ethenyl]6,11-dihydrodibenz[b,e]oxepin-11-yl]thioethyl}trifluoromethanesulfonamide: (Exemplary compound 26)

In 5 ml of tetrahydrofuran was dissolved N-t-butoxycarbonyl-N-{2-[2-[(E)-2-(6,7-difluoro-2-quinolinyl)ethenyl]-6,11-dihydrodibenz[b,e]oxepin-11-yl]thioethyl}trifluoromethanesulfonamide (0.14 g, 0.20 mmol), and 7 ml of trifluoroacetic acid was added to the solution and the mixture was stirred at room temperature for 2 hours until the mixture becomes uniform solution.

After completion of the reaction, water was added to the reaction mixture, the resulting mixture was extracted with ethyl acetate, the organic layer was washed successively with a saturated aqueous sodium hydrogen carbonate solution and a saturated aqueous sodium chloride solution, and dried over anhydrous sodium sulfate. The solvent was concentrated and the resulting residue was applied to silica gel chromatography (eluent: hexane/ethyl acetate = 9/1 (volume ratio)) to obtain 0.06 g of the desired compound as pale yellowish solid.
m.p.; 84 to 87°C
FAB-MS (m/z); 593 ($M^+$+1)
$^1$H-NMR(δ, DMSO-$d_6$); 2.63(t, J=7.1Hz, 2H), 3.29-3.36(m, 2H), 5.02(d, J=12.7Hz, 1H), 5.40(s, 1H), 6.20(d, J=12.9Hz, 1H), 6.88(d, J=8.6Hz, 1H), 7.35(d, J=16.4Hz, 1H), 7.38-7.47(m, 4H), 7.58(dd, J=8.3, 2.2Hz, 1H), 7.75 (d, J=2.2Hz, 1H), 7.78(d, J=16.4Hz, 1H), 7.86(d, J=8.8Hz, 1H), 7.93(dd, J=11.7, 7.8Hz, 1H), 8.03(dd, J=11.0, 9.0Hz, 1H), 8.36(d, J=8.8Hz, 1H), 9.57(br.s, 1H)

Example 51

(a) Ethyl 4-[2-trifluoromethanesulfonyloxy-6,11-dihydrodibenz[b,e]oxepin-11-yl]butanoate

In 4 ml of methylene chloride was dissolved ethyl 4-[2-hydroxy-6,11-dihydrodibenz[b,e]oxepin-11-yl]butanoate (0.34 g, 1.0 mmol), and then, triethylamine(0.6 ml, 4.3 mmol) and trifluoromethanesulfonic anhydride (0.4 ml, 2.6 mmol) were successively added to the solution and the mixture was stirred at room temperature for 20 minutes.

After completion of the reaction, the reaction solution was concentrated, and the resulting residue was applied to silica gel chromatography (eluent: hexane/ethyl acetate = 92/8 (volume ratio)) to obtain 0.37 g of the desired compound as pale yellowish liquid.
CI-MS(m/z); 459($M^+$+1), EI-MS(m/z); 458($M^+$)
$^1$H-NMR(δ, $CDCl_3$); 1.22(t, J=7.1Hz, 3H), 1.55 (quintet, J=7.7Hz, 2H), 2.06-2.17(m, 2H), 2.28(t, J=7.3Hz, 2H), 3.79 (t, J=7.8Hz, 1H), 4.10(q, J=7.1Hz, 2H), 4.98(d, J=14.4Hz, 1H), 5.52(d, J=14.4Hz, 1H), 6.99-7.12(m, 4H), 7.18-7.25 (m, 3H)

(b) Ethyl 4-[2-[(E)-2-(6,7-difluoro-2-quinolinyl)ethenyl]-6,11-dihydrodibenz[b,e]oxepin-11-yl]butanoate

In 5 ml of N,N-dimethylformamide was dissolved ethyl 4-[2-trifluoromethanesulfonyloxy-6,11-dihydrodibenz[b,e]oxepin-11-yl]butanoate (0.36 g, 0.79 mmol), and then, tetra-n-butyl ammonium chloride (0.27 g, 0.97 mmol), sodium hydrogen carbonate (0.33 g, 3.11 mmol), lithium bromide (0.12 g, 1.38 mmol), 6,7-difluoro-2-vinylquinoline (0.30 g, 1.57 mmol) and tetrakis(triphenylphosphine)palladium (0) (70 mg, 0.06 mmol) were successively added to the solution and the mixture was stirred at room temperature for 6 hours under argon atmosphere.

After completion of the reaction, water was added to the reaction solution, the mixture was extracted with toluene, and the organic layer was washed with a saturated aqueous sodium chloride solution and dried over anhydrous sodium sulfate. The solvent was concentrated and the resulting residue was applied to silica gel chromatography (eluent: hexane/ethyl acetate = 9/1 (volume ratio)) to obtain 91 mg of the desired compound as yellowish solid.
CI-MS(m/z); 500($M^+$+1), EI-MS(m/z); 499($M^+$)
$^1$H-NMR(δ, $CDCl_3$); 1.23(t, J=7.1Hz, 3H), 1.61 (quintet, J=7.3Hz, 2H), 2.05-2.27(m, 2H), 2.30(t, J=7.1Hz, 2H), 3.85(t, J=7.8Hz, 1H), 4.10(q, J=7.1Hz, 2H), 5.00(d, J=14.4Hz, 1H), 5.56(d, J=14.2Hz, 1H), 6.99(d, J=8.8Hz, 1H), 7.11-7.14(m, 1H), 7.19-7.24(m, 4H), 7.43-7.45(m, 2H), 7.50(dd, J=10.3, 8.3Hz, 1H), 7.61(d, J=8.6Hz, 1H), 7.64(d, J=15.9Hz, 1H), 7.80(dd, J=11.2, 7.8Hz, 1H), 8.04(d, J=8.8Hz, 1H)

(c) Sodium 4-[2-[(E)-2-(6,7-difluoro-2-quinolinyl)ethenyl]-6,11-dihydrodibenz[b,e]oxepin-11-yl]butanoate: (sodium salt of Exemplary compound 32)

In 2 ml of dioxane was dissolved ethyl 4-[2-[(E)-2-(6,7-difluoro-2-quinolinyl)ethenyl]-6,11-dihydrodibenz[b,e]oxepin-11-yl]butanoate (133 mg, 0.27 mmol), and 2 ml of an aqueous 2N-sodium hydroxide solution was added to the solution and the mixture was stirred at room temperature for 2 hours.

After completion of the reaction, 4 ml of 1N-hydrochloric acid was added to the reaction solution, the mixture was extracted with ethyl acetate, and the organic layer was washed with a saturated aqueous sodium chloride solution, dried over anhydrous sodium sulfate and concentrated. Then, the resulting concentrated residue was dissolved in 2 ml of tetrahydrofuran, and an aqueous 1N-sodium hydroxide solution (0.25 ml, 0.25 mmol) was added to the mixture and the mixture was concentrated. To the obtained residue was added diethyl ether, and the formed precipitate was collected by filtration and washed with diethyl ether and dried under reduced pressure to obtain 87 mg of the desired compound as gray solid.

m.p.; 211 to 220°C

FAB-MS(m/z); 494($M^+$+1)

$^1$H-NMR(δ, DMSO-$d_6$); 1.30-1.51(m, 2H), 1.92(t, J=7.4Hz, 2H), 2.05(q, J=7.3Hz; 2H), 4.01(t, J=7.3Hz, 1H), 5.01 (d, J=13.9Hz, 1H), 5.57(d, J=13.9Hz, 1H), 6.90(d, J=8.3Hz, 1H), 7.23-7.27(m, 4H), 7.35(d, J=16.4Hz, 1H), 7.51 (dd, J=8.3, 2.0Hz, 1H), 7.61(d, J=2.0Hz, 1H), 7.78(d, J=16.4Hz, 1H), 7.88(d, J=8.8Hz, 1H), 7.95(dd, J=12.0, 7.8Hz, 1H), 8.02(dd, J=11.2, 9.0Hz, 1H), 8.34(d, J=8.8Hz, 1H)

Example 52

(a) 3-{[2-[(E)-2-(6,7-difluoro-2-quinolinyl)ethenyl]-6,11-dihydrodibenz[b,e]oxepin-11-yl]thio}propionic acid: (optical resolution of Exemplary compound 6)

(+)-(S)-Camphor-10-sulfonic acid monohydrate (167 mg, 0.67 mmol) was added to a mixed solution comprising 10 ml of N,N-dimethylformamide and 40 ml of acetonitrile containing 3-{[2-[(E)-2-(6,7-difluoro-2-quinolinyl)ethenyl]-6,11-dihydrodibenz[b,e]oxepin-11-yl]thio}propionicacid (730mg, 1.49 mmol) obtained in Example 5(a), and the mixture was stirred at room temperature for 1 hour. The precipitated crystal was filtered off, and after adding (-)- (R)-camphor-10-sulfonic acid monohydrate (167 mg, 0.67 mmol) to the filtrate, and after the mixture was stirred at room temperature for 1 hour, precipitated crystal was collected by filtration. The resulting crystal was dissolved in 2.5 ml of dimethylsulfoxide, and then, 7.5 ml of water and sodium hydrogen carbonate (45 ml, 0.54 mmol) were added to the solution, and the mixture was stirred at room temperature for 30 minutes. The formed slurry liquid was adjusted to pH about 4.0 with an aqueous 10% acetic acid solution, and precipitated crystal was collected by filtration. The obtained crystal was dissolved in a mixed solution comprising 3 ml of N,N-dimethylformamide and 12 ml of acetonitrile, and then, (-)-(R)-camphor-10-sulfonic acid monohydrate (167 mg, 0.67 mmol) was added to the solution and the mixture was stirred at room temperature for 30 minutes, and the precipitated crystal was collected by filtration. The obtained crystal was dissolved in 1.5 ml of dimethylsulfoxide, 4.5 ml of water and sodium hydrogen carbonate (33 mg, 0.39 mmol) were added to the solution and the mixture was stirred at room temperature for 1 hour. The formed slurry liquid was adjusted to pH about 4.0 with an aqueous 10% acetic acidsolution, and after collecting the crystal, it was dried under reduced pressure to obtain 159 mg of yellowish solid. This solid was subjected to HPLC analysis (Column CHIRAL-CEL OJ-R 0.46 ϕx 15cm, eluent: $CH_3CN$/2.0 mM $H_3PO_4$-$KH_2PO_4$ buffer (pH = 4.0) = 70/30(v/v), flow rate: 1.0 ml/min, measurement wavelength: 254 nm, measurement temperature: 40°C), it was detected peaks at retension times of 6.8 and 13.2 minutes with a ratio of 95.5 : 4.5, and optical purity was 91.0% ee.

Example 53

3-{[2-[(E)-2-(5,6,7,8-tetrahydro-2-quinolinyl)ethenyl]-8-methoxycarbonyl-6,11-dihydrodibenz[b,e]oxepin-11-yl]thio}propionic acid: (Exemplary compound 1125)

In 40 ml of methylene chloride was dissolved [2-[(E)-2-(5,6,7,8-tetrahydro-2-quinolinyl)ethenyl]-11-methoxy-8-methoxycarbonyl-6,11-dihydrodibenz[b,e]oxepine (0.20 g, 0.45 mmol), and then, 3-mercaptopropionic acid (0.14 ml, 0.54 mmol) and boron trifluoride-diethyl ether complex (0.047 ml, 1.10 mmol) were successively added to the solution and the mixture was stirred at room temperature for 4 hours.

After completion of the reaction, water was added to the reaction solution, and the mixture was adjusted to pH about 6.0 with a saturated aqueous sodium hydrogen carbonate solution and extracted with chloroform. The organic layer was washed with a saturated aqueous sodium chloride solution, dried over anhydrous sodium sulfate and concentrated. To the resulting residue was added diethyl ether to form crystal, and then, the mixture was filtered. The obtained crystal was washed with diethyl ether and dried under reduced pressure to obtain 0.17 g of the desired compound as yellowish solid.
m.p.; 121 to 123°C
FAB-MS(m/z); 516($M^+$+1)
$^1$H-NMR(δ, DMSO-$d_6$); 1.60-1.90 (m, 4H), 2.60-2.90(m, 4H), 3.87(s, 3H), 5.15(d, J=12.9Hz, 1H), 5.43 (s, 1H), 6.09(d, J=12.7Hz, 1H), 6.84(d, J=8.3Hz, 1H), 7.10(d, J=15.9Hz, 1H), 7.28(d, J=8.1Hz, 1H), 7.43 (d, J=8.3Hz, 1H), 7.47(d, J=16.1Hz, 1H), 7.48 (dd, J=8.5, 2.2Hz, 1H), 7.54(d, J=7.8Hz, 1H), 7.62(d, J=2.2Hz, 1H), 7.95(dd, J=7.8, 2.0Hz, 1H), 8.04(d, J=1.7Hz, 1H)
In the same manner as in Example 2, compounds of the following Examples 54 to 58 were obtained.

Example 54

(a) 3-{[9-[(E)-2-(6,7-difluoro-2-quinolinyl)ethenyl]-6,11-dihydrodibenz[b,e]oxepin-11-yl]thio}propionic acid: (Exemplary compound 404)
Appearance; yellow greenish solid
$^1$H-NMR(δ, DMSO-$d_6$); 2.63-2.73(m, 4H), 5.00(d, J=13.2Hz, 1H), 5.33 (s, 1H), 6.10 (d, J=12.9Hz, 1H), 6.83 (d, J=8.2Hz, 1H), 6.96 (t, J=7.0Hz, 1H), 7.19(t, J=7.6Hz, 1H), 7.33(d, J=7.9Hz, 1H), 7.48(d, J=10.7Hz, 1H), 7.52(d, J=19.2Hz, 1H), 7.69(d, J=7.8Hz, 1H), 7.82-8.08(m, 5H), 8.38(d, J=8.8Hz, 1H)

(b) Sodium 3-{[9-[(E)-2-(6,7-difluoro-2-quinolinyl)ethenyl]6,11-dihydrodibenz[b,e]oxepin-11-yl]thio}propionate
Appearance; yellowish white-tinted solid
m.p.; >300°C
FAB-MS(m/z); 512 ($M^+$+1)

Example 55

(a) 3-{[3-[{E)-2-(6,7-difluoro-2-quinolinyl)ethenyl]-10,11-dihydro-5H-dibenz[a,d]cyclohepten-5-yl]thio}-2-(S)-methylpropionic acid: (Exemplary compound 385)
Appearance; yellow greenish solid
$^1$H-NMR(δ, DMSO-$d_6$); 1.05(d, J=6.8Hz, 3H), 2.41-2.73 (m, 3H), 2.87(m, 2H), 3.70-3.74(m, 2H), 5.33(s, 1H), 7.15-7.25(m, 4H), 7.33(d, J=6.4Hz, 1H), 7.46(d, J=16.4Hz, 1H), 7.57(d, J=7.8Hz, 1H), 7.72(s, 1H), 7.81(d, J=16.6Hz, 1H), 7.91(dd, J=8.6, 2.7Hz, 1H), 7.96(dd, J=9.2, 3.7Hz, 1H), 8.04(dd, J=11.1, 8.9Hz, 1H), 8.34(d, J=8.8Hz, 1H)

(b) Sodium 3-{[3-[(E)-2-(6,7-difluoro-2-quinolinyl)ethenyl]10,11-dihydro-5H-dibenz[a,d]cyclohepten-5-yl]thio}-2-(S)methylpropionate
Appearance; white powder
m.p.; 211 to 220°C
FAB-MS(m/z); 524 ($M^+$+1)

Example 56

(a) 3-{[2-[(E)-2-(6-fluoro-7-trifluoromethyl-2-quinolinyl)ethenyl]-6,11-dihydrodibenz[b,e]oxepin-11-yl]thio}propionic acid: (Exemplary compound 352)
Appearance; pale yellowish solid
FAB-MS(m/z); 540($M^+$+1)
$^1$H-NMR(δ, DMSO-$d_6$); 2.45-2.80(m, 4H), 5.02(d, J=12.9Hz, 1H), 5.37(s, 1H), 6.19(d, J=12.7Hz, 1H), 6.88(d, J=8.5Hz, 1H), 7.30-7.50(m, 5H), 7.58(dd, J=8.5, 2.2Hz, 1H), 7.72(d, J=2.2Hz, 1H), 7.84(d, J=16.4Hz, 1H), 8.06(d, J=8.5Hz, 1H), 8.08(d, J=11.2Hz, 1H), 8.35(d, J=6.8Hz, 1H), 8.44(d, J=8.8Hz, 1H), 12.0-12.7(br.s, 1H)

(b) Sodium 3-{[2-[(E)-2-(6-fluoro-7-trifluoromethyl-2-quinolinyl)ethenyl]-6,11-dihydrodibenz[b,e]oxepin-11-yl]thio} propionate
Appearance; yellowish solid
m.p.; 221 to 224°C (decomposed)
FAB-MS(m/z); 494 ($M^+$+1)

Example 57

(a) 3-{[2-[(E)-2-(6,7-difluoro-2-quinolinyl)ethenyl]-8-fluoro-6,11-dihydrodibenz[b,e]oxepin-11-yl]thio}propionic acid: (Exemplary compound 44)
Appearance; yellowish solid
$^1$H-NMR(δ, DMSO-$d_6$); 8.35(d, J=8.6Hz, 1H), 8.06-7.31(m, 9H), 7.22(td, J=9.0Hz, J=2.7Hz, 1H), 6.89(d, J=8.6Hz, 1H), 6.13(d, J=12.9Hz, 1H), 5.42(s, 1H), 5.03(d, J=12.9Hz, 1H), 2.66(t, J=5.9Hz, 2H), 2.53(t, J=6.1Hz, 2H)

(b) Sodium 3-{[2-[(E)-2-(6,7-difluoro-2-quinolinyl)ethenyl]-8-fluoro-6,11-dihydrodibenz[b,e]oxepin-11-yl]thio}propionate
Appearance; pale yellowish glossy solid
m.p.; 243 to 250°C

Example 58

3-{[2-[(E)-2-(6,7-difluoro-2-quinolinyl)ethenyl]-6,11-dihydrodibenz[b,e]oxepin-11-yl]thio}-3-methylbutanoic acid: (sodium salt of Exemplary compound 12)
Appearance; pale yellowish solid
FAB-MS(m/z); 540($M^+$+1)
$^1$H-NMR(δ, DMSO-$d_6$); 1.36(s, 3H), 1.39(s, 3H), 2.29(d, J=18.7Hz, 1H), 2.34(d, J=18.7Hz, 1H), 4.99(d, J=12.8Hz, 1H), 5.64(s, 1H), 6.24(d, J=12.8Hz, 1H), 6.80(d, J=8.5Hz, 1H), 7.30-7.80(m, 8H), 7.90(d, J=8.8Hz, 1H), 7.97(dd, J=8.1, 7.8Hz, 1H), 8.02(dd, J=11.2, 9.0Hz, 1H), 8.34(d, J=8.8Hz, 1H)

Reference example 1

(a) 2-[(E)-2-(7-chloro-6-fluoro-2-quinolinyl)ethenyl]-6,11-dihydrodibenz[b,e]oxepin-11-one

A 20 ml of acetic anhydride solution containing 7-chloro-6-fluoroquinaldine (0.82 g, 4.20 mmol) and 2-formyl-6,11-dihydrodibenz[b,e]oxepin-11-one (1.00 g, 4.20 mmol) was stirred at 125°C for 72 hours.

After completion of the reaction, the reaction mixture was concentrated under reduced pressure, and a saturated aqueous sodium hydrogen carbonate solution was added to the residue and the mixture was extracted with ethyl acetate. The organic layer was washed with a saturated aqueous sodium chloride solution, dried over anhydrous sodium sulfate and concentrated. The obtained residue was applied to silica gel chromatography (eluent: toluene to toluene/ethyl acetate = 25/1 (volume ratio)) to obtain 1.40 g of the desired compound as yellowish brown solid. $^1$H-NMR(δ, DMSO-$d_6$); 5.36(s, 2H), 7.20(d, J=8.5Hz, 1H), 7.49(d, J=8.5Hz, 1H), 7.50-7.75(m, 5H), 7.73(dd, J=8.5, 2.4Hz, 1H), 7.80(d, J=7.8Hz, 1H), 7.96(d, J=9.8Hz, 1H), 8.14(d, J=7.3Hz, 1H), 8.23(d, J=2.0Hz, 1H), 8.26 (d, J=8.5Hz, 1H)

(b) [2-[(E)-2-(7-chloro-6-fluoro-2-quinolinyl)ethenyl]-11-hydroxy-6,11-dihydrodibenz[b,e]oxepine

After suspending [2-[(E)-2-(7-chloro-6-fluoro-2-quinolinyl)ethenyl]-6,11-dihydrodibenz[b,e]oxepin-11-one (1.40 g, 3.47 mmol) in a mixed solution comprising 40 ml of tetrahydrofuran and 40 ml of methanol, sodium borohydride (0.16 g, 4.16 mmol) was added to the suspension, and the mixture was stirred at room temperature overnight.

After completion of the reaction, the reaction mixture was concentrated, and the obtained residue was applied to silica gel chromatography (eluent: toluene to toluene/ethyl acetate = 9/1 (volume ratio)) to obtain 0.77 g of the desired compound as yellowish solid.
FAB-MS(m/z); 418($M^+$+1)

$^1$H-NMR(δ, DMSO-$d_6$); 5.30(d, J=12.2Hz, 1H), 5.77(d, J=14.2Hz, 1H), 5.97(d, J=3.9Hz, 1H), 6.27(d, J=3.9Hz, 1H), 6.83(d, J=8.5Hz, 1H), 7.25-7.60(m, 5H), 7.70(dd, J=8.5, 2.2Hz, 1H), 7.80(d, J=15.6Hz, 1H), 7.92(d, J=8.8Hz, 1H)

Reference example 2

(a) [2-[(E)-2-(6,7-difluoro-2-quinolinyl)ethenyl]-6,11-dihydrodibenz[b,e]oxepin-11-one

To 15 ml of tetrahydrofuran suspension containing [(6,7-difluoro-2-quinolinyl)methyl]triphenylphosphonium bromide (1.35 g, 2.59 mmol) suspended therein was added 1.7 ml of n-butyl lithium 1.57M hexane solution at -78°C, and the mixture was stirred at the same temperature for 30 minutes. Then, a solution of 2-formyl-6,11-dihydrodibenz[b,e]oxepin-11-one (0.59 g, 2.48 mmol) dissolved in 15 ml of tetrahydrofuran was added to the above mixture at -78°C over 15 minutes, and the mixture was stirred at the same temperature for 1 hour.

After completion of the reaction, a saturated aqueous ammonium chloride solution was added to the reaction solution, and the mixture was extracted with ethyl acetate. The organic layer was washed with water, dried over anhydrous magnesium sulfate and concentrated. The obtained residue was applied to silica gel chromatography (eluent: toluene/ethyl acetate = 97/3 (volume ratio)) to obtain 0.31 g of the desired compound as yellowish solid.

CI-MS(m/z); 399($M^+$), EI-MS(m/z); 399($M^+$)

$^1$H-NMR(δ, DMSO-$d_6$); 5.37(s, 2H), 7.19(d, J=8.8Hz, 1H), 7.44(d, J=15.9Hz, 1H), 7.54-7.62 (m, 2H), 7.68(d, J=7.3Hz, 1H), 7.82(d, J=7.8Hz, 1H), 7.97-8.07(m, 5H), 8.36-8.39(m, 2H)

(b) 2-[(E)-2-(6,7-difluoro-2-quinolinyl)ethenyl]-11-hydroxy-6,11-dihydrodibenz[b,e]oxepine

The reaction was carried out in the same manner as in Reference example 1(b) except for using 2-[2-(6,7-difluoro-2-quinolinyl)ethenyl]-6,11-dihydrodibenz[b,e]oxepin-11-one (0.31 g, 0.77 mmol) in place of [2-[(E)-2-(7-chloro-6-fluoro-2-quinolinyl)ethenyl]-6,11-dihydrodibenz[b,e]oxepin-11-one to obtain 0.10 g of the desired compound as yellowish solid. CI-MS(m/z); 401($M^+$), EI-MS(m/z); 401 ($M^+$)

$^1$H-NMR(δ, DMSO-$d_6$); 5.38(d, J=12.4Hz, 1H), 5.76(d, J=12.2Hz, 1H), 5.94(s, 1H), 6.24(s, 1H), 6.82(d, J=8.5Hz, 1H), 7.28-7.50(m, 5H), 7.56(dd, J=8.5, 2.2Hz, 1H), 7.76-7.82(m, 2H), 7.88(d, J=8.8Hz, 1H), 7.93(dd, J=12.0, 8.0Hz, 1H), 8.01(dd, J=11.2, 9.0Hz, 1H), 8.33(d, J=8.5Hz, 1H)

In the same manner as in Reference example 2, compounds shown in the following Reference examples 3 to 13 were obtained.

Reference example 3

(a) 2-[(E)-2-(7-fluoro-2-quinolinyl)ethenyl]-6,11-dihydrodibenz[b,e]oxepin-11-one
Appearance; pale yellowish solid
CI-MS(m/z); 382($M^+$+1), EI-MS(m/z); 381($M^+$)
$^1$H-NMR(δ, DMSO-$d_6$); 5.38(s, 2H), 7.19(d, J=8.5Hz, 1H), 7.45(d, J=16.4Hz, 1H), 7.49(td, J=8.8, 2.7Hz, 1H), 7.57 (t, J=7.6Hz, 1H), 7.61(d, J=6.6Hz, 1H), 7.65-7.75(m, 2H), 7.82(d, J=7.6Hz, 1H), 7.91(d, J=8.5Hz, 1H), 7.94(d, J=16.4Hz, 1H), 8.00-8.15(m, 2H), 8.37(d, J=2.2Hz, 1H), 8.40(d, J=8.5Hz, 1H)

(b) 2-[(E)-2-(7-fluoro-2-quinolinyl)ethenyl]-11-hydroxy-6,11-dihydrodibenz[b,e]oxepine
Appearance; yellow brownish solid
CI-MS(m/z); 384($M^+$+1), EI-MS(m/z); 383($M^+$)
$^1$H-NMR(δ, DMSO-$d_6$); 5.29(d, J=12.5Hz, 1H), 5.78(d, J=12.2Hz, 1H), 5.96(d, J=3.7Hz, 1H), 6.26(d, J=3.9Hz, 1H), 6.83(d, J=8.3Hz, 1H), 7.25-7.55(m, 5H), 7.33(d, J=16.4Hz, 1H), 7.57(dd, J=8.5, 2.2Hz, 1H), 7.69(dd, J=10.7, 2.7Hz, 1H), 7.81(d, J=10.7Hz, 1H), 7.82(d, J=2.0Hz, 1H), 7.84(d, J=8.8Hz, 1H), 8.02(dd, J=8.8, 6.6Hz, 1H), 8.37(d, J=8.5Hz, 1H)

Reference example 4

(a) 2-[(E)-2-(7-chloro-2-quinolinyl)ethenyl]-6,11-dihydrodibenz[b,e]oxepin-11-one
Appearance; pale yellowish solid

CI-MS(m/z); 398($M^+$+1), EI-MS(m/z); 397($M^+$)
$^1$H-NMR(δ, DMSO-$d_6$); 5.38(s, 2H), 7.19(d, J=8.5Hz, 1H), 7.45(d, J=16.4Hz, 1H), 7.50-7.75 (m, 4H), 7.82(d, J=7.6Hz, 1H), 7.94(d, J=16.1Hz, 1H), 7.96(d, J=8.5Hz, 1H), 8.00(d, J=8.8Hz, 1H), 8.02(d, J=1.7Hz, 1H), 8.06(dd, J=8.5, 2.4Hz, 1H), 8.36(d, J=2.2Hz, 1H), 8.40(d, J=8.8Hz, 1H)

(b) 2-[(E) -2-(7-chloro-2-quinolinyl)ethenyl]-11-hydroxy-6,11-dihydrodibenz[b,e]oxepine
Appearance; yellowish solid
CI-MS(m/z); 399($M^+$), EI-MS(m/z); 399($M^+$)
$^1$H-NMR(δ, DMSO-$d_6$); 5.28(d, J=12.4Hz, 1H), 5.76(d, J=12.2Hz, 1H), 5.95(d, J=3.7Hz, 1H), 6.24(d, J=3.9Hz, 1H), 6.82(d, J=8.5Hz, 1H), 7.25-7.55(m, 5H), 7.57(dd, J=8.8, 2.2Hz, 2H), 7.81(d, J=16.4Hz, 1H), 7.81(d, J=2.0Hz, 1H), 7.89(d, J=8.8Hz, 1H), 7.98(d, J=8.8Hz, 1H), 8.00(d, J=1.7Hz, 1H), 8.36(d, J=8.8Hz, 1H)

Reference example 5

(a) 2-[(E)-2-(5,6,7,8-tetrahydro-2-quinolinyl)ethenyl]-6,11-dihydrodibenz[b,e]oxepin-11-one
Appearance; yellowish solid
CI-MS(m/z); 368($M^+$+1), EI-MS(m/z); 367(M+)
$^1$H-NMR(δ, DMSO-$d_6$); 5.34(s, 2H), 7.14(d, J=8.5Hz, 1H), 7.20(d, J=16.1Hz, 1H), 7.34(d, J=8.1Hz, 1H), 7.43(d, J=8.1Hz, 1H), 7.50-7.75(m, 3H), 7.59(d, J=16.1Hz, 1H), 7.81(d, J=7.1Hz, 1H), 7.93(dd, J=8.5, 2.2Hz, 1H), 7.26 (d, J=2.2Hz, 1H)

(b) 2-[(E)-2-(5,6,7,8-tetrahydro-2-quinolinyl)ethenyl]-11-hydroxy-6,11-dihydrodibenz[b,e]oxepine
Appearance; pale yellowish solid
CI-MS(m/z); 370($M^+$+1), EI-MS(m/z); 369($M^+$)
$^1$H-NMR(δ, DMSO-$d_6$); 2.70-2.90(m, 4H), 2.73(t, J=6.3Hz, 2H), 2.82(t, J=6.3Hz, 2H), 5.23(d, J=12.5Hz, 1H), 5.75 (d, J=12.2Hz, 1H), 5.89(s, 1H), 6.17(d, J=3.4Hz, 1H), 6.77(d, J=8.5Hz, 1H), 7.07(d, J=16.1Hz, 1H), 7.20-7.55(m, 8H), 7.69(d, J=2.2Hz, 1H)

Reference example 6

(a) 2-[(E)-2-(4-chloro-2-quinolinyl)ethenyl]-6,11-dihydrodibenz[b,e]oxepin-11-one
Appearance; yellowish solid
CI-MS(m/z); 398($M^+$+1), EI-MS(m/z); 397($M^+$)
$^1$H-NMR(δ, DMSO-$d_6$); 5.38(s, 2H), 7.19(d, J=8.8Hz, 1H), 7.42(d, J=16.4Hz, 1H), 7.57(td, J=7.6, 1.2Hz, 1H), 7.61 (d, J=6.6Hz, 1H), 7.65-7.75(m, 2H), 7.83(dd, J=7.3, 1.5Hz, 1H), 7.86(ddd, J=8.5, 7.1, 1.5Hz, 1H), 7.99(d, J=16.6Hz, 1H), 8.05(dd, J=8.5, 2.4Hz, 1H), 8.07(d, J=7.8Hz, 1H), 8.16(dd, J=8.5, 1.5Hz, 1H), 8.20(s, 1H), 8.38(d, J=2.2Hz, 1H)
(b) 2-[(E)-2-(4-chloro-2-quinolinyl)ethenyl]-11-hydroxy-6,11-dihydrodibenz[b,e]oxepine
Appearance; yellowish solid
CI-MS(m/z); 400($M^+$+1), EI-MS(m/z); 399($M^+$)
$^1$H-NMR(δ, DMSO-$d_6$); 5.29 (d, J=12.5Hz, 1H), 5.76(d, J=12.5Hz, 1H), 5.95(d, J=3.4Hz, 1H), 6.24(d, J=3.9Hz, 1H), 6.82(d, J=8.3Hz, 1H), 7.30(d, J=16.4Hz, 1H), 7.30-7.45(m, 3H), 7.48(dd, J=6.8, 2.0Hz, 1H), 7.57(dd, J=8.5, 2.2Hz, 1H), 7.69(ddd, J=8.3, 6.8, 1.2Hz, 1H), 7.82(d, J=2.2Hz, 1H), 7.85(ddd, J=8.3, 6.8, 1.2Hz, 1H), 7.86(d, J=16.4Hz, 1H), 8.04(d, J=8.1Hz, 1H), 8.12(s, 1H), 8.15(d, J=8.3Hz, 1H)

Reference example 7

(a) 2-[(E)-2-(5-fluoro-2-quinolinyl)ethenyl]-6,11-dihydrodibenz[b,e]oxepin-11-one
Appearance; yellowish solid
CI-MS(m/z); 382($M^+$+1), EI-MS(m/z); 381($M^+$)
$^1$H-NMR(δ, DMSO-$d_6$); 5.38(s, 2H), 7.19(d, J=8.5Hz, 1H), 7.38(ddd, J=10.0, 7.8, 1.0Hz, 1H), 7.47(d, J=16.4Hz, 1H), 7.57(td, J=7.3, 1.2Hz, 1H), 7.61(d, J=7.3Hz, 1H), 7.70(td, J=7.3, 1.5Hz, 1H), 7.75(td, J=7.8, 6.1Hz, 1H), 7.83(dd, J=7.8, 1.2Hz, 1H), 7.85(d, J=8.5Hz, 1H), 7.96(d, J=15.6Hz, 1H), 8.00(d, J=8.5Hz, 1H), 8.05(dd, J=8.8, 2.4Hz, 1H), 8.38(d, J=2.2Hz, 1H), 8.46(d, J=9.0Hz, 1H)

(b) 2-[(E)-2-(5-fluoro-2-quinolinyl)ethenyl]-11-hydroxy-6,11-dihydrodibenz[b,e]oxepine
Appearance; yellowish solid
CI-MS(m/z); 384($M^+$+1), EI-MS(m/z); 383($M^+$)
$^1$H-NMR(δ, DMSO-$d_6$); 5.28 (d, J=12.2Hz, 1H), 5.78(d, J=12.5Hz, 1H), 5.94(s, 1H), 6.83(d, J=8.3Hz, 1H), 7.25-7.55 (m, 5H), 7.58(dd, J=8.3, 2.2Hz, 1H), 7.30-7.60(m, 1H), 7.75-7.90 (m, 3H), 7.94(d, J=8.8Hz, 1H), 8.44(d, J=8.8Hz, 1H)

Reference example 8

(a) 2-[(E)-2-(5,7-difluoro-2-quinolinyl)ethenyl]-6,11-dihydrodibenz[b,e]oxepin-11-one
Appearance; pale yellowish solid
CI-MS(m/z); 400 ($M^+$+1), EI-MS(m/z); 399($M^+$)
$^1$H-NMR(δ, DMSO-$d_6$); 5.38(s, 2H), 7.20(d, J=8.5Hz, 1H), 7.46(d, J=16.4Hz, 1H), 7.45-7.65 (m, 4H), 7.70(td, J=7.6, 1.5Hz, 1H), 7.82(dd, J=7.8, 1.2Hz, 1H), 7.97(d, J=9.0Hz, 1H), 7.98 (d, J=15.4Hz, 1H), 8.05(dd, J=8.8.2.4Hz, 1H), 8.37(d, J=2.2Hz, 1H), 8.46(d, J=8.5Hz, 1H)

(b) 2-[(E)-2-(5,7-fluoro-2-quinolinyl)ethenyl]-11-hydroxy-6,11-dihydrodibenz[b,e]oxepine
Appearance; yellowish solid
CI-MS(m/z); 402($M^+$+1), EI-MS(m/z); 401($M^+$)
$^1$H-NMR(δ, DMSO-$d_6$); 5.29(d, J=12.5Hz, 1H), 5.77(d, J=12.5Hz, 1H), 5.94(s, 1H), 6.26(d, J=3.9Hz, 1H), 6.83(d, J=8.3Hz, 1H), 7.25-7.65(m, 6H), 7.82(d, J=2.2Hz, 1H), 7.87(d, J=17.3Hz, 1H), 7.91(d, J=9.0Hz, 1H), 8.43(d, J=9.0Hz, 1H)

Reference example 9

(a) 2-[(E)-2-(6-fluoro-7-trifluoromethyl-2-quinolinyl)ethenyl]-6,11-dihydrodibenz[b,e]oxepin-11-one
Appearance; yellowish solid
CI-MS (m/z); 450($M^+$+1); EI-MS(m/z); 449 ($M^+$)
$^1$H-NMR(δ, DMSO-$d_6$) ; 5.38(s, 2H), 7.20(d, J=8.5Hz, 1H), 7.47(d, J=16.4Hz, 1H), 7.57(t, J=7.6Hz, 1H), 7.61(d, J=7.8Hz, 1H), 7.70(t, J=7.3Hz, 1H), 7.82(d, J=7.6Hz, 1H), 7.99(d, J=16.4Hz, 1H), 8.07(d, J=8.1Hz, 1H), 8.10(d, J=10.3Hz, 1H), 8.13(d, J=8.1Hz, 1H), 8.30-8.45(m, 1H), 8.37(d, J=2.4Hz, 1H), 8.47(d, J=8.8Hz, 1H)

(b) 2-[(E)-2-(6-fluoro-7-trifluoromethyl-2-quinolinyl)ethenyl]-11-hydroxy-6,11-dihydrodibenz[b,e]oxepine Appearance; yellowish solid
CI-MS(m/z); 452($M^+$+1), EI-MS(m/z); 451($M^+$)
$^1$H-NMR(δ, DMSO-$d_6$) ; 5.30(d, J=12.5Hz, 1H), 5.76(d, J=12.5Hz, 1H), 5.96(br.s, 1H), 6.25(br.s, 1H), 6.83(d, J=8.5Hz, 1H), 7.30-7.55(m, 4H), 7.34(d, J=16.1Hz, 1H), 7.57(dd, J=8.5, 2.2Hz, 1H), 7.82(d, J=2.4Hz, 1H), 7.86 (d, J=16.6Hz, 1H), 8.05(d, J=8.8Hz, 1H), 8.07(d, J=11.2Hz, 1H), 8.34(d, J=6.8Hz, 1H), 8.42(d, J=8.8Hz, 1H)

Reference example 10

(a) 2-[(E)-2-(5,6,7-trifluoro-2-quinolinyl)ethenyl]-6,11-dihydrodibenz[b,e]oxepin-11-one
Appearance; yellowish solid
CI-MS(m/z); 418($M^+$+1), EI-MS(m/z); 417($M^+$)
$^1$H-NMR(δ, DMSO-$d_6$); 5.37(s, 2H), 7.18(d, J=8.5Hz, 1H), 7.44(d, J=16.4Hz, 1H), 7.57(td, J=7.6, 1.5Hz, 1H), 7.60 (d, J=7.8Hz, 1H), 7.69(td, J=7.6, 1.5Hz, 1H), 7.81(dd, J=7.6, 1.0Hz, 1H), 7.80-7.95(m, 1H), 7.96(d, J=16.1Hz, 1H), 8.03(d, J=8.8Hz, 1H), 8.04(dd, J=8.5, 2.4Hz, 1H), 8.36(d, J=2.2Hz, 1H), 8.50(d, J=8.8Hz, 1H)

(b) 2-[(E)-2-(5,6,7-trifluoro-2-quinolinyl)ethenyl]-11-hydroxy-6,11-dihydrodibenz[b,e]oxepine

Appearance; yellowish solid
CI-MS(m/z); 420($M^+$+1), EI-MS(m/z); 4l9($M^+$)
$^1$H-NMR(δ, DMSO-$d_6$); 5.29(d, J=12.5Hz, 1H), 5.76(d, J=12.2Hz, 1H), 5.94(d, J=3.7Hz, 1H), 6.25(d, J=3.9Hz, 1H), 6.82(d, J=8.3Hz, 1H), 7.33(d, J=16.4Hz, 1H), 7.30-7.45(m, 3H), 7.48(dd, J=6.8, 2.0Hz, 1H), 7.57(dd, J=8.3, 2.2Hz, 1H), 7.82(d, J=2.7Hz, 1H), 7.85(d, J=16.6Hz, 1H), 7.86(ddd, J=11.5, 7.1, 2.2Hz, 1H), 7.97(d, J=8.8Hz, 1H), 8.47 (d, J=9.0Hz, 1H)

Reference example 11

(a) 2-[(E)-2-(5-fluoro-2-benzothiazolyl)ethenyl]-6,11-dihydrodibenz[b,e]oxepin-11-one
Appearance; yellowish solid
CI-MS(m/z); 388($M^+$+1), EI-MS(m/z); 387($M^+$)
$^1$H-NMR(δ, DMSO-$d_6$); 5.38(s, 2H), 7.19(d, J=8.5Hz, 1H), 7.34(td, J=9.0, 2.4Hz, 1H), 7.50-7.65(m, 2H), 7.58(d, J=16.1Hz, 1H), 7.70(td, J=7.3, 1.2Hz, 1H), 7.75-7.80(m, 1H), 7.78(d, J=16.1Hz, 1H), 7.81(dd, J=9.8, 2.4Hz, 1H), 8.09(dd, J=8.5, 2.2Hz, 1H), 8.14(dd, J=8.8, 5.4Hz, 1H), 8.38(d, J=2.4Hz, 1H)

(b) 2-[(E)-2-(5-fluoro-2-benzothiazolyl)ethenyl]-11-hydroxy-6,11-dihydrodibenz[b,e]oxepine
Appearance; yellowish solid
CI-MS(m/z); 390($M^+$+1), EI-MS(m/z); 389($M^+$)
$^1$H-NMR(δ, DMSO-$d_6$); 5.28(d, J=12.5Hz, 1H), 5.80(d, J=12.2Hz, 1H), 5.91(d, J=3.4Hz, 1H), 6.25(d, J=3.7Hz, 1H), 6.82(d, J=8.5Hz, 1H), 7.25-7.50(m, 5H), 7.44(d, J=16.1Hz, 1H), 7.62(dd, J=8.5, 2.2Hz, 1H), 7.63(d, J=16.4Hz, 1H), 7.79(d, J=10.0, 2.4Hz, 1H), 7.84(d, J=2.2Hz, 1H), 8.11(dd, J=8.8, 5.4Hz, 1H)

Reference example 12

(a) 2-[(E)-2-(5-fluoro-2-benzoxazolyl)ethenyl]-6,11-dihydrodibenz[b,e]oxepin-11-one
Appearance; yellowish solid
CI-MS(m/z); 372($M^+$+1), EI-MS(m/z); 731($M^+$)
$^1$H-NMR(δ, DMSO-$d_6$); 5.38(s, 2H), 7.20 (d, J=8.8Hz, 1H), 7.26(ddd, J=9.8, 9.0, 2.7Hz, 1H), 7.28(d, J=16.4Hz, 1H), 7.57(td, J=7.6, 1.5Hz, 1H), 7.55-7.65(m, 1H), 7.61(dd, J=8.8, 2.7Hz, 1H), 7.70(td, J=7.3, 1.5Hz, 1H), 7.76 (dd, J=9.0, 4.4Hz, 1H), 7.80(dd, J=7.6, 1.2Hz, 1H), 7.90(d, J=16.4Hz, 1H), 8.13(dd, J=8.8, 2.4Hz, 1H), 8.38(d, J=2.4Hz, 1H)

(b) 2-[(E)-2-(5-fluoro-2-benzoxazolyl)ethenyl]-11-hydroxy-6,11-dihydrodibenz[b,e]oxepine
Appearance; yellowish brown solid
CI-MS(m/z); 374($M^+$+1), EI-MS(m/z); 373($M^+$)
$^1$H-NMR(δ, DMSO-$d_6$); 5.27(d, J=12.2Hz, 1H), 5.82 (d, J=12.2Hz, 1H), 5.89(d, J=3.2Hz, 1H), 6.24(d, J=3.9Hz, 1H), 6.83(d, J=8.3Hz, 1H), 7.13(d, J=16.4Hz, 1H), 7.24(ddd, J=9.8, 9.0, 2.7Hz, 1H), 7.30-7.50(m, 4H), 7.58(dd, J=8.8, 2.7Hz, 1H), 7.65(dd, J=8.5, 2.2Hz, 1H), 7.73(dd, J=8.8, 4.4Hz, 1H), 7.77(d, J=16.6Hz, 1H), 7.85(d, J=2.2Hz, 1H)

Reference example 13

(a) 2-[(E)-2-(6-isopropyl-5-methyl-2-pyridyl)ethenyl]-6,11-dihydrodibenz[b,e]oxepin-11-one
Appearance; yellowish solid
CI-MS (m/z); 370($M^+$+1), EI-MS(m/z); 369($M^+$)
$^1$H-NMR(δ, DMSO-$d_6$); 7.14(d, J=8.5Hz, 1H), 7.21(d, J=16.1Hz, 1H), 7.31(d, J=7.8Hz, 1H), 7.50(d, J=8.3Hz, 1H), 7.54-7.71(m, 4H), 7.80 (dd, J=7.6Hz, 1H), 7.94 (dd, J=8.5, 2.4Hz, 1H), 8.25 (d, J=2.2 Hz, 1H)

(b) 2-[(E)-2-(6-isopropyl-5-methyl-2-pyridyl)ethenyl]-11-hydroxy-6,11-dihydrodibenz[b,e]oxepine
Appearance; yellowish solid
CI-MS (m/z); 371($M^+$), EI-MS(m/z); 371($M^+$)

$^1$H-NMR (δ, DMSO-$d_6$) ; 1.24(d, J=6.6Hz, 6H), 2.30(s, 3H), 3.24-3.36(m, 1H), 5.24(d, J=12.2Hz, 1H), 5.75(d, J=12.4Hz, 1H), 5.90(s, 1H), 6.18(s, 1H), 6.77 (d, J=8.3Hz, 1H), 7.08(d, J=16.1Hz, 1H), 7.24(d, J=7.8Hz, 1H), 7.32-7.56(m, 7H), 7.68(d, J=2.0Hz, 1H)

Reference example 14

(a) 2-[(E)-2-(4-acetoxyphenyl)ethenyl]-6,7-difluoroquinoline

In 150 ml of acetic anhydride were dissolved 6,7-difluoro-2-quinaldine (17.4 g, 96.8 mmol) and 4-hydroxybenzaldehyde (11.7 g, 95.6 mmol), and the mixture was stirred at 140°C for 31 hours under nitrogen atmosphere.

After completion of the reaction, the reaction solution was allowed to cool and diluted with 50 ml of toluene, and then, precipitated crystal was collected by filtration and washed with toluene twice, and dried under reduced pressure to obtain 16.9 g of the desired compound as white solid.

CI-MS(m/z); 326($M^+$+1), EI-MS(m/z); 325($M^+$)

$^1$H-NMR(δ, $CDCl_3$); 2.32(s, 3H), 7.12-7.17 (m, 2H), 7.29(d, J=16.1Hz, 1H), 7.51(dd, J=10.3, 8.5Hz, 1H), 7.62(d, J=8.6Hz, 1H), 7.62-7.65(m, 2H), 7.69(d, J=16.1Hz, 1H), 7.81(dd, J=11.5, 7.81Hz, 1H), 8.05(d, J=8.6Hz, 1H)

(b) 6,7-difluoro-2-[(E)-2-(4-hydroxyphenyl)ethenyl]quinoline

In a mixed solution comprising 30 ml of dimethylsulfoxide and 20 ml of methanol was suspended 2-[(E)-2-(4-acetoxyphenyl)ethenyl]-6,7-difluoroquinoline (5.21g, 16.0 mmol), and a solution in which sodium hydroxide (1.13 g, 28.3 mmol) had been dissolved in 10 ml of water was added to the suspension and the mixture was stirred at room temperature for 20 minutes.

After completion of the reaction, 30 ml of 1N-hydrochloric acid was added to the reaction mixture to make the mixture acidic, and the precipitated crystal was extacted with ethyl acetate. The organic layer was washed with a saturated aqueous sodium chloride solution and dried over anhydrous sodium sulfate, and then, concentrated to obtain 4.20 g of the desired compound as yellowish solid.

CI-MS(m/z); 284($M^+$+1), EI-MS(m/z); 282($M^+$-1)

$^1$H-NMR(δ, DMSO-$d_6$); 6.81-6.84(m, 2H), 7.23(d, J=16.1Hz, 1H), 7.55-7.58(m, 2H), 7.75(d, J=16.1Hz, 1H), 7.84 (d, J=8.8Hz, 1H), 7.92(dd, J=12.0, 8.0Hz, 1H), 8.00 (dd, J=11.0, 8.8Hz, 1H), 8.32(d, J=8.8Hz, 1H), 9.81(br.s, 1H)

(c) Methyl 2-[4-[(E)-2-(6,7-difluoro-2-quinolinyl)ethenyl]phenoxymethyl]-3-cyano-benzoate

To 5 ml of dimethylsulfoxide solution containing 6,7-difluoro-2-[(E) -2- (4-hydroxyphenyl) ethenyl] quinoline (0.40 g, 1.41 mmol) was added a methyl 2-bromomethyl-3-cyanobenzoate (0.29 g, 1.14 mmol) solution dissolved in 5 ml of dimethylsulfoxide, and further potassium carbonate (0.38 g, 2.75 mmol) was added to the mixture and the mixture was stirred at 70°C for 1 hour under nitrogen atmosphere.

After completion of the reaction, water was added to the reaction mixture, the resulting mixture was extracted with ethyl acetate, and the organic layer was washed with a saturated aqueous sodium chloride solution and dried over anhydrous sodium sulfate. The solvent was concentrated and the resulting residue was applied to silica gel chromatography (eluent: toluene/ethyl acetate = 99/1 (volume ratio)) to obtain 0.52 g of the desired compound as yellowish solid.

CI-MS(m/z); 457($M^+$+1), EI-MS(m/z); 456($M^+$)

$^1$H-NMR(δ, $CDCl_3$); 3.85(s, 3H), 5.59(s, 2H), 7.01-7.04(m, 2H), 7.22(d, J=16.4Hz, 1H), 7.49(dd, J=10.0, 8.3Hz, 1H), 7.54-7.63(m, 4H), 7.66(d, J=16.1Hz, 1H), 7.80(dd, J=11.5, 7.8Hz, 1H), 7.87(dd, J=7.8, 1.5Hz, 1H), 8.03(d, J=8.6Hz, 1H), 8.10(dd, J=8.1, 1.5Hz, 1H)

(d) 3-Cyano-2-[4-[(E)-2-(6,7-difluoro-2-quinolinyl)ethenyl]phenoxymethyl]benzoic acid

In 30 ml of dioxane was suspended methyl 3-cyano-2-[4-[(E)-2-(6,7-difluoro-2-quinolinyl)ethenyl]phenoxymethyl]benzoate (0.52g, 1.14 mmol), and a sodium hydroxide (0.48 g, 12.0 mmol) solution dissolved in 1 ml of water was added to the suspension and the mixture was stirred at room temperature for 30 minutes util the solution became uniform.

After completion of the reaction, the reaction solution was made acidic with 1 ml of trifluoroacetic acid, and then, 100 ml of water was added thereto and the formed precipitate was collected by filtration, washed with water and dried under reduced pressure to obtain 0.41 g of the desired compound as yellowish solid.

(e) 7-Cyano-2-[(E)-2-(6,7-difluoro-2-quinolinyl)ethenyl]-6,11-dihydrodibenz[b,e]oxepin-11-one

In 10 ml of methylene chloride was suspended 3-cyano-2-[4-[(E)-2-(6,7-difluoro-2-quinolinyl)ethenyl]phenoxymethyl]benzoic acid (0.41 g, 0.93 mmol), trifluoroacetic acid anhydride (3.0 ml, 21.2 mmol) was added to the

suspension to make the solution uniform, and then, boron trifluoride-diethyl ether complex (2.0 ml, 16.3 mmol) was added to the mixture and the mixture was stirred at room temperature for 15 hours.

After completion of the reaction, the reaction solution was poured into a saturated aqueous sodium hydrogen carbonate solution into which sodium hydroxide (1.60 g, 40.0 mmol) had been dissolved therein. The mixture was extracted with ethyl acetate, the organic layer was washed with a saturated aqueous sodium chloride solution and dried over anhydrous sodium sulfate. The solvent was concentrated to obtain 0.38 g of the desired compound as yellowish solid.

CI-MS(m/z); 425($M^+$+1), EI-MS(m/z); 424($M^+$)

$^1$H-NMR(δ, DMSO-$d_6$); 5.29(s, 2H), 7.24(d, J=8.5Hz, 1H), 7.44(d, J=16.4Hz, 1H), 7.78(t, J=7.81Hz, 1H), 7.91(d, J=16.4Hz, 1H), 7.93-7.98(m, 2H), 8.02(dd, J=11.0, 9.0Hz, 1H), 8.06-8.10(m, 2H), 8.20(dd, J=8.0, 1.2Hz, 1H), 8.32 (d, J=2.4Hz, 1H), 8.37(d, J=8.5Hz, 1H)

(f) 7-Cyano-2-[(E)-2-(6,7-difluoro-2-quinolinyl)ethenyl]-11-hydroxy-6,11-dihydrodibenz[b,e]oxepine

The same reaction was carried out as in (b) of Reference example 1 except for using 7-cyano-2-[(E)-2-(6,7-difluoro-2-quinolinyl)ethenyl]-6,11-dihydrodibenz[b,e]oxepin-11-one (0.37 g, 0.87 mmol) in place of 2-[(E)-2-(7-chloro-6-fluoro-2-quinolinyl)ethenyl]-6,11-dihydrodibenz[b,e]oxepin-11-one to obtain 0.37 g of the desired compound as yellowish solid.

CI-MS(m/z); 427($M^+$+1), EI-MS(m/z); 426($M^+$)

$^1$H-NMR(δ, DMSO-$d_6$); 5.56(d, J=13.2Hz, 1H), 5.83(d, J=13.4Hz, 1H), 6.12(d, J=3.9Hz, 1H), 6.56(d, J=4.4Hz, 1H), 6.91(d, J=8.3Hz, 1H), 7.33(d, J=16.4Hz, 1H), 7.60(t, J=7.8Hz, 1H), 7.60-7.62(m, 1H), 7.78-7.91(m, 5H), 7.94(dd, J=12.0, 8.1Hz, 1H), 8.02(dd, J=11.0, 9.0Hz, 1H), 8.35(d, J=8.8Hz, 1H)

In the same manner as in Reference example 14(a), compounds of the following Reference example 15 was obtained.

Reference example 15

2-[(E)-2-(4-acetoxyphenyl)ethenyl]-7-chloro-6-fluoroquinoline

Appearance; pale brownish solid

CI-MS(m/z); 342($M^+$+1), EI-MS(m/z); 341($M^+$)

$^1$H-NMR(δ, $CDCl_3$); 2.30 (s, 3H), 7.21(dd, J=6.6, 2.0Hz, 2H), 7.44 (d, J=16.4Hz, 1H), 7.78(d, J=8.5Hz, 2H), 7.85(d, J=16.4Hz, 1H), 7.93(d, J=8.5Hz, 1H), 7.99(d, J=9.5Hz, 1H), 8.20(d, J=7.3Hz, 1H), 8.37(d, J=8.5Hz, 1H)

In the same manner as in Reference example 14(b), compound of the following Reference example 16 was obtained.

Reference example 16

7-chloro-6-fluoro-2-[(E)-2-(4-hydroxyphenyl)ethenyl]quinoline

Appearance; yellowish solid

CI-MS(m/z); 300($M^+$+1), EI-MS(m/z); 298($M^+$-1)

$^1$H-NMR(δ, DMSO-$d_6$) ; 6.83(d, J=8.5Hz, 2H), 7.23(d, J=16.4Hz, 1H), 7.57(d, J=8.8Hz, 2H), 7.76(d, J=16.4Hz, 1H), 7.89(d, J=8.8Hz, 1H), 7.97(d, J=10.0Hz, 1H), 8.17(d, J=7.3Hz, 1H), 8.32(d, J=8.8Hz, 1H), 9.84(br.s, 1H)

Reference example 17

Methyl 2-methyl-3-trimethylsilylethynylbenzoate

In 10 ml of tetrahydrofuran were suspended methyl 3-iodo-2-methylbenzoate (1.20g, 4.35 mmol) andcopper (I) iodide (0.21 g, 1.10 mmol), and then, triethylamine (3.0 ml, 21.5 mmol) and trimethylacetylene (1.5 ml, 10.8 mmol) were added to the suspension to form a uniform solution. Then, to the mixture was added tetrakistriphenylphosphinepalladium (0) (0.72g, 0.62 mmol), and the mixture was stirred at room temperature for 5 hours under argon atmosphere.

After completion of the reaction, water was added to the reactionmixture, the resulting mixture was extracted with ethyl acetate, and the organic layer was washed successively with a saturated aqueous ammonium chloride solution and a saturated aqueous sodium chloride solution, and dried over anhydrous sodium sulfate. The solvent was concentrated and the resulting residue was applied to silica gel chromatography (eluent: hexane/ethyl acetate = 99/1 (volume ratio)) to obtain 1.05 g of the desired compound as black brownish oily product.

CI-MS(m/z); 247($M^+$+1), EI-MS(m/z); 246($M^+$)

$^1$H-NMR(δ, $CDCl_3$); 0.27(s, 9H), 2.70(s, 3H), 3.89(s, 3H), 7.17(t, J=7.8Hz, 1H), 7.58(dd, J=7.8, 1.5Hz, 1H), 7.78(dd, J=7.8, 1.5Hz, 1H)

Reference example 18

Methyl 2-methyl-3-trifluoromethylbenzoate

In 5 ml of dimethylformamide were suspended methyl 3-iodo-2-methylbenzoate (0.80g, 2.90 mmol) and copper (I) iodide (0.12 g, 0.63 mmol), and methyl fluorosulfonyl(difluoro)acetate (1.0 ml, 7.91 mmol) was added to the suspension and the mixture was stirred at 80°C for 6 hours under argon atmosphere. After allowing to coll, copper (I) iodide (0.18g, 0.95 mmol) and methyl fluorosulfonyl(difluoro)acetate (2.0 ml, 15.8 mmol) were further added to the mixture and stirred at 80°C for 10 hours.

After completion of the reaction, pentane was added to the reaction solution, and after removing the precipitates, the filtrate was washed twice with water and once with a saturated aqueous sodium chloride solution, and dried over anhydrous sodium sulfate. The solvent was concentrated to obtain 0.62 g of the desired compound as colorless transparent oily product.
CI-MS(m/z); 219($M^+$+1), EI-MS(m/z); 218($M^+$)
$^1$H-NMR(δ, $CDCl_3$); 2.64(s, 3H), 3.93(s, 3H), 7.34(dd, J=7.8, 8.1Hz, 1H), 7.77(d, J=8.1Hz, 1H), 7.91(d, J=7.8Hz, 1H)

Reference example 19

Methyl 2-bromomethyl-3-trimethylsilylethynylbenzoate

In 15 ml of acetonitrile was dissolved methyl 2-methyl-3-trimethylsilylethynylbenzoate (1.05 g, 4.26 mmol), and N-bromosuccinimide (0.87 g, 4.89 mmol), and benzoyl peroxide (0.22 g, 0.68 mmol) was added to the solution and the mixture was refluxed for 6 hours under argon atmosphere.

After completion of the reaction, hexane was added to the reaction mixture, and the mixture was washed successively with water, a saturated aqueous sodium hydrogen carbonate solution and a saturated aqueous sodium chloride solution, and dried over anhydrous sodium sulfate. The solvent was concentrated and the resulting residue was applied to silica gel chromatography (eluent: hexane/ethyl acetate = 99.5/0.5 (volume ratio)) to obtain to obtain 1.02 g of the desired compound as pale yellowish oily product.
CI-MS(m/z); 325($M^+$+1), EI-MS(m/z); 324 ($M^+$)
$^1$H-NMR(δ, $CDCl_3$); 3.95(s, 3H), 5.19(s, 2H), 7.31(t, J=7.87Hz, 1H), 7.63(dd, J=7.8, 1.5Hz, 1H), 7.89(dd, J=7.8, 1.5Hz, 1H)

In the same manner as in Reference example 19, a compound of the following Reference example 20 was obtained.

Reference example 20

Methyl 2-bromomethyl-3-trifluoromethylbenzoate
Appearance; colorless transparent oily product
CI-MS(m/z); 297($M^+$+1), EI-MS(m/z); 217($M^+$-Br)
$^1$H-NMR(δ, $CDCl_3$); 3.99(s, 3H), 5.11(s, 2H), 7.49(dd, J=8.1, 7.6Hz, 1H), 7.82(d, J=8.1Hz, 1H), 8.05(d, J=7.6Hz, 1H)

In the same manner as in Reference example 14(c), compounds of the following Reference examples 21 to 24 were obtained.

Reference example 21

Methyl 2-[4-[(E)-2-(6,7-difluoro-2-quinolinyl)-ethenyl]phenoxymethyl]-3-ethynylbenzoate
Appearance; yellowish solid
CI-MS(m/z); 456($M^+$+1), EI-MS(m/z); 455($M^+$)
$^1$H-NMR(δ, $CDCl_3$); 3.33(s, 1H), 3.80(s, 3H), 5.59(s, 2H), 6.99-7.02(m, 2H), 7.21(d, J=16.4Hz, 1H), 7.40(t, J=7.8Hz, 1H), 7.49(dd, J=10.0, 8.3Hz, 1H), 7.56-7.59(m, 2H), 7.60(d, J=8.8Hz, 1H), 7.70(dd, J=7.8, 1.2Hz, 1H), 7.76-7.83(m, 2H), 8.02(d, J=8.8Hz, 1H)

Reference example 22

Methyl 2-[4-[(E)-2-(6,7-difluoro-2-quinolinyl)ethenyl]phenoxymethyl]-3-trifluoromethylbenzoate
Appearance; yellowish solid
CI-MS(m/z); 500($M^+$+1), EI-MS(m/z); 499($M^+$)
$^1$H-NMR(δ, $CDCl_3$); 3.77(s, 3H), 5.46(s, 2H), 6.97-7.00(m, 2H), 7.22(d, J=16.4Hz, 1H), 7.50 (dd, J=10.0, 8.3Hz, 1H), 7.57-7.60(m, 2H), 7.61(dd, J=8.1, 7.8Hz, 1H), 7.68(d, J=16.1Hz, 1H), 7.80(dd, J=11.5, 7.8Hz, 1H), 7.86(d, J=8.1Hz, 1H), 7.95(d, J=7.8Hz, 1H), 8.03(d, J=8.5Hz, 1H)

Reference example 23

Ethyl 3-fluoro-2-[4-[(E)-2-(6,7-difluoro-2-quinolinyl)ethenyl]phenoxymethyl]benzoate
Appearance; yellowish solid
CI-MS(m/z); 464($M^+$+1), EI-MS(m/z); 463 ($M^+$)
$^1$H-NMR(δ, $CDCl_3$); 1.18(t, J=7.1Hz, 3H), 4.23(q, J =7.1Hz, 2H), 5.37(s, 2H), 7.06(d, J=8.8Hz, 2H), 7.34(d, J=16.4Hz, 1H), 7.51-7.72(m, 5H), 7.81(d, J=16.4Hz, 1H), 7.86-8.05(m, 3H), 8.34(d, J=8.8Hz, 1H)

Reference example 24

Ethyl 3-fluoro-2-[4-[(E)-2-(7-chloro-6-fluoro-2-quinolinyl)ethenyl]phenoxymethyl]benzoate
Appearance; yellowish solid
CI-MS(m/z); 480($M^+$+1), EI-MS(m/z); 479($M^+$)
$^1$H-NMR(δ, $CDCl_3$); 1.18(t, J=7.1Hz, 3H), 4.23(q, J=7.1Hz, 2H), 5.37(s, 2H), 7.06(d, J=8.8Hz, 2H), 7.34(d, J=16.1Hz, 1H), 7.55-7.72(m, 5H), 7.81(d, J=16.4Hz, 1H), 7.91(d, J=8.8Hz, 1H), 7.98(d, J=9.8Hz, 1H), 8.19(d, J=7.3Hz, 1H), 8.35 (d, J=8.8Hz, 1H)

In the same manner as in Reference example 14(d), compounds of the following Reference examples 25 to 28 were obtained.

Reference example 25

2-[(E)-2-(6,7-difluoro-2-quinolinyl)ethenyl]-7-ethynyl-6,11-dihydrodibenz[b,e]oxepin-11-one
Appearance; yellowish solid
CI-MS(m/z); 424($M^+$+1), EI-MS(m/z); 423($M^+$) $^1$H-NMR(δ, DMSO-$d_6$); 3.47(s, 1H), 5.56(s, 2H), 7.21(d, J=8.5Hz, 1H), 7.44(d, J=16.4 Hz, 1H), 7.58(t, J=7, 8Hz, 1H), 7.79(dd, J=7.8, 1.5Hz, 1H), 7.84(dd, J=7.8, 1.5Hz, 1H), 7.92(d, J=16.4Hz, 1H), 7.94-8.09(m, 3H), 8.31(d, J=2.4Hz, 1H), 8.38(d, J=8.6Hz, 1H)

Reference example 26

2-[(E)-2-(6,7-difluoro-2-quinolinyl)ethenyl]-6,11-dihydro-7-trifluoromethyldibenz[b,e]oxepin-11-one
Appearance; yellowish solid
CI-MS(m/z); 468($M^+$+1), EI-MS(m/z); 467($M^+$) $^1$H-NMR(δ, $CDCl_3$); 5.41(s, 2H), 7.14(d, J=8.54Hz, 1H), 7.33(d, J=16.4Hz, 1H), 7.51(dd, J=10.3, 8.3Hz, 1H), 7.59(t, J=7.8Hz, 1H), 7.62(d, J=8.8Hz, 1H), 7.73(d, J=16.4Hz, 1H), 7.81 (dd, J=8.5, 2.4Hz, 1H), 7.82(dd, J=11.5, 7.6Hz, 1H), 7.91(d, J=7.8Hz, 1H), 8.00(d, J=7.81Hz, 1H), 8.07(d, J=8.6Hz, 1H), 8.39(d, J=2.4Hz, 1H)

Reference example 27

7-Fluoro-2-[(E)-2-(6,7-difluoro-2-quinolinyl)ethenyl]-6,11-dihydrodibenz[b,e]oxepin-11-one
Appearance; yellowish solid
CI-MS(m/z); 418($M^+$+1), EI-MS(m/z); 417($M^+$)
$^1$H-NMR(δ, $CDCl_3$); 5.45(s, 2H), 7.22(d, J=8.5Hz, 1H), 7.45(d, J=16.4Hz, 1H), 7.57-7.64(m, 3H), 7.95(d, J=16.1Hz, 1H), 8.00-8.09(m, 4H), 8.34(d, J=2.2Hz, 1H), 8.42(d, J=8.8Hz, 1H)

Reference example 28

7-Fluoro-2-[(E)-2-(7-chloro-6-fluoro-2-quinolinyl)-ethenyl] -6,11-dihydrodibenz[b,e]oxepin-11-one
Appearance; yellowish solid
CI-MS(m/z); 434($M^+$+1), EI-MS(m/z); 433($M^+$)
$^1$H-NMR(δ, $CDCl_3$); 5.45(s, 2H), 6.83(d, J=8.5Hz, 1H), 7.24(d, J=16.4Hz, 1H), 7.42-7.78(m, 3H), 7.76(d, J=16.6Hz, 1H), 7.88-8.32(m, 4H), 8.34(d, J=2.9Hz, 1H), 8.39(d, J=8.3Hz, 1H)

In the same manner as in Reference example 14(e), compounds of the following Reference examples 29 to 32 were obtained.

Reference example 29

2-[(E)-2-(6,7-difluoro-2-quinolinyl)ethenyl]-7-ethynyl-11-hydroxy-6,11-dihydrodibenz[b,e]oxepine
Appearance; yellowish solid

CI-MS(m/z); 426($M^+$+1), EI-MS(m/z); 425($M^+$)
$^1$H-NMR(δ, $CDCl_3$); 2.83(d, J=4.4Hz, 1H), 3.36(s, 1H), 5.55(d, J=13.7Hz, 1H), 5.76(d, J=4.6Hz, 1H), 5.95(d, J=13.9 Hz, 1H), 7.01(d, J=8.3Hz, 1H), 7.23(d, J=15.4Hz, 1H), 7.30(d, J=7.6Hz, 1H), 7.43-7.68(m, 7H), 7.79(dd, J=11.2, 7.6Hz, 1H), 8.04(d, J=8.6Hz, 1H)

Reference example 30

2-[(E)-2-(6,7-difluoro-2-quinolinyl)ethenyl]-11-hydroxy-6,11-dihydro-7-trifluoromethyldibenz[b,e]oxepine
Appearance; yellowish solid
CI-MS(m/z); 470($M^+$+1), EI-MS(m/z); 469 ($M^+$)
$^1$H-NMR(δ, $CDCl_3$); 2.82(s, 1H), 5.43(d, J=13.9Hz, 1H), 5.86(s, 1H), 6.12(d, J=13.7Hz, 1H), 6.97(d, J=8.5Hz, 1H), 7.23 (d, J=15.1Hz, 1H), 7.41-7.71(m, 8H), 7.78(dd, J=11.2, 7.8Hz, 1H), 8.04(d, J=8.6Hz, 1H)

Reference example 31

7-Fluoro-2-[(E)-2-(6,7-difluoro-2-quinolinyl)ethenyl]-11-hydroxy-6,11-dihydrodibenz[b,e]oxepine
Appearance; yellowish solid
CI-MS(m/z); 420($M^+$+1), EI-MS(m/z); 419 ($M^+$)
$^1$H-NMR(δ, $CDCl_3$); 5.46(d, J=13.2Hz, 1H), 5.71(d, J=14.6Hz, 1H), 6.03(d, J=4.2Hz, 1H), 6.42(d, J=4.2Hz, 1H), 6.87 (d, J=8.5Hz, 1H), 7.21-7.42(m, 3H), 7.32(d, J=16.1Hz, 1H), 7.59(d, J=8.5Hz, 1H), 7.77-7.83(m, 1H), 7.89(d, J=9.0Hz, 1H), 7.97(d, J= 9.0Hz, 1H), 8.03(d, J=11.0Hz, 1H), 8.34(d, J=8.8Hz, 1H)

Reference example 32

7-Fluoro-2-[(E)-2-(7-chloro-6-fluoro-2-quinolinyl)ethenyl]-11-hydroxy-6,11-dihydrodibenz[b,e]oxepine
Appearance; yellowish solid
CI-MS(m/z); 436($M^+$+1), EI-MS(m/z); 435($M^+$) $^1$H-NMR(δ, $CDCl_3$); 5.47(d, J=12.7Hz, 1H), 5.68(d, J=12.7Hz, 1H), 6.05 (s, 1H), 6.88(d, J=8.5Hz, 1H), 7.21-7.42(m, 4H), 7.39(d, J=16.1Hz, 1H), 7.57(m, 1H), 7.82(s, 1H), 7.98(d, J=8.8Hz, 1H), 8.02(d, J=9.8Hz, 1H), 8.21(d, J=7.3Hz, 1H), 8.40(d, J=8.5Hz, 1H)

Reference example 33

2-[(tetrahydropyran-2-yl)oxy]-6,11-dihydrodibenz[b,e]oxepin-11-one
In 100 ml of ethyl acetate was dissolved 2-hydroxy-6,11-dihydrodibenz[b,e]oxepin-11-one (9.95 g, 44.0 mmol), and dihydropyrane (25.0 ml, 274.0 mmol) and pyridinium p-toluenesulfonate (2.11 g, 8.4 mmol) were added to the solution and the mixture was stirred at room temperature for 21 hours.
After completion of the reaction, ethyl acetate was added to the reaction solution, and the mixture was washed successively with a saturated aqueous sodium hydrogen carbonate solution, and then, with a saturated aqueous sodium chloride solution, and dried over anhydrous sodium sulfate. The solvent was concentrated and the resulting residue was applied to silica gel chromatography (eluent: hexane/ethyl acetate = 97/5 (volume ratio)) to obtain 12.45 g of the desired compound as pale yellowish solid.
CI-MS(m/z); 311($M^+$+1), EI-MS(m/z); 226($M^+$-THP)
$^1$H-NMR(δ, $CDCl_3$); 1.59-1.73(m, 3H), 1.84-2.04(m, 3H), 3.64(dt, J=11.5, 4.4 Hz, 1H), 3.93(ddd, J=11.5, 9.0, 3.2Hz, 1H), 5.15(s, 2H), 5.42(t, J=3.3Hz, 1H), 9.98(d, J=8.8Hz, 1H), 7.22(dd, J=8.8, 3.2Hz, 1H), 7.33(dd, J=7.3, 1.5Hz, 1H), 7.45(dt, J=7.6, 1.5Hz, 1H), 7.54(dt, J=7.6, 1.5Hz, 1H), 7.89(d, J=3.2Hz, 1H), 7.92(dd, J=7.8, 1.5Hz, 1H)

Reference example 34

11-Hydroxy-2-[(tetrahydropyran-2-yl)oxy]-11-[(2-trimethylsilyl)ethynyl]-6,11-dihydrodibenz[b,e]oxepine
Under argon atmosphere, n-butyl lithium 1.6M-hexane solution (80.0 ml, 128.0 mmol) was added dropwise over 20 minutes to 50 ml of a diethyl ether solution containing trimethylacetylene (20.0 ml, 142.0 mmol) which had been cooled to -78°C by an acetone-dry ice bath. After stirring for 10 minutes, a solution of 2-[(tetrahydropyran-2-yl) oxy]-6,11-dihydrodibenz[b,e]oxepin-11-one (29.1 g, 93.8 mmol) dissolved in 200 ml of tetrahydrofuran was added to the mixture and the mixture was stirred at the same temperature for 1 hour and further at room temperature for 2 hours.
After completion of the reaction, a saturated aqueous ammonium chloride solution was added to the reaction solution, and the mixture was extracted with ethyl acetate. The organic layer was washed with a saturated aqueous sodium chloride solution and dried over anhydrous sodium sulfate. The solvent was concentrated to obtain 38.3 g of the desired compound as yellowish white solid (diastereomer mixture).

CI-MS(m/z); 409($M^+$+1), EI-MS(m/z); 408 ($M^+$), 324($M^+$-THP)
$^1$H-NMR(δ, $CDCl_3$); 0.30(s, 3H), 1.59-1.67(m, 3H), 1.79-2.03(m, 3H), 3.55-3.59(m, 1H), 3.86-3.93(m, 1H), 4.26(s, 0.5H), 4.30(s, 0.5H), 5.24(d, J=14.4 Hz, 0.5H), 5.25(d, J=14.7Hz, 1H), 5.31-5.35(m, 1H), 5.67(d, J=14.4Hz, 0.5H), 5.69 (d, J=14.7Hz, 0.5H), 6.93-6.95(m, 2H), 7.05-7.08(m, 1H), 7.25-7.30(m, 2H), 7.66-7.69(m, 1H), 8.09-8.12(m, 1H)

Reference example 35

11-Ethynyl-11-hydroxy-2-[(tetrahydropyran-2-yl)oxy]-6,11-dihydrodibenz[b,e]oxepine
In 200 ml of tetrahydrofuran was dissolved 11-hydroxy-2-[(tetrahydropyran-2-yl)oxy]-11-[(2-trimethylsilyl)ethynyl]-6,11-dihydrodibenz[b,e]oxepine (38.3 g, 93.8 mmol), and tetra-n-butyl ammonium fluoride 1.0 M-tetrahydrofuran solution (95.0 ml, 95.0 mmol) was added to the solution and the mixture was stirred at room temperature for 40 minutes.
After completion of the reaction, water was added to the reaction mixture, the resulting mixture was extracted with ethyl acetate, and the organic layer was washed with a saturated aqueous sodium chloride solution and dried over anhydrous sodium sulfate. The solvent was concentrated and the resulting residue was applied to silica gel chromatography (eluent: toluene/ethyl acetate = 99.5/0.5 (volume ratio)) to obtain 26.2 g of the desired compound as white solid (diastereomer mixture).
CI-MS(m/z); 319($M^+$-OH), 252 ($M^+$-THP), EI-MS(m/z); 252($M^+$-THP)
$^1$H-NMR(δ, $CDCl_3$); 1.57-1.71(m, 3H), 1.81-2.01(m, 3H), 3.06(s, 0.5H), 3.07(s, 0.5H), 3.56-3.60(m, 1H), 3.86-3.90(m, 1H), 4.21(br.s, 1H), 5.29(d, J=14.7Hz, 0.5H), 5.30(d, J=14.9Hz, 0.5H), 5.33-5.34(m, 1H), 5.47(d, J=14.9Hz, 0.5H), 5.53 (d, J=14.9Hz, 0.5H), 6.96-6.98(m, 2H), 7.04-7.07(m, 1H), 7.25-7.31(m, 2H), 7.60-7.62(m, 1H), 8.09-8.12(m, 1H)

Reference example 36

2-[2-Hydroxy-6,11-dihydrodibenz[b,e]oxepin-11-ylidene]acetaldehyde
In 10 ml of tetrahydrofuran was dissolved 11-ethynyl-11-hydroxy-2-[(tetrahydropyran-2-yl)oxy]-6,11-dihydrodibenz[b,e]oxepine (0.80g, 2.4 mmol), and 2 ml of distilled water was added to the solution. To the mixture was added trifluoroacetic acid (0.4 ml, 5.2 mmol), and the mixture was stirred at room temperature for 18 hours.
After completion of the reaction, a saturated aqueous sodium hydrogen carbonate solution was added to the reaction mixture, and the mixture was extracted with ethyl acetate. The organic layer was washed with a saturated aqueous sodium chloride solution and dried over anhydrous sodium sulfate. The solvent was concentrated and the resulting residue was applied to silica gel chromatography (eluent: toluene/ethyl acetate = 9/1 (volume ratio)) to obtain 0.42 g of the desired compound as yellowish solid (geometric isomer mixture).
CI-MS(m/z); 253($M^+$+1), EI-MS(m/z); 252($M^+$)
$^1$H-NMR(δ, $CDCl_3$); 4.91(br, 1H), 5.18(s, 2H), 6.32(d, J=7.8Hz, 0.67H), 6.58(d, J=8.3Hz, 0.33H), 6.71-6.89(m, 3H), 7.24-7.45(m, 4H), 9.57(d, J=8.1Hz, 0.33H), 9.88(d, J=7.8Hz, 0.67H)

Reference example 37

Ethyl 4-[2-hydroxy-6,11-dihydrodibenz[b,e]oxepin-11-ylidene-2-butenoic acid
Triethyl phosphonoacetate (11.0 ml, 55.4 mmol) was added to a mixture comprising 60% oily sodium hydride (2.04 g, 51.0 mmol) and 20 ml of tetrahydrofuran, and the mixture was stirred at room temperature for 15 minutes. To the solution was added 2-[2-hydroxy-6,11-dihydrodibenz[b,e]oxepine-11-ylidene]acetaldehyde (3.57 g, 14.2 mmol) dissolved in 20 ml of tetrahydrofuran, and the mixture was further stirred at room temperature for 15 minutes.
After completion of the reaction, a saturated aqueous ammonium chloride solution was added to the reaction solution, and the mixture was extracted with ethyl acetate. The organic layer was washed with a saturated aqueous sodium chloride solution and dried over anhydrous sodium sulfate. The solvent was concentrated and the resulting residue was applied to silica gel chromatography (eluent : hexane/ethyl acetate=85/15 (volume ratio)) to obtain 3.71 g of the desired compound as yellowish solid (geometric isomer mixture).
CI-MS(m/z); 323($M^+$+1), EI-MS(m/z); 322($M^+$)
$^1$H-NMR(δ, $CDCl_3$); 1.26(t, J=7.1Hz, 0.33H), 1.29(t, J=7.1Hz, 0.66H), 4.18(q, J=7.08Hz, 0.33H), 4.22(q, J=7.1Hz, 0.66H), 5.13 (s, 2H), 5.27(br, 0.33H), 5.61(br, 0.66H), 6.06(dd, J=15.4, 0.7Hz, 0.33H), 6.08(dd, J=15.4, 1.0Hz, 0.66H), 6.45(dd, J=11.7, 1.0Hz, 0.66H), 6.68-6.85 (m, 3.33H), 7.22-7.45(m, 4H), 7.41(dd, J=15.4, 11.7Hz, 0.33H), 7.76(dd, J=15.4, 11.7Hz, 0.66H)

Reference example 38

Ethyl 4-[2-hydroxy-6,11-dihydrodibenz[b,e]oxepin-11-yl]butanoate
10 mg of platinum oxide (IV) suspended in 2 ml of ethanol was added to ethyl 4-[2-hydroxy-6,11-dihydrodibenz[b,e]oxepin-11-ylidene-2-butenoate (0.50 g, 1.6 mmol) dissolved in 5 ml of ethanol, and the mixture was stirred at room temperature for 42 hours under hydrogen atmosphere.

After completion of the reaction, insoluble material was removed from the reaction solution, and the filtrate was concentrated to obtain 0.51 g of the desired compound as colorless transparent oily product.
CI-MS(m/z); 327($M^+$+1), EI-MS(m/z); 326 ($M^+$)
$^1$H-NMR(δ, $CDCl_3$); 1.22(t, J=7.1Hz, 3H), 1.55 (quintet, J=7.8Hz, 2H), 1.96-2.08(m, 1H), 2.17-2.30(m, 1H), 2.26(t, J=7.6Hz, 2H), 3.64(t, J=7.7Hz, 1H), 4.09(q, J=7.1Hz, 2H), 4.96(d, J=15.1 Hz, 1H), 5.04(br, 1H), 5.39(d, J=15.1Hz, 1H), 6.61-6.65(m, 2H), 6.90-6.93 (m, 1H), 6.97-7.00(m, 1H), 7.13-7.18(m, 3H)

Reference example 39

6,7-Difluoro-2-vinylquinoline

In a mixed solution comprising 8 ml of ethanol and triethylamine (0.2 ml, 1.4 mmol) was dissolved 6,7-difluoro-2-methylquinoline (10.64 g, 59.4 mmol), and 37% formaldehyde (4.72 g, 59.4 mmol) was added to the solution and the mixture was stirred at 60°C for 2 hours. Then, a mixed solution comprising diethylamine hydrochloride (6.60 g, 59.4 mmol), 3 ml of ethanol, 3 ml of water and triethylamine (0.4 ml, 2.8 mmol) was added to the mixture over 30 minutes, and then, the mixture was stirred at 60°C for 12 hours.

After completion of the reaction, water was added to the reaction mixture, the resulting mixture was extracted with ethyl acetate, the organic layer was washed with a saturated aqueous sodium chloride solution and dried over anhydrous magnesium sulfate. The solvent was concentrated and the resulting residue was applied to silica gel chromatography (eluent: hexane/ethyl acetate = 9/1 (volume ratio)) to obtain 2.89 g of the desired compound as pale yellowish solid.
CI-MS(m/z); 192($M^+$+1), EI-MS(m/z); 191($M^+$)
$^1$H-NMR(δ, DMSO-$d_6$); 5.69(dd, J=11.0, 1.0Hz, 1H), 6.39(dd, J=17.6, 1.0Hz, 1H), 6.95(dd, =17.6, 11.0Hz, 1H), 7.82 (d, J=8.8Hz, 1H), 6.93(dd, J=8.1, 3.9Hz, 1H), 8.00(dd, J=9.0, 2.2Hz, 1H), 8.33(d, J=8.5Hz, 1H)

Reference example 40

11-Oxo-2-trifluoromethanesulfonyloxy-6,11-dihydrodibenz[b,e]thiepine

In 20 ml of methylene chloride was dissolved 2-hydroxy-6,11-dihydrodibenz[b,e]thiepin-11-one (1.12 g, 4.6 mmol), and under ice-cooling, trifluoromethanesulfonic anhydride (2.32 ml, 13.8 mmol) and triethylamine (1.84 ml, 13.8 mmol) were added to the solution and the mixture was stirred at the same temperature for 6 hours.

After completion of the reaction, the reaction solution was concentrated and the obtained residue was applied to silica gel chromatography (eluent: toluene/ethyl acetate = 9/1 (volume ratio)) to obtain 0.95 g of the desired compound as black oily product.
CI-MS(m/z); 375($M^+$+1), EI-MS(m/z); 374 ($M^+$)
$^1$H-NMR(δ, DMSO-$d_6$); 4.35(s, 2H), 7.40-7.50(m, 2H), 7.53(d, J=7.8Hz, 1H), 7.58(d, J=7.3Hz, 1H), 7.60-7.70(m, 2H), 8.08(d, J=2.2Hz, 1H)

In the same manner as in Reference example 40, a compound of the following Reference example 41 was obtained.

Reference example 41

5-Oxo-3-trifluoromethanesulfonyloxy-10,11-dihydro-5H-dibenzo[a,d]cycloheptene
Appearance; brownish oily product
CI-MS(m/z); 357($M^+$+1), EI-MS(m/z); 356($M^+$)
$^1$H-NMR(δ, DMSO-$d_6$); 3.00-3.30(m, 4H), 7.30-7.50(m, 3H), 7.51-7.65(m, 2H), 7.70(dd, J=8.5, 2.7Hz, 1H), 7.88(d, J=2.9Hz, 1H)

Reference example 42

2-[(E)-2-(6,7-difluoro-2-quinolinyl)ethenyl]-6,11-dihydrodibenz[b,e]thiepin-11-one

In 7 ml of N,N-dimethylformamide were dissolved 11-oxo-2-trifluoromethanesulfonyloxy-6,11-dihydrodibenz [b,e]thiepin-2-yl (0.67 g, 1.8 mmol) and 6,7-difluoro-2-vinylquinoline (0.31g, 1.6 mmol), and then, palladium acetate (0.04 g, 0.2 mmol), triphenylphosphine (0.16 g, 0.6 mmol) and lithium bromide (0.84 g, 4.8 mmol) were added to the solution. Then, under nitrogen atmosphere, triethylamine (2.0 ml, 14.0 mmol) was added to the mixture and the mixture was stirred at 100°C for 4 hours.

After completion of the reaction, the reaction solution was concentrated, and the obtained residue was applied

to silica gel chromatography (eluent: toluene/ethyl acetate = 9/1 (volume ratio)) to obtain 0.62 g of the desired compound as dark brownish solid.
CI-MS(m/z); 416($M^+$+1), EI-MS(m/z); 415 ($M^+$)
$^1$H-NMR(δ, DMSO-$d_6$) ; 4.31(s, 2H), 7.30-8.20(m, 11H), 8.32(d, J=7.8Hz, 1H), 8.39(d, J=8.5Hz, 1H)
In the same manner as in Reference example 42, a compound of the following Reference example 43 was obtained.

Reference example 43

3-[(E)-2-(6,7-difluoro-2-quinolinyl)ethenyl]-10,11-dihydro-5H-dibenz[a,d]cyclohepten-5-one
Appearance; brownish solid
CI-MS(m/z); 398($M^+$+1), EI-MS(m/z); 397($M^+$)
$^1$H-NMR(δ, DMSO-$d_6$) ; 3.21(s, 4H), 7.20-7.55(m, 7H), 7.85-8.25(m, 5H), 8.38(d, J=8.5Hz, 1H)
In the same manner as in Reference example 1(b), compounds of the following Reference examples 44 to 45 were obtained.

Reference example 44

2-[(E)-2-(6,7-difluoro-2-quinolinyl)ethenyl]-11-hydroxy-6,11-dihydrodibenz[b,e]thiepine
Appearance; dark brownish solid
CI-MS(m/z); 418($M^+$+1), EI-MS(m/z); 417($M^+$)
$^1$H-NMR(δ, DMSO-$d_6$); 4.38(d, J=13.7, 1H), 4.68(d, J=13.7Hz, 1H), 6.21(s, 1H), 7.06(d, J=8.3Hz, 1H), 7.20-7.50(m, 5H), 7.34(d, J=14.9, 1H), 7.84(d, J=14.4, 1H), 7.90-8.15(m, 4H), 8.40(d, J=8.8Hz, 1H)

Reference example 45

3-[(E)-2-(6,7-difluoro-2-quinolinyl)ethenyl]-5-hydroxy-10,11-dihydro-5H-dibenz[a,d]cycloheptene
Appearance; dark brownish solid
CI-MS(m/z); 400($M^+$+1), EI-MS(m/z); 399($M^+$)
$^1$H-NMR(δ DMSO-$d_6$); 3.00-3.30(m, 4H), 5.90-6.05(br.s, 1H), 6.13(s, 1H), 7.10-7.60(m, 6H), 7.39(d, J=16.4Hz, 1H), 7.65-8.10(m, 5H), 8.35(d, J=8.8Hz, 1H)

Reference example 46

8-Bromo-2,11-dihydroxy-6,11-dihydrodibenz[b,e]oxepine
In a mixed solution comprising 400ml of tetrahydrofuran and 200 ml of methanol was dissolved 8-bromo-2-hydroxy-6,11-dihydrodibenz[b,e]oxepin-11-one (48.41 g, 0.159 mol), and then, sodium borohydride (6.00 g, 0.159 mol) was added to the solution and the mixture was stirred at room temperature for 2 hours.
After completion of the reaction, the solvent was removed under reduced pressure, water was added to the residue, and after a pH of the mixture was adjusted to about 6.0 with 1N-hydrochloric acid, the mixture was extracted with ethyl acetate. The organic layer was washed with a saturated aqueous sodium chloride solution and dried over anhydrous sodium sulfate, and the solvent was removed under reduced pressure to obtain 55.95 g of the desired compound as dark brownish oily product. This product was used as such for the next reaction of Reference example 47 without further purification.
CI-MS(m/z); 307($M^+$+1), EI-MS(m/z); 306($M^+$)
$^1$H-NMR(δ, DMSO-$d_6$) ; 5.52 (d, J=13.7Hz, 1H), 5.30(d, J=13.7Hz, 1H), 5.91(d, J=4.4Hz, 1H), 6.12(d, J=4.6Hz, 1H), 6.57(dd, J=8.8, 2.9Hz, 1H), 6.68(d, J=8.8Hz, 1H),
6.84(d, J=2.9Hz, 1H), 7.40-7.60(m, 3H), 9.01(s, 1H)

Reference example 47

8-Bromo-2-hydroxy-11-methoxy-6,11-dihydrodibenz[b,e]-oxepine
In 500 ml of methanol was dissolved 8-bromo-2,11-dihydroxy-6,11-dihydrodibenz[b,e]oxepine (55.95 g), and then, p-toluenesulfonic acidmonohydrate (3.02 g, 15.9 mmol) was added to the solution and the mixture was refluxed for 1.5 hours.
After completion of the reaction, the solvent was removed under reduced pressure, the obtained residue was applied to silica gel chromatography (gradient eluent: hexane to hexane/ethyl acetate = 9/1 (volume ratio)) to obtain 45.85 g of the desired compound as brownish oily product.

CI-MS(m/z); 321($M^+$+1), EI-MS(m/z); 320($M^+$)
$^1$H-NMR(δ, DMSO-$d_6$) ; 3.25(s, 3H), 4.95(d, J=12.9Hz, 1H), 5.16(s, 1H), 5.57(d, J=12.9Hz, 1H), 6.67(dd, J=8.8, 2.2Hz, 1H), 6.69(d, J=8.1Hz, 1H), 6.74(d, J=2.0Hz, 1H), 7.39(d, J=8.3Hz, 1H), 7.52(dd, J=8.1, 2.2Hz, 1H), 7.61(d, J=2.0Hz, 1H)

Reference example 48

8-Bromo-2-t-butyldimethylsilyloxy-11-methoxy-6,11-dihydrodibenz[b,e]oxepine
In 400 ml of methylene chloride was dissolved 8-bromo-2-hydroxy-11-methoxy-6,11-dihydrodibenz[b,e]oxepine (41.75 g, 0.130 mol), and then, t-butyldimethylsilyl chloride (29.78 g, 0.198 mol), imidazole (17.88 g, 0.263 mol) and N,N-dimethylaminopyridine (1.59 g, 0.013 mol) were added to the solution and the mixture was stirred at room temperature for 1.5 hours.
After completion of the reaction, the solvent was removed under reduced pressure, the obtained residue was applied to silica gel chromatography (gradient eluent: hexane to hexane/ethyl acetate = 50/1 (volume ratio)) to obtain 51.26 g of the desired compound as reddish oily product.
CI-MS(m/z); 435($M^+$+1), EI-MS(m/z); 434 ($M^+$)
$^1$H-NMR(δ, DMSO-$d_6$) ; 0.16(s, 6H), 0.94(s, 9H), 3.24(s, 3H), 4.96(d, J=12.7Hz, 1H), 5.20 (s, 1H), 5.66(d, J=12.5Hz, 1H), 6.65-6.75(m, 1H), 6.76(d, J=8.8Hz, 1H), 6.84(d, J=2.0Hz, 1H), 7.38(d, J=8.3Hz, 1H), 7.55(dd, J=8.1, 2.2Hz, 1H), 7.66(d, J=2.0Hz, 1H)

Reference example 49

11-Methoxy-8-methoxycarbonyl-2-t-butyldimethylsilyloxy-6,11-dihydrodibenz[b,e]oxepine
n-Butyl lithium 2.52 M-hexane solution (6.83 ml, 17.22 mmol) was added dropwise to 8-bromo-11-methoxy-2-t-butyldimethylsilyloxy-6,11-dihydrodibenz[b,e]oxepine (5.00 g, 11.48 mmol) solution dissolved in 60 ml of tetrahydrofuran at - 78°C, and the mixture was stirred at the same temperature for 30 minutes. Then, dry ice (65.0 g) was gradually added to the mixture, the mixture was gradually raised to room temperature. Then, dimethylsulfate (1.30 ml, 13.78 mmol) was added to the mixture and the mixture was refluxed for 8.5 hours.
After completion of the reaction, ethyl acetate was added to the reaction mixture, and the mixture was washed with a saturated aqueous sodium hydrogen carbonate solution, and then, with a saturated aqueous sodium chloride solution, dried over anhydrous sodium sulfate, and the solvent was removed under reduced pressure to obtain 5.44 g of the desired compound as red brownish oily product. This product was used as such for the next reaction of Reference example 50 without effecting further purification.
CI-MS(m/z); 415($M^+$+1), EI-MS (m/z) ; 414 ($M^+$)
$^1$H-NMR(δ, DMSO-$d_6$) ; 0.16(s, 6H), 0.94(s, 9H), 3.28(s, 3H), 3.87(s, 3H), 5.11(d, J=12.5Hz, 1H), 5.34(s, 1H), 5.70(d, J=12.7Hz, 1H), 6.72(d, J=8.8Hz, 1H), 6.76(d, J=8.8Hz, 1H), 6.87(d, J=2.0Hz, 1H), 7.58(d, J=7.8Hz, 1H), 7.94(dd, J=7.8, 1.7Hz, 1H), 8.00(d, J=2.0Hz, 1H)

Reference example 50

2-Hydroxy-11-methoxy-8-methoxycarbonyl-6,11-dihydrodibenz[b,e]oxepine
Tetra-n-butylammonium fluoride (6.00 ml, 20.66 mmol) was added to 11-methoxy-8-methoxycarbonyl-2-t-butyldimethylsilyloxy-6,11-dihydrodibenz[b,e]oxepine (5.44 g) solution dissolved in 250 ml of tetrahydrofuran under ice-cooling, and the mixture was stirred at the same temperature for 1 hour.
After completion of the reaction, a saturated aqueous ammonium chloride solution was added to the reaction solution, and the mixture was extracted with ethyl acetate. The organic layer was washed with a saturated aqueous sodium chloride solution, and dried over anhydrous sodium sulfate. The solvent was removed under reduced pressure, and the obtained residue was applied to silica gel chromatography (gradient eluent: toluene to toluene/ethyl acetate = 7/3 (volume ratio)) to obtain 2.47 g of the desired compound as yellowish oily product.
CI-MS(m/z); 301($M^+$+1), EI-MS(m/z); 300 ($M^+$)
$^1$H-NMR(δ, DMSO-$d_6$) ; 3.29(s, 3H), 3.86(s, 3H), 5.10(d, J=12.9Hz, 1H), 5.31(s, 1H), 5.61(d, J=12.7Hz, 1H), 6.66(dd, J=8.8, 2.4Hz, 1H), 6.70(d, J=8.1Hz, 1H), 6.77(d, J=2.4Hz, 1H), 7.59 (d, J=7.8Hz, 1H), 7.92(dd, J=7.8, 1.7Hz, 1H), 7.95 (d, J=1.7Hz, 1H), 9.06(Br.s.1H)
In the same manner as in Example 51(b), a compound of the following Reference example 51 was obtained.

Reference example 51

11-Methoxy-8-methoxycarbonyl-2-trifluoromethanesul-fonyloxy-6,11-dihydrodibenz[b,e]oxepine

CI-MS(m/z); 433($M^+$+1), EI-MS(m/z); 432($M^+$)
$^1$H-NMR(δ, DMSO-$d_6$); 3.32(s, 3H) , 3.88(s, 3H), 5.30(d, J=12.5Hz, 1H), 5.56(s, 1H), 5.80 (d, J=12.5Hz, 1H), 6.98(d, J=9.OHz, 1H), 7.38(dd, J=9.0, 3.2Hz, 1H), 7.58(d, J=2.9Hz, 1H), 7.58(d, J=8.3Hz, 1H), 7.99(dd, J=7.8, 1.7Hz, 1H) , 8.08(d, J=1.7Hz, 1H)

In the same manner as in Example 51(c), a compound of the following Reference example 52 was obtained.

Reference example 52

2-[(E)-2-(5,6,7,8-tetrahydro-2-quinolinyl)ethenyl]-11-methoxy-8-methoxycarbonyl-6,11-dihydrodibenz[b,e]oxepine CI-MS(m/z); 442($M^+$+1), EI-MS(m/z); 441($M^+$)
$^1$H-NMR(δ, DMSO-$d_6$); 1.60-1.90(m, 2H), 2.72(t, J=6.1Hz, 1H), 2.81(t, J=6.3Hz, 1H), 3.29(s, 3H) , 3.86(s1 3H), 5.16 ('d, J=12.5Hz, 1H), 5.36(s, 1H), 5.86(d, J=12.2Hz, 1H) , 6.82(d, J=8.5Hz, 1H) , 7.10(d, J=16.1Hz, 1H), 7.26(d, J=8.1Hz, 1H) , 7.41(d, J=8.1Hz, 1H) , 7.48(d, J=16.1Hz, 1H), 7.53(dd, J=8.5, 2.2Hz, 1H) , 7.58(d, J=8.1Hz, 1H) , 7.68(d, J=2.0Hz, 1H) , 7.96(dd, J=7.8, 1.7Hz, 1H) , 8.05(d, J=1.7Hz, 1H)

Reference example 53

9-[(E)-2-(6,7-difluoro-2-quinolinyl)ethenyl]-6,11-di-hydrodibenz[b,e]oxepin-11-one

Palladium acetate (0.67 g, 3.0 mmol) and triphenylphosphine (0.33 g, 13 mmol) were added to 40 ml of N,N-di-methylformamide solution containing 9-bromo-6,11-dihydro-dibenz[b,e]oxepin-11-one (2.0 g, 6.9 mmol) and 6,7-difluoro-2-vinylquinoline (1.58 g, 8.3 mmol), and then, under nitrogen atmosphere, triethylamine (6.0 ml, 42 mmol) was added to the solution and the mixture was stirred at 100°C for 4 hours.

After completion of the reaction, water was added to the reaction mixture, the resulting mixture was extracted with ethyl acetate, the organic layer was washed with a saturated aqueous sodium chloride solution and dried over anhydrous sodium sulfate. The residue obtained by concentrating the solvent was applied to silica gel column chromatography (eluent: hexane/ethyl acetate = 3/1 (volume ratio)) to obtain 0.97 g of the desired compound as ocherous solid.
CI-MS(m/z); 400($M^+$+1), EI-MS(m/z); 399($M^+$)
$^1$H-NMR (δ, DMSO-$d_6$); 5.34(s, 2H), 7.14(d, J=9.0Hz, 1H) , 7.20(t, J=8.1Hz, 1H), 7.57-7.67 (m, 3H), 7.91-8.08(m, 6H), 8.14(dd, J=8.1, 1.7Hz, 1H), 8.40(d, J=8.6Hz, 1H)

In the same manner as in Reference example 1(b), a compound of the following Reference example 54 was obtained.

Reference example 54

9-[(E)-2-(6,7-difluoro-2-quinolinyl)ethenyl]-11-hydroxy-6,11-dihydrodibenz[b,e]oxepine
Appearance; ocherous solid
CI-MS(m/z); 402($M^+$+1), EI-MS(m/z); 401 ($M^+$)
$^1$H-NMR(δ, DMSO-$d_6$); 5.24(d, J=12.7Hz, 1H), 5.71(d, J=12.5Hz, 1H), 5.92(s, 1H), 6.21(br.s, 1H), 6.39(d, J=9.3Hz, 1H), 6.93(t, J=7.6Hz, 1H) , 7.18(t, J=8.3Hz, 1H) , 7.42-7.52(m, 3H), 7.66(d, J=7.8Hz, 1H), 7.84-8.27(m. 5H), 8.38(d, J=8.6Hz, 1H)

Reference example 55

5-Fluoroquinaldine

Croton aldehyde (23.5 ml, 0.28 mol) was added dropwise over 50 minutes to a mixed solution comprising 40 ml of water and 145 ml of conc. hydrochloric acid dissolved therein 3-fluoroaniline (30.0 g, 0.27 mol) under reflux, and the mixture was refluxed for 2 hours.

After completion of the reaction, the reaction mixture was cooled, and washed with methylene chloride. Then, toluene was added to the aqueous layer, and a pH of the layer was adjusted to about 9.0 by a 30% aqueous sodium hydroxide solution and the mixture was extracted with toluene. The organic layer was washed with a saturated aqueous sodium chloride solution and dried over anhydrous sodium sulfate, and the mixture was concentrated. The obtained residue was applied to silica gel column chromatography (gradient eluent: toluene to toluene/ethyl acetate=10/1 (volume ratio)) to obtain 1.04 g of the desired compound as yellowish oily product.
CI-MS(m/z); 162($M^+$+1), EI-MS(m/z); 161($M^+$)
$^1$H-NMR(δ, DMSO-$d_6$); 2.69(s, 3H), 7.37(ddd, J=10.0, 7.8, 1.0Hz, 1R), 7.52(d, J=8.8Hz, 1H), 7.71(td, J=7.8, 6.1Hz, 1H), 7.80(d, J=8.5Hz, 1H), 8.37(d, J=8.5Hz, 1H)

Reference example 56

2-Bromomethyl-5-fluoroquinoline

To 40 ml of ethyl acetate solution containing 5-fluoroquinaldine (2.03 g, 12.6 mmol) were added N-bromosuccinimide (4.05 g, 22.8 mmol) and 2,2'-azobis(isobutyro-nitrile) (0.37 g, 2.25 mmol) divided into several times while refluxing for 12 hours.

After completion of the reaction, the reaction solution was cooled, and the mixture was washed successively with a saturated aqueous sodium hydrogen carbonate solution, an aqueous sodium thiosulfate solution, and then, with a saturated aqueous sodium chloride solution, and dried over anhydrous sodium sulfate. A residue obtained by concentration was applied to silica gel column chromatography (eluent: hexane/ethyl acetate = 50/1 (volume ratio)) to obtain 2.20 g of the desired compound as yellowish solid.
CI-MS(m/z); 240($M^+$+1), EI-MS(m/z); 239($M^+$)
$^1$H-NMR(δ, DMSO-$d_6$); 4.89(s, 2H), 7.47(ddd, J=10.0, 7.8, 1.0H 1H), 7.79(d, J=8.8Hz, 1H), 7.75-7.85(m, 1H), 7.88(d, J=8.5Hz, 1H), 8.54(d, J=8.5Hz, 1H)

Reference example 57

(5-Fluoroquinolin-2-yl)triphenylphosphonium bromide

Triphenylphosphine (3.36 g, 12.8 mmol) was added to 15 ml of acetonitrile solution containing 2-bromomethyl-5-fluoroquinoline (2.20 g, 9.2 mmol), and the mixture was stirred at 60°C for 5 hours.

After completion of the reaction, the solvent was removed from the reaction solution under reduced pressure, and diethyl ether was added to the residue to crystallize the product. The crystal was obtained by filtration, washed with diethyl ether, and dried under reduced pressure to obtain 5.46 g of the desired compound as brownish solid.
CI-MS(m/z); 421($M^+$-HBr) , EI-MS(m/z); 421($M^+$-HBr)

In the same manner as in Reference example 55, compounds of the following Reference examples 58 to 60 were obtained.

Reference example 58

5,7-Difluoroquinaldine
Appearance; brownish solid
CI-MS(m/z); 180($M^+$+1), EI-MS(m/z); 179($M^+$)
$^1$H-NMR(δ, DMSO-$d_6$) ; 2.69(s, 3H), 7.40-7.60(m, 3H), 8.34(d, J=8.8Hz, 1H)

Reference example 59

5,6,7-Trifluoroquinaldine
Appearance; brownish solid
CI-MS(m/z); 198($M^+$+1), EI-MS(m/z); 197($M^+$)
$^1$H-NMR(δ, DMSO-$d_6$); 2.68(s, 3H) , 7.56(d, J=8.5Hz, 1H), 7.81(ddd, J=11.7, 7.1, 2.4Hz, 1H), 8.38(d, J=8.8Hz, 1H)

Reference example 60

6-Fluoro-7-trifluoromethylquinaldine
Appearance; brownish solid
CI-MS(m/z); 230($M^+$+1), EI-MS(m/z); 229($M^+$)
$^1$H-NMR(δ, DMSO-$d_6$); 2.87(s, 3H), 7.89(d, J=8.8Hz, 1H), 8.28(d, J=11.2Hz, 1H), 8.66(d, J=6.8Hz, 1H), 8.70(d, J=8.5Hz, 1H)

In the same manner as in Reference example 56, a compound of the following Reference examples 61 to 63 were obtained.

Reference example 61

2-Bromomethyl-5,7-difluoroquinaldine
Appearance; brownish solid
CI-MS(m/z); 180($M^+$+1), EI-MS(m/z); 179 ($M^+$)
$^1$H-NMR(δ, DMSO-$d_6$); 4.87(s, 2H), 7.55-7.75(m, 2H), 7.76(d, J=8.8Hz, 1H), 8.53(d, J=8.5Hz, 1H)

Reference example 62

2-Bromomethyl-5,6,7-trifluoroquinaldine
Appearance; brownish solid
CI-MS(m/z); 180($M^+$+1) , EI-MS(m/z); 179($M^+$)
$^1$H-NMR (δ, DMSO-$d_6$); 4.88 (s, 2H) , 7.82 (d, J=8.8Hz, 1H) , 7.95(ddd, J=11.5, 7.1, 2.4Hz, 1H), 8.56(d, J=8.8Hz, 1H)

Reference example 63

2-Bromomethyl-6-fluoro-7-trifluoromethylquinaldine
Appearance; brownish solid
CI-MS(m/z); 308($M^+$+1), EI-MS(m/z); 307($M^+$)
$^1$H-NMR(δ, DMSO-$d_6$) ; 4.90(s, 2H), 7.90(d, J=8.5Hz, 1H) , 8.15(d, J=11.5Hz, 1H), 8.42(d, J=7.1Hz, 1H), 8.52(d, J=8.5Hz, 1H)
In the same manner as in Reference example 57, compounds of the following Reference examples 64 to 66 were obtained.

Reference example 64

(5,7-Difluoroquinolin-2-yl)triphenylphosphonium bromide
Appearance; pale brownish solid
CI-MS(m/z); 439($M^+$-HBr), EI-MS(m/z); 439($M^+$-HBr)

Reference example 65

(5,6,7-Trifluoroquinolin-2-yl)triphenylphosphonium bromide
Appearance; brownish solid
CI-MS(m/z); 439($M^+$-HBr), EI-MS(m/z); 439($M^+$-HBr)

Reference example 66

(6-Fluoro-7-trifluoromethylquinolin-2-yl)triphenyl-phosphonium bromide
Appearance; brownish solid
In the same manner as in Reference example 2, a compound of the following Reference example 67 was obtained.
Reference example 67

(a) 2-[(E)-2-(6,7-difluoro-2-quinolinyl)ethenyl-7-fluoro-6,11-dihydrodibenz[b,e]oxepin-11-one
Appearance; pale yellowish solid
$^1$H-NMR(δ DMSO-$d_6$); 8.39-8.36(m, 2H), 8.05-7.90(m, 5H) , 7.53(dd, J=9.0, 2.4Hz, 1H), 7.47-7.38(m, 3H), 7.21(d, J=8.6Hz, 1H), 5.37(s, 2H)

(b) 2-[(E)-2-(6,7-difluoro-2-quinolinyl)ethenyl]-7-fluoro-11-hydroxy-6,11-dihydrodibenz[b,e]oxepine
Appearance; pale yellowish solid
$^1$H-NMR(δ, DMSO-$d_6$); 8.35(d, J=8.6Hz, 1H) , 8.05-7.29(m, 9H) , 7.18(td, J=9.0, 2.7Hz, 1H), 6.84(d, J=8.5Hz, 1H), 6.25(br.s, 1H), 5.93(s, 1H), 5.73(d, J=12.5Hz, 1H) , 5.27(d, J=12.5Hz, 1H)

In the same manner as in Reference example 19, a compound of the following Reference example 68 was obtained.

Reference example 68

Ethyl 2-bromomethyl-4-fluorobenzoate
Appearance; red brownish oily product
CI-MS(m/z); 261($M^+$+1)
$^1$H-NMR(δ, CDCl3); 8.02(dd, J=4.3, 2.7Hz, 1H), 7.19(dd, J=9.3, 2.7Hz, 1H), 7.05(td, J=7.8, 2.7Hz, 1H), 4.93(s, 2H), 4.40(q, J=7.1Hz, 2H), 1.42(t, J=7.1Hz, 3H)
In the same manner as in Reference example 14(c), a compound of the following Reference example 69 was

obtained.

Reference example 69

Ethyl 2-{4-(6,7-difluoroquinolin-2-ylethenyl)phenoxy-methyl}-4-fluorobenzoate
Appearance; pale yellowish solid
CI-MS(m/z); 464($M^+$+1)
$^1$H-NMR(δ, DMSO-$d_6$); 8.35-7.06(m, 13H), 5.48(S, 2H), 4.30(q, J=7.1Hz, 2H), 1.30(t, J=7.1Hz, 3H)

In the same manner as in Reference example 14(d), a compound of the following Reference example 70 was obtained.

Reference example 70

2-{4-(6,7-Difluoroquinolin-2-ylethenyl)phenoxy-methyl}-4-fluorobenzoic acid
Appearance; yellowish solid
CI-MS(m/z); 436($M^+$+1)
$^1$H-NMR(δ, DMSO-$d_6$) ; 8.34-6.92(m, 13H) , 5.52(S, 2H)

## Claims

1. A tricyclic compound represented by the formula (I):

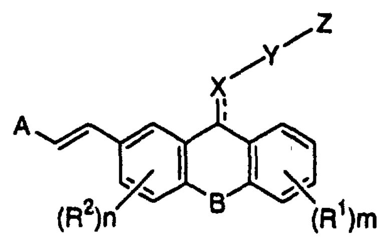

(I)

wherein $R^1$ represents a hydrogen atom, a halogen atom, a hydroxyl group, a nitro group, a cyano group, a carbamoyl group, a formyl group, a carboxyl group, a $C_1$-$C_4$ alkoxycarbonyl group, a 1H-tetrazol-5-yl group, $C_1$-$C_4$ alkyl group, a fluoro $C_1$-$C_4$ alkyl group, a hydroxy $C_1$-$C_4$ alkyl group, a $C_2$-$C_4$ alkenyl group, a $C_2$-$C_4$ alkynyl group, a $C_1$-$C_4$ alkoxy group, a fluoro $C_1$-$C_4$ alkoxy group, a $C_1$-$C_4$ alkylthio group, a $C_1$-$C_4$ alkyisulfinyl group or a $C_1$-$C_4$ alkylsulfonyl group, $R^2$ represents a hydrogen atom, a halogen atom, a nitro group, a cyano group, $C_1$-$C_4$ alkyl group or a $C_1$-$C_4$ alkoxy group, A represents a 5-membered or a 6-membered heteroaromatic ring group containing 1 to 3 hetero atoms selected from the group consisting of a nitrogen atom, an oxygen atom and a sulfur atom, or a fused heteroaromatic ring group in which the heteroaromatic ring group and a benzene ring are fused, said heteroaromatic ring group or fused heteroaromatic ring group may have a halogen atom, a nitro group, a cyano group, a $C_1$-$C_4$ alkyl group,a fluoro $C_1$-$C_4$ alkyl group, a $C_1$-$C_4$ alkoxy group,a fluoro $C_1$-$C_4$ alkoxy group, a $C_1$-$C_4$ alkylthio group,a fluoro $C_1$-$C_4$ alkylthio group or a $C_3$-$C_4$ alkylene group as a substituent(s), B represents a formula: $-OCH_2-$, a formula: $-CH_2CH_2-$, a formula: $-SCH_2-$, a formula: $-CH_2O-$ or a formula: $-CH_2S-$, X represents an oxygen atom, a sulfuratom, amethylene group or a formula: =CH-, Y represents a $C_1$-$C_{10}$ alkylene group, phenylene group or a group of a formula (a):

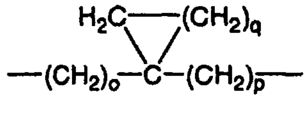

(a)

wherein o and p each is an integer of 0 to 2, and q is an integer of 1 to 4,
each of which may havea halogen atom, a $C_1$-$C_4$ alkyl group or a $C_1$-$C_4$ alkoxy group as a substituent(s), Z represents a carboxyl group which may be protected; a 1H-tetrazol-5-yl group; a formula: $-SO_3H$ group; a formula:

$-NH-SO_2-R^3$; or a formula: $-CO-NH-SO_2-R^3$

wherein $R^3$ represents a $C_1$-$C_4$ alkyl group, a fluoro$C_1$-$C_4$ alkyl group or a phenyl group which may have at least one substituent selected from the group consisting of a halogen atom, a $C_1$-$C_4$ alkyl group,a fluoro $C_1$-$C_4$ alkyl group, a $C_1$-$C_4$ alkoxy group, a fluoro $C_1$-$C_4$ alkoxy group, a nitro group and a cyano group as a substituent(s), ------ represents a single bond or a double bond,

m is an integer of 1 to 4, and when m is 2 or more, then $R^1$ may be the same or different from each other, and n is an integer of 1 to 3, and when n is 2 or more, then $R^2$ may be the same or different from each other, or a pharmaceutically acceptable salt thereof.

**2.** A tricyclic compound or a pharmaceutically acceptable salt thereof according to Claim 1, wherein $R^1$ of the compound represented by the formula (I) is selected from the group consisting of a hydrogen atom, a fluorine atom, a chlorine atom, a bromine atom, a hydroxyl group, a nitro group, a cyano group, a carbamoyl group, a formyl group, a carboxyl group, a methoxycarbonyl group, an ethoxycarbonyl group, a propoxy-carbonyl group, an isopropoxycarbonyl group, a 1H-tetrazol-5-yl group, amethyl group, an ethyl group, a propyl group, an isopropyl group, a fluoromethyl group, a difluoromethyl group, a trifluoromethyl group, a 2-fluoroethyl group, a hydroxymethyl group, a 1-hydroxyethyl group, a 2-hydroxyethyl group, a 1-hydroxy-1-methylethyl group, a 1-hydroxypropyl group, a 2-hydroxypropyl group, a vinyl group, a 1-propenyl group, an allyl group, a 1-butenyl group, a 2-butenyl group, a 2-methyl-1-propenyl group, an ethynyl group, a 1-propynyl group, a 1-butynyl group, a methoxy group, an ethoxy group, a propoxy group, an isopropoxy group, a fluoromethoxy group, a difluoromethoxy group, a trifluoromethoxy group, a 2-fluoroethoxy group, a methylthio group, an ethylthio group, a propylthio group, an isopropylthio group, a methylsulfinyl group, an ethylsulfinyl group, a propylsulfinyl group, an isopropylsulfinyl group, a methylsulfonyl group, an ethyl-sulfonyl group, a propylsulfonyl group and an isopropylsulfonyl group.

**3.** A tricyclic compound or a pharmaceutically acceptable salt thereof according to Claim 1, wherein $R^1$ of the compound represented by the formula (I) is selected from the group consisting of a hydrogen atom, a fluorine atom, a chlorine atom, a hydroxyl group, a nitro group, a cyano group, a carbamoyl group, a formyl group, a methoxycarbonyl group, an ethoxycarbonyl group, a 1H-tetrazol-5-yl group, a methyl group, an ethyl group, a fluoromethyl group, a difluoromethyl group, a trifluoromethyl group, a hydroxymethyl group, a 1-hydroxyethyl group, a 1-hydroxy-1-methylethyl group, a 1-hydroxypropyl group, avinyl group, a 1-propenyl group, an allyl group, an ethynyl group, a 1-propynyl group, a 1-butynyl group, a methoxy group, an ethoxy group, a fluoromethoxy group, a difluoromethoxy group, a trifluoromethoxy group, a methylthio group, an ethylthio group, a methylsulfinylgroup, an ethylsulfinylgroup, amethylsulfonyl group and an ethylsulfonyl group.

**4.** A tricyclic compound or a pharmaceutically acceptable salt thereof according to Claim 1, wherein $R^1$ of the compound represented by the formula (I) is selected from the group consisting of a hydrogen atom, a fluorine atom, a nitro group, a'cyano group, a formyl group, a methoxycarbonyl group, a 1H-tetrazol-5-yl group, a methyl group, a difluoromethyl group, a trifluoromethyl group, a hydroxymethyl group, a 1-hydroxy-1-methylethyl group, a vinyl group, an ethynyl group, a methoxy group, a difluoromethoxy group, a trifluoromethoxy group, a methylthio group, a methylsulfinyl group and a methylsulfonyl group.

**5.** A tricyclic compound or a pharmaceutically acceptable salt thereof according to Claim 1, wherein $R^1$ of the compound represented by the formula (I) is selected from the group consisting of a hydrogen atom, a fluorine atom, a cyano group, a trifluoromethyl group and an ethynyl group.

**6.** A tricyclic compound or a pharmaceutically acceptable salt thereof according to Claim 1, wherein $R^2$ of the compound represented by the formula (I) is selected from the group consisting of a hydrogen atom, a fluorine atom, a chlorine atom, a nitro group, a cyano group, a methyl group, an ethyl group, a methoxy group and an ethoxy group.

**7.** A tricyclic compound or a pharmaceutically acceptable salt thereof according to Claim 1, wherein $R^2$ of the compound represented by the formula (I) is selected from the group consisting of a hydrogen atom, a fluorine atom, a chlorine atom, methyl group and methoxy group.

**8.** A tricyclic compound or a pharmaceutically acceptable salt thereof according to Claim 1, wherein $R^2$ of the compound represented by the formula (I) is a hydrogen atom.

**9.** A tricyclic compound or a pharmaceutically acceptable salt thereof according to Claim 1, wherein the ring shown by A of the compound represented by the formula (I) is selected from the group consisting of furan, thiophene, oxazole, thiazole, imidazole, pyrazole, thiadiazole, pyridine, pyrimidine, pyridazine, pyrazine, benzofuran, benzothiophene, benzoxazole, benzothiazole, benzimidazole, quinoline, quinazoline and quinoxaline rings.

**10.** A tricyclic compound or a pharmaceutically acceptable salt thereof according to Claim 1, wherein the ring shown by A of the compound represented by the formula (I) is selected from the group consisting of oxazole, thiazole, imidazole, pyrazole, thiadiazole, pyridine, pyrimidine, pyridazine, pyrazine, benzoxazole, benzothiazole, benzimidazole, quinoline, quinazoline and quinoxaline rings.

**11.** A tricyclic compound or a pharmaceutically acceptable salt thereof according to Claim 1, wherein the ring shown by A of the compound represented by the formula (I) is selected from the group consisting of thiazole, thiadiazole, pyridine, pyrimidine, benzoxazole, benzothiazole, quinoline and quinazoline rings.

**12.** A tricyclic compound or a pharmaceutically acceptable salt thereof according to Claim 1, wherein the ring shown by A of the compound represented by the formula (I) is selected from the group consisting of pyridine, benzothiazole and quinoline rings.

**13.** A tricyclic compound or a pharmaceutically acceptable salt thereof according to any one of Claims 9 to 12, wherein said heteroaromatic ring group or fused heteroaromatic ring group is substituted by at least one substituent selected from the group consisting of fluorine, chlorine, bromine atoms, nitro, cyano, methyl, ethyl, propyl, isopropyl, butyl, isobutyl, t-butyl, fluoromethyl, difluoromethyl, trifluoromethyl, 2-fluoroethyl, methoxy, ethoxy, propoxy, isopropoxy, fluoromethoxy, difluoromethoxy, trifluoromethoxy, 2-fluoroethoxy, methylthio, ethylthio, propylthio, isopropylthio, trimethylene and tetramethylene groups.

**14.** A tricyclic compound or a pharmaceutically acceptable salt thereof according to any one of Claims 9 to 12, wherein said heteroaromatic ring group or fused heteroaromatic ring group is substituted by at least one substituent selected from the group consisting of fluorine, chlorine atoms, nitro, cyano, methyl, ethyl, isopropyl, t-butyl, fluoromethyl, difluoromethyl, trifluoromethyl, methoxy, ethoxy, fluoromethoxy, difluoromethoxy, trifluoromethoxy, methylthio, ethylthio, trimethylene and tetramethylene groups.

**15.** A tricyclic compound or a pharmaceutically acceptable salt thereof according to any one of Claims 9 to 12, wherein said heteroaromatic ring group or fused heteroaromatic ring group is substituted by at least one substituent selected from the group consisting of fluorine, chlorine atoms, nitro, cyano, methyl, isopropyl, t-butyl, difluoromethyl, trifluoromethyl, methoxy, difluoromethoxy, trifluoromethoxy, methylthio and tetramethylene groups.

**16.** A tricyclic compound or a pharmaceutically acceptable salt thereof according to any one of Claims 9 to 12, wherein said heteroaromatic ring group or fused heteroaromatic ring group is substituted by at least one substituent selected from the group consisting of fluorine, chlorine atoms, trifluoromethyl and tetramethylene groups.

**17.** A tricyclic compound or a pharmaceutically acceptable salt thereof according to Claim 1, wherein A of the compound represented by the formula (I) is selected from the group consisting of 2-oxazolyl, 2-thiazolyl, 2- or 4-imidazolyl, 3-pyrazolyl, 1,3,4-thiadiazol-2-yl, 2-pyridyl, 2- or 4-pyrimidinyl, 3-pyridazinyl, 2-pyrazinyl, 2-benzoxazolyl, 2-benzothiazolyl, 2-benzimidazolyl, quinolin-2-yl, quinazolin-2-yl, quinoxaline-2-yl, 4-methyl-2-thiazolyl, 4-ethyl-2-thiazolyl, 4-isopropyl-2-thiazolyl, 4-t-butyl-2-thiazolyl, 4-trifluoromethyl-2-thiazolyl, 5-methyl-1,3,4-thiadiazol-2-yl, 5-ethyl-1,3,4-thiadiazol-2-yl, 5-isopropyl-1,3,4-thiadiazol-2-yl, 5-t-butyl-1,3,4-thia-diazol-2-yl, 5-trifluoromethyl-1,3,4-thiadiazol-2-yl, 5,6-difluoro-2-pyridyl, 5,6-dichloro-2-pyridyl, 5,6-di-methyl-2-pyridyl, 5,6-diethyl-2-pyridyl, 6-trifluoromethyl-2-pyridyl, 6-methylthio-2-pyridyl, 5,6-dihydroclopenta[b]pyridin-2-yl, 5,6,7,8- tetrahydroquinolin-2-yl, 5,6-difluoro-2-pyrimidinyl, 5,6-dichloro-2-pyrimidinyl, 5,6-dimethyl-2-pyrimidinyl, 6-trifluoromethyl-2-pyrimidin-yl, 5,6-dihydrocyclopenta[d]pyrimidine-2-yl, 5,6,7,8-tetrahydroquinazolin-2-yl, 6-fluoro-2-benzoxazolyl, 5-fluoro-2-benzoxazolyl, 5,6-difluoro-2-benzoxazolyl, 6-chloro-2-benzoxazolyl, 5-chloro-2-benzoxazolyl, 5,6-dichloro-2-benzoxazolyl, 5-chloro-6-fluoro-2-benzoxazolyl, 5-methyl-2-benzoxazolyl, 5-cyano-2-benzoxazolyl, 5-trifluoromethyl-2-benzoxazolyl, 5-methylthio-2-benzoxazolyl, 6-fluoro-2-benzothiazolyl, 5-fluoro-2-benzothiazolyl, 5,6-difluoro-2-benzothiazolyl, 6-chloro-2-benzothiazolyl, 5-chloro-2-benzothiazolyl, 5,6-dichloro-2-benzothiazolyl, 5-chloro-6-fluoro-2-benzothiazolyl, 5-methyl-2-benzothiazolyl, 5-cyano-2-benzothiazolyl, 5-trifluoromethyl-2-benzothiazolyl, 5-methylthio-2-benzothiazolyl, 5-fluoroquinolin-2-yl, 6-fluoroquinolin-2-yl, 7-fluoroquinolin-2-yl, 5-chloroquinolin-2-yl, 6-chloro-quinolin-2-yl, 7-chloroquinolin-2-yl, 7-methylquinolin-2-yl, 7-trifluoromethylquinolin-2-yl, 7-methoxyquinolin-2-yl, 7-difluoromethoxyquinolin-2-yl, 7-trifluoromethoxyquinolin-2- yl, 5,7-difluoroquinolin-2-yl, 6,7-difluoroquinolin-2-yl, 5,7-dichloroquinolin-2-yl, 6,7-dichloroquinolin-2-yl, 5-chloro-7-fluoroquinolin-2-yl, 6-chloro-7-fluoroquinolin-2-yl, 7-chloro-5-fluoroquinolin-2-yl, 7-chloro-6-fluoro-quinolin-2-yl, 7-chloro-6-cyano-quinolin-2-yl, 7-cyano-6-fluoroquinolin-2-yl, 6-fluoro-7-trifluoromethylquinolin-2-yl, 5,6,7-trifluoroquinolin-2-yl, 5-fluoroquinazolin-2-yl, 6-fluoroquinazolin-2-yl, 7-fluoroquinazolin-2-yl, 5-chloro-quinazolin-2-yl, 6-chloroquinazolin-2-yl, 7-chloroquinazol-in-2-yl, 7-methylquinazolin-2-yl, 7-trifluoromethyl-quinazolin-2-yl, 7-methoxyquinazolin-2-yl, 7-difluoro-methoxyquinazolin-2-yl, 7-trifluoromethoxyquinazolin-2-yl, 5,7-difluoroquinazolin-2-yl, 6,7-difluoroquinazolin-2-yl, 5,7-dichloroquinazolin-2-yl, 6,7-dichloroquinazolin-2-yl, 5-chloro-7-fluoroquinazolin-2-yl, 6-chloro-7-fluoroquin-azolin-2-yl, 7-chloro-5-fluoroquinazolin-2-yl, 7-chloro-6-fluoroquinazolin-2-yl, 7-chloro-6-cyano-quinazolin-2-yl, 7-cyano-6-fluoroquinazolin-2-yl, 6-fluoro-7-trifluoro-methylquinazolin-2-yl and 5,6,7-trifluoroquinazolin-2-yl groups.

**18.** A tricyclic compound or a pharmaceutically acceptable salt thereof according to Claim 1, wherein A of the compound represented by the formula (I) is selected from the group consisting of 2-thiazolyl, 1,3,4-thiadiazol-2-yl, 2-pyridyl, 2-pyrimidinyl, 2-benzoxazolyl, 2-benzothiazolyl, quinolin-2-yl, quinazolin-2-yl, 4-methyl-2-thiazolyl, 4-isopropyl-2-thiazolyl, 4-t-butyl-2-thiazolyl, 4-trifluoromethyl-2-thiazolyl, 5-methyl-1,3,4-thiadiazol-2-yl, 5-isopropyl-1,3,4-thiadiazol-2-yl, 5-t-butyl-1,3,4-thiadiazol-2-yl, 5-trifluoromethyl-1,3,4-thiadiazol-2-yl, 5,6-difluoro-2-pyridyl, 5,6-dichloro-2-pyridyl, 5,6-dimethyl-2-pyridyl, 5,6-dihydroclopenta[b]pyridin-2-yl, 5,6,7,8-tetrahydro-quinolin-2-yl, 5,6-difluoro-2-pyrimidinyl, 5,6-dichloro-2-pyrimidinyl, 5,6-dimethyl-2-pyrimidinyl, 6-trifluoro-methyl-2-pyrimidinyl, 5,6-dihydrocyclopenta[d]pyrimidin-2-yl, 5,6,7,8-tetrahydroquinazolin-2-yl, 6-fluoro-2-benzoxazolyl, 5-fluoro-2-benzoxazolyl, 5,6-difluoro-2-benzoxazolyl, 6-chloro-2-benzoxazolyl, 5-chloro-2-benzoxazolyl, 5,6-dichloro-2-benzoxazolyl, 5-chloro-6-fluoro-2-benzoxazolyl, 5-methyl-2-benzoxazolyl, 5-cyano-2-benzoxazolyl, 5-trifluoromethyl-2-benzoxazolyl, 5-methyl-thio-2-benzoxazolyl, 6-fluoro-2-benzothiazolyl, 5-fluoro-2-benzothiazolyl, 5,6-difluoro-2-benzothiazolyl, 6-chloro-2-benzothiazolyl, 5-chloro-2-benzothiazolyl, 5,6-dichloro-2-benzothiazolyl, 5-chloro-6-fluoro-2-benzothiazolyl, 5-methyl-2-benzothiazolyl, 5-cyano-2-benzothiazolyl, 5-trifluoromethyl-2-benzothiazolyl, 5-methylthio-2-benzo-thiazolyl, 5-fluoroquinolin-2-yl, 6-fluoroquinolin-2-yl, 7-fluoroquinolin-2-yl, 5-chloroquinolin-2-yl, 6-chloro-quinolin-2-yl, 7-chloroquinolin-2-yl, 7-methylquinolin-2-yl, 7-trifluoromethylquinolin-2-yl, 7-methoxyquinolin-2-yl, 7-difluoromethoxyquinolin-2-yl, 7-trifluoromethoxy-quinolin-2-yl, 5,7-difluoroquinolin-2-yl, 6,7-difluoro-quinolin-2-yl, 5,7-dichloroquinolin-2-yl, 6,7-dichloro-quinolin-2-yl, 5-chloro-7-fluoroquinolin-2-yl, 6-chloro-7-fluoroquinolin-2-yl, 7-chloro-5-fluoroquinolin-2-yl, 7-chloro-6-fluoroquinolin-2-yl, 7-chloro-6-cyano-quinolin-2-yl, 7-cyano-6-fluoroquinolin-2-yl, 6-fluoro-7-trifluoro-methylquinolin-2-yl, 5,6,7-trifluoroquinolin-2-yl, 5-fluoroquinazolin-2-yl, 6-fluoroquinazolin-2-yl, 7-fluoro-quinazolin-2-yl, 5-chloroquinazolin-2-yl, 6-chloroquinazol-in-2-yl, 7-chloroquinazolin-2-yl, 7-methylquinazolin-2-yl, 7-trifluoromethylquinazolin-2-yl, 7-methoxyquinazolin-2-yl, 7-difluoromethoxyquinazolin-2-yl, 7-trifluoromethoxyquin-azolin-2-yl, 5,7-difluoroquinazolin-2-yl, 6,7-difluoroquinazolin-2-yl, 5,7-dichloroquinazolin-2-yl, 6,7-dichloro-quinazolin-2-yl, 5-chloro-7-fluoroquinazolin-2-yl, 6-chloro-7-fluoroquinazolin-2-yl, 7-chloro-5-fluoroquinazolin-2-yl, 7-chloro-6-fluoroquinazolin-2-yl, 7-chloro-6-cyano-quina-zolin-2-yl, 7-cyano-6-fluoroquinazolin-2-yl, 6-fluoro-7-trifluoromethylquinazolin-2-yl and 5,6,7-trifluoroquin-azolin-2-yl groups.

**19.** A tricyclic compound or a pharmaceutically acceptable salt thereof according to Claim 1, wherein A of the compound represented by the formula (I) is selected from the group consisting of 2-pyridyl, 2-benzothiazolyl, quinolin-2-yl, 5,6-difluoro-2- pyridyl, 5,6-dichloro-2-pyridyl, 5,6-di-methyl-2-pyridyl, 5,6,7,8-tetrahydroquinolin-2-yl, 6-fluoro-2-benzothiazolyl, 5-fluoro-2-benzothiazolyl, 5,6-difluoro-2-benzothiazolyl, 6-chloro-2-benzothiazolyl, 5-chloro-2-benzothiazolyl, 5,6-dichloro-2-benzothiazolyl, 5-chloro-6-fluoro-2-benzothiazolyl, 5-methyl-2-benzothia-zolyl, 5-cyano-2-benzothiazolyl, 5-trifluoromethyl-2-benzo-thiazolyl, 5-methylthio-2-benzothiazolyl, 5-fluoroquinolin-2-yl, 6-fluoroquinolin-2-yl, 7-fluoroquinolin-2-yl, 5-chloroquinolin-2-yl, 6-chloroquinolin-2-yl, 7-chloro-quinolin-2-yl, 7-methylquinolin-2-yl, 7-trifluoromethyl-quinolin-2-yl, 7-methoxyquinolin-2-yl, 7-difluoromethoxy-quinolin-2-yl, 7-trifluoromethoxyquinolin-2-yl, 5,7-di-fluoroquinolin-2-yl, 6,7-difluoroquinolin-2-yl, 5,7-di-chloroquinolin-2-yl, 6,7-dichloroquinolin-2-yl, 5-chloro-7-fluoroquinolin-2-yl, 6-chloro-7-fluoroquinolin-2-yl, 7-chloro-5-fluoroquinolin-2-yl, 7-chloro-6-fluoroquinolin-2-yl, 7-chloro-6-cyano-quinolin-2-yl, 7-cyano-6-fluoro-quinolin-2-yl, 6-fluoro-7-trifluoromethylquinolin-2-yl and 5,6,7-trifluoroquinolin-2-yl groups.

**20.** A tricyclic compound or a pharmaceutically acceptable salt thereof according to Claim 1, wherein A of the compound represented by the formula (I) is selected from the group consisting of 7-fluoroquinolin-2-yl, 7-chloroquinolin-2-yl, 6,7-difluoroquinolin-2-yl, 6,7-dichloroquinolin-2-yl, 7-chloro-6-fluoroquinolin-2-yl, 6-fluoro-7-trifluoromethyl-quinolin-2-yl and 5,6,7,8-tetrahydroquinolin-2-yl groups.

**21.** A tricyclic compound or a pharmaceutically acceptable salt thereof according to any one of Claims 1 to 20, wherein B in the formula (I) is a formula: $-OCH_2-$, a formula: $-CH_2O-$or a formula: $-CH_2CH_2-$.

**22.** A tricyclic compound or a pharmaceutically acceptable salt thereof according to any one of Claims 1 to 20, wherein X in the formula (I) is a methylene group, a sulfur or oxygen atom.

**23.** A tricyclic compound or a pharmaceutically acceptable salt thereof according to Claim 1, wherein Y in the formula (I) is a methylene, ethylene, trimethylene, tetramethylene, pentamethylene, fluoromethylene, difluoromethylene, 1-fluoroethylene, 2-fluoroethylene, 1,1-difluoroethylene, 2,2-difluoroethylene, 1-methylethylene, 2-methylethylene, 1,1-dimethylethylene, 2,2-dimethylethylene, 1-ethylethylene, 2-ethylethylene, 1-methoxyethylene, 2-methoxyethylene, 1-fluorotrimethylene, 2-fluorotrimethylene, 3-fluorotrimethylene, 1,1-difluorotrimethylene, 2,2-difluorotrimethylene, 3,3-difluorotrimethylene, 1-methyltrimethylene, 2-methyltrimethylene, 1,1-dimethyltrimethylene, 2,2-dimethyltrimethylene, 3,3-dimethyltrimethylene, 2,2-diethyl-trimethylene, 2-methoxytrimethylene, 3-methoxytrimethylene, 2,2-dimethoxytrimethylene, 3,3-dimethoxytrimethylene, 1,2-phenylene, 1, 3-phenylene group, a group shown by a group (a) wherein o=0, p=0 and q=1, a group wherein o=0, p=1 and q=1, a group wherein o=0, p=1 and q=2, a group wherein o=1, p=0 and q=1, a group wherein o=1, p=1 and q=1 and a group wherein o=1, p=1 and q=2.

**24.** A tricyclic compound or a pharmaceutically acceptable salt thereof according to Claim 1, wherein Y in the formula (I) is a methylene, ethylene, trimethylene, fluoromethylene, difluoromethylene, 1-fluoroethylene, 2-fluoroethylene, 1,1-difluoroethylene, 2,2-difluoroethylene, 1-methylethylene, 2-methylethylene, 1,1-dimethylethylene, 2,2-dimethylethylene, 1-ethylethylene, 2-ethylethylene, 1-fluorotrimethylene, 2-fluorotrimethylene, 3-fluorotrimethylene, 1,1-difluorotrimethylene, 2,2-difluorotrimethylene, 3,3-difluorotrimethylene, 1-methyltrimethylene, 2-methyltrimethylene, 1,1-dimethyltrimethylene, 2,2-dimethyltrimethylene, 3,3-dimethyltrimethylene, 1,2-phenylene group, , in a group shown by the formula (a), a group wherein o=1, p=0 and q=1, a group wherein o=1, p=1 and q=1 and a group wherein o=1, p=1 and q=2.

**25.** A tricyclic compound or a pharmaceutically acceptable salt thereof according to Claim 1, wherein Y in the formula (I) is a methylene, ethylene, trimethylene, difluoromethylene, 1-fluoroethylene, 2-fluoroethylene, 1,1-difluoroethylene, 2,2-difluoroethylene, 1-methylethylene, 2-methylethylene, 1,1-dimethylethylene, 2,2-dimethylethylene, 1-ethylethylene, 2-ethylethylene, 2,2-difluorotrimethylene, 1-methyltrimethylene, 2-methyltrimethylene, 1,1-dimethyltrimethylene, 2,2-dimethyltrimethylene, 3,3-dimethyltrimethylene, 1.2-phenylene and in a group shown by the formula (a), a group wherein o=1, p=1 and q=1.

**26.** A tricyclic compound or a pharmaceutically acceptable salt thereof according to Claim 1, wherein Y in the formula (I) is a methylene, ethylene, trimethylene, 1-methylethylene, 2-methylethylene, 1,1-dimethylethylene, 2,2-dimethylethylene, 1-ethylethylene, 2-ethylethylene, 1,2-phenylene and in a group shown by the formula (a), a group wherein o=1, p=1 and q=1.

**27.** A tricyclic compound or a pharmaceutically acceptable salt thereof according to Claim 1, wherein Z in the formula (I) is selected from the group consisting of a carboxyl, a 1H-tetrazol-5-yl, a formula: $-SO_3H$, methanesulfonylamino, ethanesulfonylamino, trifluoromethanesulfonylamino, phenylsulfonylamino, p-fluorophenylsulfonylamino, p-chloro-phenylsulfonylamino, o-methylphenylsulfonylamino, p-methylphenylsulfonylamino, p-trifluoromethylphenylsulfonylamino, o-methoxyphenylsulfonylamino, p-methoxyphenylsulfonylamino, p-difluoromethoxyphenylsulfonylamino, p-trifluoromethoxy-phenylsulfonylamino, p-nitrophenylsulfonylamino, p-cyano-phenylsulfonylamino, methanesulfonylaminocarbonyl, ethane-sulfonylaminocarbonyl, trifluoromethanesulfonylaminocarbonyl, phenylsulfonylaminocarbonyl, p-fluorophenyl-sulfonylaminocarbonyl, p-chlorophenylsulfonylaminocarbonyl, o-methylphenylsulfonylaminocarbonyl, p-methylphenylsulfonylaminocarbonyl, p-trifluoromethylphenylsulfonyl-aminocarbonyl, o-methoxyphenylsulfonylaminocarbonyl, p-methoxyphenylsulfonylaminocarbonyl, p-difluoromethoxy-phenylsulfonylaminocarbonyl, p-trifluoromethoxyphenyl-sulfonylaminocarbonyl, p-nitrophenylsulfonylaminocarbonyl and p-cyanophenylsulfonylaminocarbonyl groups.

**28.** A tricyclic compound or a pharmaceutically acceptable salt thereof according to Claim 1, wherein Z in the formula (I) is selected from the group consisting of a carboxyl, a 1H-tetrazol-5-yl, a formula: $-SO_3H$, methanesulfonylamino, trifluoromethanesulfonylamino, phenylsulfonylamino, o-methylphenylsulfonylamino, p-methylphenylsulfonylamino, methanesulfonylaminocarbonyl, trifluoromethanesulfonyl-aminocarbonyl, phenylsulfonylaminocarbonyl, o-methylphenylsulfonylaminocarbonyl and p-methylphenylsulfonylaminocarbonyl groups.

**29.** A tricyclic compound or a pharmaceutically acceptable salt thereof according to Claim 1, wherein Z in the formula (I) is selected from the group consisting of a carboxyl, a formula: $-SO_3H$, methanesulfonylamino, trifluoromethanesulfonylamino, methanesulfonylaminocarbonyl and trifluoromethanesulfonylaminocarbonyl groups.

**30.** A tricyclic compound or a pharmaceutically acceptable salt thereof according to any one of Claims 1 to 5, wherein m in the formula (I) is an integer of 1, 2 or 3.

**31.** A tricyclic compound or a pharmaceutically acceptable salt thereof according to any one of Claims 1 and 6 to 8, wherein n in the formula (I) is an integer of 1 or 2.

**32.** A tricyclic compound or a pharmaceutically acceptable salt thereof according to Claim 1, wherein the compound represented by the formula (I) is at least one selected from the group consisting of:

[2-[(E)-2-(6,7-difluoro-2-quinolinyl)ethenyl]-6,11-dihydro-dibenz[b,e]oxepin-11-yl]oxyacetic acid,
[2-[(E)-2-(6,7-difluoro-2-quinolinyl)ethenyl]-6,11-dihydro-dibenz[b,e]oxepin-11-yl]thioacetic acid,
3-{[2-[(E)-2-(6,7-difluoro-2-quinolinyl)ethenyl]-6,11-di-hydrodibenz[b,e]oxepin-11-yl]thio}propionic acid,
3-{[2-[(E)-2-(6,7-difluoro-2-quinolinyl)ethenyl]-6.11-di-hydrodibenz[b,e]oxepin-11-yl]thio}-2-methylpropionic acid,
3-{[2-[(E)-2-(6,7-difluoro-2-quinolinyl)ethenyl]-6,11-di-hydrodibenz[b,e]oxepin-11-yl]thio}-2,2-dimethylpropionic acid,
3-{[2-[(E)-2-(6,7-difluoro-2-quinolinyl)ethenyl]-6,11-di-hydrodibenz[b,e]oxepin-11-yl]thio}-2-ethylpropionic acid,
3-{[2-[(E)-2-(6,7-difluoro-2-quinolinyl)ethenyl]-6,11-di-hydrodibenz[b,e]oxepin-11-yl]thio}-3,3-dimethylpropionic acid,
{1-[[2-[(E)-2-(6,7-difluoro-2-quinolinyl)ethenyl]-6,11-di-hydrodibenz[b,e]oxepin-11-yl]thiomethyl]cyclopropyl} acetic acid,
2-{(2-[(E)-2-(6,7-difluoro-2-quinolinyl)ethenyl]-6,11-di-hydrodibenz[b,e]oxepin-11-yl]thio}benzoic acid,
[2-((E)-2-(6,7-difluoro-2-quinolinyl)ethenyl]-6,11-di-hydrodibenz[b,e]oxepin-11-yl]thio-N-methanesulfonylacet-amide,
3-[2-[(E)-2-(6,7-difluoro-2-quinolinyl)ethenyl]-6,11-di-hydrodibenz[b,e]oxepin-11-yl]thio-N-methanesulfonylpropionamide,
2-{[2-[(E)-2-(6,7-difluoro-2-quinolinyl)ethenyl]-6,11-di-hydrodibenz[b,e]oxepin-11-yl]thio}ethanesulfonic acid,
4-[2-[(E)-2-(6,7-difluoro-2-quinolinyl)ethenyl]-6,11-di-hydrodibenz[b,e]oxepin-11-yl]butanoic acid,
3-{[2-[(E)-2-(6,7-difluoro-2-quinolinyl)ethenyl]-7-fluoro-6,11-dihydrodibenz[b,e]oxepin-11-yl]thio}propionic acid,
{1-[[2-[(E)-2-(6,7-difluoro-2-quinolinyl)ethenyl]-7-fluoro-6,11-dihydrodibenz[b,e]oxepin-11-yl]thiomethyl]cyclo-propyl}acetic acid,
3-{[2-[(E)-2-(6,7-difluoro-2-quinolinyl)ethenyl]-8-fluoro-6,11-dihydrodibenz[b,e]oxepin-11-yl]thio}propionic acid,
3-{[7-cyano-2-[(E)-2-(6,7-difluoro-2-quinolinyl)ethenyl]-6,11-dihydrodibenz[b,e]oxepin-11-yl]thio]propionic acid,
3-{[2-[(E)-2-(6,7-difluoro-2-quinolinyl)ethenyl]-7-tri-fluoromethyl-6,11-dihydrodibenz[b,e]oxepin-11-yl]thio}-propionic acid,
3-{[7-ethynyl-2-[(E)-2-(6,7-difluoro-2-quinolinyl)-ethenyl]-6,11-dihydrodibenz[b,e]oxepin-11-yl]thio]-propionic acid,
3-{(2-[(E)-2-(7-chloro-6-fluoro-2-quinolinyl)ethenyl]-6,11-dihydrodibenz[b,e]oxepin-11-yl]thio}propionic acid,
3-{[2-[(E)-2-(7-chloro-6-fluoro-2-quinolinyl)ethenyl]-7-fluoro-6,11-dihydrodibenz[b,e]oxepin-11-yl]thio}propionic acid,
{1-[[2-[(E)-2-(7-chloro-6-fluoro-2-quinolinyl)ethenyl]-7-fluoro-6,11-dihydrodibenz[b,e]oxepin-11-yl]thiomethyl]-cyclopropyl}acetic acid,
3-{[2-[(E)-2-(7-fluoro-2-quinolinyl)ethenyl]-6,11-dihydro-dibenz[b,e]oxepin-11-yl]thio}propionic acid,
3-{[2-[(E)-2-(7-fluoro-2-quinolinyl)ethenyl]-6,11-dihydro-dibenz[b,e]oxepin-11-yl]thio}-2-methylpropionic acid,
{1-[(2-[(E)-2-(7-chloro-6-fluoro-2-quinolinyl)ethenyl]-6,11-dihydrodibenz[b,e]oxepin-11-yl]thiomethyl]cyclopropyl}acetic acid,
{2-[(E)-2-(7-chloro-2-quinolinyl)ethenyl]-6,11-dihydrodi-benz[b,e]oxepine-11-yl}thioacetic acid,
3-{(2-[(E)-2-(7-chloro-2-quinolinyl)ethenyl]-6,11-dihydro-dibenz[b,e]oxepin-11-yl]thio}propionic acid,
3-{[2-[(E)-2-(7-chloro-2-quinolinyl)ethenyl]-6,11-dihydro-dibenz[b,e]oxepin-11-yl]thio}-2-methylpropionic acid,
{1-[[2-[(E)-2-(7-chloro-2-quinolinyl)ethenyl]-6,11-dihydro-dibenz(b,e]oxepin-11-yl]thiomethyl]cyclopropyl}acetic acid,
3-{[2-[(E)-2-(5,6,7,8-tetrahydro-2-quinolinyl)ethenyl]-6,11-dihydrodibenz[b,e]oxepin-11-yl]thio}propionic acid,
3-{[2-[(E)-2-(5,6,7,8-tetrahydro-2-quinolinyl)ethenyl]-6,11-dihydrodibenz[b,e]oxepin-11-yl]thio}-2-methylpropionic acid,
{1-[[2-[(E)-2-(5,6,7,8-tetrahydro-2-quinolinyl)ethenyl]-6,11-dihydrodibenz[b,e]oxepin-11-yl]thiomethyl]cyclopropyl} acetic acid,
3-{[2-[(E)-2-(6-fluoro-7-trifluoromethyl-2-quinolinyl)-ethenyl]-6,11-dihydrodibenz[b,ejoxepin-11-yl]thio}propionic acid,
3-{[3-[(E)-2-(6,7-difluoro-2-quinolinyl)ethenyl]-10,11-di-hydro-5H-dibenz[a,d]cyclohepten-5-yl]thio}propionic acid,
3-{[3-[(E)-2-(6,7-difluoro-2-quinolinyl)ethenyl]-10,11-di-hydro-5H-dibenz[a,d]cyclohepten-5-yl]thio}-2-methylpro-pionic acid, and
3-{[9-[(E)-2-(6,7-difluoro-2-quinolinyl)ethenyl]-6,11-di-hydrodibenz[b,e]oxepin-11-yl]thio}propionic acid.

## INTERNATIONAL SEARCH REPORT

International application No.
PCT/JP00/09406

A. CLASSIFICATION OF SUBJECT MATTER
Int.Cl$^7$ C07D215/12, 14, 18, 405/06, 417/06, 413/06, 409/06, A61K31/55, 31/47, A61P43/00, 37/08, 29/00

According to International Patent Classification (IPC) or to both national classification and IPC

B. FIELDS SEARCHED

Minimum documentation searched (classification system followed by classification symbols)
Int.Cl$^7$ C07D215/12, 14, 18, 405/06, 417/06, 413/06, 409/06, A61K31/55, 31/47, A61P43/00, 37/08, 29/00

Documentation searched other than minimum documentation to the extent that such documents are included in the fields searched

Electronic data base consulted during the international search (name of data base and, where practicable, search terms used)
REGISTRY(STN), CA(STN), CAOLD(STN), CAPLUS(STN)

C. DOCUMENTS CONSIDERED TO BE RELEVANT

| Category* | Citation of document, with indication, where appropriate, of the relevant passages | Relevant to claim No. |
|---|---|---|
| A | EP, 685478, A1 (UBE INDUSTRIES, LTD.), 06 December, 1995 (06.12.95) & WO, 94/19345, A1 & JP, 06-518813, A & US, 5591752, A & CN, 1117730, A | 1-32 |
| A | EP, 468785, A2 (MERCK FROSST CANADA INC.), 29 January, 1992 (29.01.92) & JP, 05-105678, A & US, 5221678, A | 1-32 |
| A | WO, 95/18107, A1 (MERCK & CO., INC.), 06 July, 1995 (06.07.95) & JP, 09-507235, A & EP, 737186, A1 & US, 5614632, A & CN, 1139429, A | 1-32 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27 February, 2001 (27.02.01) | 13 March, 2001 (13.03.01) |
| Name and mailing address of the ISA/ Japanese Patent Office | Authorized officer |
| Facsimile No. | Telephone No. |

Form PCT/ISA/210 (second sheet) (July 1992)